# EUROPEAN PATENT APPLICATION

(11) **EP 4 663 628 A1**
(43) Date of publication of application: **17.12.2025**
(21) Application number: 24752984.5
(22) Date of filing: 08.02.2024
(51) Int. Cl.: C07D 213/54, A61K 31/444, A61K 31/4545, A61K 31/4439, A61K 31/5377, A61P 35/00, C07D 401/12, C07D 471/04, C07D 213/16, C07D 401/14

(54) **NEW COMPOUND AND PHARMACEUTICAL COMPOSITION COMPRISING SAME**

(30) Priority: 10.02.2023 KR 20230018333
(71) Applicant: Samjin Pharmaceutical Co., Ltd., Seoul 04054 (KR)
(72) Inventor: AHN, Soyeon, Seoul 07605 (KR); KIM, Jiwon, Seoul 07791 (KR); LEE, Goeun, Seoul 07537 (KR); HWANG, Kyungrim, Seoul 07791 (KR); WOO, Juhyun, Goyang-si, Gyeonggi-do 10595 (KR); LEE, Soo Min, Seoul 06277 (KR); KWON, Ho Seok, Suwon-si, Gyeonggi-do 16557 (KR); LEE, Jae Woong, Suwon-si, Gyeonggi-do 16713 (KR); KIM, Hyun Tae, Suwon-si, Gyeonggi-do 16687 (KR)
(74) Representative: HGF
(86) International application number: PCT/IB2024/051151
(87) International publication number: WO 2024/166024

(57) **Abstract**

The present invention relates to a new urea compound, an optical isomer thereof, a pharmaceutically acceptable salt of them, or a hydrate or solvate of them, and a new compound exhibits NAMPT inhibitory activity and may effectively prevent or treat cancer.

## Description

### Technical Field

The present invention relates to a compound represented by formula I below, an optical isomer thereof, a pharmaceutically acceptable salt thereof, or a solvate or hydrate thereof, and a use thereof.

### Background

Nicotinamide phosphoribosyltransferase (NAMPT) is an enzyme which induces the synthesis of nicotinamide mononucleotide (NMN) from nicotinamide (NAM) and 5'phosphoribosyl-l'-pyrophosphate (PRPP), playing a key role in the cyclic biosynthetic pathway of nicotinamide adenine dinucleotide (NAD+). NAD is essential for several signaling pathways including mono-ADP-ribosylation in both the immune system and G-protein-coupled receptor signaling, among other poly-ADP-ribosylation in DNA repair, and NAD is also essential for the deacetylase activity of sirtuin.

NAD+ is produced by two distinct biosynthetic pathways: the salvage and de novo synthesis pathways. As a rate-limiting enzyme in the NAD+ salvage pathway, NAMPT is biologically essential and may be associated with a number of diverse diseases.

Thus, there is a need to develop a drug capable of more effectively inhibiting NAMPT.

### Related Art References

### Patent Documents

Korean Patent Publication 10-2015-0014250

### Detailed Description of the Invention

### Technical Problem

The present invention provides a novel urea compound, an optical isomer thereof, a pharmaceutically acceptable salt of them, or a hydrate or solvate of them.

The present invention provides a pharmaceutical composition including a novel urea compound, an optical isomer thereof, a pharmaceutically acceptable salt of them, or a hydrate or solvate of them.

The present invention provides a method for preparing a novel urea compound, an optical isomer thereof, a pharmaceutically acceptable salt of them, or a hydrate or solvate of them.

The present invention provides a pharmaceutical composition for preventing or treating diseases (e.g., cancer) which may be treated by inhibiting NAMPT, including a novel urea compound, an optical isomer thereof, a pharmaceutically acceptable salt of them, or a hydrate or solvate of them as an active ingredient.

Another object of the present invention is to provide a method for preventing or treating diseases (e.g., cancer) which may be treated by inhibiting NAMPT, including administering a novel urea compound, an optical isomer thereof, a pharmaceutically acceptable salt of them, or a hydrate or solvate of them into an individual in need.

Another object of the present invention is to provide a novel urea compound, an optical isomer thereof, a pharmaceutically acceptable salt of them, or a hydrate or solvate of them for preventing or treating diseases (e.g., cancer) which may be treated by inhibiting NAMPT.

Another object of the present invention is to provide a use of a novel urea compound, an optical isomer thereof, a pharmaceutically acceptable salt of them, or a hydrate or solvate of them in preparation of a medication for preventing or treating cancer diseases (e.g., cancer) which may be treated by inhibiting NAMPT.

### Technical Solution

The present inventors have identified a compound having a novel structure with NAMPT inhibitory activity and have used the same in preventing and/or treating diseases which may be treated by inhibiting NAMPT, thereby completing the present invention.

Hereinafter, the present invention will be described in more detail. All the combinations of various elements disclosed in the present invention fall within the scope of the present invention. In addition, it cannot be seen that the scope of the present invention is limited to the specific description below.

The present invention provides a urea compound represented by formula I below, an optical isomer thereof, a pharmaceutically acceptable salt of them, or a hydrate or solvate of them.

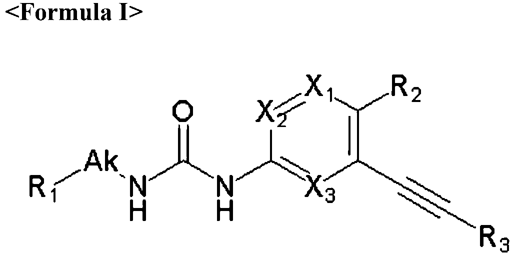

In above formula I,
R₁ is NHYx (the Yₓ may be 3- to 6-membered heteroaryl, in which at least one H of the heteroaryl may be substituted), aryl (at least one -H of aryl and heteroaryl may be substituted with a substituent), or heteroaryl (at least one -H of aryl and heteroaryl may be substituted with a substituent);
R₂ may be any substituents except -H, such as aryl, heteroaryl, cycloalkyl, heterocycloalkyl, bicycloalkyl, biheterocycloalkyl, alkylamine, C₁₋₁₀ amide, C₁₋₁₀ amine or phenoxy, and the group of the R₂ may be unsubstituted or at least one H may be substituted;
Ak is -(CH₂)n- or C₃₋₆ cycloalkylene (in which -H of the -(CH₂)n- or cycloalkylene group may be unsubstituted or at least one -H may be each independently substituted with a substituent, and n is 0 to 5);
X₁, X₂ and X₃ are each independently -CH- or -N-; and
R₃ is aryl or heteroaryl (at least one -H of the aryl or heteroaryl may be substituted with another substituent).

In the present specification, in the expression "Cx" of a functional group, x represents the number of carbons (C), and Cx-y may mean an integer of carbon number x or more and carbon number y or less.

In the present invention, the term "substituted" represents a moiety having a substituent which replaces -H of at least one carbon (-C-) or nitrogen (-N-) of a main chain. The "substitution" or "substituted with~" is defined to include implicit conditions, in which the substitution follows a permitted valency of a substituted atom and a substituent and induces a compound stabilized by substitution, for example, a compound which is not naturally modified by rearrangement, cyclization, removal, etc.

In the present specification, a "single bond" means a case in which adjacent atoms or groups of atoms are directly bonded to each other.

In the present specification, "alkyl" may mean a linear (or straight-chain) saturated hydrocarbon group or a branched (or side-chain) saturated hydrocarbon group unless otherwise specified, and may include at least one selected from methyl, ethyl, n-propyl, isopropyl, n-butyl, sec-butyl, isobutyl, tert-butyl, n-pentyl, n-hexyl, n-heptyl, and the like, but is not limited thereto.

In the present invention, "alkylene" may mean a divalent functional group which is induced from alkyl as defined above, unless otherwise stated. For example, C1 alkylene may be methylene (-CH₂-).

In the present specification, "alkenyl" may mean an unsaturated hydrocarbon group including at least one double bond between carbons, unless otherwise stated, and may mean an unsaturated hydrocarbon group including a straight chain or branched chain and a cis and trans type, unless otherwise stated. In the present specification, "alkylene" may mean a divalent functional group which is induced from alkyl as defined above, unless otherwise stated.

In the present specification, "alkynyl" may mean an unsaturated hydrocarbon group including at least one triple bond between carbons, unless otherwise specified.

In the present specification, "aryl" may include a monocyclic aromatic, dicyclic aromatic, or polycyclic aromatic one, unless otherwise specified, and may include a case in which at least one of two or more rings is aromatic, which may be at least one selected from phenyl, biphenyl, naphthalenyl, and the like, but is not limited thereto.

In the present specification, "heteroaryl" may mean a polycyclic ring structure in which a monocyclic, bicyclic or polycyclic hetero-ring or two or more rings, with at least one carbon atom of said aryl group substituted with nitrogen (N), oxygen (O) or sulfur (S), share at least one pair of atoms, and may also include a case in which at least one of the two or more rings is aromatic. Heteroaryl may be at least one selected from pyridinyl, pyrimidinyl, furanyl, thiophenyl, triazolyl, tetrazolyl, benzothiazolyl, benzothiophenyl, quinolinyl, indolyl, isoindolyl, benzofuranyl, benzopyrrolyl, furanyl, pyrrolyl, thiazolyl, isothiazolyl, imidazolyl, pyrazolyl, oxazolyl, imidazolyl, isoxazolyl, pyrazinyl, pyridazinyl, pyrimidinyl, isoquinolinyl, benzooxazolyl, benzoimidazolyl, dihydrobenzothiophenyl, purinyl, indolizinyl, pyrrolyl, quinazolyl, imidazopyridinyl, benzoxazolyl, imidazoimidazolyl, imidazotriazolyl, chromenyl, and the like, but is not limited thereto.

In the present specification, "cycloalkyl" may mean a saturated hydrocarbon ring having at least three specified carbon atoms including a ring, and the saturated hydrocarbon ring may be meant to include both monocyclic and polycyclic ring structures, and may also include bicycloalkyl, and "bicycloalkyl" may mean a ring structure in which a saturated hydrocarbon ring generally having at least three specified carbon atoms including a ring shares at least one pair of carbon atoms with at least two rings, and may be at least one selected from cyclopentyl, cyclohexyl, cycloheptyl, and cyclooctyl, 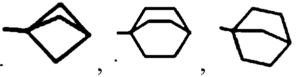 and the like, but is not limited thereto.

In the present specification, "heterocycloalkyl" may mean a polycyclic ring structure in which a saturated monocyclic and polycyclic hetero ring or at least two rings, containing one to five hetero atoms independently selected from nitrogen (N), oxygen (O) and sulfur (S), share at least one pair of carbon atoms. The heterocycloalkyl may be at least one selected from oxiranyl, oxetanyl, morpholinyl, pyrrolidinyl, piperidinyl, piperazinyl, tetrahydrofuranyl, tetrahydrothiophenyl, tetrahydropyranyl, azabicyclohexanyl, azabicycloheptanyl, tetrahydrothiopyranyl, and the like, but is not limited thereto.
1) The present invention provides a urea compound represented by formula I below, a stereoisomer thereof, a pharmaceutically acceptable salt thereof, or a hydrate or solvate of them.

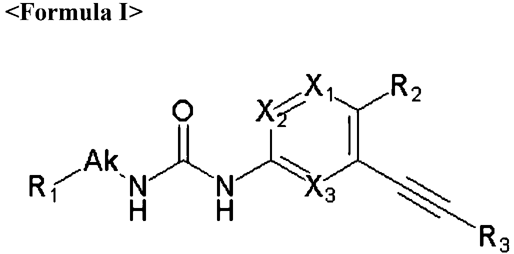

In compounds represented by above formula I, it may be provided that R₁, R₂, R₃, Ak, X₁, X₂ and X₃ are as follows, respectively:
In embodiments of the present invention, R₁ may be
6- to 12-membered aryl (e.g., C₆₋₁₂ aryl); or
5- to 12-membered heteroaryl including at least one heteroatom selected from the group consisting of N, O and S.

In this case, it may be provided that -H of aryl or heteroaryl, which is the R₁, is each independently unsubstituted or at least one H is each independently substituted with -NH₂, - (C₁₋₅ alkyl)NH₂, -NH-(C₁₋₅ alkyl), -N(C₁₋₅ alkyl)₂, -OH, -NO₂, C₁₋₅ alkyl, -F, -Cl, -Br, or -I.

2) In above 1), it may be specifically provided that the R₁ is 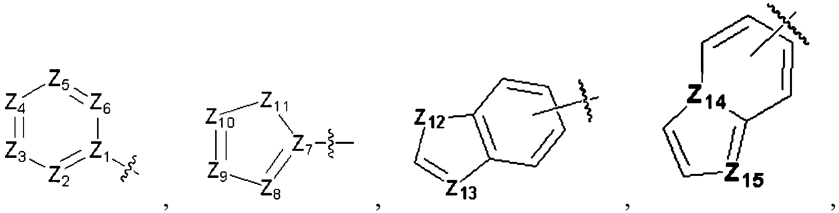 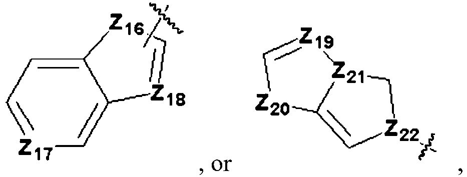 in which the Z₁ to Z₂₂ may be each independently -CH-, -CH₂, -N-, -NH, -O- or -S-, in which -H of the above group listed as the R₁ may be unsubstituted or at least one H may be each independently substituted with -NH₂, -(C₁₋₅ alkyl)NH₂, -NH-(C₁₋₅ alkyl), -N(C₁₋₅ alkyl)₂ - OH, -NO_{2,-} C₁₋₅ alkyl, -F, -Cl, -Br, or -I.

3) In above 1) or 2), it may be specifically provided that the R₁ is 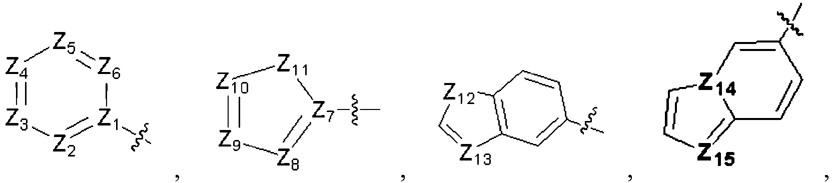 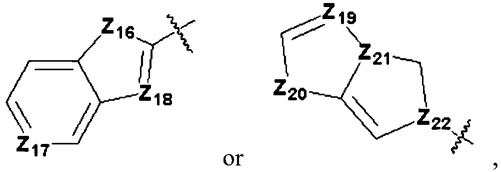 in which the Z₁ to Z₂₂ may be each independently -CH, -CH₂, -N-, -NH-, -O- or -S-, in which -H of the above group listed as the R₁ may be each independently unsubstituted or at least one H may be each independently substituted with -NH₂, -(C₁₋₅ alkyl)NH₂, -NH-(C₁₋₅ alkyl), -N(C₁₋₅ alkyl)₂ -OH, -NO₂, C₁₋₅ alkyl, -F, -Cl, -Br, or -I.

4) In any one of above 1) to 3), it may be more specifically provided that R₁ is 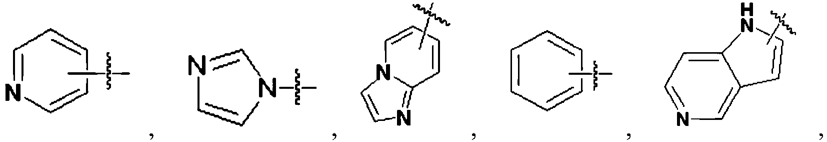 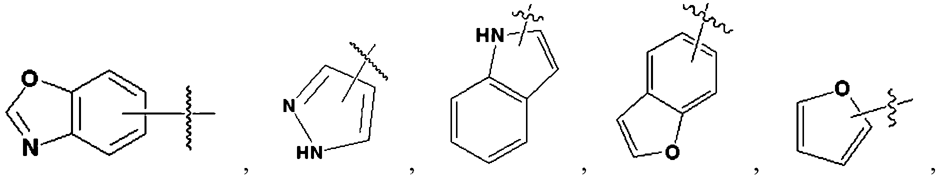 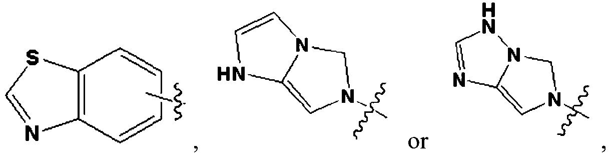 in which -H of the above group listed as the R₁ may be each independently unsubstituted or at least one -H may be each independently substituted with -NH₂, -(C₁₋₅ alkyl)NH₂, -NH-(C₁₋₅ alkyl), -N(C₁₋₅ alkyl)₂, -OH, -NO₂, C₁₋₅ alkyl, -F, -Cl, -Br, or -I.

5) In any one of above 1) to 4), it may be much more specifically provided that R₁ may be 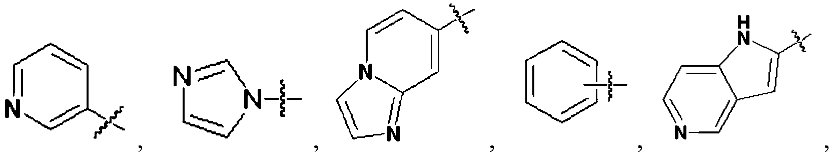 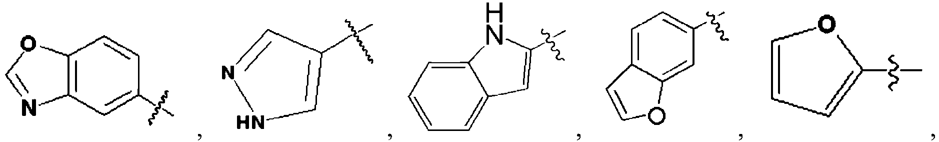 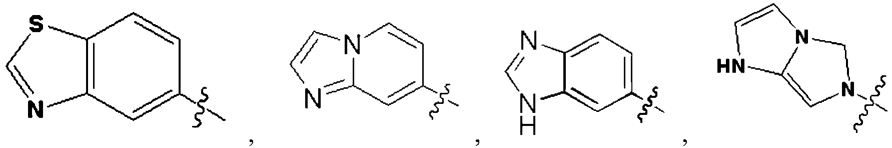 or 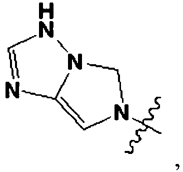 in which -H of the above group listed as the R₁ may be unsubstituted or at least one -H may be each independently substituted with -NH₂, -(C₁₋₅ alkyl)NH₂, -NH(C₁₋₅ alkyl), -N(C₁₋₅ alkyl)₂, -OH, -NO₂, C₁₋₅ alkyl, -F, -Cl, -Br, or -I.

6) In any one of above 1) to 5), according to embodiments of the present invention, it may be provided that R₂ may be 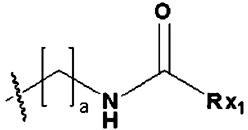 (the Rx₁ is C₁₋₅ alkyl; C₂₋₅ alkenyl; 6- to 12-membered aryl (e.g., C₆₋₁₂ aryl); 3- to 10-membered cycloalkyl (e.g., C₃₋₁₀ cycloalkyl); 3- to 10-membered heterocycloalkyl including at least one heteroatom selected from the group consisting of N, O and S; or 5- to 12-membered heteroaryl including at least one heteroatom selected from the group consisting of N, O and S); -NRx₂Rx₃(Rx₂ or Rx₃ is each independently H or C₁₋₆ alkyl); 6- to 12-membered aryl (e.g., C₆₋₁₂ aryl); 5- to 12-membered heteroaryl including at least one heteroatom selected from the group consisting of N, O and S; -ORx₄ (Rx₄ is 3- to 7-membered aryl); 3- to 10-membered heterocycloalkyl including at least one heteroatom selected from the group consisting of N, O and S; 3- to 10-membered cycloalkyl (e.g., C₃₋₁₀ cycloalkyl); 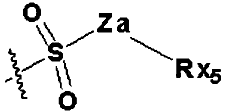 (Za may be a single bond, -NH or -CH₂-, and Rx₅ is 5- to 12-membered heteroaryl including at least one heteroatom selected from the group consisting of N, O and S, 6- to 12-membered aryl (e.g., C₆₋₁₂ aryl), 3- to 10-membered cycloalkyl (e.g., C₃₋₁₀ cycloalkyl), or 3- to 10-membered heterocycloalkyl including at least one heteroatom selected from the group consisting of N, O and S, or -NYaYb {Ya or Yb is each independently H or C₁₋₆ alkyl}); 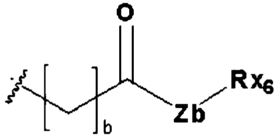 (Zb may be a single bond, -NH, -NHCH₂-, -NH(CH₂)₂ -CH₂-, - (CH₂)₂-, -(CH₂)₃-, or -CH₂NH-, Rx₆ is 5- to 12-membered heteroaryl including at least one heteroatom selected from the group consisting of N, O and S, 6- to 12-membered aryl (e.g., C₆₋₁₂ aryl), 3- to 10-membered heterocycloalkyl including at least one heteroatom selected from the group consisting of N, O and S, -NYcYd {Yc or Yd may be each independently H, C₁₋₆ alkyl or C₃₋₆cycloalkyl} or 3- to 10-membered cycloalkyl (e.g., C₃₋₁₀ cycloalkyl), and b is an integer of 0 to 4);

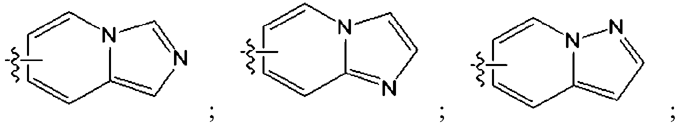

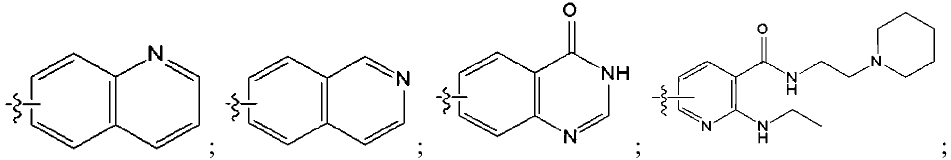

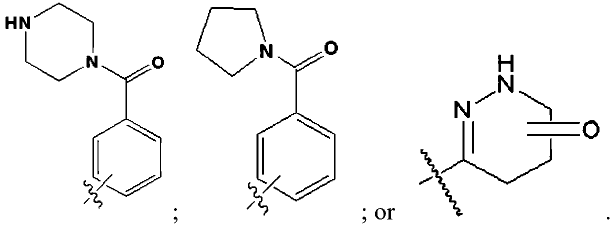

In this case, it may be provided that -H of each group of the R₂ is each independently unsubstituted or at least one -H is each independently substituted with one selected from the group consisting of C₁₋₅ alkyl (-H of the alkyl may be unsubstituted or at least one -H may be each independently substituted with one selected from the group consisting of phenyl, pyridinyl, morpholinyl, piperazine and piperidine); -(C₁₋₅ alkyl)NH₂; -NH₂; -NH(C₁₋₅ alkyl); -N(C₁₋₅ alkyl)₂; -NHCH₃; -N(CH₃)₂; -NHCH₂CH₃; -OH; -NO₂; -F; -Cl; -Br; -I; -CHF₂; -CF₃; -C₁₋₅ alkoxy; -C(=O)N(CH₃)₂; -C(=O)NHCH₃; -C(=O)NH₂; -NHC(=O)CH₃; -C(=O)CH₂CH₃; 6- to 12-membered aryl (e.g., C₆₋₁₂ aryl), in which -H of the aryl may be unsubstituted or at least one -H may be substituted with one selected from the group consisting of -C₁₋₃ alkyl and -CF₃); 5-to 12-membered heteroaryl including at least one heteroatom selected from the group consisting of N, O and S (-H of the heteroaryl may be unsubstituted or at least one -H may be substituted with -C₁₋₃ alkyl and -CF₃); 3- to 10-membered cycloalkyl (e.g., C₃₋₁₀ cycloalkyl); 3- to 10-membered heterocycloalkyl including at least one heteroatom selected from the group consisting of N, O and S (-H of the heterocycloalkyl may be unsubstituted or at least one -H may be substituted with one selected from the group consisting of -C₁₋₃ alkyl and -CF₃); 3- to 10-membered cycloalkoxy; -S(=O)₂NHCH₃; -S(=O)_{2,} -S(=O)₂-CH₃, -C(=O)NH₂; 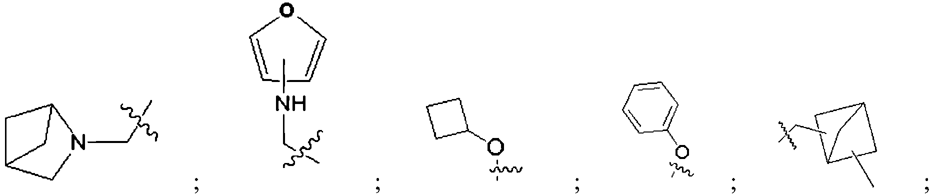 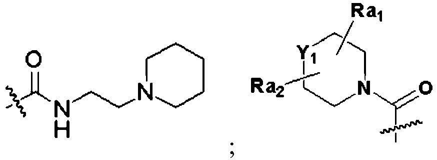 (the Ra₁ and Ra₂ may be each independently H, F, Cl, Br, I or -C₁₋₅ alkyl, and Y₁ may be one selected from -CH₂, -NH, - and -O-), 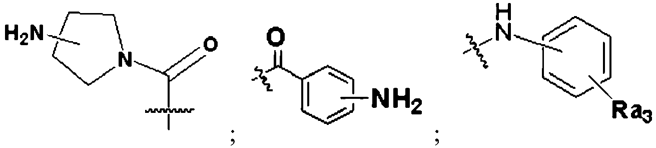 (the Ra₃ may be -H, -C₁₋₅ alkyl or -CF₃);

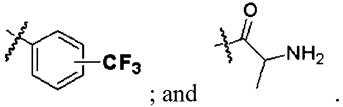

7) In any one of above 1) to 6), according to embodiments of the present invention, it may be provided that the R₂ is 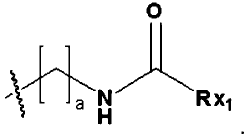 In this case, it may be provided that Rx₁ is -C₁₋₅ alkyl, -C₂₋₅ alkenyl; 6- to 12-membered aryl (e.g., C₆₋₁₂ aryl); 3- to 10-membered cycloalkyl (e.g., C₃₋₁₀ cycloalkyl); 3- to 10-membered heterocycloalkyl including at least one heteroatom selected from the group consisting of N, O and S; or 5- to 12-membered heteroaryl including at least one heteroatom selected from the group consisting of N, O and S. In this case, it may be provided that -H of the 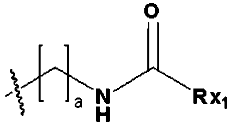 is unsubstituted or at least one -H is each independently substituted with -C₁₋₅ alkyl (-H of the alkyl may be unsubstituted or at least one -H may be each independently substituted with one selected from the group consisting of phenyl, pyridinyl, morpholinyl, piperazine and piperidine), -morpholinyl, piperazinyl, piperidinyl, -phenyl (-H of the -morpholinyl, -piperazinyl, -piperidinyl, and -phenyl may be unsubstituted or at least one -H may be each independently substituted with one selected from the group consisting of -C₁₋₃ alkyl and -CF₃), -NH₂, -F, -Cl, -Br or -I, and a is 0, 1, 2, 3 or 4.

8) In any one of above 1) to 6), according to embodiments of the present invention, it may be provided that the R₂ is -NRx₂Rx_{3.} In this case, it may be provided that Rx₂ or Rx₃ is each independently H or C₁₋₆ alkyl, and if the Rx₂ or Rx₃ is alkyl, -H of the Rx₂ and Rx₃ is unsubstituted or at least one -H is each independently substituted with C₁₋₆ alkyl, -F, -Cl, -Br or -I.

9) In any one of above 1) to 6), according to embodiments of the present invention, it may be provided that the R₂ is 6- to 12-membered aryl (e.g., C₆₋₁₂ aryl). It may be provided that -H of the aryl is unsubstituted or at least one -H is each independently substituted with one selected from the group consisting of C₁₋₅ alkyl (-H of the alkyl may be unsubstituted or at least one -H may be each independently substituted with one selected from the group consisting of phenyl, pyridinyl, morpholinyl, piperazine and piperidine), F, -Cl, -Br, -I, -morpholinyl, - piperazinyl, -piperidinyl (-H of the -morpholinyl, -piperazinyl and -piperidinyl is unsubstituted or at least one -H may be each independently substituted with one selected from the group consisting of -C₁₋₃ alkyl and -CF₃), -cyclobutoxy, -cyclopropoxy, -cyclopentoxy, 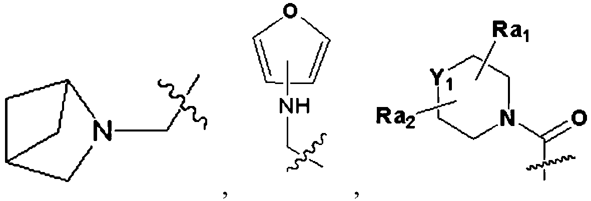 (the Ra₁ and Ra₂ may be each independently -H, -F, -Cl, -Br-, -I or -C₁₋₅ alkyl, and Y₁ may be one selected from -CH₂, -NH, - and -O-), 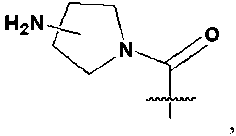 -C₁₋₃ alkoxy, -C(=O)-N(CH₃)₂ and -C(=O)NH₂. 10) In any one of above 1) to 6), according to embodiments of the present invention, it may be provided that the R₂ is 5- to 12-membered heteroaryl including at least one heteroatom selected from the group consisting of N, O and S. It may be provided that -H of the heteroaryl is unsubstituted or at least one -H is each independently substituted with one selected from the group consisting of -C₁₋₅ alkyl, -F, -Cl, -Br, -I, -NH₂, -NH(C₁₋₅ alkyl), -N(C₁₋₅ alkyl)₂, -NHCH₃, -N(CH₃)₂, -NHCH₂CH₃, -C(=O)NH₂, -C(=O)N(CH₃)₂ and -C(=O)NH(CH₃).

11) In any one of above 1) to 6), according to embodiments of the present invention, it may be provided that the R₂ is -ORx₄. It may be provided that the Rx₄ is 6- to 7-membered aryl (e.g., C₆₋₇ aryl such as phenyl), and -H of the -ORx is unsubstituted or at least one -H is each independently substituted with one selected from the group consisting of -C₁₋₅ alkyl, -F, - Cl, -Br, -I, -NH₂, -NH(C₁₋₅ alkyl), -N(C₁₋₅ alkyl)₂, -NHCH₃, -N(CH₃)₂, -NHCH₂CH₃, - C(=O)NH₂, -C(=O)N(CH₃)₂ and -C(=O)NH(CH₃).

12) In any one of above 1) to 6), according to embodiments of the present invention, it may be provided that the R₂ is 3- to 10-membered heterocycloalkyl including at least one heteroatom selected from the group consisting of N, O and S. In this case, it may be provided that -H of the heterocycloalkyl is unsubstituted or at least one -H is each independently substituted with one selected from the group consisting of -C₁₋₅ alkyl (-H of the alkyl may be unsubstituted or at least one -H may be each independently substituted with one selected from the group consisting of phenyl, pyridinyl, morpholinyl, piperazine and piperidine), pyridinyl, phenyl (-H of the pyridinyl and phenyl may be unsubstituted or at least one -H may be each independently substituted with one selected from the group consisting of C₁₋₃ alkyl and -CF₃), 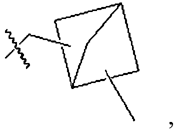 -NHC(=O)CH₃, -C(=O)CH₂CH₃, 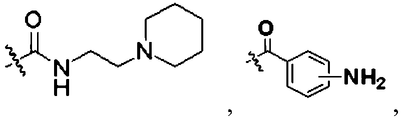 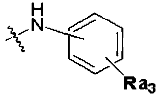 (the Ra₃ may be H, -C₁₋₃ alkyl or -CF₃), -OH, -S(=O)₂CH₃, -NH₂, -NH(C₁₋₅ alkyl), -N(C₁₋₅ alkyl)₂, -NHCH₃, -N(CH₃)₂ and -NHCH₂CH₃.

13) In any one of above 1) to 6), according to embodiments of the present invention, it may be provided that the R₂ is 3- to 10-membered cycloalkyl (e.g., C₃₋₁₀ cycloalkyl).

14) In any one of above 1) to 6), according to embodiments of the present invention, it may be provided that the R₂ is 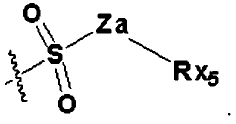 In this case, it may be provided that Za is a single bond, -NH or -CH₂-, and Rx₅ is 5- to 12-membered heteroaryl including at least one heteroatom selected from the group consisting of N, O and S, 6- to 10-membered aryl (e.g., C₆₋₁₂ aryl), 3- to 10-membered cycloalkyl, 5- to 12-membered heterocycloalkyl including at least one heteroatom selected from the group consisting of N, O and S, or -NYaYb (Ya or Yb is each independently H or C₁₋₆ alkyl). In this case, it may be provided that -H of the 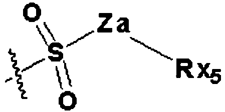 is unsubstituted or at least one -H is each independently substituted with C₁₋₅ alkyl (-H of the alkyl may be unsubstituted or at least one -H may be each independently substituted with one selected from the group consisting of phenyl, pyridinyl, morpholinyl, piperazine and piperidine), -CF₃, 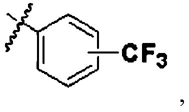 pyridinyl, pyrimidinyl, piperazinyl, morpholinyl, piperidinyl (-H of the pyridinyl, pyrimidinyl, piperidinyl, piperazinyl and morpholinyl may be unsubstituted or at least one -H may be substituted with one selected from the group consisting of -C₁₋₃ alkyl and -CF₃) 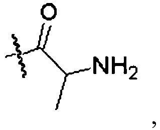 -NH₂, -F, -Cl, -Br or -I.

15) In any one of above 1) to 6), according to embodiments of the present invention, it may be provided that the R₂ is 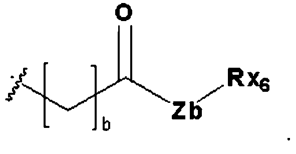 . In this case, it may be provided that Zb is a single bond, -NH, -NHCH₂-, -NH(CH₂)₂ -CH₂-, -(CH₂)₂-, -(CH₂)₃-, -CH₂NH-, Rx₆ is 5- to 12-membered heteroaryl including at least one heteroatom selected from the group consisting of N, O and S, 6- to 10-membered aryl (e.g., C₆₋₁₂ aryl such as phenyl), 3- to 10-membered heterocycloalkyl including at least one heteroatom selected from the group consisting of N, O and S, -NYcYd (Yc or Yd may be each independently H, -C₁₋₆ alkyl or C₃₋₆ cycloalkyl) or 3- to 10-membered cycloalkyl (e.g., C₃₋₁₀ cycloalkyl such as cyclopropyl or cyclohexyl), and b is an integer of 0 to 4. In this case, it may be provided that -H of the 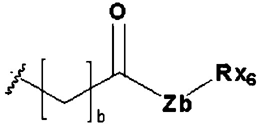 is unsubstituted or at least one -H is each independently substituted with -C₁₋₅ alkyl (-H of the alkyl may be unsubstituted or at least one -H may be each independently substituted with one selected from the group consisting of phenyl, pyridinyl, morpholinyl, piperazine and piperidine), -CF₃, 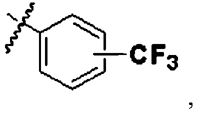 pyridinyl, pyrimidinyl, piperidinyl, piperazinyl, -morpholinyl (-H of pyridinyl, pyrimidinyl, piperidinyl, piperazinyl and morpholinyl may be unsubstituted or at least one -H may be each independently substituted with one selected from the group consisting of -C₁₋₃ alkyl and -CF₃) 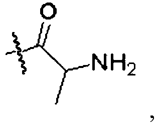 -NH₂, -F, -Cl, -Br or -I.

16) In any one of above 1) to 6), according to embodiments of the present invention, it may be provided that the R₂ is 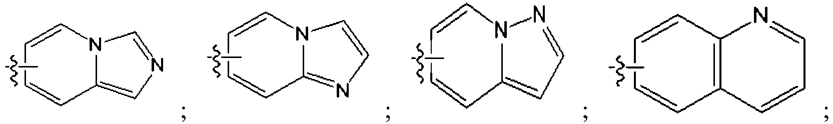 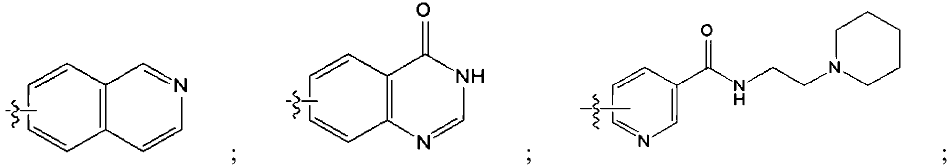 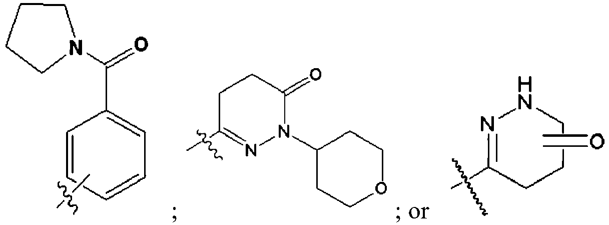 . In this case, it may be provided that -H of the group is unsubstituted or at least one -H is each independently substituted with one selected from the group consisting of C₁₋₅ alkyl, -NH₂, -NHCH₂CH₃, -(C₁₋₅ alkyl)NH₂, -F, -Cl, -Br, and -I.

17) In any one of above 1) to 16), according to embodiments of the present invention, it may be provided that R₂ is 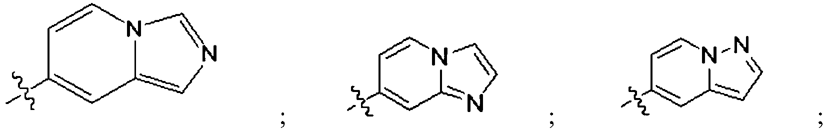 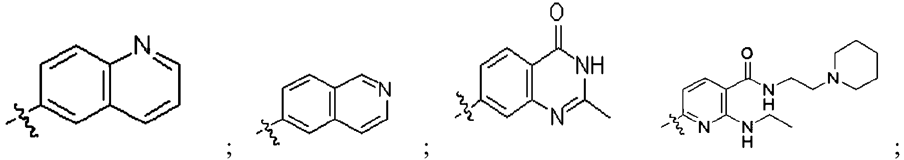 or 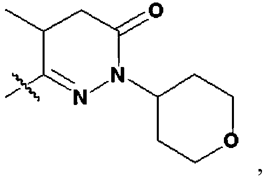 in which -H of the group may be unsubstituted or at least one -H may be each independently substituted with one selected from the group consisting of -C₁₋₅ alkyl, -NH₂, -NHCH₂CH₃, -F, -Cl, -Br, and -I.

18) In any one of above 1) to 17), according to embodiments of the present invention, it may be provided that R₃ is 6- to 12-membered aryl (e.g., C₆₋₁₂ aryl); 5- to 12-membered heteroaryl including at least one heteroatom selected from the group consisting of N, O and S; 3- to 10-membered heterocycloalkyl including at least one heteroatom selected from the group consisting of N, O and S; or 3- to 10-membered cycloalkyl (e.g., C₃₋₁₀ heterocycloalkyl).

It may be provided that the above group listed as the R₃ is each independently unsubstituted or at least one H is each independently substituted with -NH₂, -OH, -NO₂, C₁₋₃ alkyl, -F, -Cl, -Br, -I, -C₁₋₃alkylamine, 6- to 12-membered aryl, 5- to 10-membered heteroaryl including at least one heteroatom selected from the group consisting of N, O and S, or 3- to 10-membered heterocycloalkyl including at least one heteroatom selected from the group consisting of N, O and S, C₅₋₁₂ bicycloalkyl, 3- to 8-membered cycloalkyl, -CHF₂, or -CF₃.

19) In any one of above 1) to 18), according to embodiments of the present invention, it may be provided that -H of each group listed above as the R₃ is each independently unsubstituted or at least one -H is each independently substituted with one group selected from the group consisting of 1) to 18) below:
1) C₁₋₅ alkyl;
2) -F, -Cl, -Br, or -I
3) 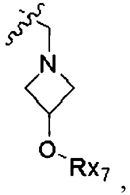 in which Rx₇ may be C₁₋₃ alkyl or -CF₃;
4) 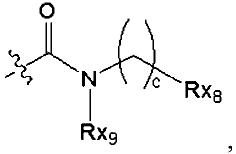 in which c is 0, 1, 2 or 3, Rx₈ may be C₁₋₅alkyl, -NH₂, - NHCH₃, -N(CH₃)₂, piperidinyl, piperazinyl, morpholinyl, 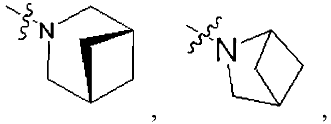 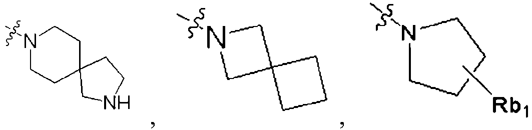 (the Rb₁ may be -NH₂ or -CH₂OH), 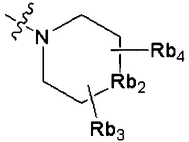 (the Rb₂ may be -CH₂-, -NH- or -O-, and Rb₃ and Rb₄ are each independently -H, -F, -Cl, -Br, or -I), pyridinyl, pyrimidinyl or pyrrolyl, and Rx₉ may be -H or C₁₋₅ alkyl;
5) -COOH;
6) -NRx₁₀Rx₁₁, in which Rx₁₀ or Rx₁₁ may be each independently -H or -CH₃;
7) -CF₃;
8) -CN;
9) Morpholinyl or morpholidinyl in which at least one -H is each independently substituted with C₁₋₅ alkyl, -F, -Cl, -Br, I, -C(=O)NHCH₃, -C(=O)CH₃ or -C(=O)CH=CH₂;
10) Piperidinyl or piperidinyl in which at least one -H is each independently substituted with C₁₋₅ alkyl, -F, -Cl, -Br, I, -C(=O)NHCH₃, -C(=O)CH₃ or -C(=O)CH=CH₂;
11) Piperazinyl or piperazinyl in which at least one -H is each independently substituted with C₁₋₅ alkyl, -F, -Cl, -Br, I, -C(=O)NHCH₃, -C(=O)CH₃ or -C(=O)CH=CH₂;
12) -ORx₁₂, in which R₁₂Rx₁₂ may be -CF₃; C₁₋₃ alkyl;
13) -OH;
14) 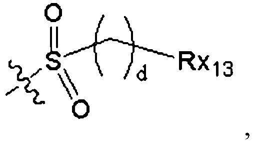 in which d may be 0, 1, 2 or 3, and Rx₁₃ is -NH₂, - NH(CH₃), -N(CH₃)₂pyrazolyl, methylpyrazolyl, pyrazolyl, piperazinyl, piperidinyl, or morpholinyl;
15) 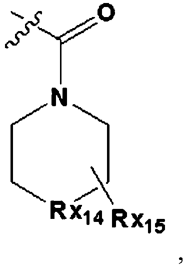 in which Rx₁₄ may be -CH₂-, -NH or -O-, and Rx₁₅ may be -H, C₁₋₅ alkyl, -NH₂, -F, -Cl, -Br, or -I;
16) 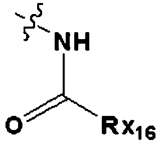 , in which Rx₁₆ may be C₁₋₅ alkyl or -CH₂CH₂N(CH₃)₂,
17) 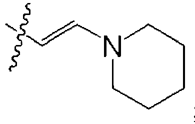 or
18) -(C₁₋₃ alkyl)-NH₂.

20) In any one of above 1) to 19), according to embodiments of the present invention, it may be provided that the R₃ is 6- to 8-membered aryl, -H of the aryl is unsubstituted or at least one -H is each independently substituted with one group selected from the group consisting of above 1) to 18).

21) In any one of above 1) to 20), according to embodiments of the present invention, it may be provided that the R₃ is phenyl, -H of the aryl is unsubstituted or at least one -H is each independently substituted with one group selected from the group consisting of above 1) to 18).

22) In any one of above 1) to 21), according to embodiments of the present invention, it may be provided that the Ak is -(CH₂)n- or 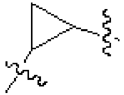 , in which -H of the -(CH₂)n- or 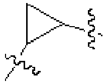 may be each independently unsubstituted or at least one -H is each independently substituted with NH₂, -OH, -NO₂, -C₁₋₅ alkyl, -CH₂NH₂, -CF₃, OCF₃, -CN, -F, -Cl, -Br, or -I, and n may be 1, 2 or 3.

23) In any one of above 1) to 22), according to embodiments of the present invention, it may be provided in above formula I that X₁, X₂ and X₃ are each independently -CH or -N.

24) In any one of above 1) to 23), according to embodiments of the present invention, it may be provided that the urea compound represented by above formula I is a compound represented by formula I-1 below.

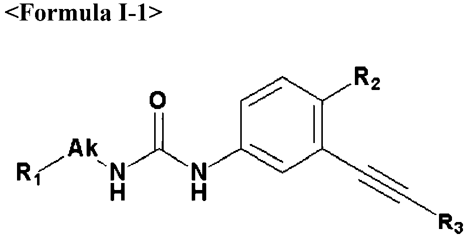

According to embodiments of the present invention, it may be provided in compounds represented by above formula I or I-1 that R₁, R₂, R₃, Ak, X₁, X₂ and X₃ are as follows, respectively:
In embodiments of the present invention, it may be provided that R₁ is pyridinyl; pyrimidinyl; pyrazolyl; imidazolyl; pyrrolyl; furanyl; phenyl; phenolic; 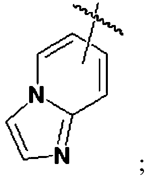 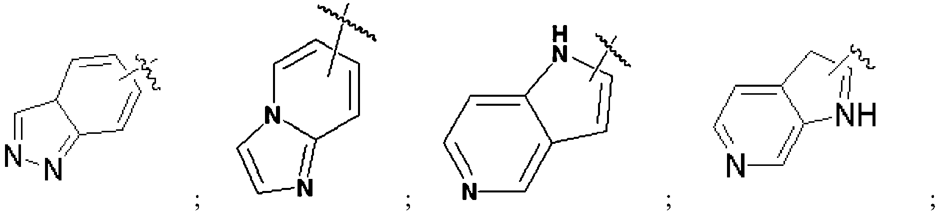 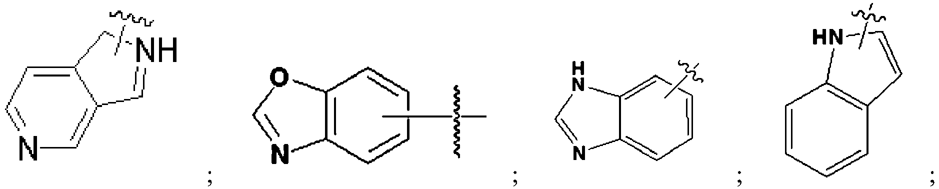 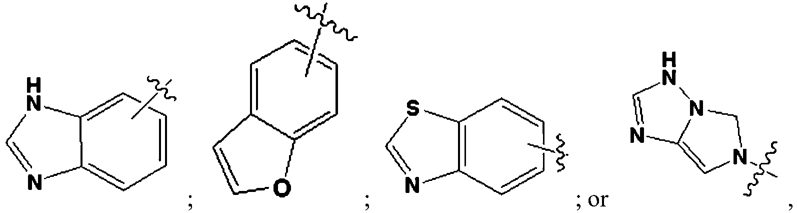 in which -H of each group of R₁ listed above may be each independently unsubstituted or at least one H may be each independently substituted with -NH₂, -(C₁₋₃ alkyl)NH₂, -NH(C₁₋₃ alkyl), -N(C₁₋₃ alkyl)₂, -OH, -NO₂, C₁₋₅ alkyl, -F, -Cl, -Br, or -I.

In embodiments of the present invention, it may be provided that the R₂ is
pyridinyl; pyrimidinyl; phenyl; piperidinyl; piperazinyl; morpholinyl; pyrazolyl, imidazolyl, phenolic, phenyl; -CH₂C(=O)NH₂; -C(=O)NH₂; -C(=O)NHCH₂CH₃; - CH₂NHC(=O)CH₂CH₃; -NH₂; -NHCH₃, -NHCH₂CH₂CH₃; 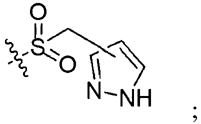 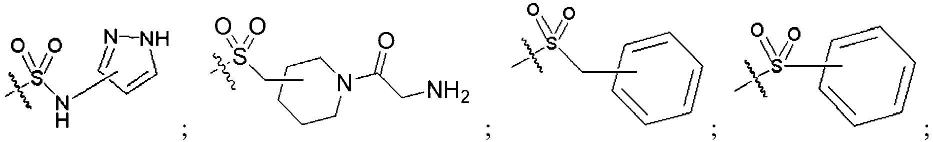 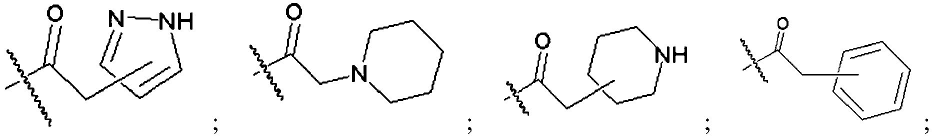 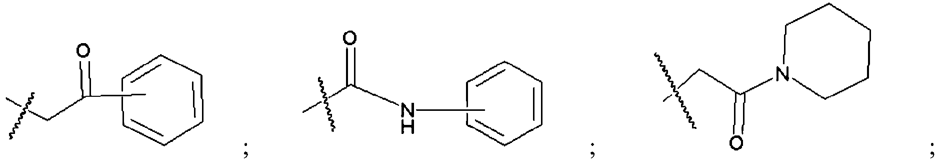 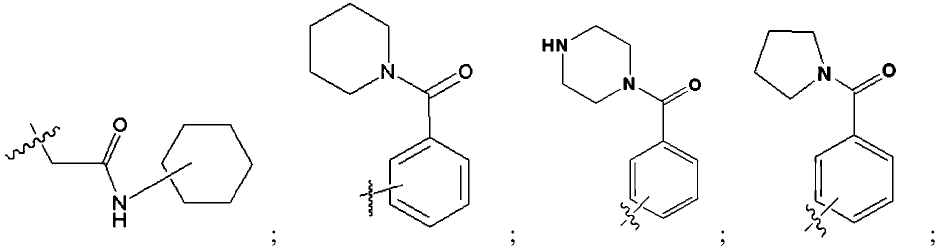 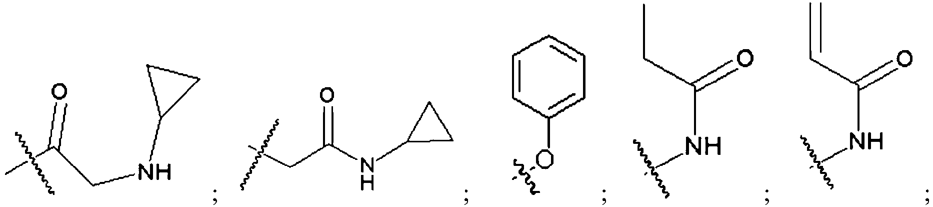 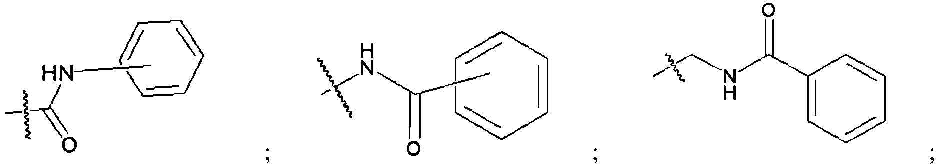 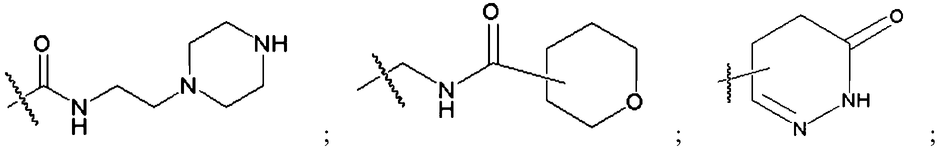 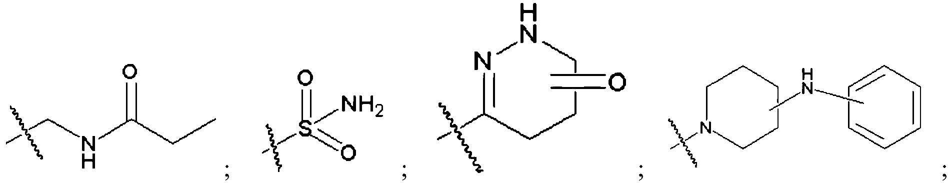 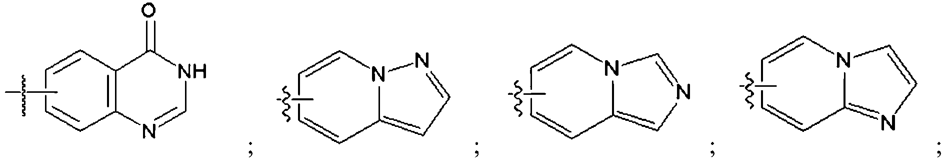 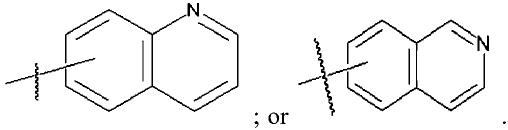 In this case, it may be provided that -H of each group of R₂ listed above is each independently unsubstituted or at least one -H is each independently substituted with -NH₂, -NH(C₁₋₅ alkyl), -N(C₁₋₅ alkyl)₂, -NHCH₃, -N(CH₃)₂, - NHCH₂CH₃, -OH, -NO₂, -S(=O)₂CH₃, C₁₋₅ alkyl (-H of the alkyl may be unsubstituted or at least one -H may be each independently substituted with one selected from the group consisting of phenyl, pyridinyl, morpholinyl, piperazine and piperidine), -morpholidinyl-piperidinyl, - piperazinyl, phenyl, pyridinyl, pyrimidinyl (-H of the -morpholidinyl, -piperidinyl, -piperazinyl, -phenyl, -pyridinyl and -pyrimidinyl may be unsubstituted or at least one -H may be each independently substituted with one selected from the group consisting of -C₁₋₃ alkyl and -CF₃), cyclohexyl, -cyclobutoxy, -cyclopropoxy, -cyclopentoxy, -F, -Cl, -Br, -I, -CHF₂, -CF₃, -C₁₋₃ alkoxy, -NHC(=O)CH₃, -C(=O)CH₂CH₃, -C(=O)N(CH₃)₂, -C(=O)NH(CH₃), -C(=O)NH₂, - S(=O)₂NHCH₃; -S(=O)₂, -S(=O)₂CH₃, -trifluoromethylphenyl, 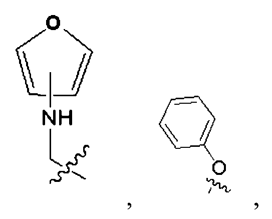 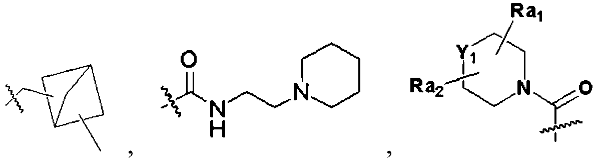 (the Ra₁ and Ra₂ may be each independently H, F, Cl, Br-, I or -C₁₋₅ alkyl, and Y₁ may be one selected from -CH₂, -NH, - and -O-), 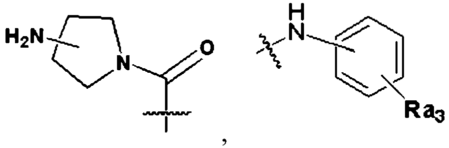 (Ra₃ may be -H, -C₁₋₅ alkyl or -CF₃),

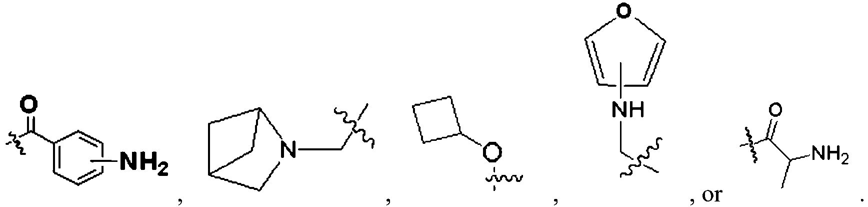

R₃ may be phenyl. In this case, it may be provided that -H of each group of R₃ listed above is each independently unsubstituted or at least one -H is each independently substituted with one group selected from the group consisting of 1) to 18) below:
1) C₁₋₅ alkyl;
2) -F, -Cl, -Br, or -I
3) 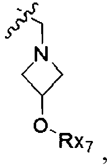 in which Rx7 may be -CF₃;
4) 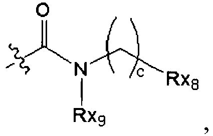
   in which if c is 0 (in which Rx₈ is directly bonded to -N), Rx₈ may be C₁₋₅ alkyl or piperidine, and Rx₉ may be -H, C₁₋₅ alkyl;
   if c is 1, Rx₈ is 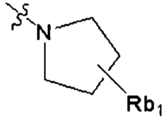 (the Rb₁ is -NH₂ or -CH₂OH), and Rx₉ is -H or C₁₋₅ alkyl;
   if c is 2, Rx₈ is C₁₋₃ alkyl, -NH₂, -NHCH₃, -N(CH₃)₂, piperidinyl, difluoropiperidinyl, piperazinyl, morpholinyl, 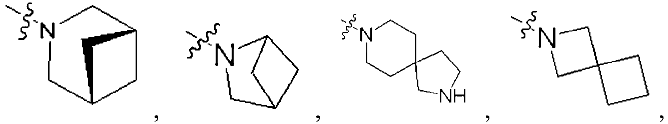 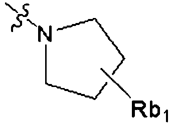 (the Rb₁ may be -NH₂ or -CH₂OH), pyridinyl, pyrrolyl, and Rx₉ may be -H or C₁₋₅ alkyl;
5) -COOH;
6) -NRx₁₀Rx₁₁, in which Rx₁₀ and Rx₁₁ may be each independently -H or -CH₃;
7) -CF₃;
8) -CN;
9) Morpholidinyl or morpholidinyl in which at least one -H is each independently substituted with C₁₋₃ alkyl, -F, -Cl, -Br , -I, -C(=O)NHCH₃, -C(=O)CH₃ or -C(=O)CH=CH₂;
10) Piperidinyl or piperidinyl in which at least one -H is each independently substituted with C₁₋₃ alkyl, -F, -Cl, -Br, -I, -C(=O)NHCH₃, -C(=O)CH₃ or -C(=O)CH=CH₂;
11) Piperazinyl or piperazinyl in which at least one -H is each independently substituted with C₁₋₃ alkyl, -F, -Cl, -Br, I, -C(=O)NHCH₃, -C(=O)CH₃ or -C(=O)CH=CH₂;
12) -ORx₁₂, in which R₁₂ Rx₁₂ may be -CF₃; C₁₋₃ alkyl;
13) -OH;
14) 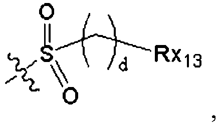 in which d may be 1, 2 or 3, and Rx₁₃ may be -NH₂, - NH(CH₃), -N(CH₃)₂ pyrazolyl, methylpyrazolyl, pyrazolyl, piperazinyl, piperidinyl, or morpholinyl;
15) 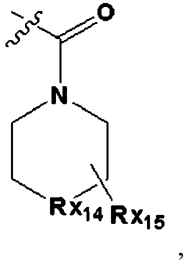 , in which Rx₁₄ may be -CH₂-, NH- or -O-, and Rx₁₅ may be -H, C₁₋₅ alkyl, -NH₂, -F, -Cl, -Br, or I;
16) 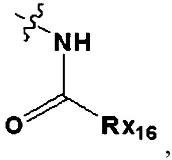 in which Rx₁₆ may be C₁₋₅ alkyl or -CH₂CH₂N(CH₃)₂;
17) 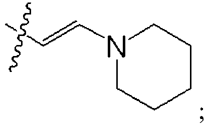 or
18) Ethylamine.

It may be provided that Ak is -CH₂-, -(CH₂)₂-, -(CH₂)₃- or 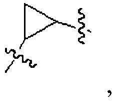 -H of -CH₂-, - (CH₂)₂-, -(CH₂)₃- or 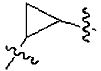 is each independently unsubstituted or at least one -H is each independently substituted with -NH₂, -OH, -NO₂, C₁₋₅ alkyl, -CH₂NH₂, -CF₃, OCF₃, -CN, -F, - Cl, -Br, or -I, and
X₁, X₂ and X₃ are -CH-.

25) In any one of above 1) to 24), according to embodiments of the present invention, it may be specifically provided in above formula I or I-1 that R₁, R₂, R₃, Ak, X₁, X₂ and X₃ are as follows, respectively:
In embodiments of the present invention, it may be provided in above formula I or I-1 that R₁ is one of groups in Table 1 below:

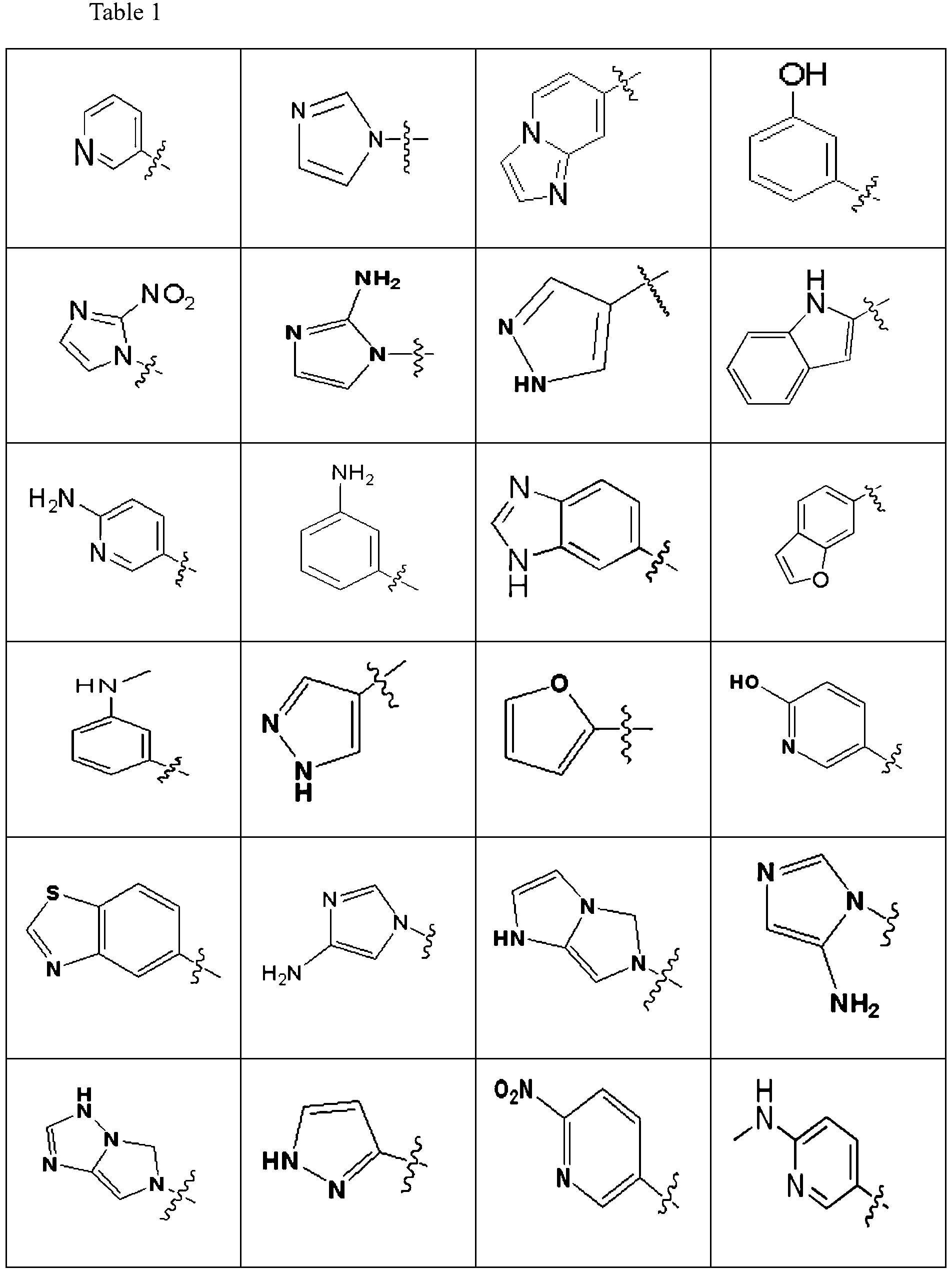

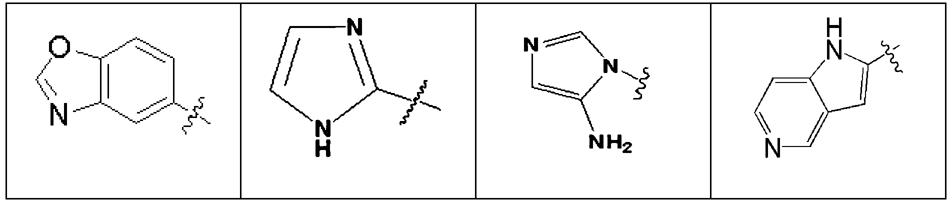

It may be provided that -H of groups listed in above Table 1 is unsubstituted or at least one H is each independently substituted with -NH₂, -OH, -NO₂, -CH₂NH₂, -CF₃, OCF₃, -CN, - C₁₋₅ alkyl, -F, -Cl, -Br, or -I.

In embodiments of the present invention, it may be provided in above formula I or I-1 that R₂ is one of groups in the following Table:

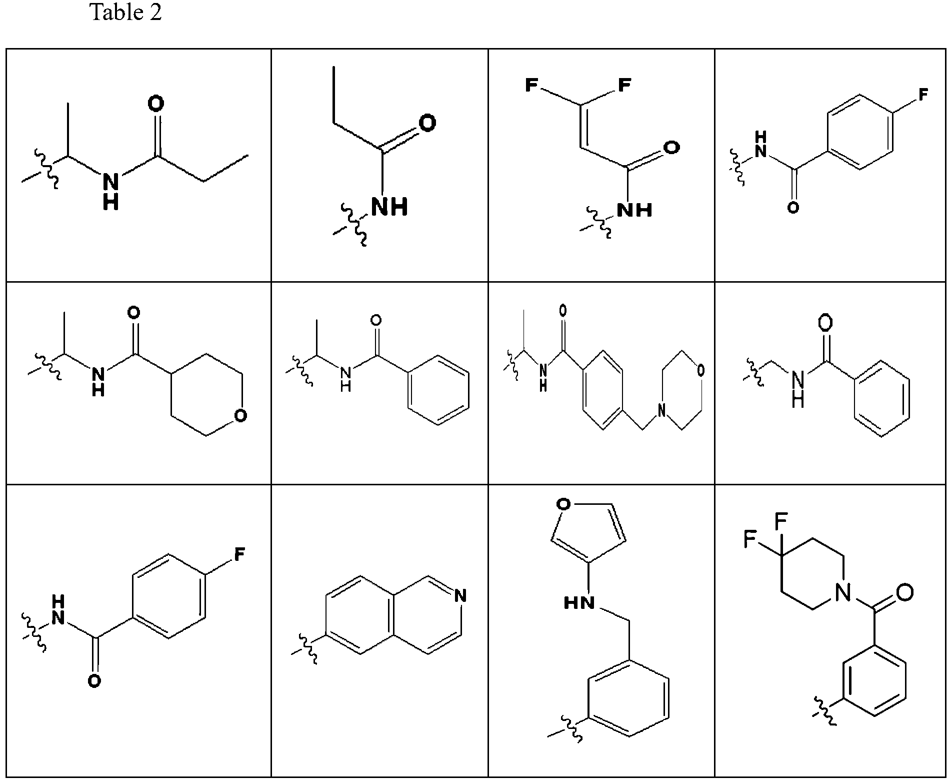

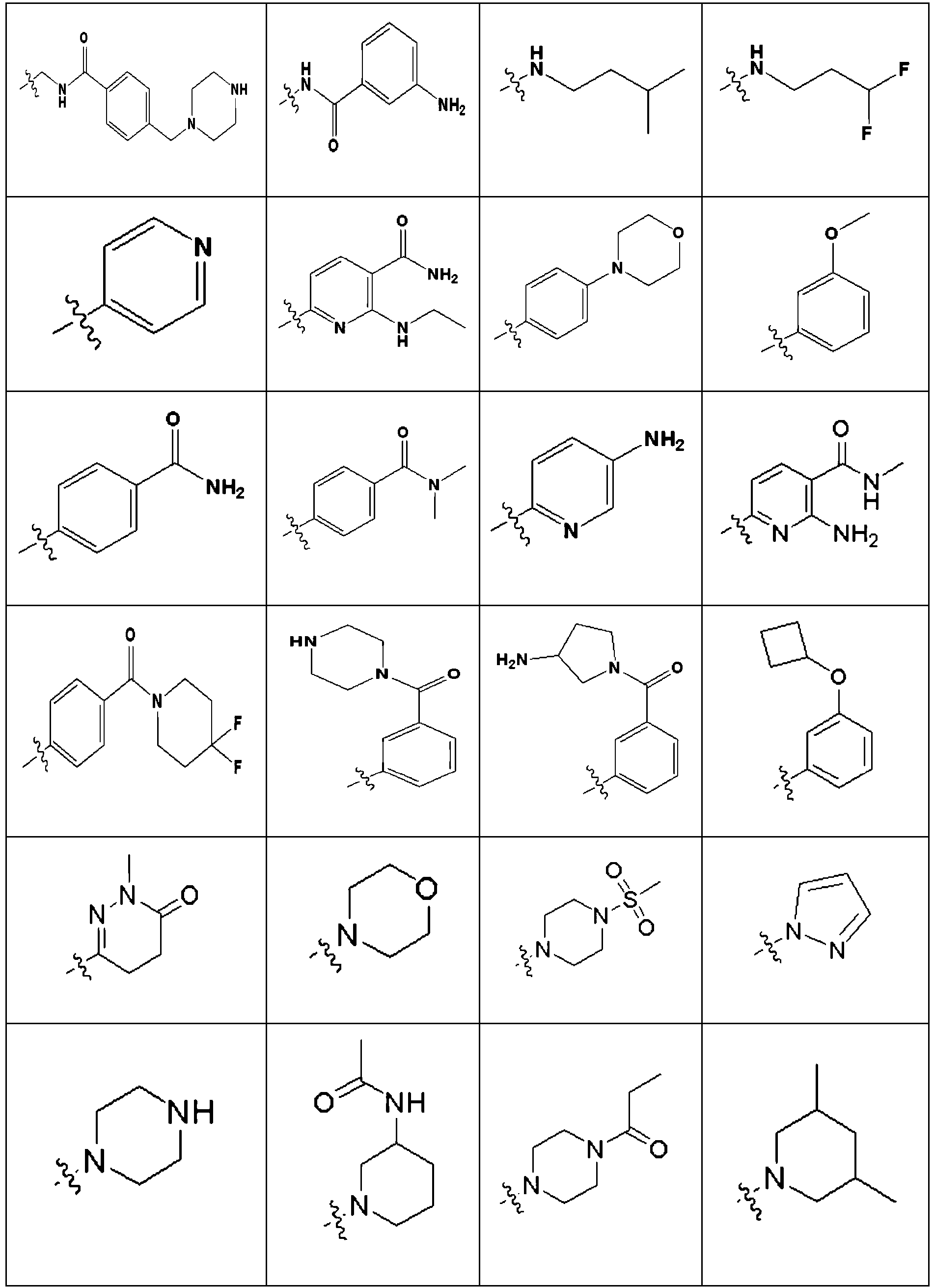

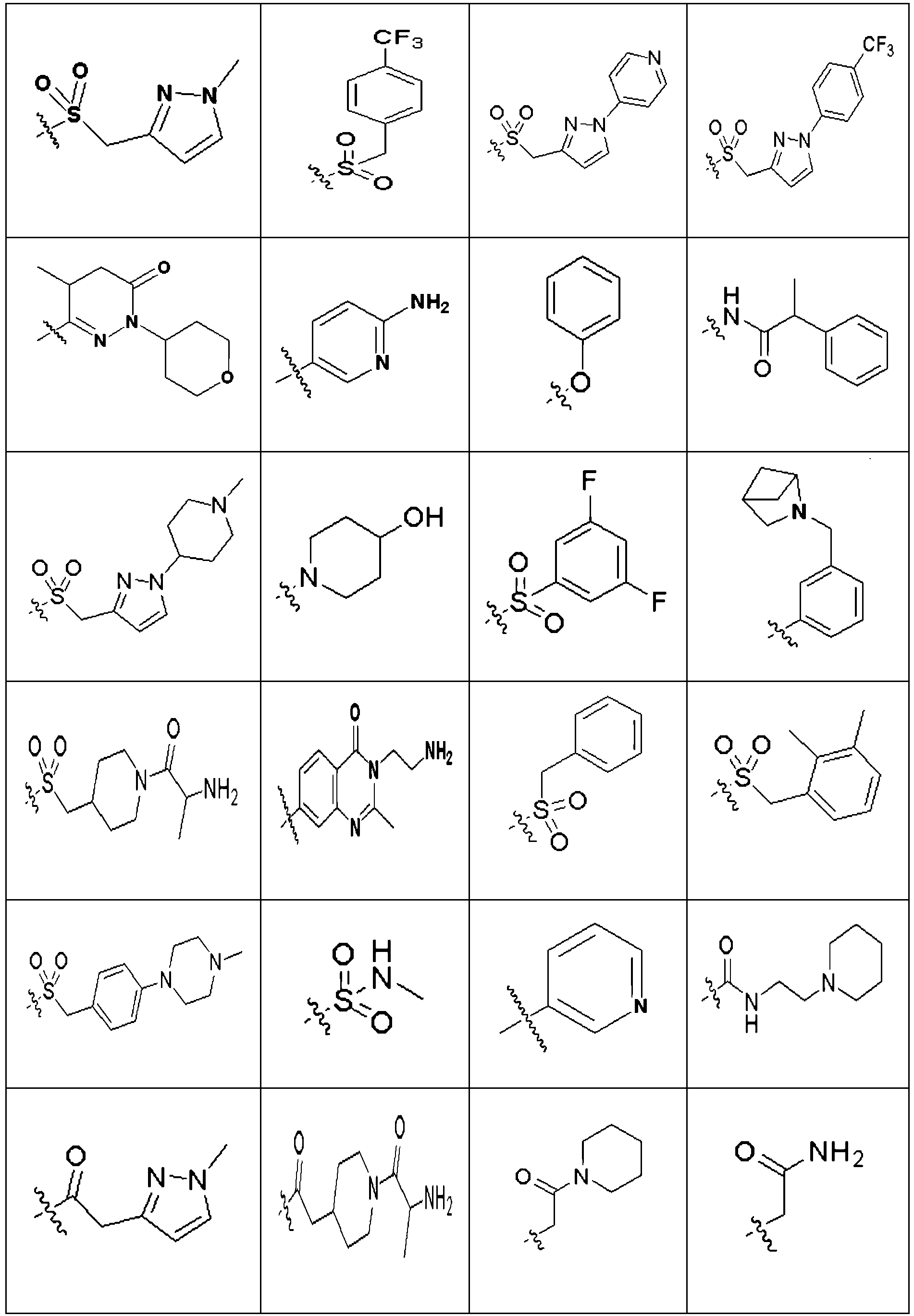

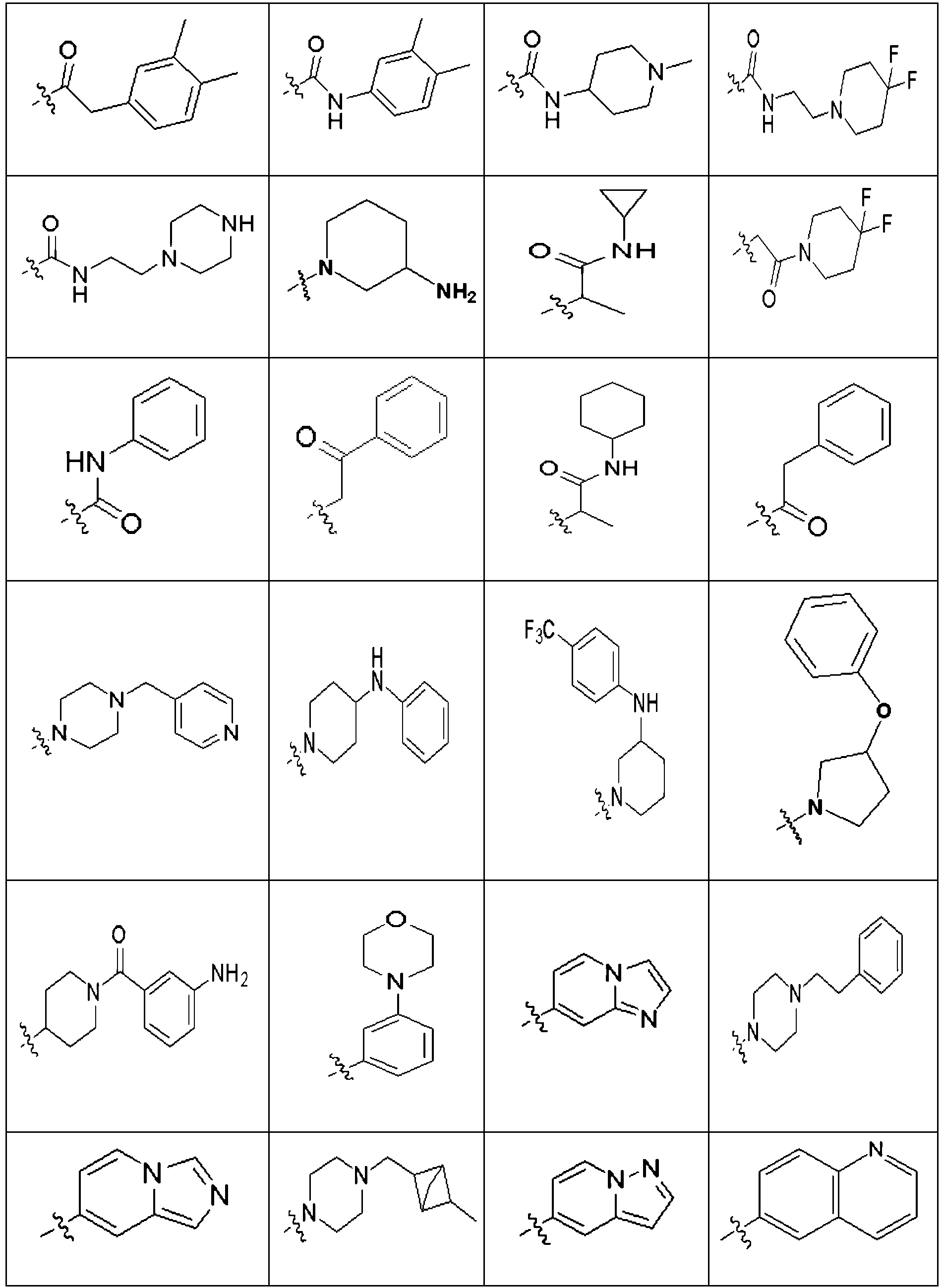

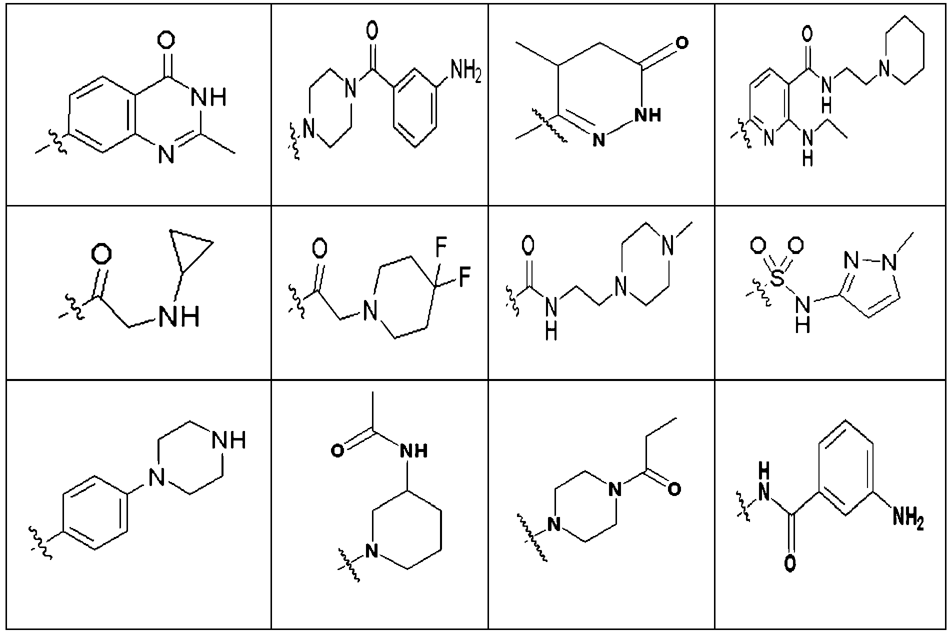

It may be provided that the groups listed in above Table 2 are unsubstituted or at least one H is each independently substituted with -NH₂, -OH, NO₂, -C₁₋₅ alkyl, -CH₂NH₂, -CF₃, - OCF₃, -CN, -F, -Cl, -Br, or -I,

In embodiments of the present invention, it may be provided in above formula I or I-1 that R₃ is one of groups in the following Table:

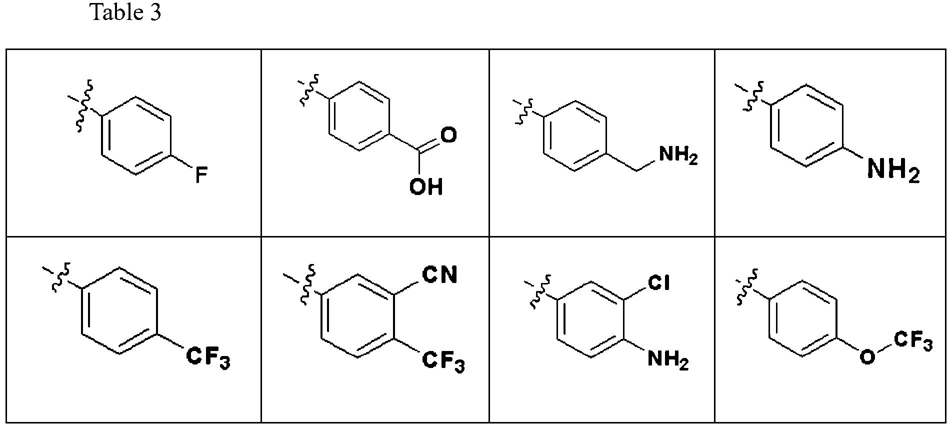

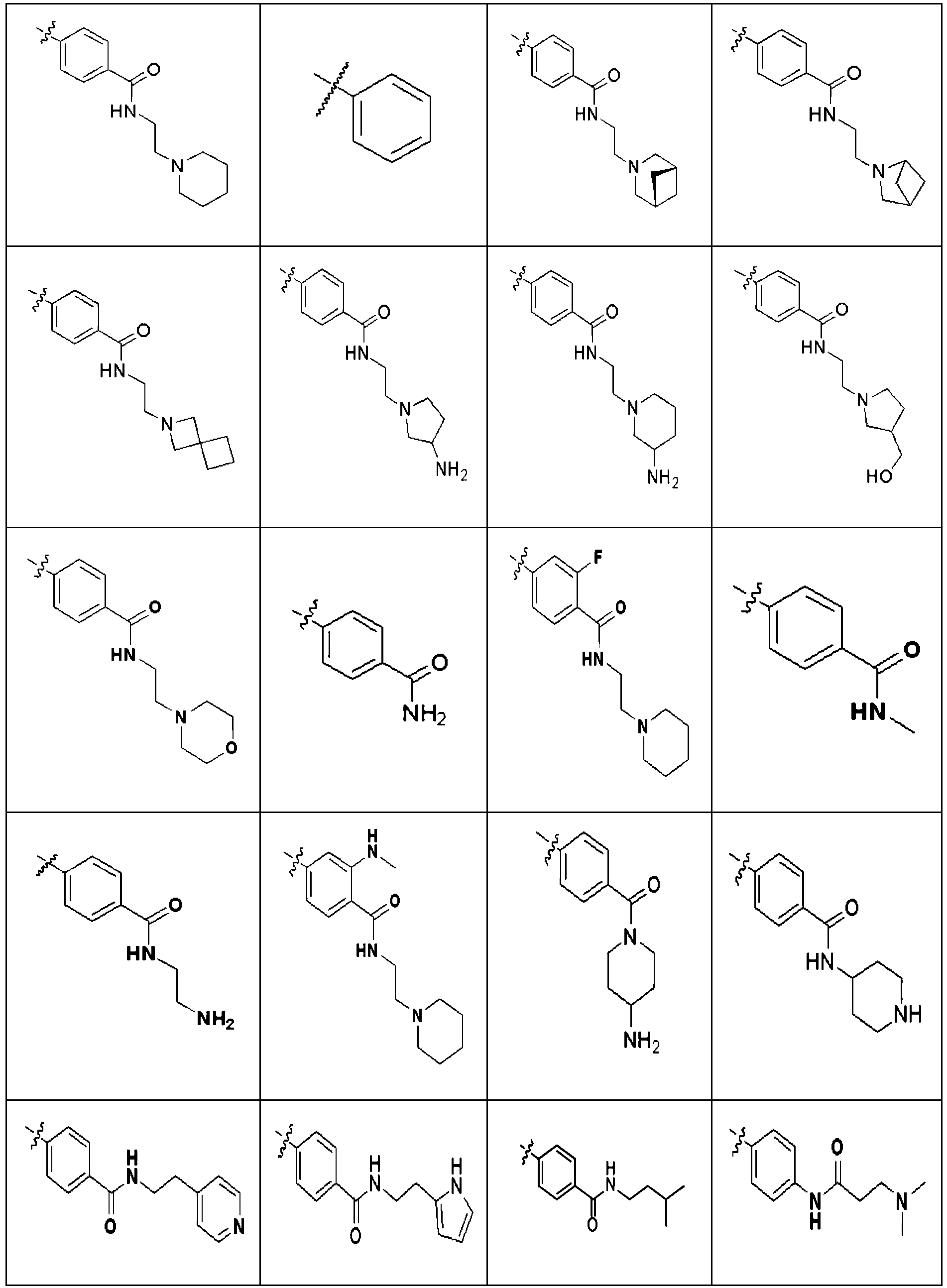

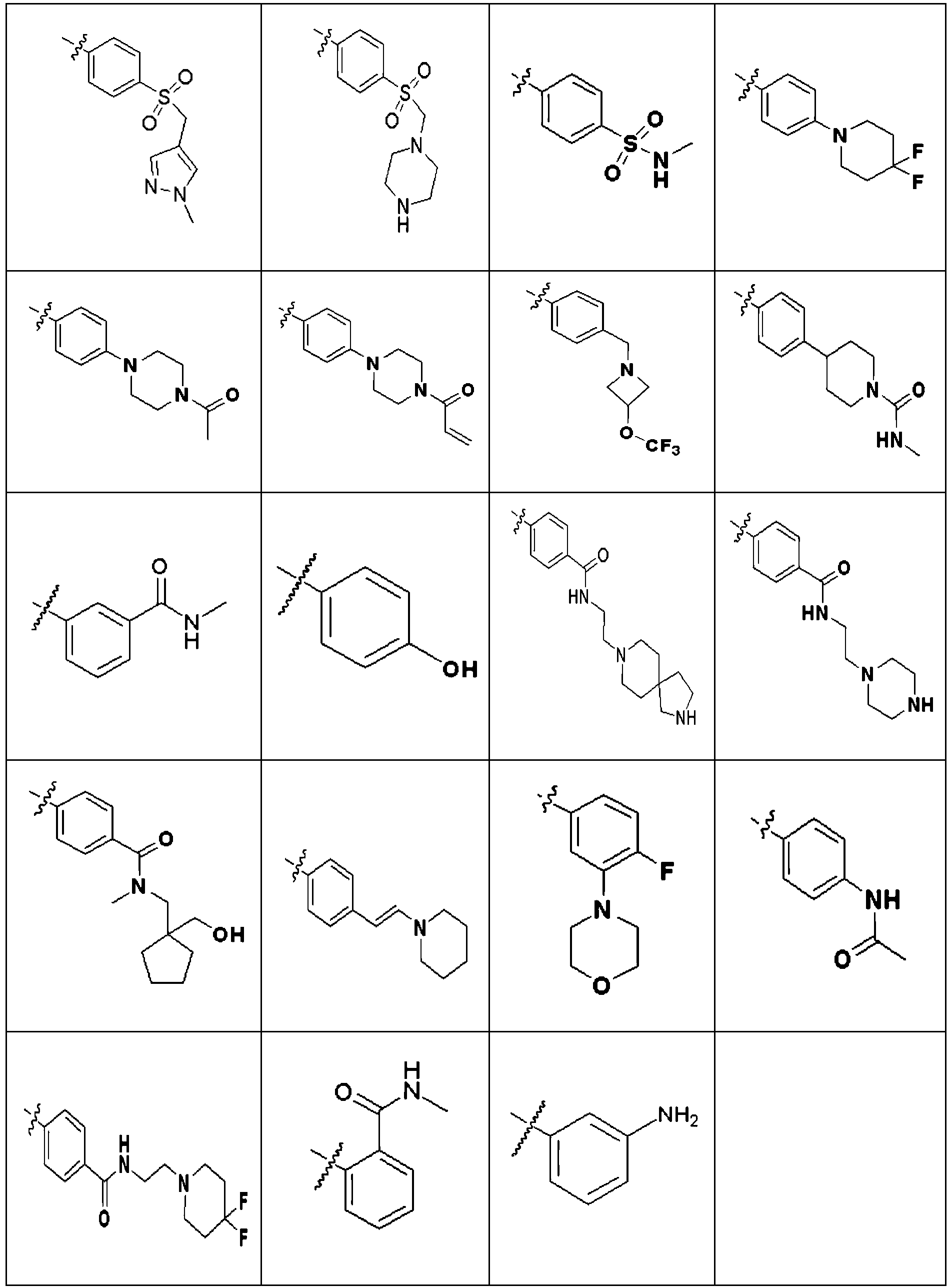

It may be provided that the groups listed in above Table 3 are unsubstituted or at least one H is each independently substituted with -NH₂, -OH, -NO₂, -C₁₋₅ alkyl, -CH₂NH₂, -CF₃, - OCF₃, -CN, -F, -Cl, -Br, or -I.

In embodiments of the present invention, it may be provided in above formula I or I-1 that Ak is -(CH₂)n- or 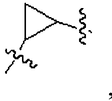 in which -H of the -(CH₂)n- or 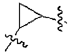 may be each independently unsubstituted or at least one -H is each independently substituted with -NH₂, - OH, -NO₂, C₁₋₅ alkyl, -CH₂NH₂, -CF₃, OCF₃, -CN, -F, -Cl, -Br, or -I, and n may be 1, 2 or 3.

In embodiments of the present invention, it may be provided that X₁, X₂ and X₃ are each independently -CH- or -N-, specifically -CH-.

26) In any one of above 1) to 25), according to embodiments of the present invention, it may be provided that the urea compound represented by above formula I-1 is a compound represented by formula I-2 below.

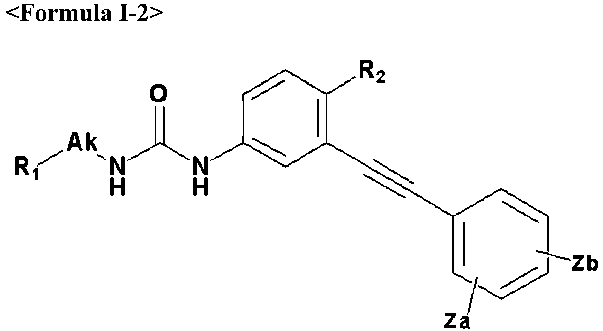

In above formula I-2,
R₁, R₂ and Ak are the same as defined in above formula I-1, and
Za and Zb are each independently -H or one selected from the group consisting of 1) to 18) below:
   1) C₁₋₅ alkyl;
   2) -F, -Cl, -Br, or -I
   3) 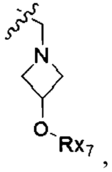 in which Rx₇ may be C₁₋₅ alkyl or -CF₃;
   4) 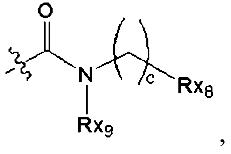 in which c is 0, 1, 2 or 3, Rx₈ may be C₁₋₅ alkyl, -NH₂, - NHCH₃, -N(CH₃)₂, piperidinyl, piperazinyl, morpholinyl, 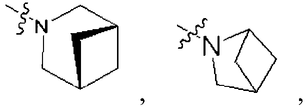 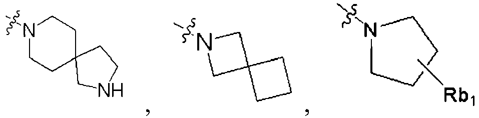 (the Rb₁ may be -NH₂ or -CH₂OH), or 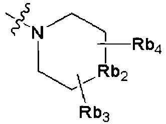 (the Rb₂ may be -CH₂-, -NH- or -O-, and Rb₃ and Rb₄ are each independently -H, -F, -Cl, -Br, or -I), pyrimidinyl or pyrrolyl, and Rx₉ may be -H or C₁₋₅ alkyl;
   5) -COOH;
   6) -NRx₁₀Rx₁₁, in which Rx₁₀ or Rx₁₁ may be each independently -H or -CH₃;
   7) -CF₃;
   8) -CN;
   9) Morpholinyl or morpholidinyl in which at least one -H is each independently substituted with C₁₋₅ alkyl, -F, -Cl, -Br, I, -C(=O)NHCH₃, -C(=O)CH₃ or -C(=O)CH=CH₂;
   10) Piperidinyl or piperidinyl in which at least one -H is each independently substituted with C₁₋₅ alkyl, -F, -Cl, -Br, I, -C(=O)NHCH₃, -C(=O)CH₃ or -C(=O)CH=CH₂;
   11) Piperazinyl or piperazinyl in which at least one -H is each independently substituted with C₁₋₅ alkyl, -F, -Cl, -Br, I, -C(=O)NHCH₃, -C(=O)CH₃ or -C(=O)CH=CH₂;
   12) -ORx₁₂, in which R₁₂Rx₁₂ may be -CF₃; C₁₋₃ alkyl;
   13) -OH;
   14) 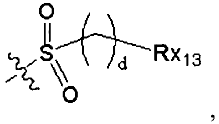 in which d may be 0, 1, 2 or 3, and Rx₁₃ may be -NH₂, - NH(CH₃), -N(CH₃)₂ pyrazolyl, methylpyrazolyl, pyrazolyl, piperazinyl, piperidinyl, or morpholinyl;
   15) 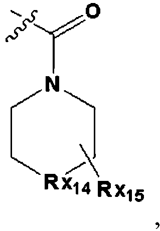 in which Rx₁₄ may be -CH₂-, NH- or -O-, and Rx₁₅ may be -H, linear or branched C₁₋₅ alkyl, -NH₂, -F, -Cl, -Br, or -I;
   16) 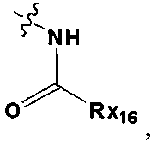 in which Rx₁₆ may be C₁₋₅ alkyl or -CH₂CH₂N(CH₃)₂,
   17) 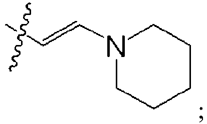 or
   18) -(C₁₋₃ alkyl)-NH₂.

27) In any one of above 1) to 25), according to embodiments of the present invention, a urea compound represented by formula I-2 below, an optical isomer thereof, a pharmaceutically acceptable salt of them, or a hydrate or solvate of them may be provided: 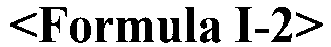 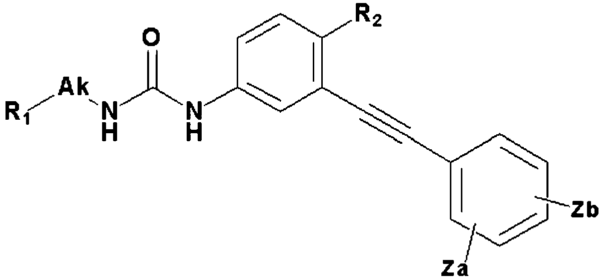 in above formula I-2, R₁ may be pyridinyl; pyrimidinyl; imidazolyl; pyrazolyl; phenyl; 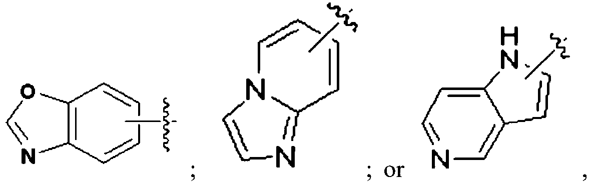 in which -H of R₁ may be each independently unsubstituted or at least one -H may be each independently substituted with morpholinyl, piperidinyl, piperazinyl, -NH₂, -(C₁₋₃ alkyl)NH₂, -NH-(C₁₋₃ alkyl), -N(C₁₋₃ alkyl)₂, -OH, -NO₂, C₁₋₅ alkyl, -F, -Cl, -Br, or -I,
R₂ is pyridinyl; pyrimidinyl; phenyl; piperazinyl; piperidinyl; 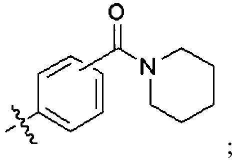 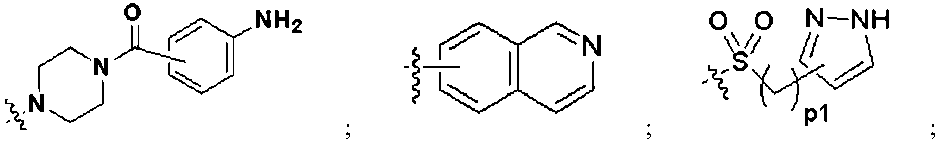 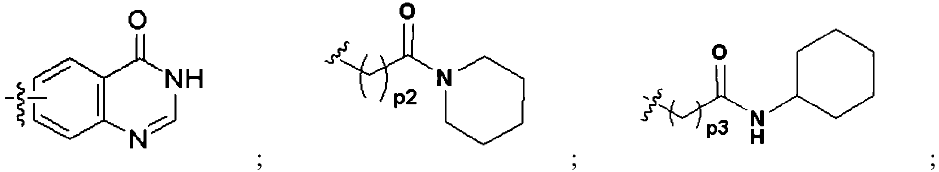 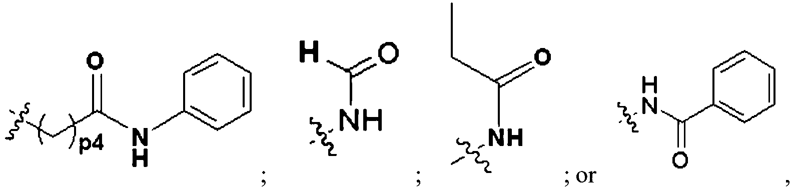 and the p1, p2, p3, or p4 are each independently an integer of 0 to 3, in which -H of R₂ may be each independently unsubstituted or at least one -H may be each independently substituted with morpholinyl, piperidinyl, piperazinyl, -NH₂, -(C₁₋₃ alkyl)NH₂, -NH-(C₁₋₃ alkyl), -N(C₁₋₃ alkyl)₂, -OH, -NO₂, C₁₋₅ alkyl, -F, -Cl, -Br, or -I,
Za or Zb may be each independently -H, morpholinyl, piperidinyl, piperazinyl, - NHC(=O)(C₁₋₃ alkyl), -NH₂, -(C₁₋₃ alkyl)NH₂, -NH(C₁₋₃ alkyl), -N(C₁₋₃ alkyl)₂, -OH, -NO₂, - C₁₋₅ alkyl, -F, -Cl, -Br, -I, 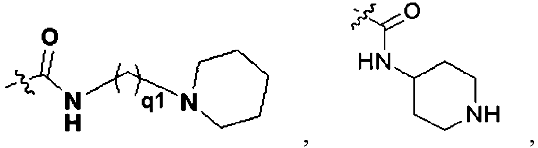 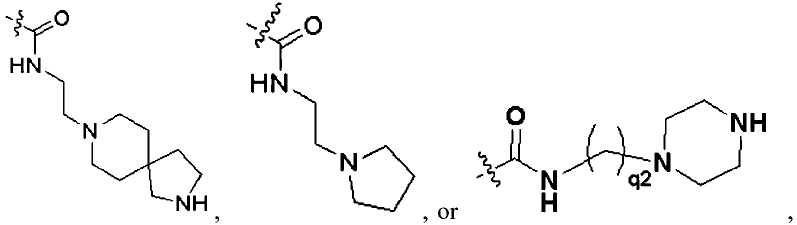 and the ql or q2 may be each independently an integer of 0 to 3, in which -H of the Za or Zb may be each independently unsubstituted or at least one -H may be each independently substituted with -C₁₋₅ alkyl, -NH₂, -(C₁₋₃ alkyl)NH₂, -NH-(C₁₋₃ alkyl), or -N(C₁₋₃alkyl)₂, and
the Ak is -(CH₂)n-, in which the n may be 1, 2 or 3.

28) In any one of above 1) to 26), the present invention provides compounds 1 to 189 described in Table 4 below, optical isomers thereof, pharmaceutically acceptable salts of them, or hydrates or solvates of them:

**Table 4**

| **No.** | **Compound** |
|---|---|
| **1** | 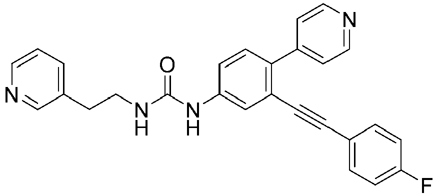 |
| **2** | 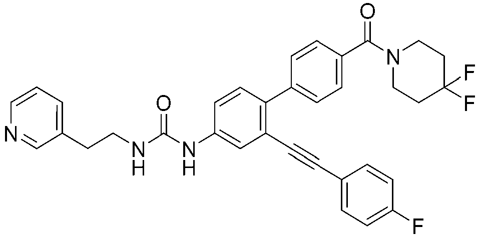 |
| **3** | 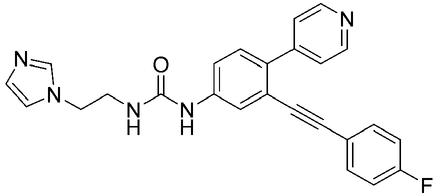 |
| **4** | 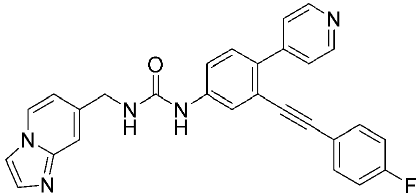 |
| **5** | 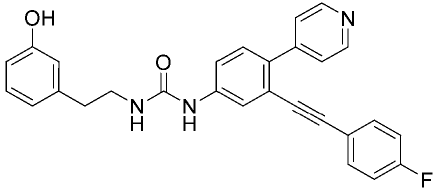 |
| **6** | 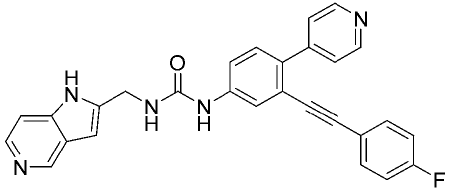 |
| **7** | 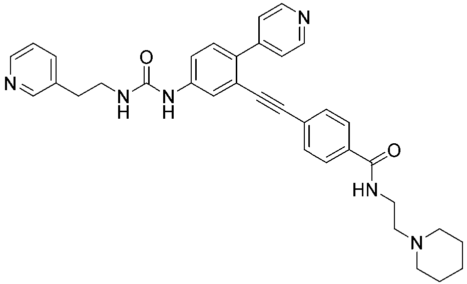 |
| **8** | 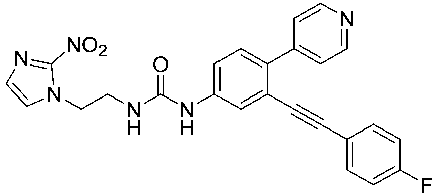 |
| **9** | 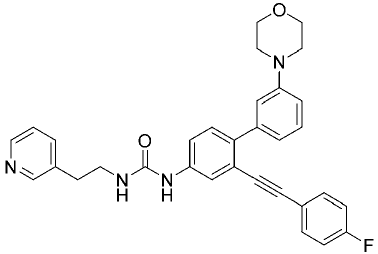 |
| **10** | 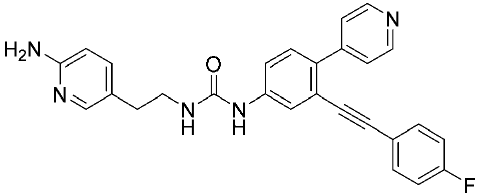 |
| **11** | 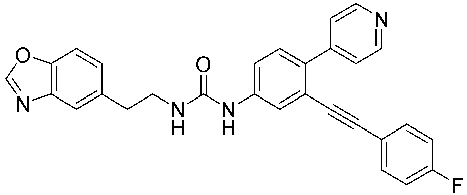 |
| **12** | 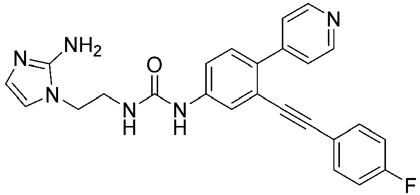 |
| **13** | 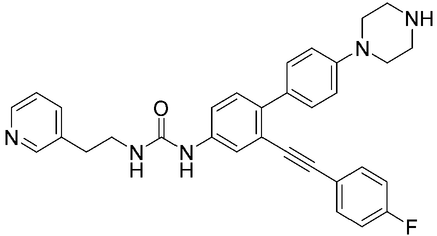 |
| **14** | 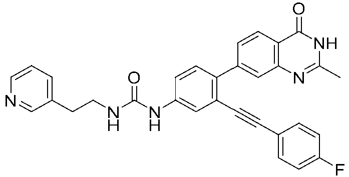 |
| **15** | 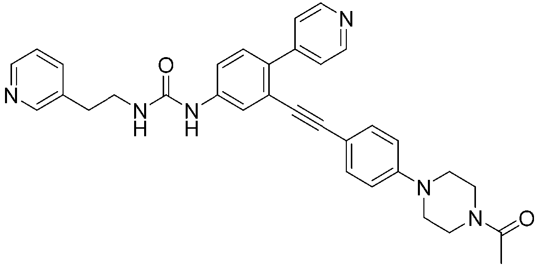 |
| **16** | 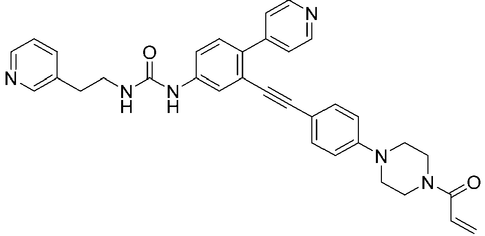 |
| **17** | 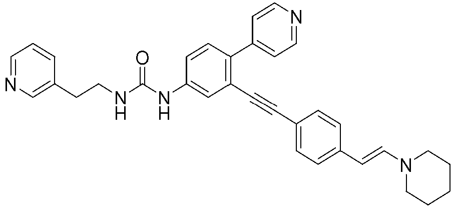 |
| **18** | 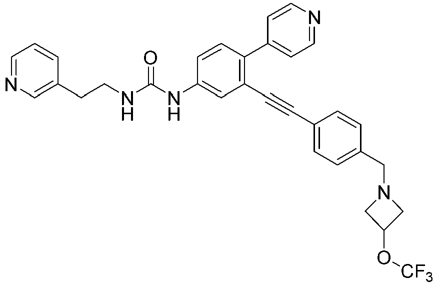 |
| **19** | 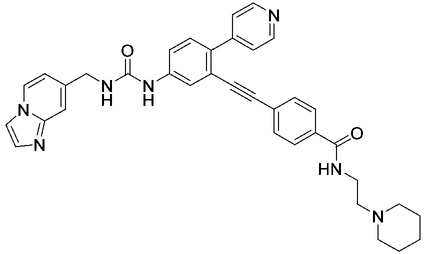 |
| **20** | 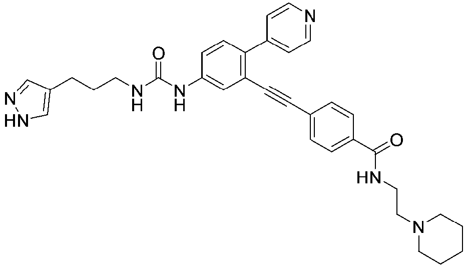 |
| **21** | 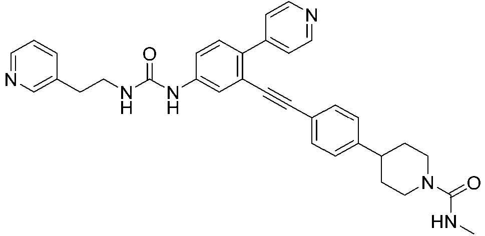 |
| **22** | 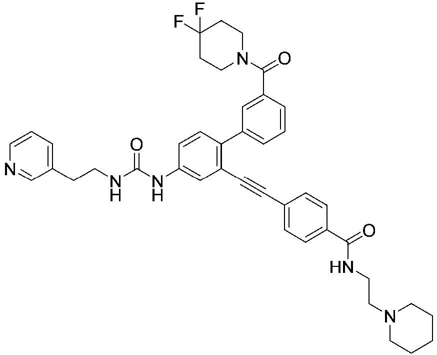 |
| **23** | 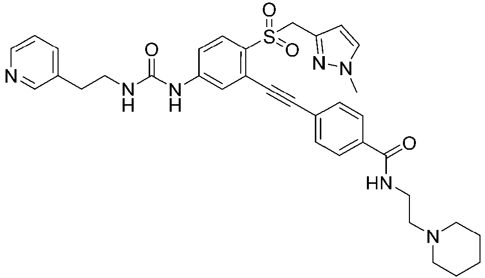 |
| **24** | 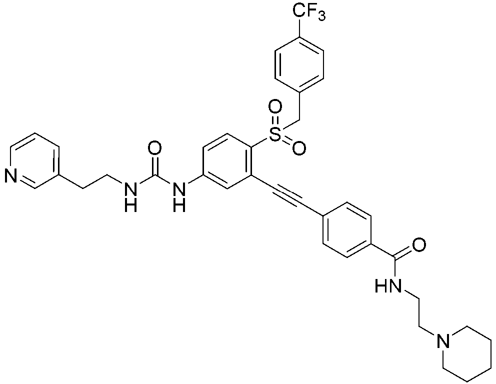 |
| **25** | 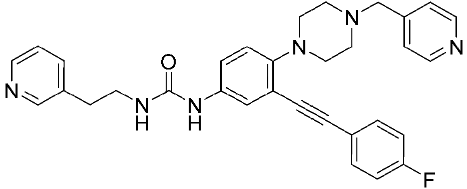 |
| **26** | 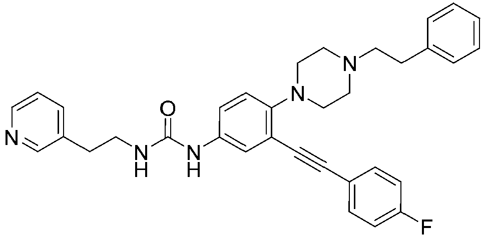 |
| **27** | 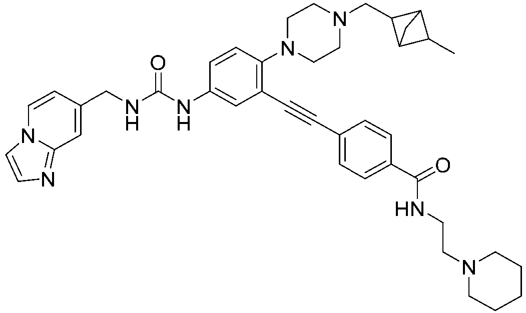 |
| **28** | 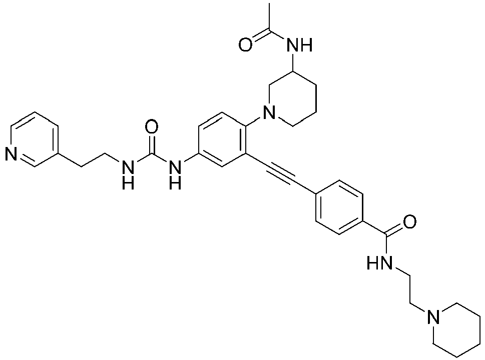 |
| **29** | 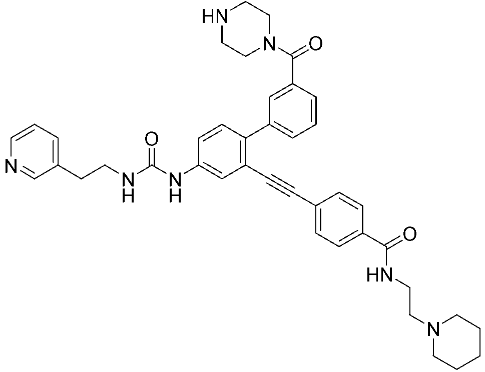 |
| **30** | 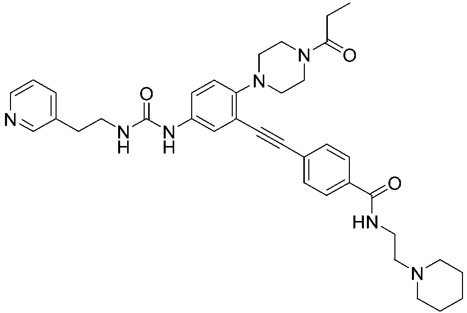 |
| **31** | 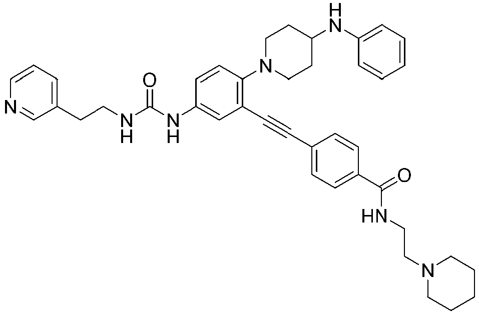 |
| **32** | 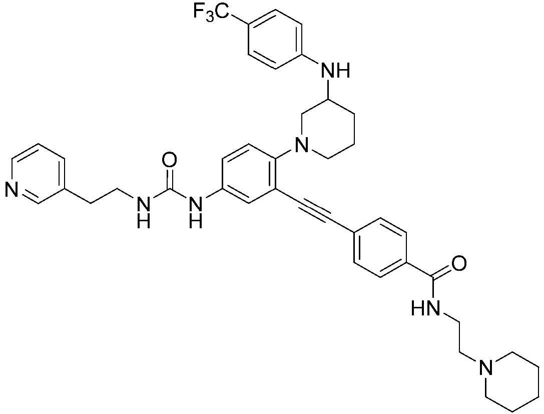 |
| **33** | 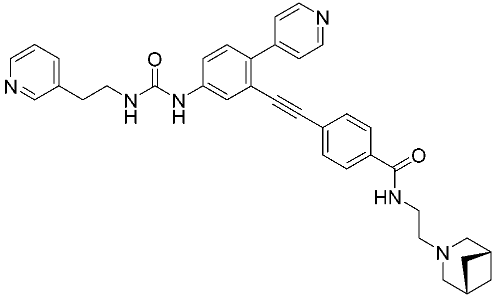 |
| **34** | 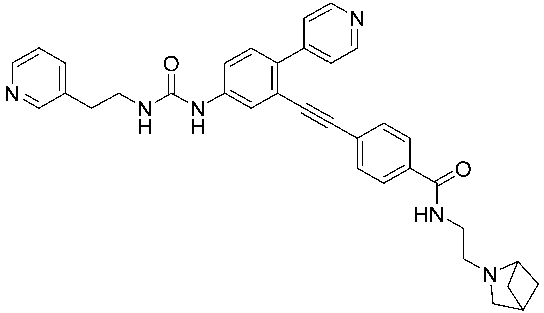 |
| **35** | 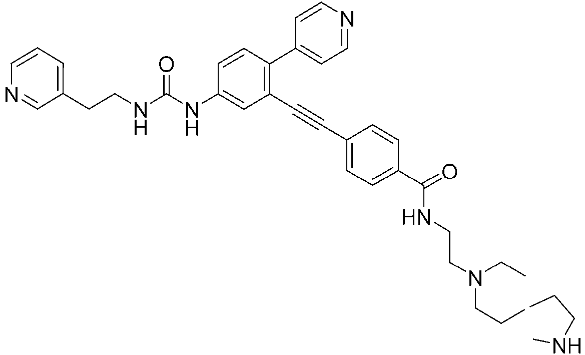 |
| **36** | 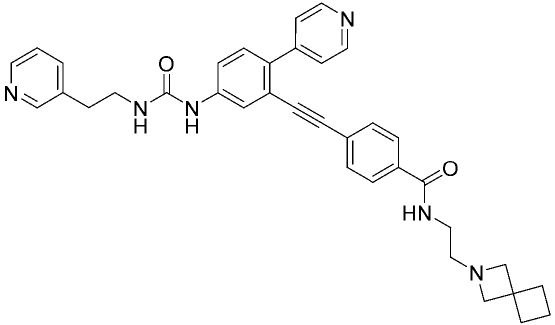 |
| **37** | 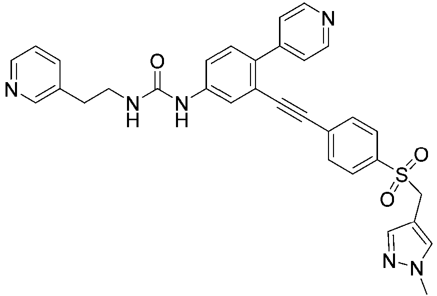 |
| **38** | 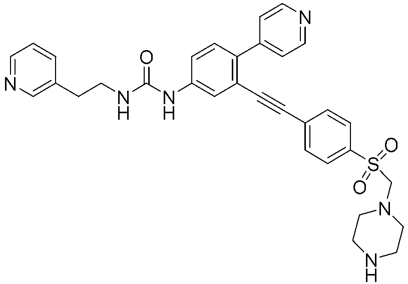 |
| **39** | 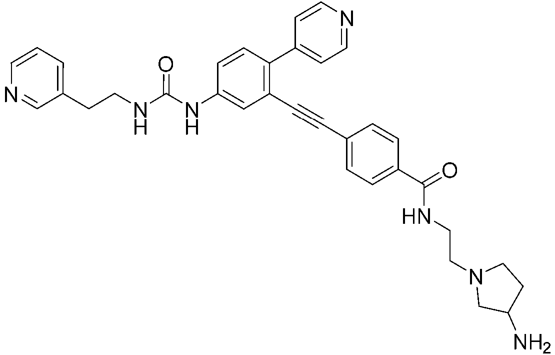 |
| **40** | 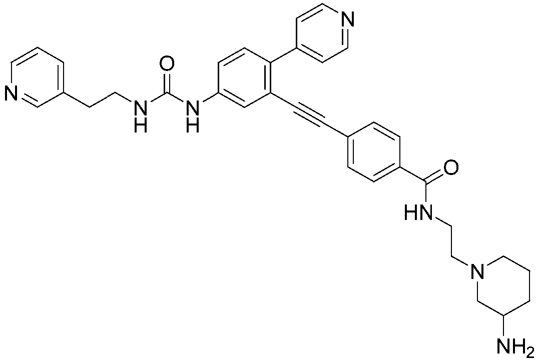 |
| **41** | 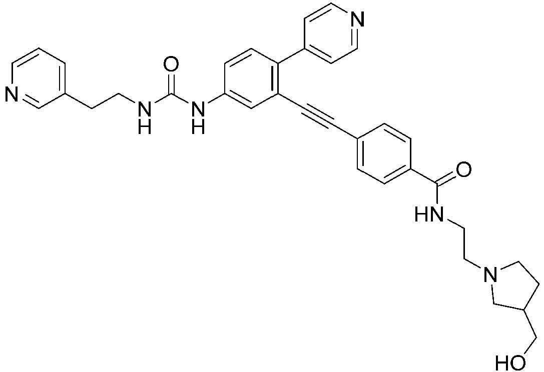 |
| **42** | 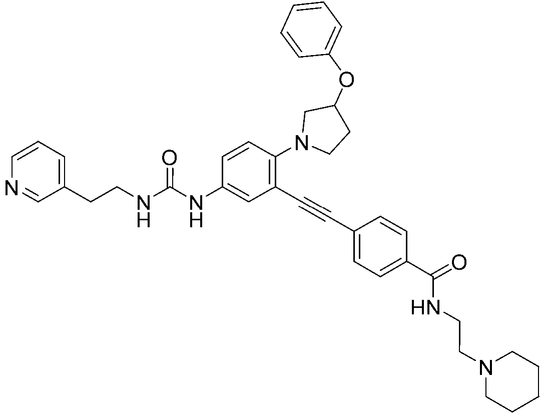 |
| **43** | 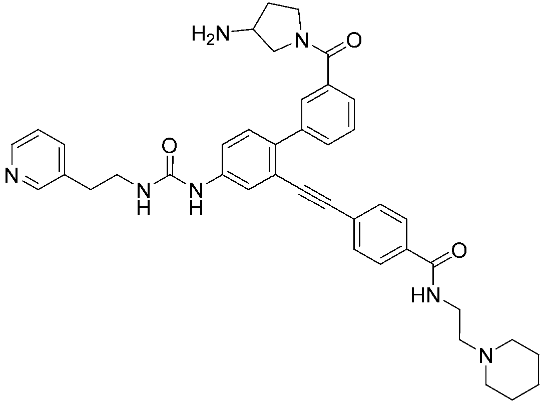 |
| **44** | 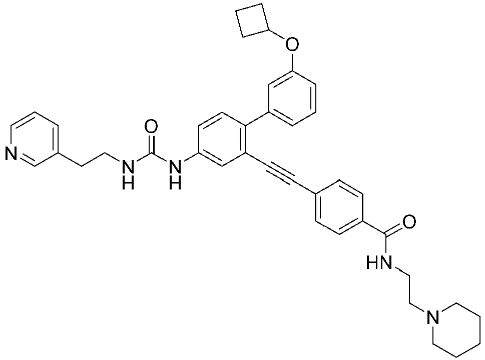 |
| **45** | 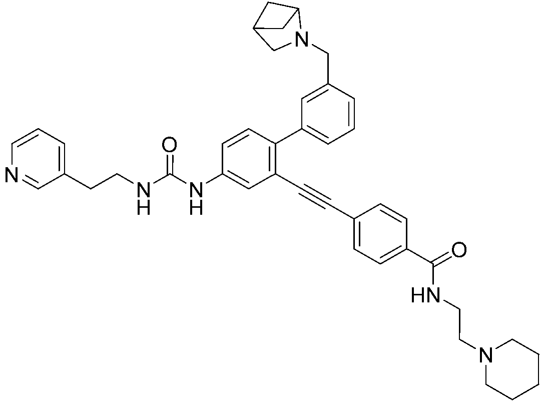 |
| **46** | 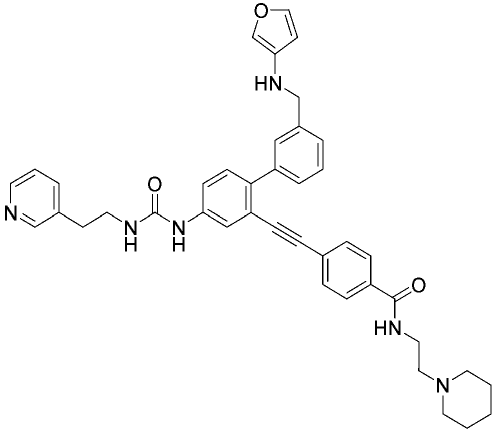 |
| **47** | 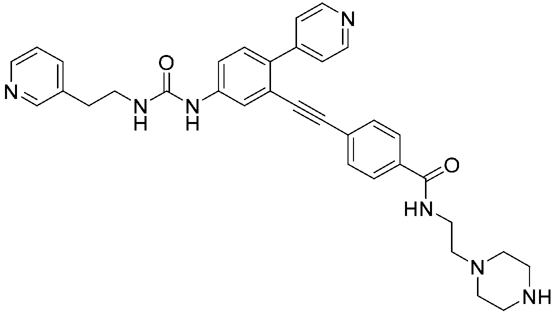 |
| **48** | 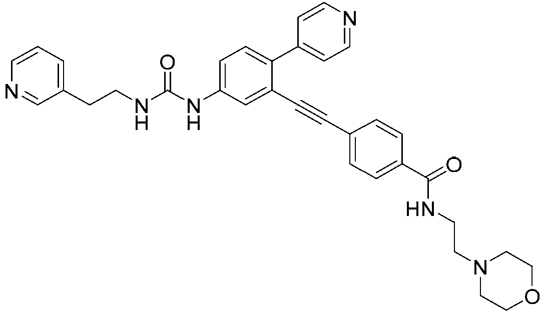 |
| **49** | 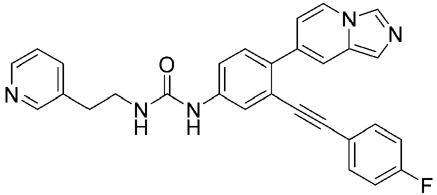 |
| **50** | 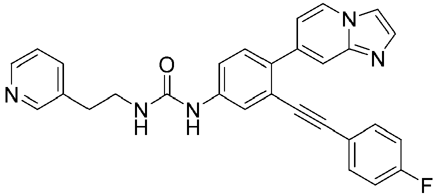 |
| **51** | 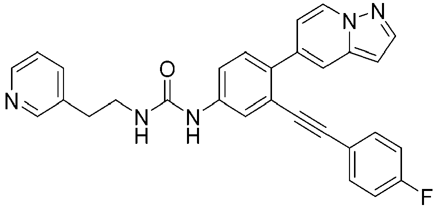 |
| **52** | 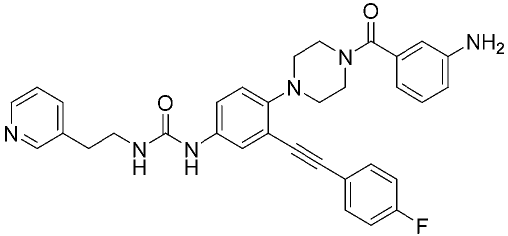 |
| **53** | 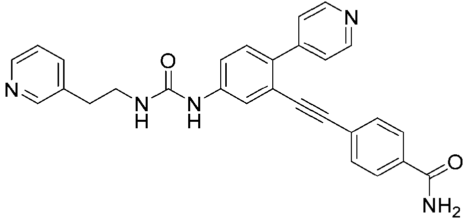 |
| **54** | 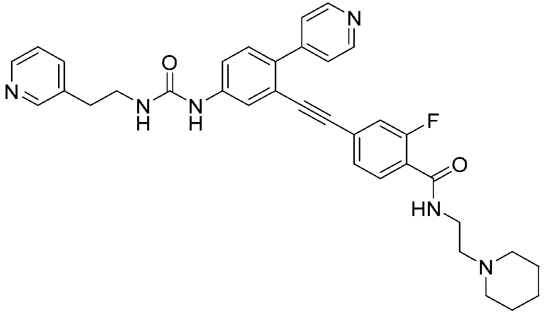 |
| **55** | 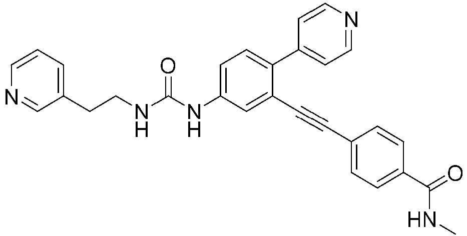 |
| **56** | 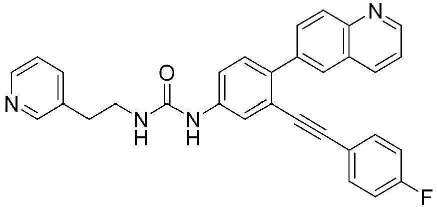 |
| **57** | 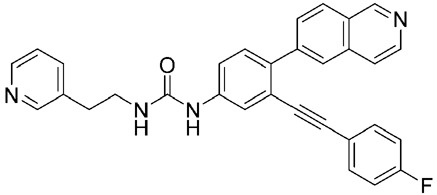 |
| **58** | 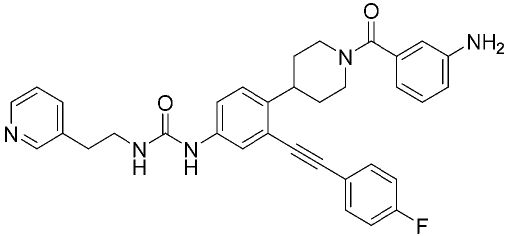 |
| **59** | 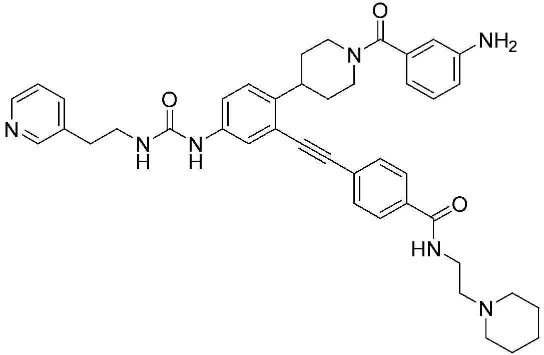 |
| **60** | 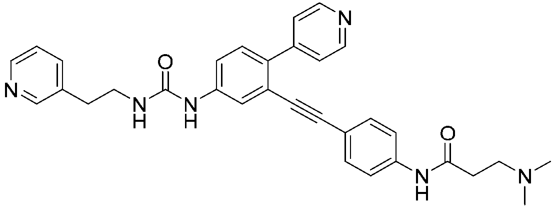 |
| **61** | 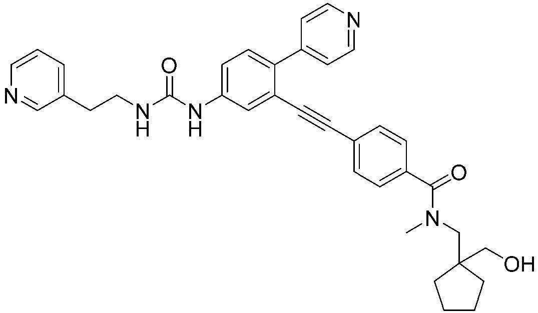 |
| **62** | 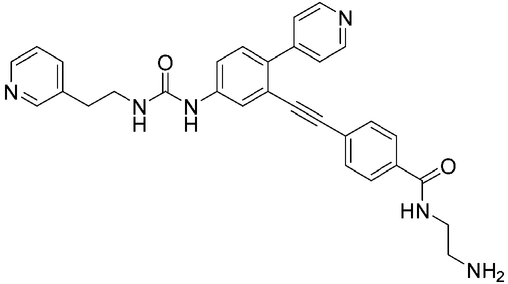 |
| **63** | 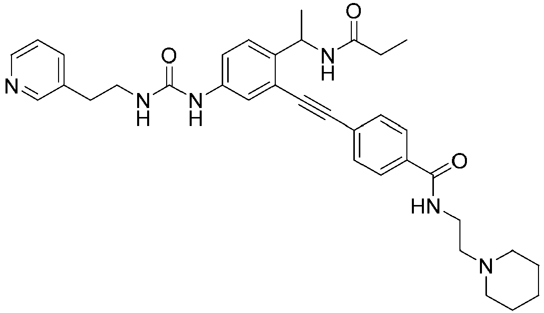 |
| **64** | 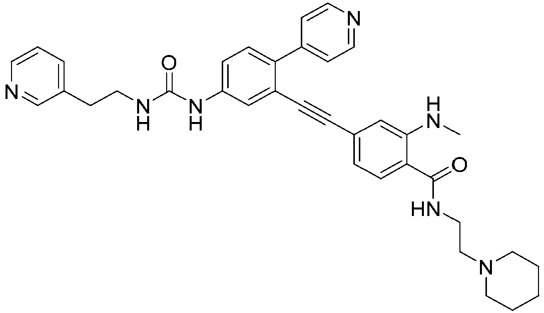 |
| **65** | 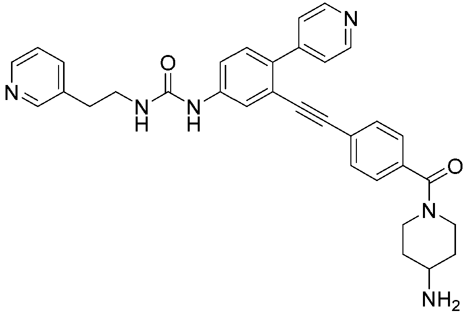 |
| **66** | 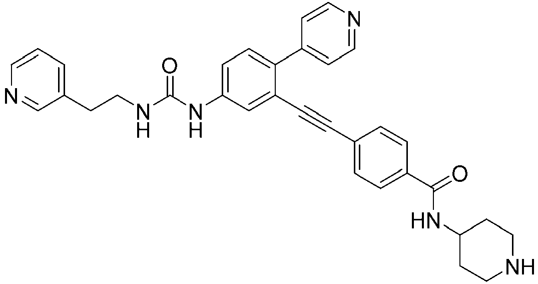 |
| **67** | 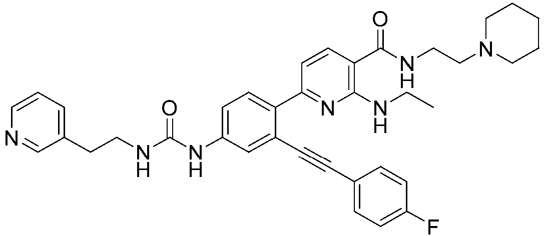 |
| **68** | 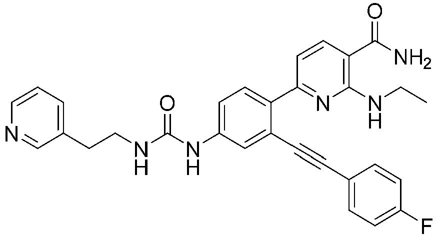 |
| **69** | 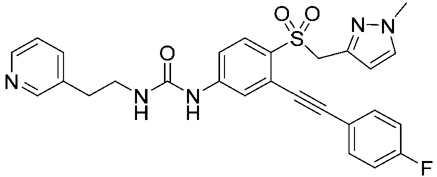 |
| **70** | 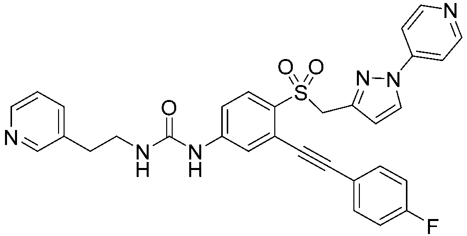 |
| **71** | 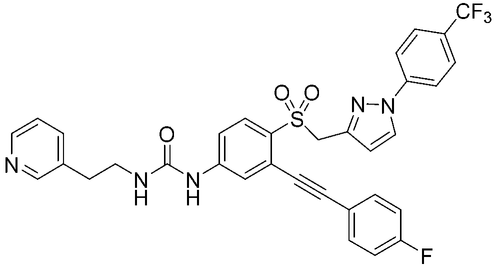 |
| **72** | 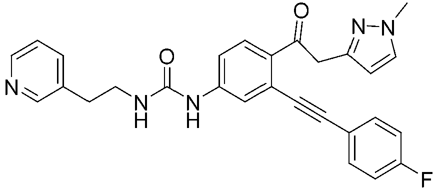 |
| **73** | 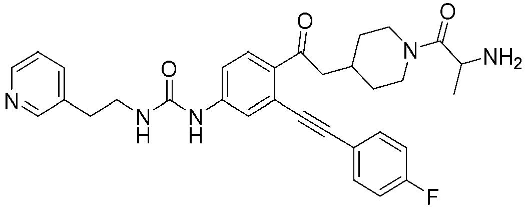 |
| **74** | 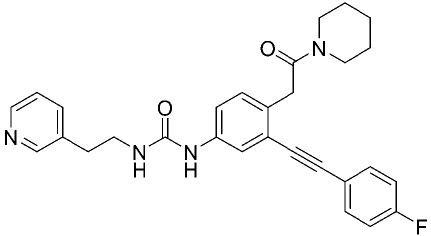 |
| **75** | 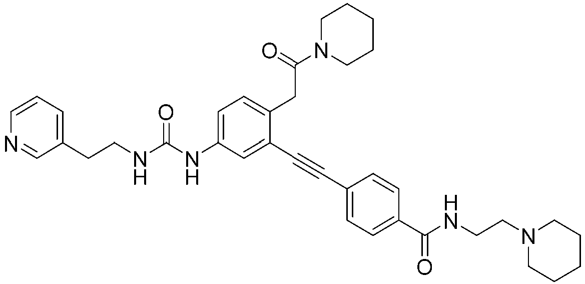 |
| **76** | 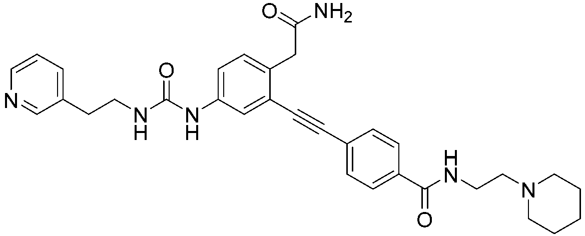 |
| **77** | 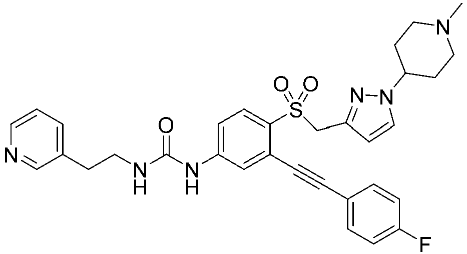 |
| **78** | 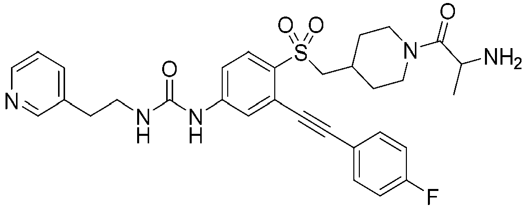 |
| **79** | 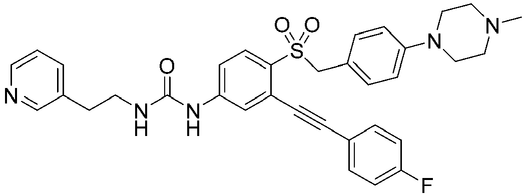 |
| **80** | 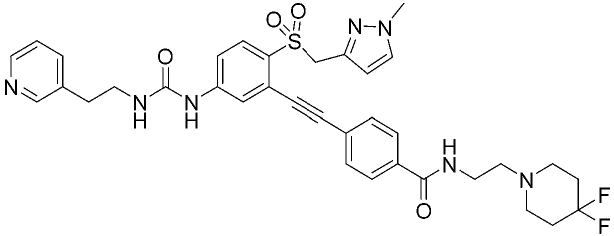 |
| **81** | 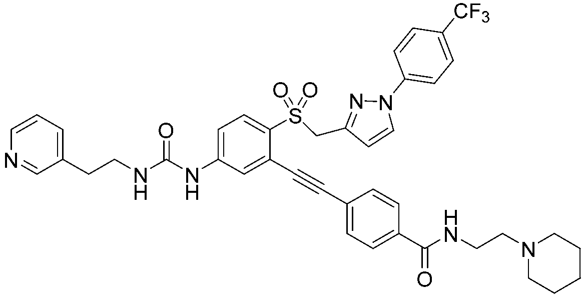 |
| **82** | 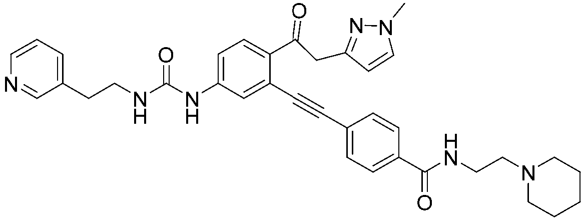 |
| **83** | 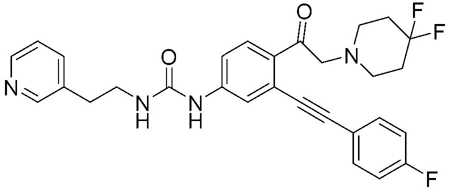 |
| **84** | 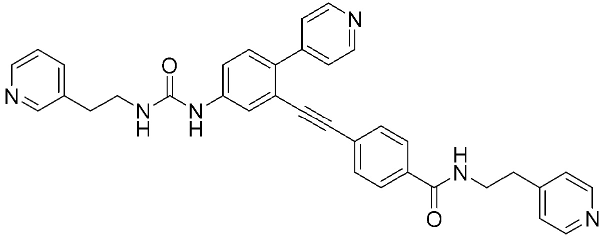 |
| **85** | 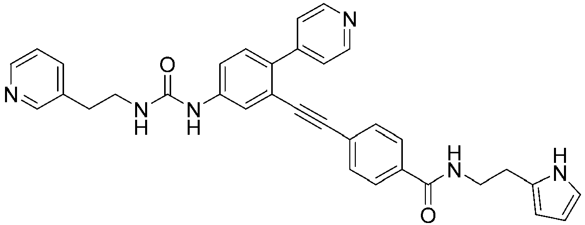 |
| **86** | 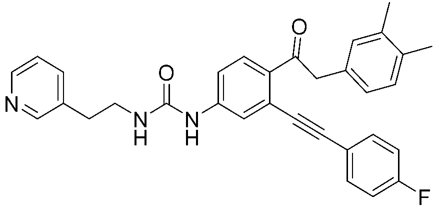 |
| **87** | 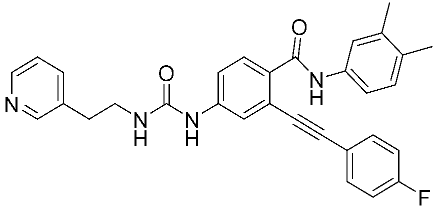 |
| **88** | 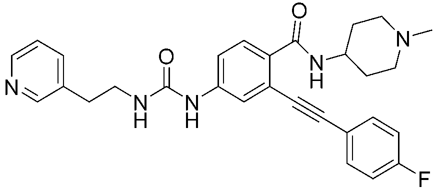 |
| **89** | 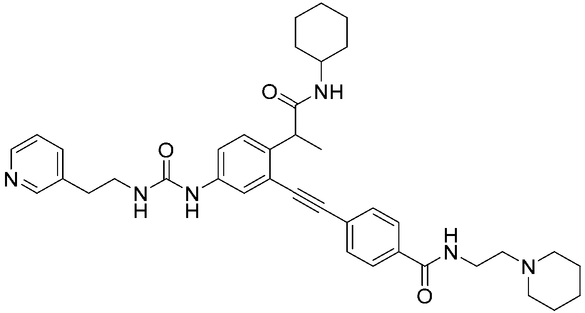 |
| **90** | 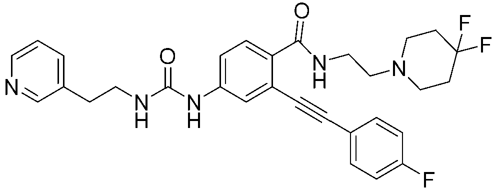 |
| **91** | 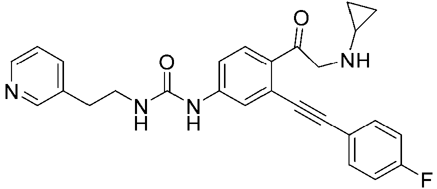 |
| **92** | 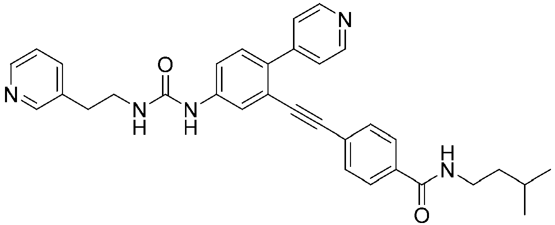 |
| **93** | 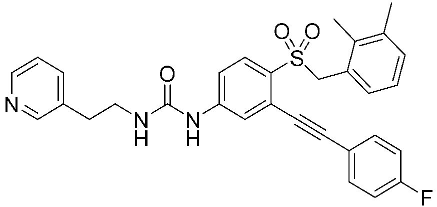 |
| **94** | 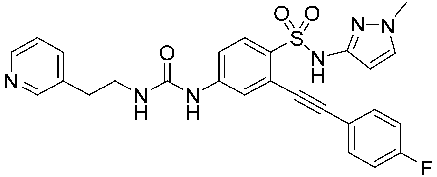 |
| **95** | 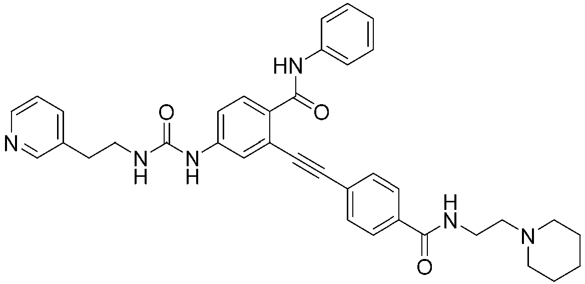 |
| **96** | 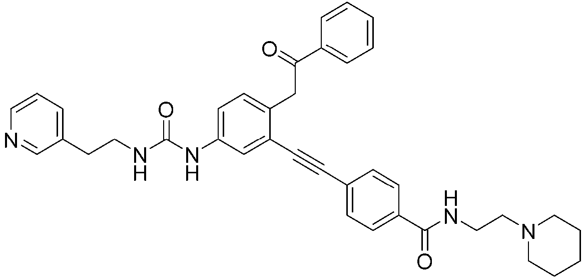 |
| **97** | 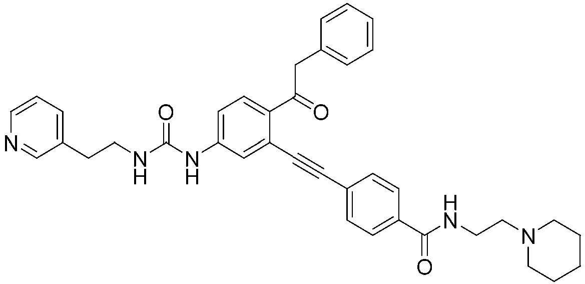 |
| **98** | 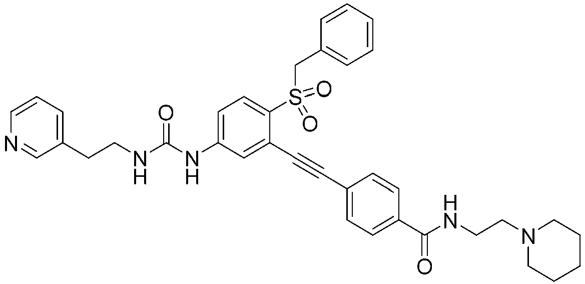 |
| **99** | 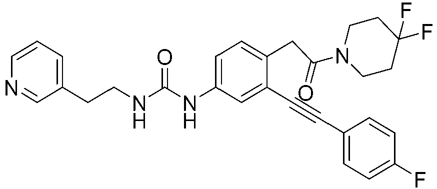 |
| **100** | 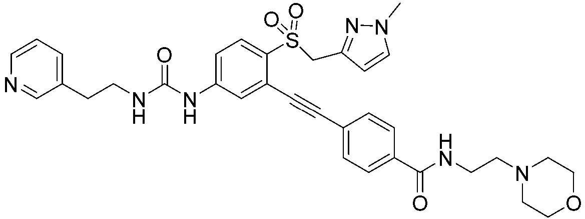 |
| **101** | 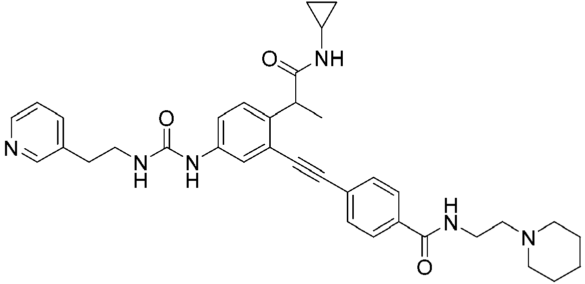 |
| **102** | 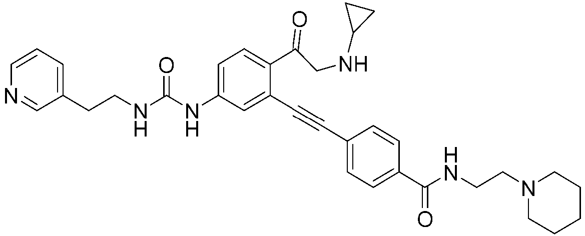 |
| **103** | 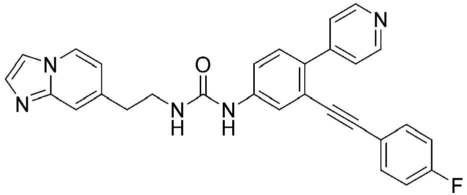 |
| **104** | 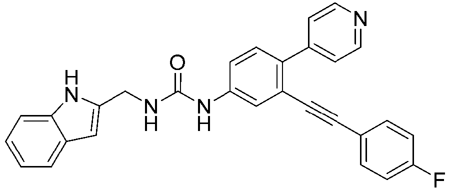 |
| **105** | 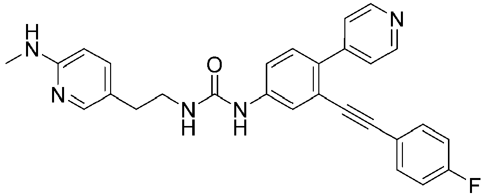 |
| **106** | 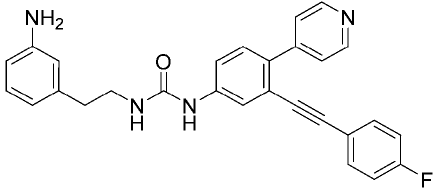 |
| **107** | 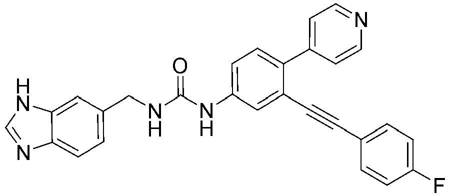 |
| **108** | 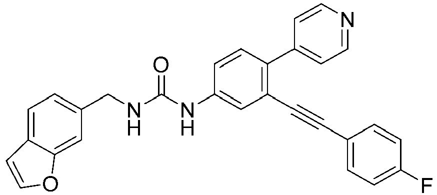 |
| **109** | 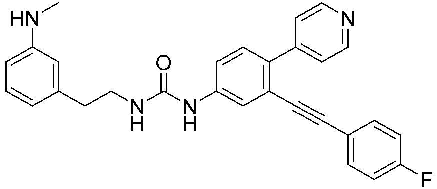 |
| **110** | 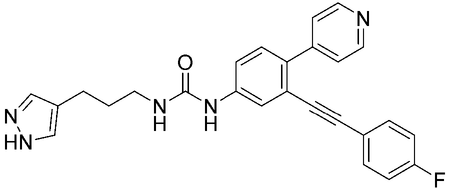 |
| **111** | 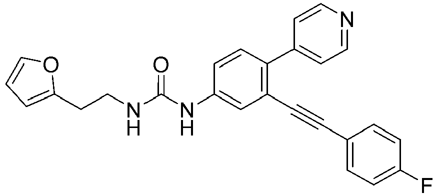 |
| **112** | 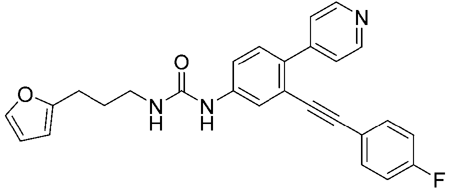 |
| **113** | 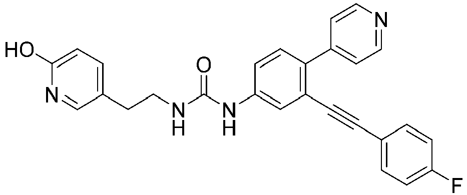 |
| **114** | 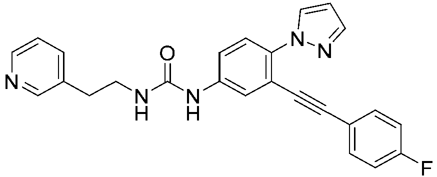 |
| **115** | 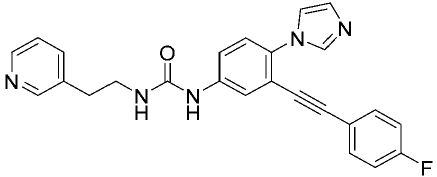 |
| **116** | 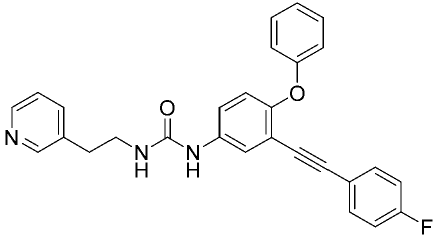 |
| **117** | 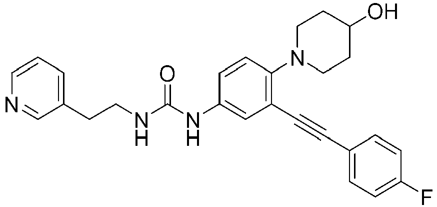 |
| **118** | 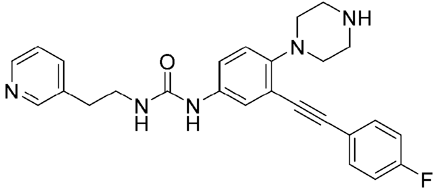 |
| **119** | 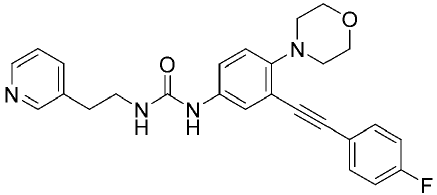 |
| **120** | 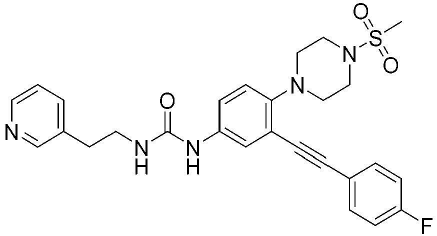 |
| **121** | 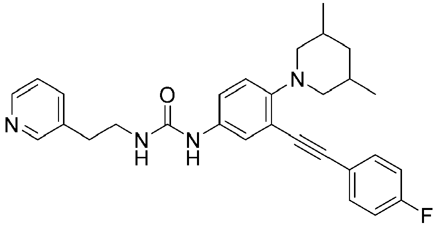 |
| **122** | 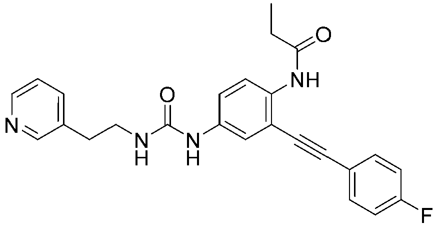 |
| **123** | 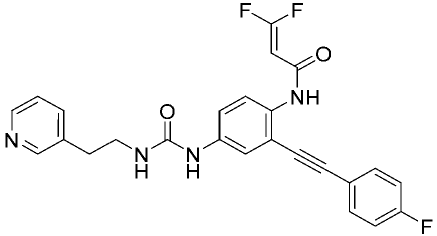 |
| **124** | 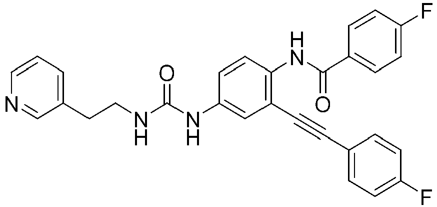 |
| **125** | 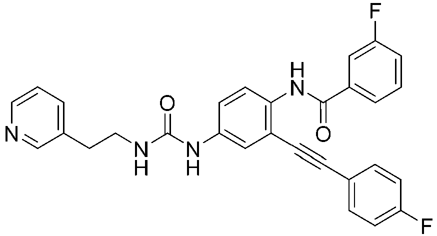 |
| **126** | 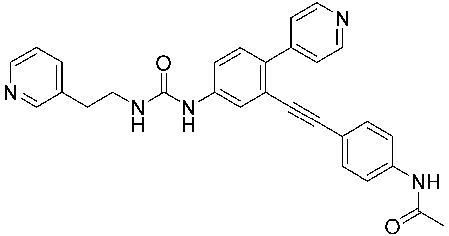 |
| **127** | 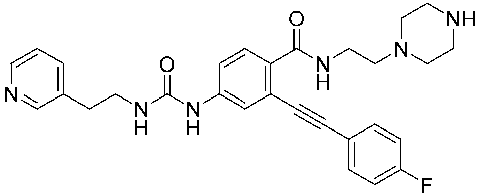 |
| **128** | 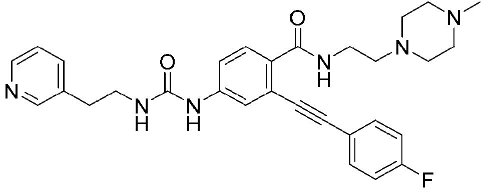 |
| **129** | 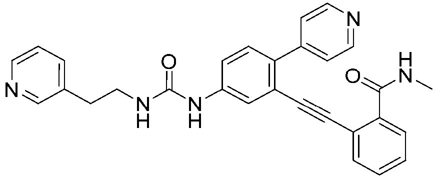 |
| **130** | 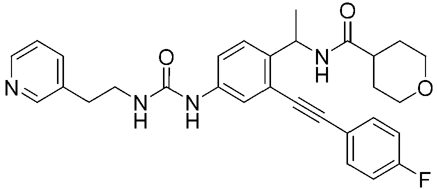 |
| **131** | 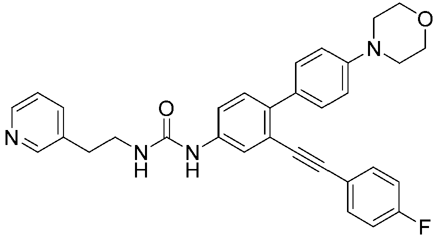 |
| **132** | 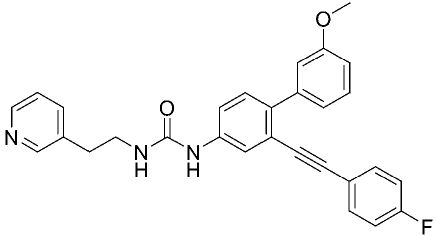 |
| **133** | 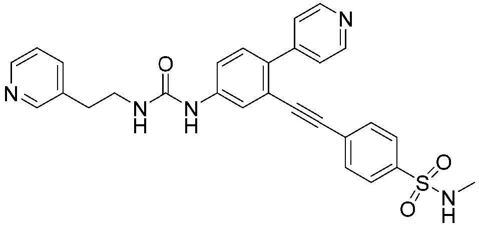 |
| **134** | 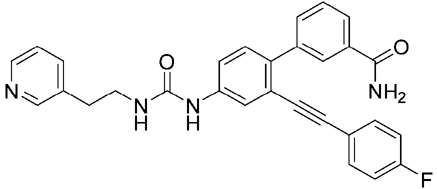 |
| **135** | 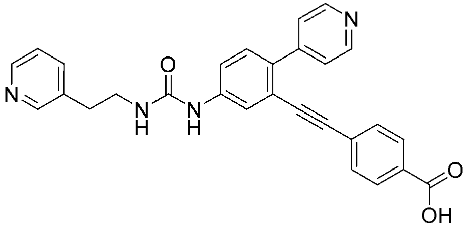 |
| **136** | 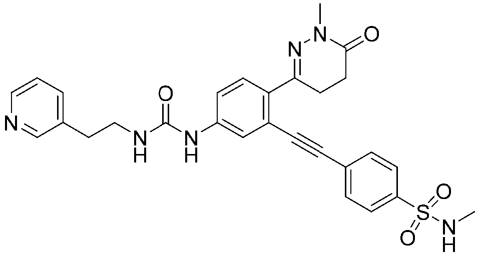 |
| **137** | 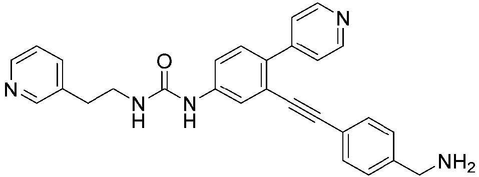 |
| **138** | 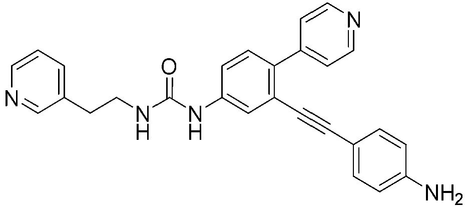 |
| **139** | 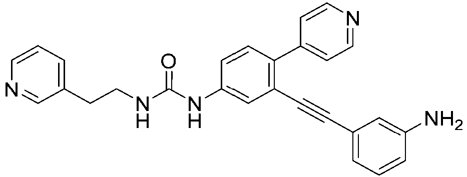 |
| **140** | 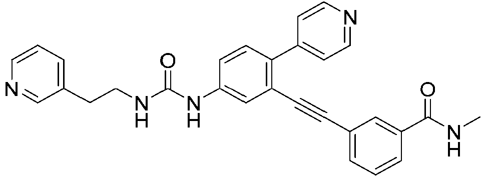 |
| **141** | 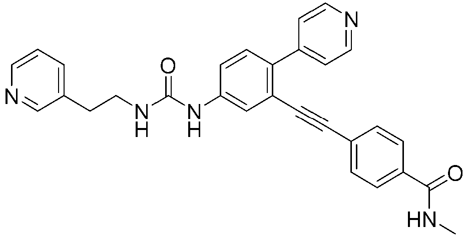 |
| **142** | 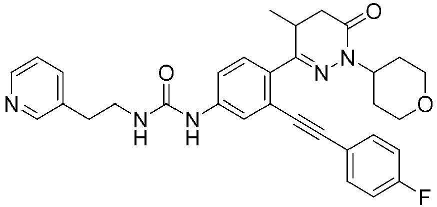 |
| **143** | 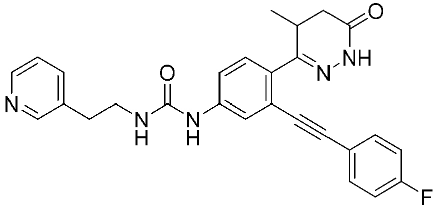 |
| **144** | 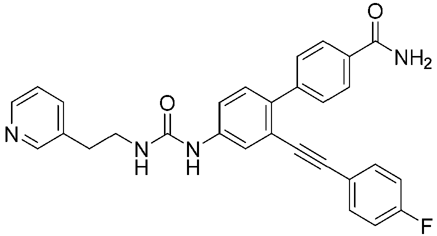 |
| **145** | 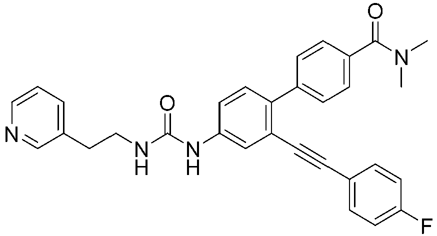 |
| **146** | 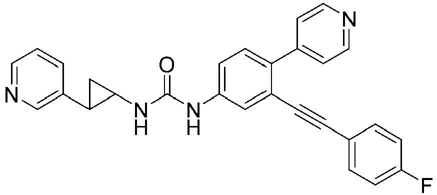 |
| **147** | 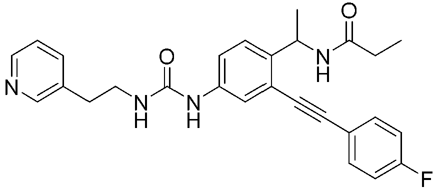 |
| **148** | 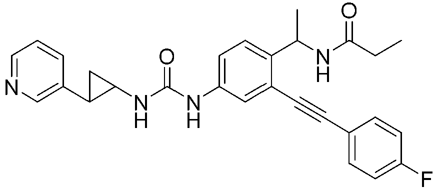 |
| **149** | 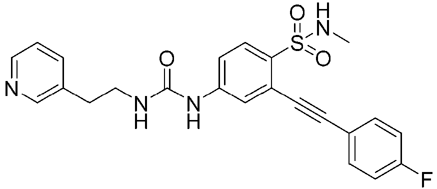 |
| **150** | 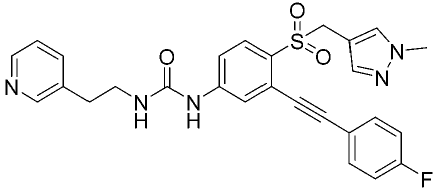 |
| **151** | 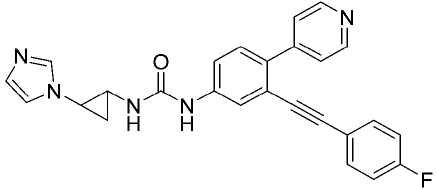 |
| **152** | 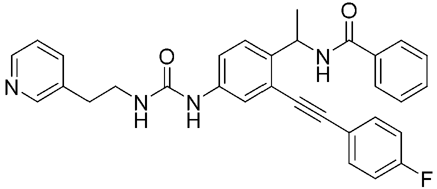 |
| **153** | 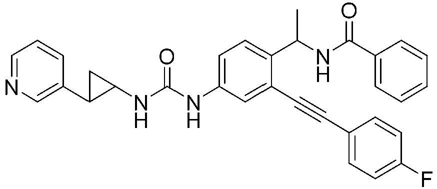 |
| **154** | 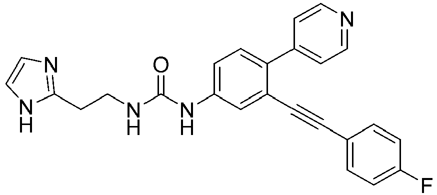 |
| **155** | 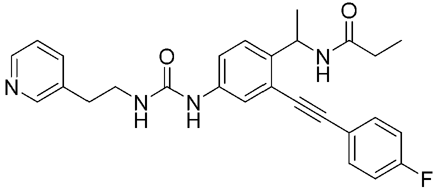 |
| **156** | 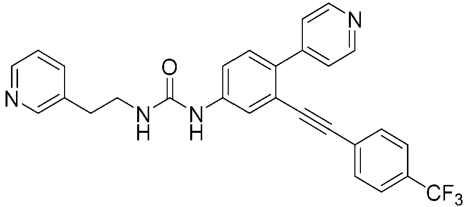 |
| **157** | 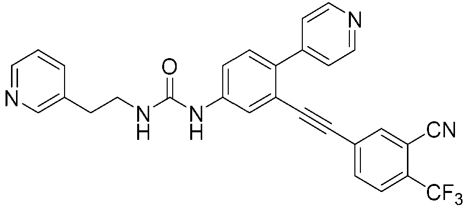 |
| **158** | 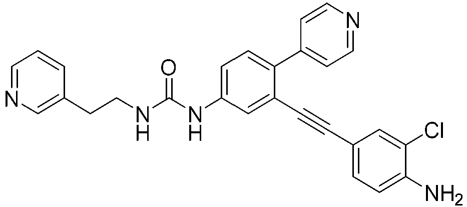 |
| **159** | 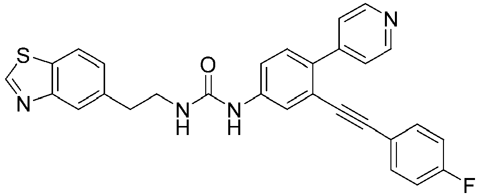 |
| **160** | 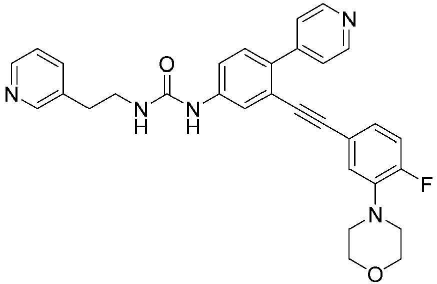 |
| **161** | 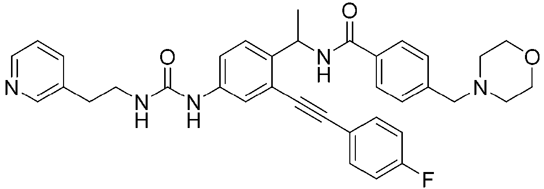 |
| **162** | 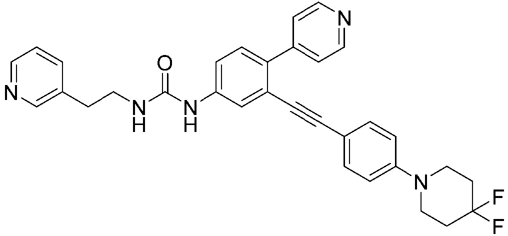 |
| **163** | 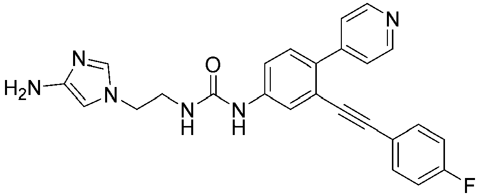 |
| **164** | 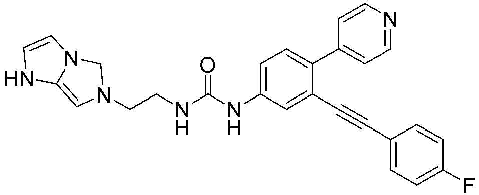 |
| **165** | 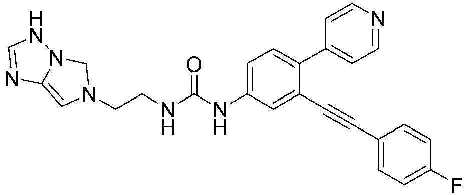 |
| **166** | 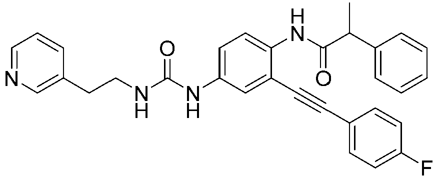 |
| **167** | 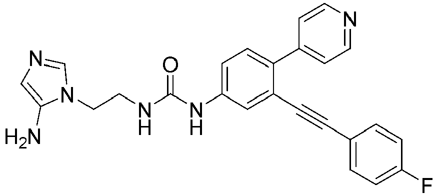 |
| **168** | 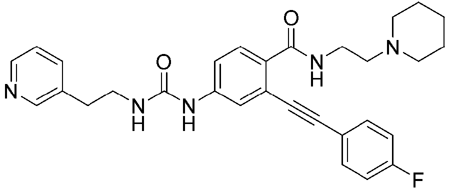 |
| **169** | 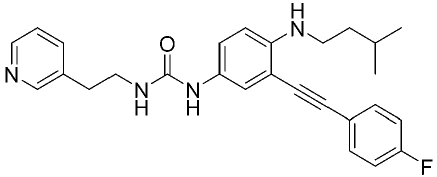 |
| **170** | 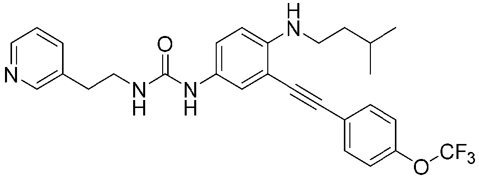 |
| **171** | 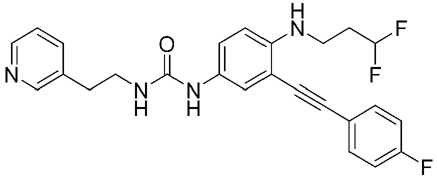 |
| **172** | 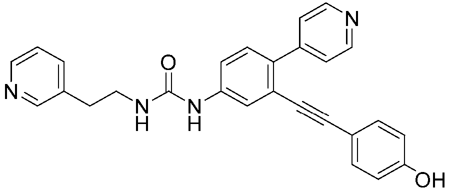 |
| **173** | 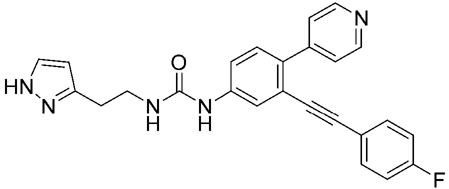 |
| **174** | 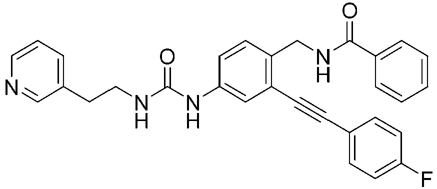 |
| **175** | 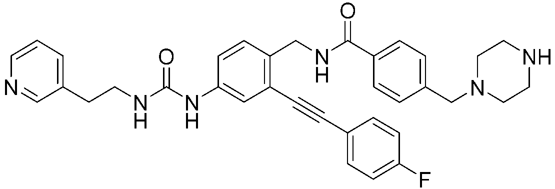 |
| **176** | 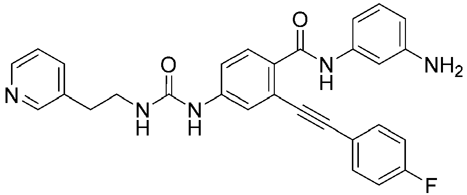 |
| **177** | 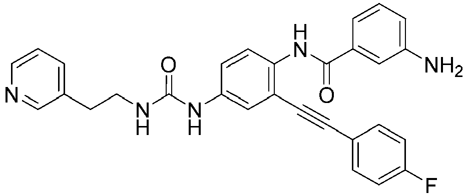 |
| **178** | 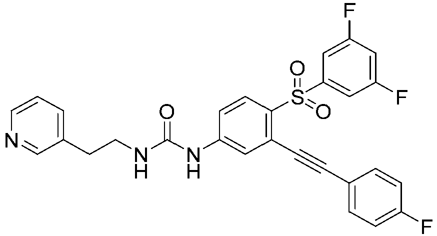 |
| **179** | 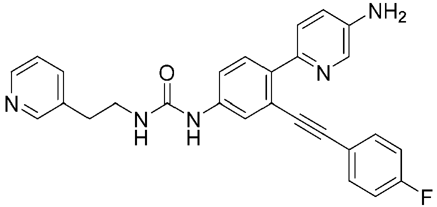 |
| **180** | 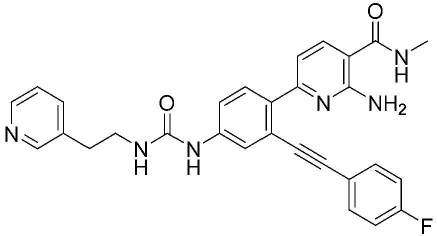 |
| **181** | 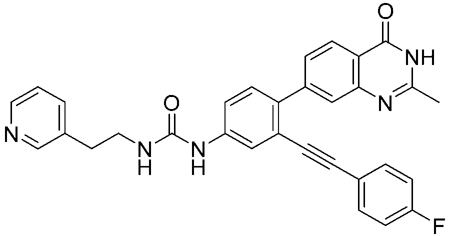 |
| **182** | 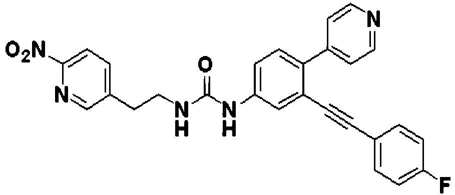 |
| **183** | 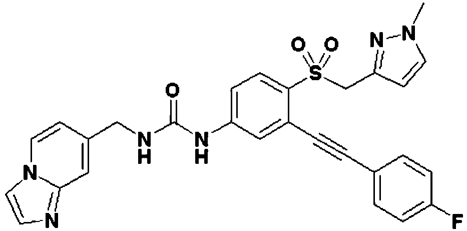 |
| **184** | |
| | 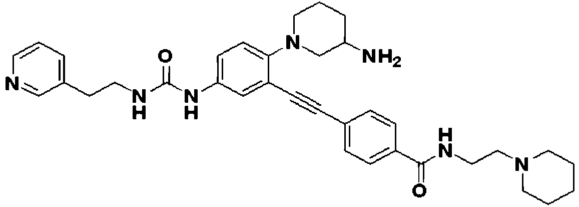 |
| **185** | 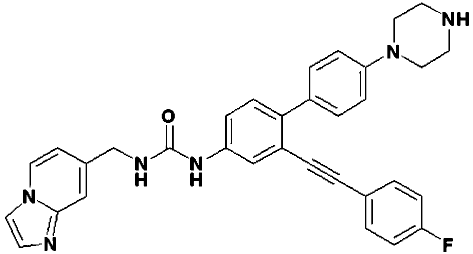 |
| **186** | 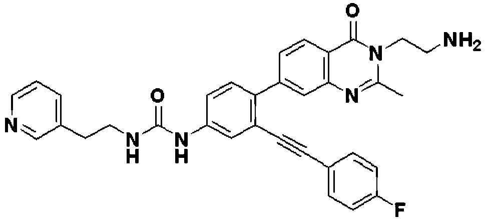 |
| **187** | 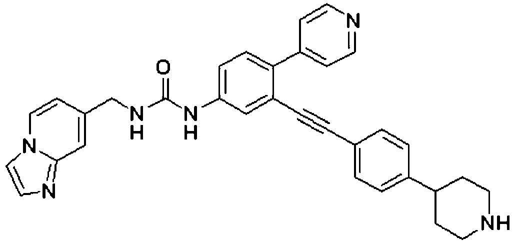 |
| **188** | |
| | 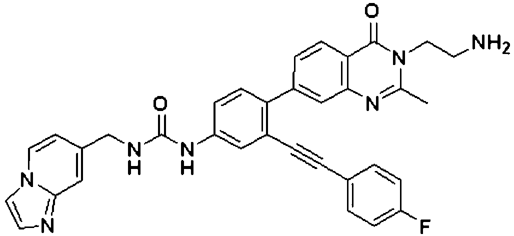 |
| **189** | 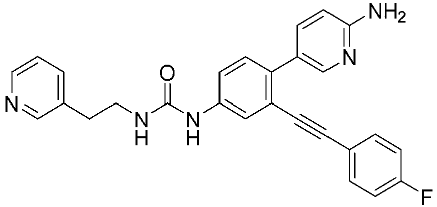 |

The compound represented by above formula I of the present invention, the compound represented by formula I-1, the compound represented by formula I-2, compounds 1 to 189 of above Table 4, optical isomers thereof, pharmaceutically acceptable salts of them, or hydrates or solvates of them exhibit NAMPT inhibitory activity.

The compound represented by above formula I of the present invention, the compound represented by formula I-1, the compound represented by formula I-2, compounds 1 to 189 of above Table 4, optical isomers thereof, pharmaceutically acceptable salts thereof, or hydrates or solvates of them may effectively inhibit NAMPT at low concentrations and may effectively prevent or treat diseases which may be treated by inhibiting NAMPT.

The compound represented by above formula I of the present invention, the compound represented by formula I-1, the compound represented by formula I-2, compounds 1 to 189 of above Table 4, optical isomers thereof, pharmaceutically acceptable salts of them, or hydrates or solvates of them may sufficiently lower NAD concentrations in cells.

The compound represented by above formula I of the present invention, the compound represented by formula I-1, the compound represented by formula I-2, compounds 1 to 189 of above Table 4, optical isomers thereof, pharmaceutically acceptable salts of them, or hydrates or solvates of them may exhibit high cytotoxicity against cancer cells and may sufficiently kill cancer cells at low concentrations.

The compound represented by above formula I of the present invention, the compound represented by formula I-1, the compound represented by formula I-2, compounds 1 to 189 of above Table 4, optical isomers thereof, pharmaceutically acceptable salts thereof, or hydrates or solvates of them may exhibit high cytotoxicity against gastric cancer cells or colorectal cancer cells, and may sufficiently kill gastric cancer cells or colorectal cancer cells at low concentrations.

The compound represented by above formula I of the present invention, the compound represented by formula I-1, the compound represented by formula I-2, compounds 1 to 189 of above Table 4, optical isomers thereof, pharmaceutically acceptable salts thereof, or hydrates or solvates of them may have little or no side effects and may exhibit excellent pharmacokinetic properties. For example, the compound represented by above formula I, the compound represented by formula I-1, the compound represented by formula I-2, compounds 1 to 188 of above Table 4, optical isomers thereof, pharmaceutically acceptable salts thereof, or hydrates or solvates of them may exhibit excellent absorption rates (e.g., high bioavailability), may be appropriately distributed to each tissue upon administration so as to exhibit desired pharmacological effects (e.g., preventive or therapeutic effects on diseases which may be treated by inhibiting NAMPT) in each tissue, and may be effectively metabolized in the body.

In the present invention, optical isomers may include not only enantiomers, but also mixtures of enantiomers as well as racemates.

In the present invention, "pharmaceutically acceptable salts" may mean salts conventionally used in a pharmaceutical industry, for example, inorganic ion salts prepared from calcium, potassium, sodium, magnesium or the like; inorganic acid salts prepared from hydrochloric acid, nitric acid, phosphoric acid, bromic acid, iodic acid, perchloric acid, sulfuric acid or the like; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbric acid, carbonic acid, vanillic acid or the like; sulfonic acid salts prepared from methanesulfonic acid, ethanesulfonic acid, benzenesulfonic acid, p-toluenesulfonic acid, naphthalenesulfonic acid or the like; amino acid salts prepared from glycine, arginine, lysine, or the like; amine salts prepared from trimethylamine, triethylamine, ammonia, pyridine, picoline, or the like; and the like, but types of salt meant in the present invention are not limited to those listed salts.

The "hydrate" of the present invention may refer to one in which the compound represented by above formula I, the compound represented by formula I-1, the compound represented by formula I-2, compounds 1 to 189 of above Table 4, and water are bound by a non-covalent intermolecular force, and may include a stoichiometric or non-stoichiometric amount of water. Specifically, said hydrate may include water at a ratio of about 0.25 mol to about 10 mol based on 1 mol of an active ingredient, and more specifically include about 0.5 mol, about 1 mol, about 1.5 mol, about 2 mol, about 2.5 mol, about 3 mol, about 5 mol, etc.

The "solvate" of the present invention may refer to one in which the compound represented by above formula I, the compound represented by formula I-1, the compound represented by formula I-2, compounds 1 to 189 of above Table 4, and a solvent other than water are bound by an intermolecular force, and may include a stoichiometric or non-stoichiometric amount of the solvent. Specifically, said solvate may include a solvent molecule at a ratio of about 0.25 mol to about 10 mol based on 1 mol of an active component, and more specifically include about 0.5 mol, about 1 mol, about 1.5 mol, about 2 mol, about 2.5 mol, about 3 mol, about 5 mol, etc.

The present invention provides a pharmaceutical composition including the compound represented by above formula I, the compound represented by formula I-1, the compound represented by formula I-2, compounds 1 to 189 of above Table 4, optical isomers thereof, pharmaceutically acceptable salts thereof, or hydrates or solvates thereof.

The pharmaceutical composition including the compound represented by above formula I, the compound represented by formula I-1, the compound represented by formula I-2, compounds 1 to 189 of above Table 4, optical isomers thereof, pharmaceutically acceptable salts thereof, or hydrates or solvates thereof exhibit NAMPT inhibitory activity and may be used to prevent or treat diseases which may be treated by inhibiting NAMPT.

The pharmaceutical composition including the compound represented by above formula I of the present invention, the compound represented by formula I-1, the compound represented by formula I-2, compounds 1 to 189 of above Table 4, optical isomers thereof, pharmaceutically acceptable salts thereof, or hydrates or solvates of them may show an effect of preventing or treating cancer.

In embodiments of the present invention, cancer may be a solid cancer or hematological cancer, and said cancer may be skin cancer (e.g., melanoma), lymphoma, breast cancer, cervical cancer, uterine cancer, gastrointestinal cancer, lung cancer, ovarian cancer, prostate cancer, colorectal cancer, colon cancer, rectal cancer, oral cancer, brain cancer, head and neck cancer, throat cancer, testicular cancer, kidney cancer, pancreatic cancer, bone cancer, spleen cancer, liver cancer, bladder cancer, larynx cancer, nasal cavity cancer, AIDS-related cancer, esophageal cancer, gastric cancer, stomach cancer, colorectal cancer, virus-related cancer (one selected from nasopharyngeal cancer, vaginal cancer, vulvar cancer, penile cancer, Kaposi sarcoma, Burkitt lymphoma, T-cell lymphoma and Merkel cell carcinoma), cancer of blood and bone marrow, such as multiple myeloma, acute and chronic leukemia, for example, lymphoblastic leukemia, myeloid leukemia, lymphocytic leukemia, and myelocytic leukemia, and the like.

The pharmaceutical composition of the present invention may further include a pharmaceutically acceptable additive. The pharmaceutically acceptable additive may be an additive commonly used in the pharmaceutical field, and may be appropriately selected by those skilled in the art. For example, said pharmaceutically acceptable additive may include lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil and the like, but is not limited thereto. In addition, the pharmaceutical composition of the present invention may further include lubricant, humectant, a sweetening agent, a flavoring agent, emulsifier, a suspending agent, preservative, etc. in addition to said ingredients.

Furthermore, the pharmaceutical composition of the present invention may be formulated into an oral dosage form such as tablet, powder, granule, pill, capsule, suspension, emulsion, liquid for internal use, emulsion, syrup, etc., as well as a form of external application, suppository and sterile solution for injection by using a pharmaceutically acceptable carrier and excipient, and thus may be prepared in a unit dose form or prepared by being inserted into a multi-dose container. The preparations may be prepared according to a conventional method used for formulation in the art or a method disclosed in Remington's Pharmaceutical Science (19th ed., 1995), and may be formulated into various preparations depending on each disease or ingredient.

The pharmaceutical composition of the present invention may be orally or parenterally administered (for example, applied intravenously, hypodermically, intraperitoneally or locally) according to an intended method, in which a dosage thereof varies depending on a patient's condition and weight, a degree of disease, a drug form, and an administration route and time, but may be appropriately selected by those skilled in the art. Specifically, a daily dosage of the compound represented by above formula I of the present invention, the compound represented by formula I-1, the compound represented by formula I-2, compounds 1 to 189 of above table 4, optical isomers thereof, pharmaceutically acceptable salts thereof, or hydrates or solvates thereof may be about 0.1 to about 1000 mg/kg based on the weight of the compound represented by above formula I, and may be administered at one time a day or several times a day by dividing the daily dosage thereof.

In addition to the compound represented by above formula I, the compound represented by formula I-1, the compound represented by formula I-2, compounds 1 to 189 of above Table 4, optical isomers thereof, pharmaceutically acceptable salts thereof, or hydrates or solvates of them, the pharmaceutical composition of the present invention may further include at least one ingredient which may exhibit the same or similar medicinal effects or may bring synergy to medicinal effects in combination.

The present invention provides a use of the compound represented by above formula I, the compound represented by formula I-1, the compound represented by formula I-2, compounds 1 to 189 of above Table 4, optical isomers thereof, pharmaceutically acceptable salts thereof, or hydrates or solvates thereof for preventing or treating diseases (e.g., cancer) which may be treated by inhibiting NAMPT.

The present invention provides a use of the compound represented by above formula I, the compound represented by formula I-1, the compound represented by formula I-2, compounds 1 to 189 of above Table 4, optical isomers thereof, pharmaceutically acceptable salts thereof, or hydrates or solvates of them, in preparation of a medication for preventing or treating diseases (e.g., cancer) which may be treated by inhibiting NAMPT.

The present invention provides a method for preventing or treating diseases (e.g., cancer) which may be treated by inhibiting NAMPT, including administering the compound represented by above formula I, the compound represented by formula I-1, the compound represented by formula I-2, compounds 1 to 189 of above Table 4, optical isomers thereof, pharmaceutically acceptable salts thereof, or hydrates or solvates thereof into an individual in need.

The compound represented by above formula I of the present invention, the compound represented by formula I-1, the compound represented by formula I-2, compounds 1 to 189 of above Table 4, optical isomers thereof, pharmaceutically acceptable salts thereof, or hydrates or solvates thereof may be administered in a therapeutically effective amount thereof.

In the present invention, the "therapeutically effective amount" may refer to an amount enough to treat a disease at a reasonable benefit/risk ratio applicable to medical treatment, and a level of effective dose may be determined according to factors including a patient's disease type, severity, activity of a drug, sensitivity to the drug, an administration time, an administration route and excretion rate, a treatment period and a concurrently used drug, as well as other factors well known in a medical field. Specifically, the therapeutically effective amount may refer to an amount effective in preventing or treating diseases (e.g., cancer) which may be treated by inhibiting NAMPT.

In the present invention, "individual" refers to a subject, whose disease needs to be prevented or treated, and more specifically to a mammal such as a human, monkey, mouse, dog, cat, horse, cow, etc., but is not limited thereto.

As used herein, the term "prevention" means all the acts, which inhibit diseases (e.g., cancer) which may be treated by inhibiting NAMPT or delay the occurrence thereof by administering the compound according to the present invention.

As used herein, the term "treatment" refers to all the acts, by which a symptom of diseases (e.g., cancer) which may be treated by inhibiting NAMPT gets better or takes a favorable turn, or a speed of such progression is delayed by administering the compound according to the present invention.

Matters mentioned in each item of the present invention, that is, the urea compound, use, composition and therapeutic method may be equally applied, if not contradictory to each other. Besides, the terms and abbreviations used in the present specification may have their original meanings, unless defined otherwise.

### ADVANTAGEOUS EFFECTS

A urea compound of the present invention, an optical isomer thereof, a pharmaceutically acceptable salt of them, or a hydrate or solvate of them can inhibit NAMPT, and thus have excellent preventive or therapeutic effects on NAMPT-related diseases. Thus, the urea compound of the present invention, the optical isomer thereof, the pharmaceutically acceptable salt of them, or the hydrate or solvate of them is advantageously used in preventing or treating diseases (e.g., cancer) which may be treated by inhibiting NAMPT.

### DETAILED DESCRIPTION OF THE EXEMPLARY EMBODIMENTS

Hereinafter, the present invention will be described in more detail through preparation examples and exemplary examples. However, the following preparation examples and exemplary examples are provided for the purpose of illustrating the present invention, and thus the present invention is not limited to the preparation examples and exemplary examples.

Each of the compounds according to the present invention was synthesized by a method described below, and a conventional method derived by combining the following specific synthesis methods may be used by those skilled in the art.

Each of the compounds used in the synthesis was purchased from a supplier outside or synthesized by using an organic synthesis method apparent to those skilled in the art or, and was used without a separate purification process. The compound of each exemplary example was identified through 400 MHz 1H-NMR (BRUKER, 400-MR), LC-Mass (Waters, SQD2) analysis.

### Example 1: Synthesis of compound 1 (synthesis reaction formula 1)

### Synthesis of 1-(3-((4-fluorophenyl)ethynyl)-4-(pyridin-4-yl)phenyl)-3-(2-(pyridin-3-yl)ethyl)urea

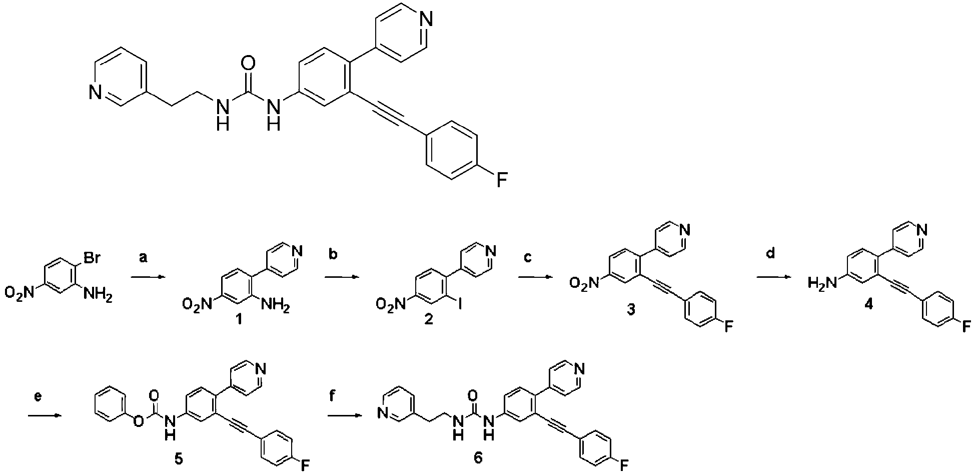

Reaction Formula 1. (a) 4-pyridine boronic acid, Pd[PPh₃]₄, sodium carbonate (Na₂CO₃), toluene, ethanol (EtOH), reflux, 1-3 hours; (b) sodium nitrite (NaNO₂), KI, HCl, water, 0°C, 1 hour (1h); (c) Pd[PPh₃]₄, CuI, TEA, ACN, RT, 1 hour; (d) Fe, AcOH, 80°C, 2 hours; (e) phenyl chloroformate, pyridine, THF, RT, 2 hours; (f) 2-(pyridin-3-yl)ethan-1-amine, TEA, THF, reflux, 24 hours

### Step 1 (a) Synthesis of 5-nitro-2-(pyridin-4-yl)aniline

A mixture of Pd[PPh₃]₄ (3.15 g, 0.2 eq) and sodium carbonate (Na₂CO₃) aqueous solution (2 M, 16.5 ml, 2.4 eq) dissolved in toluene (40 mL) and ethanol (EtOH, 30 mL) was rapidly added to a mixture of 2-bromo-5-nitroaniline (3 g, 13.8 mmol, 1.0 eq) and 4-pyridine boronic acid (2.55 g, 1.5 eq) dissolved in toluene (40 mL) and ethanol (EtOH, 30 mL). To the reaction solution, 16.5 ml of distilled water was added and stirred under reflux for one to three hours in a nitrogen environment, then cooled to room temperature, and concentrated under reduced pressure. The obtained residue was dissolved in distilled water and EA, diluted, and filtered through celite. The organic layer was dried over magnesium sulfate and concentrated under reduced pressure. The obtained residue was purified by column chromatography (silica gel, 30-70% hexane/ethyl acetate) to obtain the title target compound (2.77 g). (yellow solid, yield 93%)

### Step 2 (b) Synthesis of 4-(2-iodo-4-nitrophenyl)pyridine

A solution of sodium nitrite (0.48 g, 1.5 eq) dissolved in water (13 mL) was slowly added dropwise to a suspension of 5-nitro-2-(pyridin-4-yl)aniline (1 g, 4.65 mmol, 1 eq), concentrated hydrochloric acid aqueous solution (12 mL) and water (12 mL) at 0°C. The reaction mixture became transparent by the end of the addition of the sodium nitrite solution. After addition, the reaction mixture was stirred at 0°C for 10 minutes. Then, a solution of potassium iodide (2.31 g, 2.0 eq) dissolved in water (12 mL) was added to the mixture at 0°C. A very viscous reddish-brown mixture was formed, which turned dark brown. The reaction mixture was stirred at room temperature for one hour, treated with saturated potassium carbonate aqueous solution (pH>8), and extracted with ethyl acetate. The organic layer was washed with water, dried over magnesium sulfate, and concentrated under reduced pressure. The obtained residue was purified by column chromatography (silica gel, hexane/ethyl acetate, 1:1) to obtain the title target compound (0.84 g, yield 55%).

### Step 3 (c) Synthesis of 4-(2-((4-fluorophenyl)ethynyl)-4-nitrophenyl)pyridine)

Triethylamine (0.1 mL, 3 eq), CuI (1.5 mg, 3 mol%), and Pd[PPh₃]₄ (9.2 mg, 3 mol%) were added to a solution of 4-(2-iodo-4-nitrophenyl)pyridine (87 mg, 0.27 mmol, 1.0 eq) dissolved in MeCN (2 mL). The mixture was stirred at room temperature for five minutes under a N₂ atmosphere. Then, 4-fluorophenyl-acetylene (38 mg, 1.2 eq) was added. The reaction mixture was stirred at room temperature for one hour and concentrated under reduced pressure. The obtained residue was purified by column chromatography (silica gel, hexane/ethyl acetate) to obtain the target compound (82 mg, yield 96.5%).

### Step 4 (d) Synthesis of 3-((4-fluorophenyl)ethynyl)-4-(pyridin-4-yl)aniline

A mixture of 4-(2-((4-fluorophenyl)ethynyl)-4-nitrophenyl)pyridine (0.08 g, 0.25 mmol, 1.0 eq), iron (0.14 g, 10 eq), and AcOH (2 mL) was stirred at 80°C for one to two hours. The catalyst was removed by celite filtration, and the filtrate was concentrated under reduced pressure. The obtained black viscous oil residue was basified with potassium carbonate aqueous solution, extracted with ethyl acetate, and the organic layer was concentrated under reduced pressure. The target compound was obtained as a crude product.

### Step 5 (e) Synthesis of benzyl (3-((4-fluorophenyl)ethynyl)-4-(pyridin-4-yl)phenyl)carbamate

3-((4-fluorophenyl)ethynyl)-4-(pyridin-4-yl)aniline (90 mg, 1.0 eq) and pyridine (40 uL, 2 eq) were dissolved in tetrahydrofuran (2 mL), and then the reaction solution was cooled with ice, phenyl chloroformate (47 uL, 1.5 eq) was added, and the mixture was stirred at room temperature for two hours. After the reaction was completed, water (0.1 mL) was added to the reaction mixture, and the reaction mixture was concentrated under reduced pressure. The obtained residue was diluted with water and ethyl acetate. The organic layer was washed with water, dried over magnesium sulfate, and concentrated under reduced pressure. The target compound was obtained as a crude product.

### Step 6 (f) Synthesis of 1-(3-((4-fluorophenyl)ethynyl)-4-(pyridin-4-yl)phenyl)-3-(2-(pyridin-3-yl)ethyl)urea

Benzyl (3-((4-fluorophenyl)ethynyl)-4-(pyridin-4-yl)phenyl)carbamate (1.0 eq), 3-(2-aminoethyl)pyridine (2 eq) and triethylamine (3 eq) were dissolved in THF (4 mL) and the resulting mixture was heated at 80°C for 24 hours. The reaction mixture was concentrated under reduced pressure, and the obtained residue was purified by column chromatography (silica gel, 0-5% dichloromethane/methanol) to obtain the title target compound.

¹H NMR (400 MHz, CDCl₃) 8.65-8.66 (m, 2H), 8.44-8.45 (m, 2H), 7.66 (d, J=2.1 Hz, 1H), 7.60 (dt, *J* = 7.8, 1.8 Hz, 2H), 7.54 (m, 2H), 7.27-7.37 (m, 5H), 6.97-7.02 (m, 2H), 6.94 (s, 1H), 5.08 (t, *J =* 5.8 Hz, 1H), 3.61 (q, *J =* 6.4 Hz, 2H), 2.91 (t, *J =* 6.6 Hz, 2H); MS(ESI) m/z MH⁺ 437.

The reactants may be changed in substantially the same manner as in the synthesis method of above Example 1, so as to synthesize compounds 12, 103, 104, 105, 106, 107, 108, 109, 111, 112, 113, 146, 151, 154, 159, 163, 164, 165, 167, and 173.

### Example 2: Synthesis of compound 3

### Synthesis of 1-(2-(1H-imidazol-1-yl)ethyl)-3-(3-((4-fluorophenyl)ethynyl)-4-(pyridin-4-yl)phenyl)urea

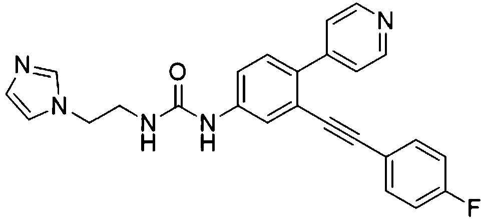

Compound 3 was prepared in substantially the same manner as in the synthesis of compound 1, except that 1H-imidazol-1-ethanamine was used instead of 3-(2-aminoethyl)pyridine in step 6) compared to the synthesis of compound 1 in Example 1.

¹H NMR (400 MHz, MeOD) δ 8.59-8.61 (m, 2H), 7.78 (d, *J=* 2.1 Hz, 1H), 7.74 - 7.66 (m, 3H), 7.36 - 7.48 (m, 4H), 7.19 (m, 1H), 7.10 (m, 2H), 7.00 (m, 1H), 4.20 (t, *J =* 6.0 Hz, 2H), 3.58 (t, *J =* 6.0 Hz, 2H); MS(ESI) m/z MH⁺ 426.

### Example 3: Synthesis of compound 4

### Synthesis of 1-(3-((4-fluorophenyl)ethynyl)-4-(pyridin-4-yl)phenyl)-3-(imidazo[1,2-a]pyridin-7-ylmethyl)urea

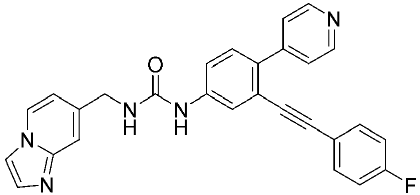

Compound 4 was prepared in substantially the same manner as in the synthesis of compound 1, except that 1-{imidazo[1,2-a]pyridin-7-yl}methanamine dihydrochloride was used instead of 3-(2-aminoethyl)pyridine in step 6) compared to the synthesis of compound 1 in Example 1.

¹H NMR (400 MHz, MeOD) δ 8.59-8.61 (m, 2H), 8.40 (d, *J =* 7.0 Hz, 1H), 7.83 (d, *J* = 2.2 Hz, 1H), 7.79 (m, 1H), 7.71-7.72 (m, 2H), 7.51 - 7.54 (m, 2H), 7.43 - 7.48 (m, 2H), 7.35 - 7.39 (m, 2H), 7.07 - 7.11 (m, 2H), 6.93 (dd, *J =* 7.0, 1.6 Hz, 1H), 4.49 (s, 2H); MS(ESI) m/z MH⁺ 462.

### Example 4: Synthesis of compound 5

### Synthesis of 1-(3-((4-fluorophenyl)ethynyl)-4-(pyridin-4-yl)phenyl)-3-(3-hydroxyphenethyl)urea

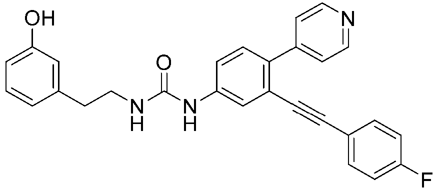

Compound 5 was prepared in substantially the same manner as in the synthesis of compound 1, except that 3-(2-aminoethyl)phenol hydrobromide was used instead of 3-(2-aminoethyl)pyridine in step 6) compared to the synthesis of compound 1 in Example 1.

¹H NMR (400 MHz, MeOD) δ 8.60 (d, *J* = 6.1 Hz, 2H), 7.79 (d, *J* = 2.2 Hz, 1H), 7.71-7.72 (m, 2H), 7.36 - 7.46 (m, 4H), 7.08-7.15 (m, 2H), 6.70 - 6.75 (m, 2H), 6.65 (dd, *J =* 8.1, 2.1 Hz, 1H), 3.46 (t, *J =* 7.1 Hz, 2H), 2.78 (t, *J =* 7.0 Hz, 2H); MS(ESI) m/z MH⁺ 452.

### Example 5: Synthesis of compound 6

### Synthesis of 1-((1H-pyrrolo[3,2-c]pyridin-2-yl)methyl)-3-(3-((4-fluorophenyl)ethynyl)-4-(pyridin-4-yl)phenyl)urea

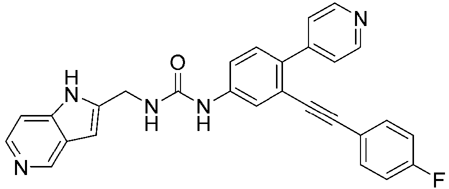

Compound 6 was prepared in substantially the same manner as in the synthesis of compound 1, except that 1-{1H-pyrrolo[3,2-c]pyridin-2-yl}methanamine dihydrochloride was used instead of 3-(2-aminoethyl)pyridine in step 6) compared to the synthesis of compound 1 in Example 1.

¹H NMR (400 MHz, DMSO) δ 11.45 (s, 1H), 9.05 (s, 1H), 8.75 (s, 1H), 8.65-8.67 (m, 2H), 8.12 (d, *J* = 5.7 Hz, 1H), 7.93 (d, *J =* 1.9 Hz, 1H), 7.64-7.68 (m, 2H), 7.44-7.52 (m, 4H), 7.35 (d, *J* = 5.7 Hz, 1H), 7.24-7.28 (m, 2H), 6.85 (t, *J =* 5.8 Hz, 1H), 6.45 (s, 1H), 4.49 (d, *J =* 5.7 Hz, 2H); MS(ESI) m/z MH⁺ 462.

### Example 6: Synthesis of compound 8

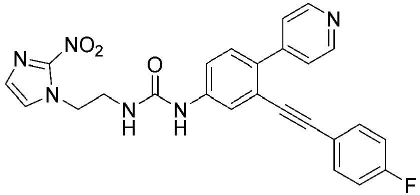

### Synthesis of 1-(3-((4-fluorophenyl)ethynyl)-4-(pyridin-4-yl)phenyl)-3-(2-(2-nitro-1H-imidazol-1-yl)ethyl)urea

Compound 8 (0.044 g, 38%) was prepared in substantially the same manner as in the synthesis of compound 1, except that 2-(2-nitro-1H-imidazol-1-yl)ethan-1-amine hydrochloride was used instead of 3-(2-aminoethyl)pyridine in step 6) compared to the synthesis of compound 1 in Example 1.

¹H NMR (400 MHz, CDCl₃) δ 8.52 (d, *J =* 5.4 Hz, 2H), 8.19 (s, 1H), 7.67 (s, 1H), 7.44 (d, *J* = 5.4 Hz, 2H), 7.27 (s, 1H), 7.21 - 7.16 (m, 3H), 7.09 (s, 1H), 7.02 (s, 1H), 6.88 (t, *J =* 8.6 Hz, 2H), 6.10 (t, *J =* 5.7 Hz, 1H), 4.52 (t, *J =* 5.7 Hz, 2H), 3.53 (q, *J =* 5.8 Hz, 2H). MS(ESI) m/z MH⁺ 471

### Example 7: Synthesis of compound 2 (synthesis reaction formula 2)

### Synthesis of 1-(4'-(4,4-difluoropiperidine-1-carbonyl)-2-((4-fluorophenyl)ethynyl)-[1,1'-biphenyl]-4-yl)-3-(2-(pyridin-3-yl)ethyl)urea

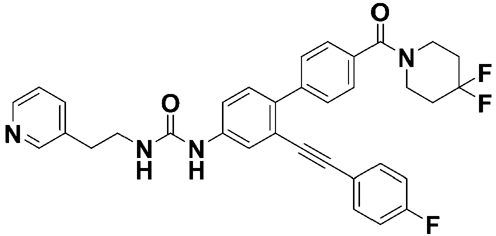

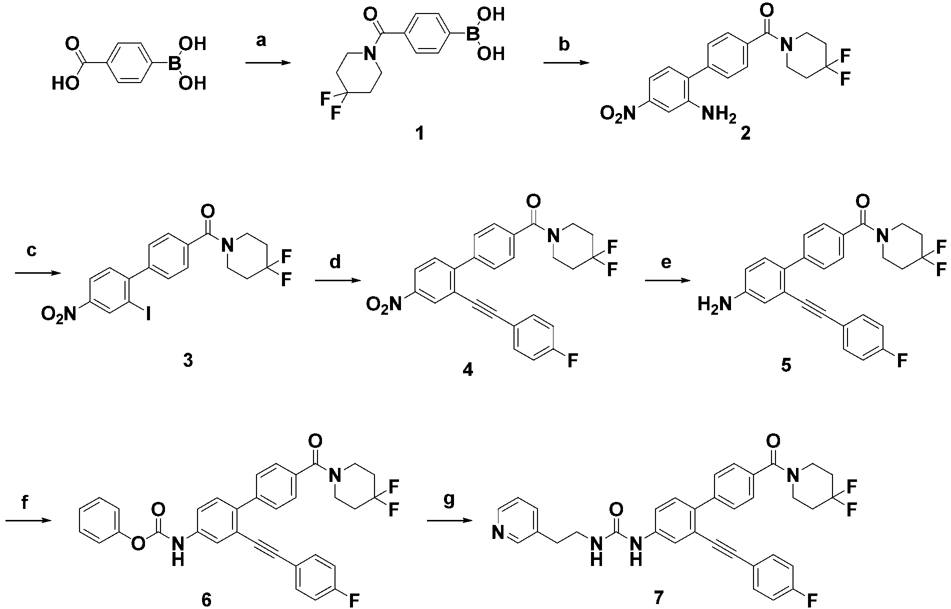

Reaction Formula 2. (a) 4,4-difluoropiperidine hydrochloride, HATU, TEA, DCM, RT, 2 hours; (b) 2-bromo-5-nitroaniline, PdCl₂(dppf), K₂CO₃, dioxane:water (3:1 (v/v)), 90°C, 2 hours; (c) sodium carbonate (NaCO₂), KI, HCl, water, 0°C, 1 hour; (d) PdCl₂(PPh₃)₂, CuI, TEA, ACN, 90°C, 3 hours; (e) Fe, AcOH, reflux, 1 hour; (f) phenyl chloroformate, pyridine, THF, 0°C- RT, 2 hours; (g) 2-(pyridin-3-yl)ethan-1-amine, TEA, THF, 80°C, 4 hours

### Step 1 (a) Synthesis of (4-(4,4-difluoropiperidine-1-carbonyl)phenyl)boronic acid

4-carboxyphenylboronic acid (0.5 g, 3 mmol, 1.0 eq) was dissolved in DCM (15 ml, 0.2 M), and then 4,4-difluoropiperidine hydrochloride (0.57 g, 3.6 mmol, 1.2 eq), HATU (1.48 g, 3.9 mmol, 1.3 eq), and TEA (1.7 ml, 12 mmol) were added and stirred at room temperature for two hours. Saturated sodium bicarbonate (Sat. NaHCO₃) aqueous solution was added and extracted with DCM. The organic layer was concentrated under reduced pressure, and then subjected to the following reaction of step 2) without separation.

### Step 2 (b) Synthesis of (2'-amino-4'-nitro-[1,1'-biphenyl]-4-yl)(4,4-difluoropiperidin-1-yl)methanone

2-bromo-5-nitroaniline (0.5 g, 2.3 mmol, 1.0 eq) was dissolved in 1,4-dioxane:water (3:1 (v/v), 12 ml, 0.2 M), and then (4-(4,4-difluoropiperidine-1-carbonyl)phenyl)boronic acid (0.8 g, 3 mmol, 1.3 eq), PdCl₂(dppf) (0.34 g, 0.46 mmol, 0.2 eq), and K₂CO₃ (0.7 g, 5.06 mmol, 2.2 eq) were added and stirred at 90°C for two hours. After cooling to room temperature and filtering, water and ethyl acetate (EA) were added to the filtrate and the organic layer was extracted. The organic layer was dried over MgSO₄, filtered, concentrated and then purified by column chromatography (silica gel, EA/HX) to obtain the title compound (1.226 g, 68%).

### Step 3 (c) Synthesis of (4,4-difluoropiperidin-1-yl)(2'-iodo-4'-nitro-[1,1'-biphenyl]-4-yl)methanone

0.9 ml of water and 0.9 ml of concentrated HCl were added to (2'-amino-4'-nitro-[1,1'-biphenyl]-4-yl)(4,4-difluoropiperidin-1-yl)methanone (0.311 g, 0.861 mmol, 1 eq), and then NaNO₂ (0.059 g, 0.861 mmol, 1 eq) dissolved in 0.9 ml of water and KI (0.143 g, 0.861 mmol, 1 eq) dissolved in 0.9 ml of water were added at 0°C and stirred for one hour. Saturated sodium bicarbonate (Sat. NaHCO₃) aqueous solution was added, neutralized and extracted with EA. The organic layer was dried over MgSO₄, filtered, concentrated and then purified by column chromatography (silica gel, EA/HX) to obtain the title compound (0.089 g, 22%).

### Step 4 (d) Synthesis of (4,4-difluoropiperidin-1-yl)(2'-((4-fluorophenyl)ethynyl)-4'-nitro-[1,1'-biphenyl]-4-yl)methanone

(4,4-difluoropiperidin-1-yl)(2'-iodo-4'-nitro-[1,1'-biphenyl]-4-yl)methanone (0.089 g, 0.188 mmol, 1 eq) was dissolved in acetonitrile (ACN, 2 ml, 0.1 M), and then triethylamine (TEA, 0.06 ml, 0.414 mmol, 2.2 eq), PdCl₂ (PPh₃)₂ (4 mg, 0.006 mmol, 3 mol%), and CuI (2 mg, 0.008 mmol, 4 mol%) were added and stirred at room temperature for five minutes. 1-ethynyl-4-fluorobenzene (0.032 ml, 0.283 mmol, 1.5 eq) was added and stirred at 90°C for three hours. The resulting mixture was cooled to room temperature, concentrated, and then purified by column chromatography (silica gel, EA/HX) to obtain the title compound (0.075 g, 86%).

### Step 5 (e) Synthesis of (4'-amino-2'-((4-fluorophenyl)ethynyl)-[1,1'-biphenyl]-4-yl)(4,4-difluoropiperidin-1-yl)methanone

(4,4-difluoropiperidin-1-yl)(2'-((4-fluorophenyl)ethynyl)-4'-nitro-[1,1'-biphenyl]-4-yl)methanone (0.075 g, 0.161 mmol, 1 eq) was dissolved in acetic acid (AcOH, 1.3 ml, 0.13 M), and then Fe (0.090 g, 1.61 mmol, 10 eq) was added and refluxed for one hour. The resulting mixture was cooled to room temperature and then filtered through a celite filter, and water was added to the filtrate and extracted with dichloromethane (DCM). The organic layer was dried over MgSO₄, filtered, concentrated and then purified by column chromatography (silica gel, EA/HX) to obtain the title compound (0.030 g, 43%).

### Step 6 (f) Synthesis of phenyl (4'-(4,4-difluoropiperidine-1-carbonyl)-2-((4-fluorophenyl)ethynyl)-[1,1'-biphenyl]-4-yl)carbamate

(4'-amino-2'-((4-fluorophenyl)ethynyl)-[1,1'-biphenyl]-4-yl)(4,4-difluoropiperidin-1-yl)methanone (0.030 g, 0.069 mmol, 1 eq) was dissolved in tetrahydrofuran (THF, 1 ml, 0.1 M), and then pyridine (0.011 ml, 0.138 mmol, 2 eq) and phenyl chloroformate (0.013 ml, 0.104 mmol, 1.5 eq) were added at 0°C and stirred at room temperature for two hours. 0.1 ml of water was added, stirred for 10 minutes, concentrated, and then purified by column chromatography (silica gel, EA/HX) to obtain the title compound (0.027 g, 71%).

### Step 7 (g) Synthesis of 1-(4'-(4,4-difluoropiperidine-1-carbonyl)-2-((4-fluorophenyl)ethynyl)-[1,1'-biphenyl]-4-yl)-3-(2-(pyridin-3-yl)ethyl)urea

Phenyl (4'-(4,4-difluoropiperidine-1-carbonyl)-2-((4-fluorophenyl)ethynyl)-[1,1'-biphenyl]-4-yl)carbamate (0.027 g, 0.049 mmol, 1 eq) was dissolved in THF (1 ml, 0.06 M), and then 2-(pyridin-3-yl)ethan-1-amine (0.011 ml, 0.097 mmol, 2 eq) and TEA (0.02 ml, 0.147 mmol, 3 eq) were added and stirred at 80°C for four hours. The reaction mixture was concentrated and purified by column chromatography (silica gel, MC/MeOH) to obtain the title compound (0.020 g, 70%).

¹H NMR (400 MHz, MeOD) δ 8.47 (s, 1H), 8.41 (d, *J =* 4.8 Hz, 1H), 7.80 (d, *J =* 7.8 Hz, 1H), 7.74 (s, 1H), 7.72 (s, 2H), 7.57 - 7.52 (m, 2H), 7.44 - 7.39 (m, 2H), 7.38 - 7.32 (m, 3H), 7.08 (t, *J =* 8.7 Hz, 2H), 3.98 - 3.57 (m, 4H), 3.50 (t, *J =* 7.0 Hz, 2H), 2.92 (t, *J =* 6.9 Hz, 2H), 2.07 (s, 4H). MS(ESI) m/z MH⁺ 583

The reactants may be changed in substantially the same manner as in the synthesis method of above Example 7, so as to synthesize compounds 22, 29, 58, 59, 67, and 68.

### Example 8: Synthesis of compound 7 (synthesis reaction formula 3)

### Synthesis of N-(2-(piperidin-1-yl)ethyl)-4-((5-(3-(2-(pyridin-3-yl)ethyl)ureido)-2-(pyridin-4-yl)phenyl)ethynyl)benzamide

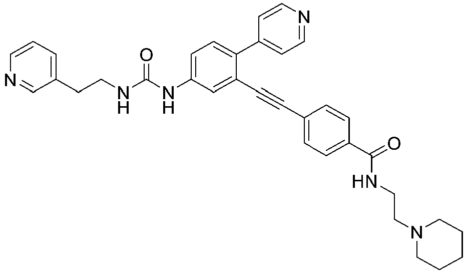

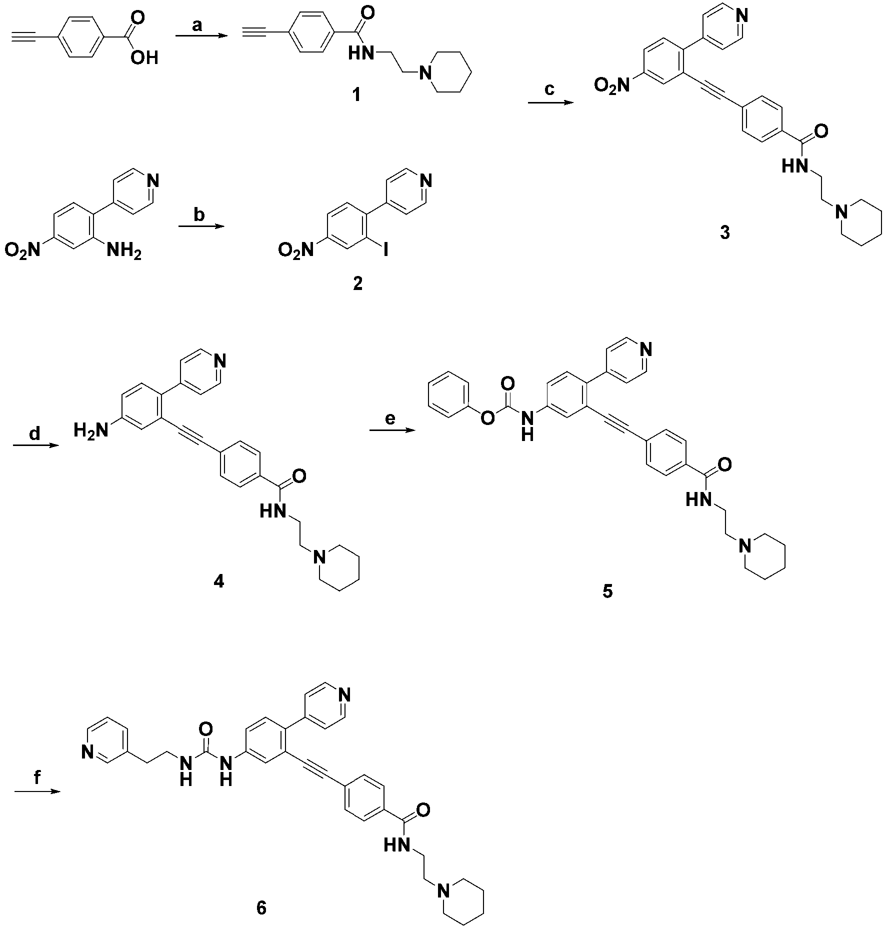

Reaction Formula 3. Reagents and conditions: (a) 2-(piperidin-1-yl)ethan-1-amine, HATU, DIPEA, DMF, RT, overnight; (b) NaNO₂, KI, HCl, H₂O, 5°C, 1 hour (1h); (c) PdCl₂(PPh₃)₂, CuI, TEA, ACN, 60°C, 5 hours (5h); (d) Zn, NH₄Cl, 1,4-dioxane, water (H₂O), RT, overnight; (e) phenyl chloroformate, pyridine, THF, 0°C to RT, overnight; (f) 2-(pyridin-3-yl)ethan-1-amine, TEA, THF, RT, overnight

### Step 1 (a) Synthesis of 4-ethynyl-N-(2-(piperidin-1-yl)ethyl)benzamide

4-ethynylbenzoic acid (0.5 g, 3.42 mmol, 1 eq) was dissolved in DMF (11 ml, 0.3 M), and then 2-(piperidin-1-yl)ethan-1-amine (0.657 g, 5.13 eq, 1.5 eq), HATU (1.950 g, 5.13 mmol, 1.5 eq) and DIPEA (1.326 g, 10.26 mmol, 3 eq) were added and stirred at room temperature overnight. After extracting with EA and H₂O, the organic layer was dried over MgSO₄, filtered, concentrated and then purified by column chromatography (silica gel, MeOH/DCM) to obtain the title compound (0.788 g, 89.7%).

### Step 2 (b) Synthesis of 4-(2-iodo-4-nitrophenyl)pyridine

4.5 ml of water and 4.5 ml of concentrated HCl were added to 5-nitro-2-(pyridin-4-yl)aniline (1.00 g, 4.64 mmol, 1 eq), and then NaNO₂ (0.326 g, 4.73 mmol, 1.0 eq) dissolved in 4.5 ml of water at 5°C was slowly added dropwise and stirred. The resulting mixture was stirred for 10 minutes after the addition, and KI (1.464 g, 8.82 mmol, 2 eq) dissolved in 4.5 ml of water was added, warmed to room temperature, and stirred for 30 minutes. Saturated K₂CO₃ aqueous solution was added, neutralized to pH 8 or higher, and extracted with DCM. The organic layer was dried over MgSO₄, filtered, concentrated and then purified by column chromatography (silica gel, EA/Hex) to obtain the title compound (0.952 g, 62.9%).

### Step 3 (c) Synthesis of 4-((5-nitro-2-(pyridin-4-yl)phenyl)ethynyl)-N-(2-(piperidin-1-yl)ethyl)benzamide

4-(2-iodo-4-nitrophenyl)pyridine (0.5 g, 1.53 mmol, 1 eq), 4-ethynyl-N-(2-(piperidin-1-yl)ethynyl)benzamide (0.588 g, 2.295 mmol, 1.5 eq), PdCl₂(PPh₃)₂ (0.0322 g, 0.046 mmol, 0.03 eq), and CuI (0.00876 g, 0.046 mmol, 0.03 eq) were added and degassed with nitrogen (N₂) gas. After that, ACN (3.06 ml, 0.5 M) and TEA (0.309 g, 3.06 mmol, 2 eq) were added, heated to 60°C, and stirred for five hours. After completion of the reaction, the resulting mixture was extracted with EA and H₂O, and the organic layer was dried over MgSO₄, filtered, concentrated and then purified by column chromatography (silica gel, MeOH/DCM) to obtain the title compound (0.29 g, 41.7%).

### Step 4 (d) Synthesis of 4-((5-amino-2-(pyridin-4-yl)phenyl)ethynyl)-N-(2-(piperidin-1-yl)ethyl)benzamide

4-((5-nitro-2-(pyridin-4-yl)phenyl)ethynyl)-N-(2-(piperidin-1-yl)ethyl)benzamide (0.29 g, 0.64 mmol, 1 eq) was dissolved in 1,4-dioxane:water (H₂O) (3:1 (v/v), 1.5 ml, 0.4 M), and then Zn (0.418 g, 6.4 mmol, 10 eq) and NH₄Cl (0.342 g, 6.4 mmol, 10 eq) were added and stirred at room temperature overnight. The resulting mixture was washed with MeOH and filtered under reduced pressure. The filtrate was concentrated and then subjected to the following reaction of step 5) without purification.

### Step 5 (e) Synthesis of phenyl (3-((4-((2-(piperidin-1-yl)ethyl)carbamoyl)phenyl)ethynyl)-4-(pyridin-4-yl)phenyl)carbamate

4-((5-amino-2-(pyridin-4-yl)phenyl)ethynyl)-N-(2-(piperidin-1-yl)ethyl)benzamide (0.272 g, 0.64 mmol, 1 eq) was dissolved in THF (3 ml, 0.2 M), and then pyridine (0.063 g, 0.8 mmol, 1.25 eq) was added. After cooling to 0°C, phenyl chloroformate (0.180 g, 1.152 mmol, 1.8 eq) was added dropwise. After slowly heating to room temperature, the resulting mixture was stirred for two hours. After completion of the reaction, the resulting mixture was concentrated, neutralized with 1N NaOH, and then extracted with EA and water (H₂O). The organic layer was dried over MgSO₄, filtered, concentrated and then purified by column chromatography (silica gel, MeOH/DCM) to obtain the title compound (0.115 g, 31.9%).

### Step 6 (f) Synthesis of N-(2-(piperidin-1-yl)ethyl)-4-((5-(3-(2-(pyridin-3-yl)ethyl)ureido)-2-(pyridin-4-yl)phenyl)ethynyl)benzamide

Phenyl (3-((4-((2-(piperidin-1-yl)ethyl)carbamoyl)phenyl)ethynyl)-4-(pyridin-4-yl)phenyl)carbamate (0.115 g, 0.21 mmol, 1 eq) was dissolved in THF (2 ml, 0.1 M), and then 2-(pyridin-3-yl)ethan-1-amine (0.0513 g, 0.42 mmol, 2 eq) and TEA (0.064 g, 0.63 mmol, 3 eq) were added and stirred at room temperature overnight. After completion of the reaction, the resulting mixture was concentrated and purified by reverse-phase column chromatography (0.1% formic acid in H₂O/ACN) to obtain the title compound (0.016 g, 13.7%).

¹H NMR (400 MHz, DMSO) δ 8.91 (s, 1H), 8.68 (s, 2H), 8.57 - 8.42 (m, 3H), 8.20 (s, 1H), 7.94 (s, 1H), 7.86 (d, *J =* 8.0 Hz, 2H), 7.72 - 7.63 (m, 3H), 7.53 - 7.43 (m, 4H), 7.39 - 7.33 (m, 1H), 6.42 (s, 1H), 3.41 (m, 4H), 2.82 (t, *J =* 6.6 Hz, 2H), 2.55 (m, 5H), 1.54 (m, 4H), 1.41 (m, 2H); MS(ESI) m/z MH+ 573.1

The reactants may be changed in substantially the same manner as in the synthesis method of above Example 8, so as to synthesize compounds 19, 20, 44, 45, 46, 54, 64, 33, 34, 36, 41, 48, 61, 62, 84, 85, and 92.

### Example 9: Synthesis of compound 9 (synthesis reaction formula 4)

### Synthesis of 1-(2-((4-fluorophenyl)ethynyl)-3'-morpholino-[1,1'-biphenyl]-4-yl)-3-(2-(pyridin-3-yl)ethyl)urea

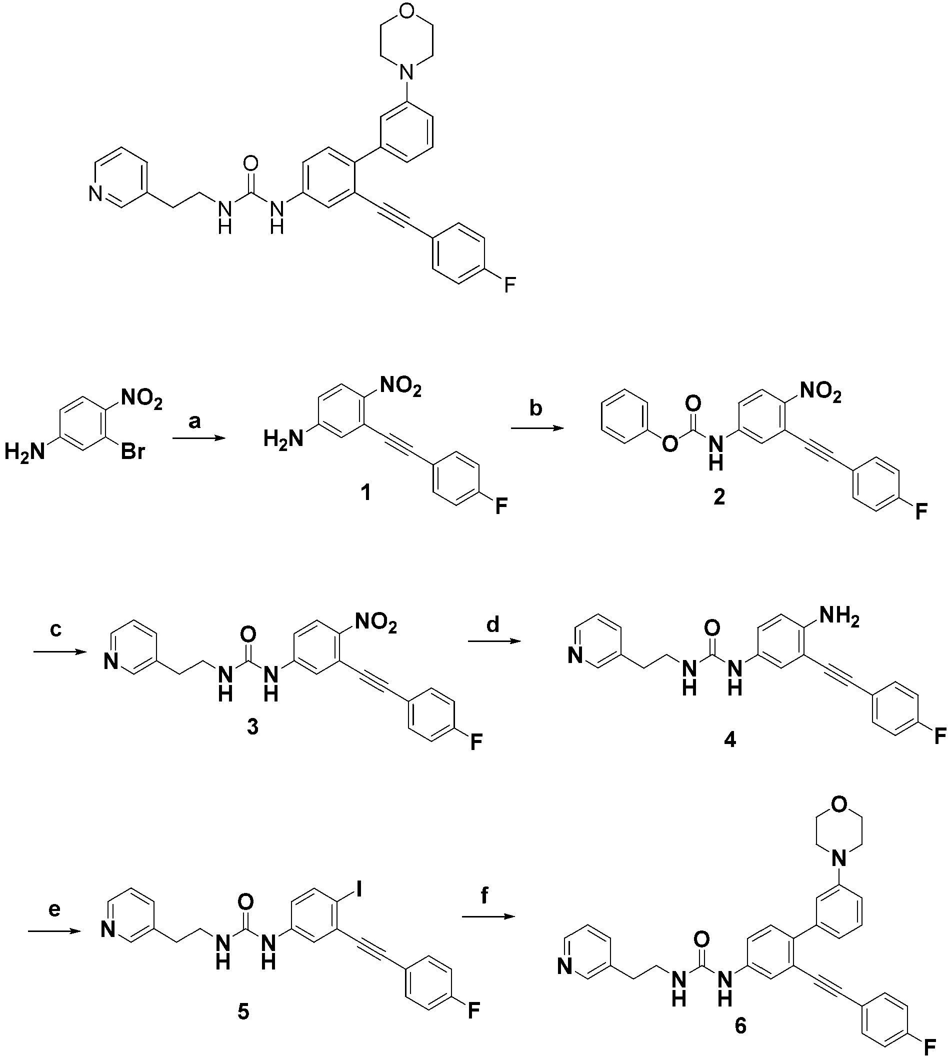

Reaction Formula 4. Reagents and conditions: (a) 1-ethynyl-4-fluorobenzene, TEA, CuI, PdCl₂(PPh₃)₂, ACN, reflux, 1 hour; (b) phenyl chloroformate, pyridine, THF, 0°C, 2 hours; (c) 2-(pyridin-3-yl)ethan-1-amine, TEA, THF, 80°C, 2 hours; (d) Fe, AcOH, reflux, 1 hour; (e) NaNO₂, KI, pTSA, ACN, 5°C, 1 hour; (f) (3-morpholinophenyl)boronic acid, PdCl₂(dppf), K₂CO₃, 1,4-dioxane, H₂O, 90°C, 4 hours

### Step 1 (a) Synthesis of 3-((4-fluorophenyl)ethynyl)-4-nitroaniline

3-bromo-4-nitroaniline (2.17 g, 0.01 mol, 1 eq) was dissolved in ACN (100 ml, 0.1 M), and then TEA (3 ml, 0.22 mol, 2.2 eq), PdCl₂(PPh₃)₂ (210 mg, 0.3 mmol, 3 mol%), and CuI (80 mg, 0.4 mmol, 4 mol%) were added and stirred at room temperature for five minutes. 1-ethynyl-4-fluorobenzene (1.72 ml, 0.015 mmol, 1.5 eq) was added and stirred at 90°C for one hour. The resulting mixture was cooled to room temperature, concentrated, and purified by column chromatography (silica gel, EA/HX) to obtain the title compound (1.44 g, 56%).

### Step 2 (b) Synthesis of phenyl (3-((4-fluorophenyl)ethynyl)-4-nitrophenyl)carbamate

3-((4-fluorophenyl)ethynyl)-4-nitroaniline (1.436 g, 5.60 mmol, 1 eq) was dissolved in THF (56 ml, 0.1 M), and then pyridine (0.9 ml, 11.2 mmol, 2 eq) and phenyl chloroformate (1.06 ml, 8.41 mmol, 1.5 eq) were added dropwise at 0°C and stirred at room temperature for two hours. Water was added, stirred for 10 minutes, concentrated, and then purified by column chromatography (silica gel, EA/HX) to obtain the title compound (1.968g, 93.4%).

### Step 3 (c) Synthesis of 1-(3-((4-fluorophenyl)ethynyl)-4-nitrophenyl)-3-(2-(pyridin-3-yl)ethyl)urea

Phenyl (3-((4-fluorophenyl)ethynyl)-4-nitrophenyl)carbamate (1 g, 2.66 mmol, 1 eq) was dissolved in THF (1 ml, 0.06 M), and then 2-(pyridin-3-yl)ethan-1-amine (0.62 ml, 5.32mmol, 2 eq) and TEA (1.11ml, 7.98 mmol, 3 eq) were added and stirred at 80°C for four hours. The reaction mixture was concentrated and purified by column chromatography (silica gel, MC/MeOH) to obtain the title compound (0.954 g, 96%).

### Step 4 (d) Synthesis of 1-(4-amino-3-((4-fluorophenyl)ethynyl)phenyl)-3-(2-(pyridin-3-yl)ethyl)urea

1-(3-((4-fluorophenyl)ethynyl)-4-nitrophenyl)-3-(2-(pyridin-3-yl)ethyl)urea (0.954 g, 2.55 mmol, 1 eq) was dissolved in AcOH (20 ml, 0.13 M), and then Fe (1.42 g, 25.5 mmol, 10 eq) was added and refluxed for one hour. The resulting mixture was cooled to room temperature and then filtered through a celite filter, and water was added to the filtrate and extracted with DCM. The organic layer was dried over MgSO₄, filtered, concentrated and then purified by column chromatography (silica gel, EA/HX) to obtain the title compound (0.859 g, 90%).

¹H NMR (400 MHz, DMSO) δ 8.46 (d, *J=* 1.5 Hz, 1H), 8.43 (d, *J =* 3.9 Hz, 1H), 8.06 (s, 1H), 7.39 - 7.31 (m, 2H), 7.26 (t, *J =* 8.8 Hz, 2H), 6.98 (dd, *J* = 8.7, 2.4 Hz, 1H), 6.64 (d, *J* = 8.7 Hz, 1H), 6.06 - 5.99 (m, 1H), 5.18 (s, 2H), 3.36 - 3.30 (m, 2H), 2.77 (t, *J =* 7.0 Hz, 2H).

### Step 5 (e) Synthesis of 1-(3-((4-fluorophenyl)ethynyl)-4-iodophenyl)-3-(2-(pyridin-3-yl)ethyl)urea

1-(4-amino-3-((4-fluorophenyl)ethynyl)phenyl)-3-(2-(pyridin-3-yl)ethyl)urea (0.05 g, 0.134 mmol, 1 eq) was dissolved in ACN (1.34 ml, 0.1 M), and then p-toluenesulfonic acid (pTSA, 0.069 g, 0.402 mmol, 3 eq) was added, and then NaNO₂ (0.01 g, 0.136 mmol, 1.02 eq) dissolved in 0.15 ml of water and KI (0.045 g, 0.268 mmol, 2 eq) dissolved in 0.15 ml of water were added at 0°C. After reaction at room temperature for one hour, saturated sodium bicarbonate (Sat. NaHCO₃) aqueous solution was added, neutralized to pH 8 or higher, and extracted with EA. The organic layer was dried over MgSO₄, filtered, concentrated and then purified by column chromatography (silica gel, MeOH/DCM) to obtain the title compound (0.038 g, 58.4%).

### Step 6 (f) Synthesis of 1-(2-((4-fluorophenyl)ethynyl)-3'-morpholino-[1,1'-biphenyl]-4-yl)-3-(2-(pyridin-3-yl)ethyl)urea

1-(3-((4-fluorophenyl)ethynyl)-4-iodophenyl)-3-(2-(pyridin-3-yl)ethyl)urea (0.05 g, 0.1 mmol, 1 eq) was dissolved in 1,4-dioxane:water (H₂O) (3:1 (v/v), 0.5 ml, 0.2 M), and then (3-morpholinophenyl)boronic acid (0.041 g, 0.2 mmol, 2 eq), PdCl₂(dppf) (0.0146 g, 0.02 mmol, 0.2 eq), and K₂CO₃ (0.0304 g, 0.22 mmol, 2.2 eq) were added, heated to 90°C and stirred for four hours. After completion of the reaction, the resulting mixture was washed with EA, filtered through a celite filter, and extracted with EA and H₂O. The organic layer was dried over MgSO₄, filtered, concentrated and then filtered by prep-LC (0.1% formic acid in H₂O/ACN) to obtain the title compound (0.078 g, 15.0%).

¹H NMR (400 MHz, DMSO) δ 8.74 (s, 1H), 8.48 (s, 1H), 8.45 (d, *J =* 4.4 Hz, 1H), 7.84 (m, 1H), 7.69 (m, 1H), 7.42 (dd, *J =* 8.3, 5.6 Hz, 2H), 7.37 (s, 2H), 7.33 (m, 2H), 7.26 (t, *J =* 8.8 Hz, 2H), 7.18 (s, 1H), 7.04 (d, *J =* 7.4 Hz, 1H), 6.96 (d, *J =* 8.6 Hz, 1H), 6.30 (t, *J* = 5.5 Hz, 1H), 3.74 - 3.70 (m, 4H), 3.39 (dd, *J =* 12.9, 6.6 Hz, 2H), 3.15 - 3.10 (m, 4H), 2.81 (t, *J =* 6.9 Hz, 2H).; MS(ESI) m/z MH- 519.48

The reactants may be changed in substantially the same manner as in the synthesis method of above Example 9, so as to synthesize compounds 49, 50, 51, 56, 131, 132, 134, 142, 143, 144, 145, 179, and 180.

### Example 10: Synthesis of compound 13 (synthesis reaction formula 5)

### Synthesis of 1-(2-((4-fluorophenyl)ethynyl)-4'-(piperazin-1-yl)-[1,1'-biphenyl]-4-yl)-3-(2-(pyridin-3-yl)ethyl)urea

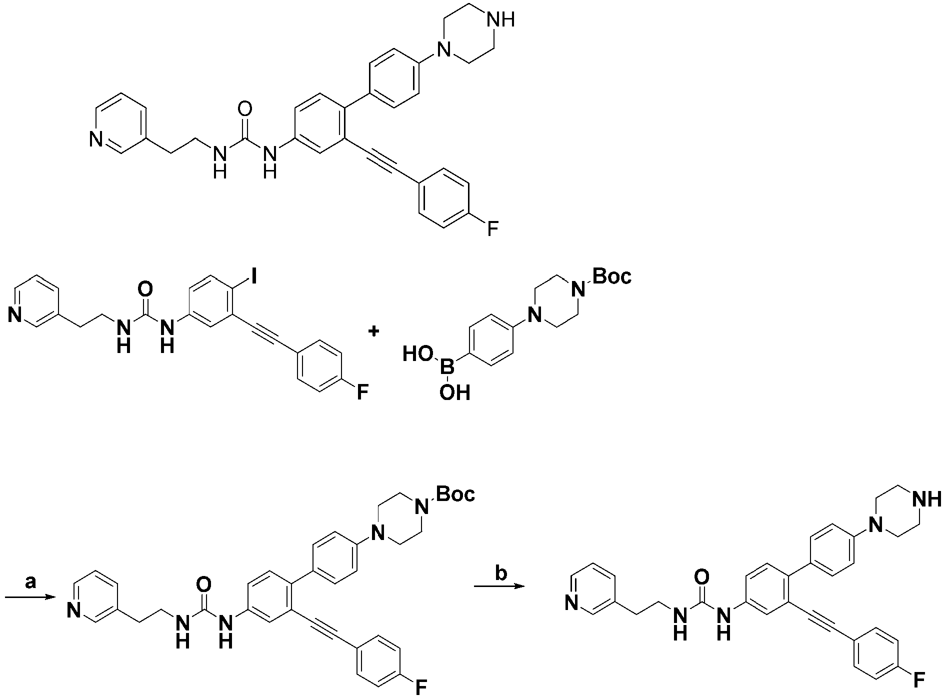

Reaction Formula 5. (a) PdCl₂(dppf), K₂CO₃, 1,4-dioxane, water (H₂O), 90°C, 4 hours; (b) TFA, DCM, RT, overnight.

### Step 1 (a) Synthesis of tert-butyl 4-(2'-((4-fluorophenyl)ethynyl)-4'-(3-(2-(pyridin-3-yl)ethynyl)ureido)-[1,1'-biphenyl]-4-yl)piperazin-1-carboxylate

1-(3-((4-fluorophenyl)ethynyl)-4-iodophenyl)-3-(2-(pyridin-3-yl)ethyl)urea (0.05 g, 0.1 mmol, 1 eq) was dissolved in 1,4-dioxane:water (H₂O) (3:1 (v/v), 0.5 ml, 0.2 M), and then (4-(4-(tert-butoxycarbonyl)piperazin-1-yl)phenyl)boronic acid (0.061 g, 0.2 mmol, 2 eq), PdCl₂(dppf) (0.0146 g, 0.02 mmol, 0.2 eq), and K₂CO₃(0.0304 g, 0.22 mmol, 2.2 eq) were added, heated to 90°C and stirred for four hours. After completion of the reaction, the resulting mixture was washed with EA, filtered through a celite filter, and extracted with EA and H₂O. The organic layer was dried over MgSO₄, filtered, concentrated and then purified by column chromatography (silica gel, MeOH/DCM) to obtain the title compound (0.043 g, 69.4%).

### Step 2 (b) Synthesis of 1-(2-((4-fluorophenyl)ethynyl)-4'-(piperazin-1-yl)-[1,1'-biphenyl]-4-yl)-3-(2-(pyridin-3-yl)ethyl)urea

Tert-butyl 4-(2'-((4-fluorophenyl)ethynyl)-4'-(3-(2-(pyridin-3-yl)ethyl)ureido)-[1,1'-biphenyl]-4-yl)piperazin-1-carboxylate (0.047 g, 0.075 mmol, 1 eq) was dissolved in DCM (0.5 ml), and then trifluoacetic acid (0.083 g, 0.75 mmol, 10 eq) was added and stirred at room temperature overnight. After completion of the reaction, the resulting mixture was concentrated and purified by reverse-phase column chromatography (0.1% formic acid in H2O/acetonitrile) to obtain the title compound (0.017 g, 43.6%).

¹H NMR (400 MHz, DMSO) δ 8.76 (s, 1H), 8.47 (d, *J =* 15.1 Hz, 2H), 7.81 (s, 1H), 7.69 (d, *J =* 7.6 Hz, 1H), 7.52 (d, *J =* 8.2 Hz, 2H), 7.48 - 7.43 (m, 2H), 7.35 (d, *J* = 7.6 Hz, 2H), 7.26 (dd, *J =* 16.4, 8.2 Hz, 3H), 7.04 (d, *J =* 8.1 Hz, 2H), 6.38 (s, 1H), 3.42 - 3.35 (m, 3H), 3.26 (s, 4H), 3.07 (s, 4H), 2.81 (t, *J =* 6.7 Hz, 2H).; MS(ESI) m/z MH- 518.45

### Example 11: Synthesis of compound 14 (synthesis reaction formula 6)

### Synthesis of 1-(3-((4-fluorophenyl)ethynyl)-4-(2-methyl-4-oxo-3,4-dihydroquinazolin-7-yl)phenyl)-3-(2-(pyridin-3-yl)ethyl)urea

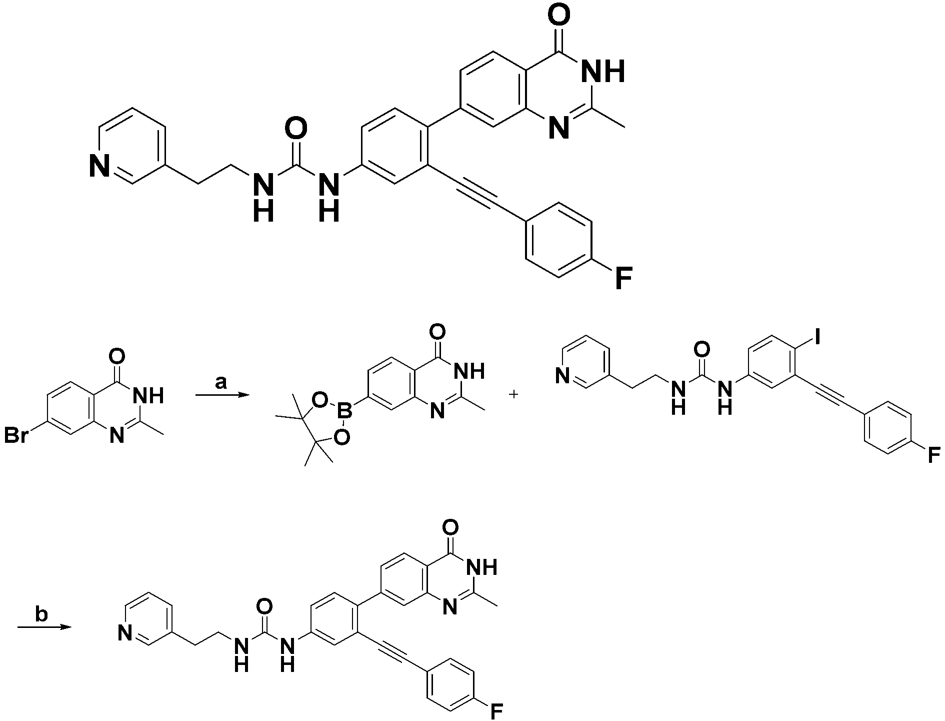

Reaction Formula 6. (a) PdCl₂(dppf), bis(pinacolato)diboron, KOAc, dioxane, 60°C, 16 hours (16h); (b) PdCl₂(dppf), K₂CO₃, 1,4-dioxane, water (H₂O), 90°C, 4 hours

### Step 1 (a) Synthesis of 2-methyl-7-(4,4,5,5-tetramethyl-1,3,2-dioxaborolan-2-yl)quinazolin-4(3H)-one

7-bromo-2-methylquinazolin-4(3H)-one (0.24 g, 1 mmol, 1 eq) was dissolved in dioxane (10 ml, 0.1 M), and then bis(pinacolato)diboron (0.5 g, 2 mmol, 2 eq), potassium acetate (0.2 g, 2 mmol, 2 eq) and PdCl₂(dppf) (0.15 g, 0.2 mmol, 0.2 eq) were added and stirred at 60°C for 16 hours. The reaction mixture was concentrated under reduced pressure and purified by column chromatography (silica gel, EA/HX) to obtain the title compound (0.075 g, 26%).

### Step 2 (b) Synthesis of 1-(3-((4-fluorophenyl)ethynyl)-4-(2-methyl-4-oxo-3,4-dihydroquinazolin-7-yl)phenyl)-3-(2-(pyridin-3-yl)ethyl)urea

The title compound (7.38 g, 22%) was obtained in substantially the same manner as in step 6) of compound 9 in Example 9, except that 2-methyl-7-(4,4,5,5-tetramethyl-1,3,2-dioxaborolan-2-yl)quinazolin-4(3H)-one was used instead of (3-morpholinophenyl)boronic acid in step 6) compared to the synthesis of compound 9 in Example 9.

¹H NMR (400 MHz, MeOD) δ 8.50 (s, 1H), 8.43 (d, *J=* 4.5 Hz, 1H), 8.26 (d, *J=* 8.3 Hz, 1H), 7.90 (s, 1H), 7.85 - 7.76 (m, 3H), 7.48 - 7.41 (m, 3H), 7.37 (dd, *J=* 7.8, 5.7 Hz, 2H), 7.08 (t, *J* = 8.5 Hz, 2H), 3.53 (t, *J =* 6.9 Hz, 2H), 2.94 (t, *J=* 7.0 Hz, 2H), 2.50 (s, 3H). MS(ESI) m/z MH+ 518

### Example 12: Synthesis of compound 11 (synthesis reaction formula 7)

### Synthesis of 1-(2-(benzo[d]oxazol-5-yl)ethyl)-3-(3-((4-fluorophenyl)ethynyl)-4-(pyridin-4-yl)phenyl)urea

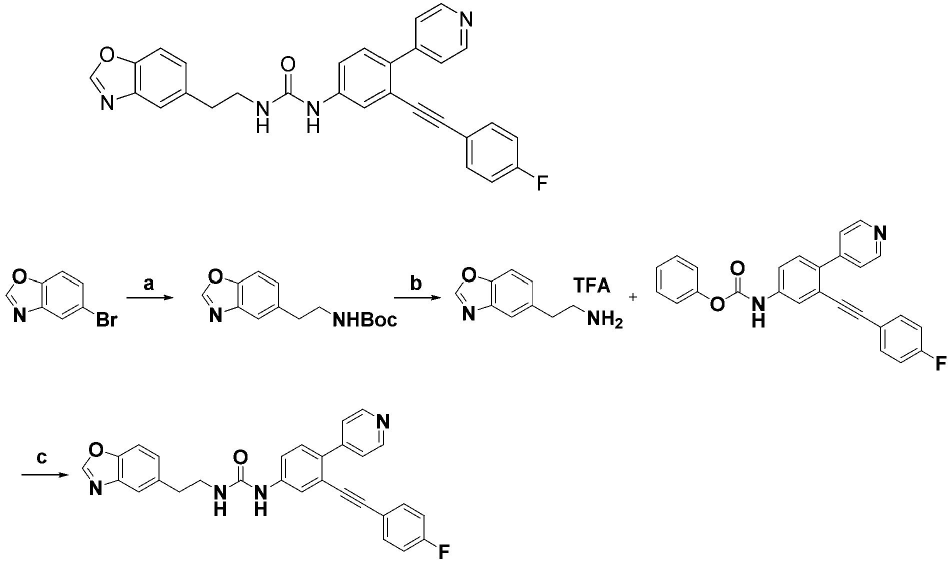

Reaction Formula 7. (a) potassium (2-((tert-butoxycarbonyl)amino)ethyl)trifluoroborate, Cs₂CO₃, Pd(OAc)₂, RuPhos, toluene/water; (b) TFA, DCM (c) TEA, THF, 24 hours, reflux

### Step 1 (a) Synthesis of tert-butyl (2-(benzo[d]oxazol-5-yl)ethyl)carbamate

A mixture of 5-bromobenzo[d]oxazole (0.2 g, 1 eq), potassium (2-((tert-butoxycarbonyl)amino)ethyl)trifluoroborate (0.28 g, 1.1 eq) and cesium carbonate (0.977 g, 3 eq) in toluene (6 mL) and water (2 mL) was degassed twice with nitrogen. Then, palladium (II) acetate (11 mg, 0.05 eq) and RuPhos (46 mg, 0.1 eq) were added, and the mixture was heated to 95°C overnight under nitrogen. The reactant was cooled to room temperature and water was added, and then the resulting mixture was extracted twice with EtOAc. Then, the combined organic layers were washed with brine, dried over anhydrous MgSO₄, filtered, and concentrated under reduced pressure. After that, the title compound (40 mg, yield 15%) was obtained by purification with (hexane/ethyl acetate = 1/5).

### Step 2 (b) Synthesis of 2-(benzo[d]oxazol-5-yl)ethan-1-amine

TFA (0.11 mL, 10 eq) was added to a solution of tert-butyl (2-(benzo[d]oxazol-5-yl)ethyl)carbamate (0.04 g, 1 eq) in DCM (5 mL), and the mixture was stirred at room temperature for two hours. Subsequently, the reactant was concentrated under reduced pressure to obtain 2-(benzo[d]oxazol-5-yl)ethan-1-amine as a crude product, which was further dried under high vacuum.

### Step 3 (c) Synthesis of 1-(2-(benzo[d]oxazol-5-yl)ethyl)-3-(3-((4-fluorophenyl)ethynyl)-4-(pyridin-4-yl)phenyl)urea

The title compound was obtained in substantially the same manner as in the synthesis of compound 1 in Example 1, except that 2-(benzo[d]oxazol-5-yl)ethan-1-amine was used instead of 3-(2-aminoethyl)pyridine in step 6) compared to the synthesis of compound 1 in Example 1.

¹H NMR (400 MHz, MeOD) δ 8.60 (d, *J =* 5.1 Hz, 2H), 8.45 (s, 1H), 7.77 - 7.61 (m, 5H), 7.45 - 7.34 (m, 5H), 7.10 (t, *J=* 8.6 Hz, 2H), 3.53 (t, *J =* 7.0 Hz, 2H), 3.01 (t, *J* = 7.0 Hz, 2H).

### Example 13: Synthesis of compound 118 (synthesis reaction formula 8)

### Synthesis of 1-(3-((4-fluorophenyl)ethynyl)-4-(piperazin-1-yl)phenyl)-3-(2-(pyridin-3-yl)ethyl)urea

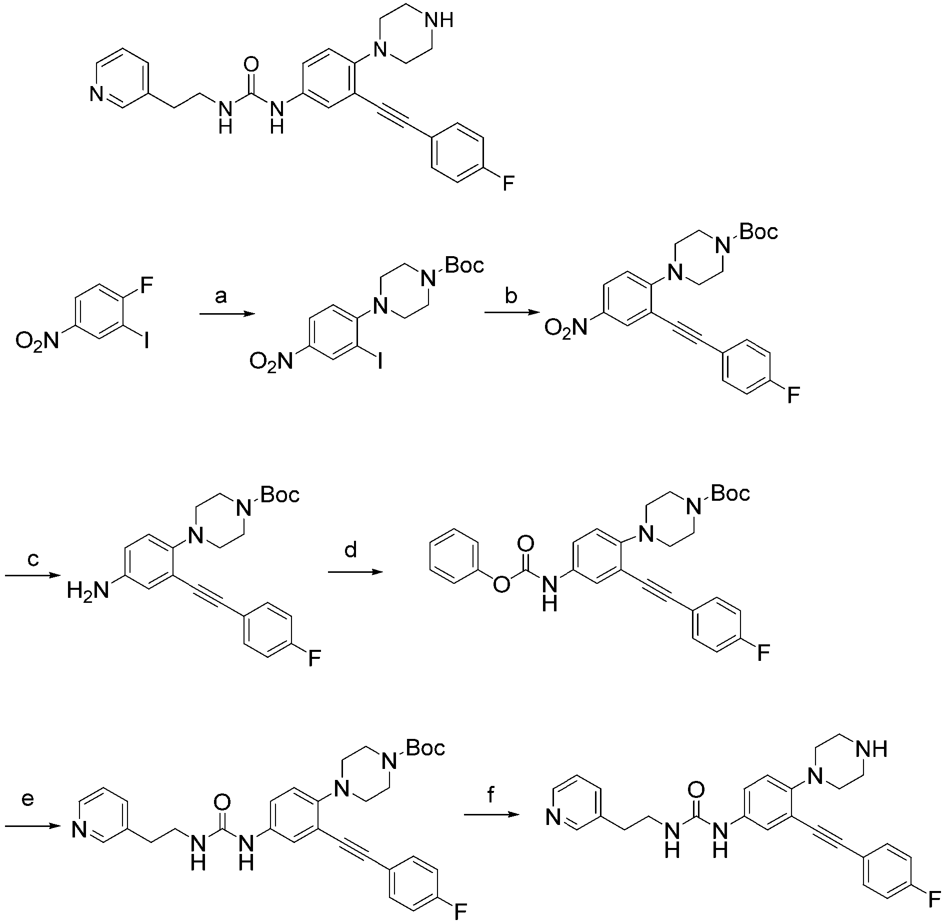

Reaction Formula 8. (a) tert-butyl piperazin-1-carboxylate, Cs₂CO₃, DMF, 80°C, 24 hours; (b) 1-ethynyl-4-fluorobenzene, PdCl₂(PPh₃)₂, CuI, TEA, ACN, 90°C, 2 hours; (c) Zn, NH₄Cl, 1,4-dioxane, water (H₂O), RT, 1 hour; (d) phenyl chloroformate, pyridine, THF, 0°C, 2 hours; (e) 2-(pyridin-3-yl)ethan-1-amine, TEA, THF, reflux, 24 hours; (f) 4N HCl in dioxane, 1,4-dioxane, RT, 24 hours

### Step 1 (a) Synthesis of tert-butyl 4-(2-iodo-4-nitrophenyl)piperazine-1-carboxylate

1-fluoro-2-iodo-4-nitrobenzene (0.1 g, 0.375 mmol, 1.0 eq) was dissolved in DMF (1 mL), and then Cs₂CO₃ (0.15g, 0.45 mmol, 1.2 eq) and tert-butyl piperazin-1-carboxylate (0.07 g, 0.375 mmol, 1.0 eq) were added and stirred at 80°C overnight. After completion of the reaction, the resulting mixture was extracted with EA and H₂O. The organic layer was dried over MgSO₄, filtered, concentrated and then purified by column chromatography (silica gel, EA/HX) to obtain the title compound (0.114 g, 70.4%).

¹H NMR (400 MHz, CDCl3) δ 8.71 (d, J = 2.2 Hz, 1H), 8.21 (dd, J = 8.8, 2.2 Hz, 1H), 7.00 (d, J = 8.8 Hz, 1H), 3.70 - 3.61 (m, 4H), 3.11 - 3.03 (m, 4H), 1.49 (s, 9H).

### Step 2 (b) Synthesis of tert-butyl 4-(2-((4-fluorophenyl)ethynyl)-4-nitrophenyl)piperazine-1-carboxylate

Tert-butyl 4-(2-iodo-4-nitrophenyl)piperazin-1-carboxylate (0.114 g, 0.263 mmol, 1 eq) was dissolved in ACN (3 ml, 0.1 M), and then TEA (0.08 ml, 0.579 mmol, 2.2 eq), PdCl₂(PPh₃)₂ (5.5 mg, 0.008 mmol, 3 mol%), and CuI (2 mg, 0.011 mmol, 4 mol%) were added and stirred at room temperature for five minutes. 1-ethynyl-4-fluorobenzene (0.045 ml, 0.395 mmol, 1.5 eq) was added and stirred at 90°C for two hours. The resulting mixture was cooled to room temperature, concentrated, and purified by column chromatography (silica gel, EA/HX) to obtain the title compound (0.109 g, 97.3%).

¹H NMR (400 MHz, CDCl3) δ 8.39 (d, J = 2.5 Hz, 1H), 8.15 (dd, J = 9.1, 2.6 Hz, 1H), 7.52 (dd, J = 8.4, 5.4 Hz, 2H), 7.12 (t, J = 8.6 Hz, 2H), 6.95 (d, J = 9.1 Hz, 1H), 3.72 - 3.65 (m, 4H), 3.47 - 3.38 (m, 4H), 1.52 (s, 8H).

### Step 3 (c) Synthesis of tert-butyl 4-(4-amino-2-((4-fluorophenyl)ethynyl)phenyl)piperazine-1-carboxylate

Tert-butyl 4-(2-((4-fluorophenyl)ethynyl)-4-nitrophenyl)piperazin-1-carboxylate (0.109 g, 0.256 mmol, 1 eq) was dissolved in 1,4-dioxane:water (H₂O) (3:1 (v/v), 3 ml, 0.4 M), and then Zn (0.14 g, 2.56 mmol, 10 eq) and NH₄Cl (0.137 g, 2.56 mmol, 10 eq) were added and stirred at room temperature for one hour. The resulting mixture was washed with MeOH and filtered under reduced pressure. The filtrate was concentrated and extracted with EA and H₂O. The organic layer was dried over MgSO₄, filtered, concentrated, and then subjected to the following reaction of step 4) without purification.

¹H NMR (400 MHz, CDCl3) δ 7.47 (dd, J = 8.2, 5.6 Hz, 2H), 7.26 - 7.26 (m, 1H), 7.05 (t, J = 8.5 Hz, 2H), 6.90 (s, 1H), 6.68 (d, J = 6.2 Hz, 1H), 3.64 (s, 4H), 3.09 (s, 4H), 1.48 (s, 9H).

### Step 4 (d) Synthesis of tert-butyl 4-(2-((4-fluorophenyl)ethynyl)-4-((phenoxycarbonyl)amino)phenyl)piperazine-1-carboxylate

Tert-butyl 4-(4-amino-2-((4-fluorophenyl)ethynyl)phenyl)piperazin-1-carboxylate (0.114 g, 0.256 mmol, 1 eq) was dissolved in THF (3 ml, 0.1 M), and then pyridine (0.04 ml, 0.512 mmol, 2 eq) and phenyl chloroformate (0.049 ml, 0.384 mmol, 1.5 eq) were added at 0°C and stirred at room temperature for two hours. Water was added, stirred for 10 minutes, concentrated, and then purified by column chromatography (silica gel, EA/HX) to obtain the title compound (0.093g, 70.5%).

¹H NMR (400 MHz, CDCl3) δ 7.66 (s, 1H), 7.51 - 7.44 (m, 2H), 7.44 - 7.35 (m, 3H), 7.25 - 7.22 (m, 2H), 7.19 (d, J = 7.8 Hz, 3H), 7.06 (t, J = 8.6 Hz, 2H), 6.87 (s, 1H), 3.67 (s, 4H), 3.18 (s, 4H), 1.48 (s, 9H).

### Step 5 (e) Synthesis of tert-butyl 4-(2-((4-fluorophenyl)ethynyl)-4-(3-(2-(pyridin-3-yl)ethyl)ureido)phenyl)piperazin-1-carboxylate

Tert-butyl 4-(2-((4-fluorophenyl)ethynyl)-4-((phenoxycarbonyl)amino)phenyl)piperazine-1-carboxylate (0.093 g, 0.180 mmol, 1 eq) was dissolved in THF (2 ml, 0.1 M), and then 2-(pyridin-3-yl)ethan-1-amine (0.042 ml, 0.36 mmol, 2 eq) and TEA (0.075 ml, 0.54 mmol, 3 eq) were added and stirred at 80°C for four hours. The reaction mixture was concentrated and purified by column chromatography (silica gel, MC/MeOH) to obtain the title compound (0.089g, 90.8%).

¹H NMR (400 MHz, CDCl3) δ 8.52 (s, 1H), 8.43 (d, J = 4.2 Hz, 1H), 7.62 (d, J = 7.7 Hz, 1H), 7.51 - 7.44 (m, 2H), 7.41 (s, 1H), 7.30 (d, J = 6.9 Hz, 1H), 7.17 (d, J = 7.8 Hz, 1H), 7.06 (t, J = 8.6 Hz, 2H), 6.84 (d, J = 8.7 Hz, 1H), 6.63 (s, 1H), 5.02 (s, 1H), 3.61 (s, 4H), 3.56 - 3.47 (m, 2H), 3.10 (s, 4H), 2.87 (t, J = 6.5 Hz, 2H), 1.48 (s, 9H).

### Step 6 (f) Synthesis of 1-(3-((4-fluorophenyl)ethynyl)-4-(piperazin-1-yl)phenyl)-3-(2-(pyridin-3-yl)ethyl)urea

Tert-butyl 4-(2-((4-fluorophenyl)ethynyl)-4-(3-(2-(pyridin-3-yl)ethyl)ureido)phenyl)piperazin-1-carboxylate (0.089 g, 0.164 mmol, 1 eq) was dissolved in 1,4-dioxane (2 ml, 0.1 M), and then 4N HCl in dioxane (0.16 ml, 0.655 mmol, 4 eq) was added and stirred overnight. The compound was obtained after washing with 1,4-dioxane and filtering under reduced pressure.

¹H NMR (400 MHz, MeOD) δ 8.45 (s, 1H), 8.39 (d, J = 4.4 Hz, 1H), 7.77 (d, J = 7.8 Hz, 1H), 7.56 - 7.48 (m, 3H), 7.39 (dd, J = 7.6, 5.0 Hz, 1H), 7.24 (dd, J = 8.7, 2.4 Hz, 1H), 7.14 (t, J = 8.7 Hz, 2H), 6.95 (d, J = 8.8 Hz, 1H), 3.46 (t, J = 6.9 Hz, 2H), 3.15 - 3.09 (m, 4H), 3.05 - 2.99 (m, 4H), 2.89 (t, J = 6.9 Hz, 2H).

The reactants may be changed in substantially the same manner as in the synthesis method of above Example 13, so as to synthesize compounds 25, 26, 27, 28, 30, 31, 32, 42, 114, 115, 116, 117, 119, 120, 121, 169, and 171.

### Example 14: Synthesis of compound 52 (synthesis reaction formula 9)

### Synthesis of 1-(4-(4-(3-aminobenzoyl)piperazin-1-yl)-3-((4-fluorophenyl)ethynyl)phenyl)-3-(2-(pyridin-3-yl)ethyl)urea

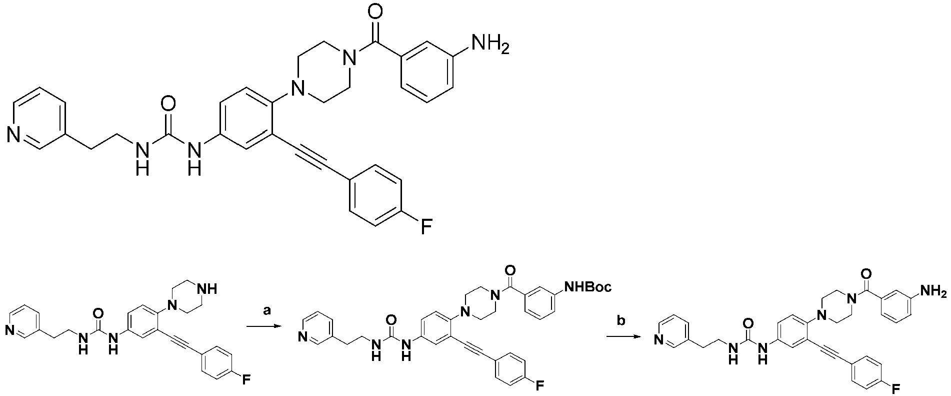

Reaction Formula 9. (a) 3-((tert-butoxycarbonyl)amino)benzoic acid, DIPEA, HATU, DCM, RT, 24 hours; (b) 4N HCl in dioxane, 1,4-dioxane, RT, 24 hours;

### Step 1 (a) Synthesis of tert-butyl (3-(4-(2-((4-fluorophenyl)ethynyl)-4-(3-(2-(pyridin-3-yl)ethyl)ureido)phenyl)piperazine-1-carbonyl)phenyl)carbamate

3-((tert-butoxycarbonyl)amino)benzoic acid (0.024 g, 0.1 mmol, 1 eq) was dissolved in DCM (1 ml, 0.3 M), and then HATU (0.046 g, 0.12 mmol, 1.2 eq), DIPEA (0.05 ml, 0.3 mmol, 3 eq), and 1-(3-((4-fluorophenyl)ethynyl)-4-(piperazin-1-yl)phenyl)-3-(2-(pyridin-3-yl)ethyl)urea (0.044 g, 0.1 mmol, 1 eq) were added and stirred at room temperature for 24 hours. The reaction mixture was concentrated and purified by column chromatography (silica gel, EA). (0.022 g, 33.3%)

¹H NMR (400 MHz, CDCl3) δ 8.64 - 8.42 (m, 2H), 8.02 (s, 1H), 7.78 (d, J = 7.8 Hz, 1H), 7.72 (s, 2H), 7.52 (s, J = 8.4 Hz, 1H), 7.49 - 7.33 (m, 8H), 7.20 (d, J = 7.6 Hz, 1H), 7.11 - 7.03 (m, 4H), 6.93 (s, 1H), 6.85 (d, J = 8.4 Hz, 1H), 6.68 (d, J = 12.4 Hz, 2H), 5.07 (s, 1H), 3.98 (s, 2H), 3.64 (s, 2H), 3.55 (s, 2H), 3.24 (s, 2H), 3.13 (s, 2H), 2.92 (s, 2H), 1.54 (s, 9H).

### Step 2 (b) Synthesis of 1-(4-(4-(3-aminobenzoyl)piperazin-1-yl)-3-((4-fluorophenyl)ethynyl)phenyl)-3-(2-(pyridin-3-yl)ethyl)urea

Tert-butyl (3-(4-(2-((4-fluorophenyl)ethynyl)-4-(3-(2-(pyridin-3-yl)ethyl)ureido)phenyl)piperazin-1-carbonyl)phenyl)carbamate (0.05 g, 0.075 mmol, 1 eq) was dissolved in 1,4-dioxane (1 ml), and then 4N HCl in dioxane (0.1 ml, 0.377 mmol, 5 eq) was added and stirred overnight. Saturated sodium bicarbonate (Sat. NaHCO₃) aqueous solution was added, neutralized and extracted with EA. The organic layer was dried over MgSO₄, filtered, concentrated and then purified by column chromatography (silica gel, MeOH/DCM) to obtain the title compound (0.018 g, 42.8%).

¹H NMR (400 MHz, MeOD) δ 8.46 (s, 1H), 8.41 (d, J = 4.2 Hz, 1H), 7.79 (d, J = 7.8 Hz, 1H), 7.61 - 7.52 (m, 3H), 7.44 - 7.39 (m, 1H), 7.27 (dd, J = 8.8, 2.6 Hz, 1H), 7.22 - 7.12 (m, 3H), 7.01 - 6.97 (m, 1H), 6.80 (dd, J = 7.7, 1.9 Hz, 1H), 6.77 - 6.74 (m, 1H), 6.72 - 6.68 (m, 1H), 3.95 (s, 2H), 3.67 (s, 2H), 3.48 (t, J = 7.0 Hz, 2H), 3.24 (s, 2H), 3.13 (s, 2H), 2.90 (t, J = 7.0 Hz, 2H).

### Example 15: Synthesis of compound 186 (synthesis reaction formula 10)

### Synthesis of 1-(4-(3-(2-aminoethyl)-2-methyl-4-oxo-3,4-dihydroquinazolin-7-yl)-3-((4-fluorophenyl)ethynyl)phenyl)-3-(2-(pyridin-3-yl)ethyl)urea

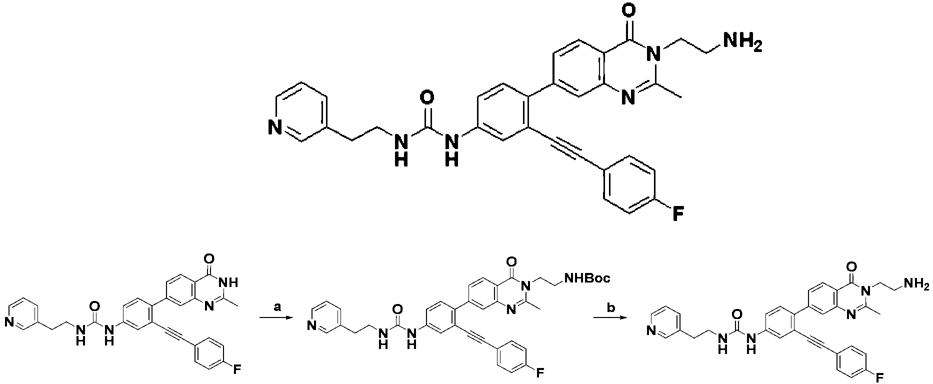

Reaction Formula 10. (a) tert-butyl (2-bromoethyl)carbamate, Cs₂CO₃, DMF, 80°C, 24 hours; (b) 4N HCl in dioxane, 1,4-dioxane, RT, 24 hours

### Step 1 (a) Synthesis of tert-butyl (2-(7-(2-((4-fluorophenyl)ethynyl)-4-(3-(2-(pyridin-3-yl)ethyl)ureido)phenyl)-2-methyl-4-oxoquinazolin-3(4H)-yl)ethyl)carbamate

1-(3-((4-fluorophenyl)ethynyl)-4-(2-methyl-4-oxo-3,4-dihydroquinazolin-7-yl)phenyl)-3-(2-(pyridin-3-yl)ethyl)urea (0.064 g, 0.124 mmol, 1.0 eq) was dissolved in DMF (1 mL), and then Cs₂CO₃ (0.081 g, 0.248 mmol, 1.2 eq) and tert-butyl (2-bromoethyl)carbamate (0.033 g, 0.15 mmol, 1.2 eq) were added and stirred at 80°C overnight. After completion of the reaction, the resulting mixture was extracted with EA and H₂O. The organic layer was dried over MgSO₄, filtered, concentrated and then purified by column chromatography (silica gel, DCM/MeOH) to obtain the title compound (0.049 g, 59.8%).

¹H NMR (400 MHz, CDCl3) δ 8.49 (s, 2H), 8.24 (d, J = 8.3 Hz, 1H), 7.84 (s, 1H), 7.77 (d, J = 9.5 Hz, 1H), 7.68 (s, 2H), 7.54 - 7.48 (m, 1H), 7.37 (s, 2H), 7.35 - 7.29 (m, 3H), 7.04 (t, J = 8.7 Hz, 1H), 7.00 - 6.92 (m, 3H), 5.22 (s, 1H), 5.00 (t, J = 5.9 Hz, 1H), 4.27 (t, J = 6.0 Hz, 2H), 3.58 (s, 2H), 3.54 - 3.47 (m, 2H), 2.92 - 2.84 (m, 2H), 2.72 (s, 3H), 1.39 (s, 9H).

### Step 2 (b) Synthesis of 1-(4-(3-(2-aminoethyl)-2-methyl-4-oxo-3,4-dihydroquinazolin-7-yl)-3-((4-fluorophenyl)ethynyl)phenyl)-3-(2-(pyridin-3-yl)ethyl)urea

Tert-butyl (2-(7-(2-((4-fluorophenyl)ethynyl)-4-(3-(2-(pyridin-3-yl)ethyl)ureido)phenyl)-2-methyl-4-oxoquinazolin-3(4H)-yl)ethyl)carbamate (0.05 g, 0.075 mmol, 1 eq) was dissolved in 1,4-dioxane (1 ml), and then 4N HCl in dioxane (0.1 ml, 0.377 mmol, 5 eq) was added and stirred overnight. Saturated sodium bicarbonate (Sat. NaHCO₃) aqueous solution was added, neutralized and extracted with EA. The organic layer was dried over MgSO₄, filtered, concentrated and then purified by preparative HPLC (0.1% formic acid/acetonitrile) to obtain the title compound (0.002 g, 0.5%).

¹H NMR (400 MHz, MeOD) δ 8.50 (s, 5H), 8.44 (s, 1H), 8.30 (d, J = 8.3 Hz, 1H), 7.93 (s, 1H), 7.80 (dd, J = 12.0, 5.0 Hz, 3H), 7.51 - 7.36 (m, 5H), 7.08 (t, J = 8.7 Hz, 2H), 4.49 (t, J = 6.2 Hz, 2H), 3.62 (s, 1H), 3.53 (t, J = 7.0 Hz, 2H), 3.41 - 3.36 (m, 2H), 2.94 (t, J = 6.9 Hz, 2H), 2.73 (s, 3H).

### Example 16: Synthesis of compound 40 (synthesis reaction formula 11)

### Synthesis of N-(2-(3-aminopiperidin-1-yl)ethyl)-4-((5-(3-(2-(pyridin-3-yl)ethyl)ureido)-2-(pyridin-4-yl)phenyl)ethynyl)benzamide

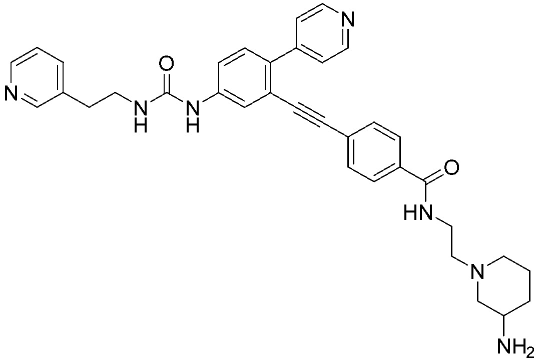

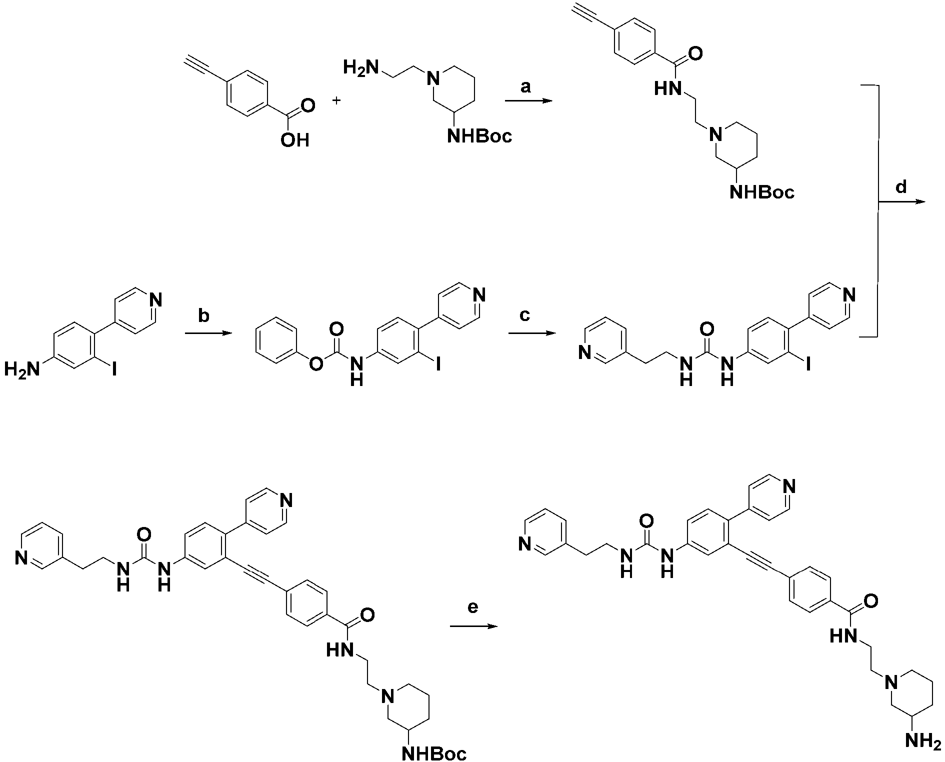

Reaction Formula 11. Reagents and conditions: (a) HATU, DIPEA, DMF, RT, overnight; (b) phenyl chloroformate, pyridine, THF, 0°C, 2 hours; (c) 2-(pyridin-3-yl)ethan-1-amine, TEA, THF, reflux, 24 hours; (d) PdCl₂(PPh₃)₂, CuI, TEA, ACN, 60°C, 5 hours; (e) 4N HCl in dioxane, 1,4-dioxane, RT, 2 hours

### Step 1 (a) Synthesis of tert-butyl (1-(2-(4-ethynylbenzamido)ethyl)piperidin-3-yl)carbamate

4-ethynylbenzoic acid (0.15 g, 1 mmol, 1 eq) was dissolved in DMF (3 ml, 0.3 M), and then tert-butyl (1-(2-aminoethyl)piperidin-3-yl)carbamate (0.243 g, 1 mmol, 1.5 eq), HATU (0.456 g, 1.2 mmol, 1.2 eq) and DIPEA (0.52 ml, 3 mmol, 3 eq) were added and stirred at room temperature overnight. The resulting mixture was washed with water and filtered to obtain the title compound (0.371g, 74%).

¹H NMR (400 MHz, CDCl3) δ 7.74 (d, J = 8.3 Hz, 2H), 7.55 (d, J = 8.3 Hz, 2H), 6.76 (s, 1H), 4.72 (s, 1H), 3.72 (s, 1H), 3.53 (dt, J = 10.5, 5.3 Hz, 2H), 2.75 (s, 1H), 2.56 (t, J = 5.3 Hz, 2H), 2.36 - 2.09 (m, 2H), 1.84 - 1.69 (m, 3H), 1.43 (s, 9H).

### Step 2 (b) Synthesis of phenyl (3-iodo-4-(pyridin-4-yl)phenyl)carbamate

3-iodo-4-(pyridin-4-yl)aniline (0.592 g, 2 mmol, 1 eq) was dissolved in THF (20 ml, 0.1 M), and then pyridine (0.32 ml g, 4 mmol, 2 eq) and phenyl chloroformate (0.38 ml, 3 mmol, 1.5 eq) were added dropwise and stirred. After completion of the reaction, the resulting mixture was concentrated and extracted with EA and H₂O. The organic layer was dried over MgSO₄, and then subjected to the following reaction of step 3) without purification.

¹H NMR (400 MHz, CDCl3) δ 8.79 (d, J = 6.0 Hz, 2H), 8.20 (s, 1H), 7.91 (d, J = 6.2 Hz, 2H), 7.63 (d, J = 8.0 Hz, 1H), 7.38 - 7.33 (m, 3H), 7.24 (d, J = 8.6 Hz, 1H), 7.13 (d, J = 7.6 Hz, 2H).

### Step 3 (c) Synthesis of 1-(3-iodo-4-(pyridin-4-yl)phenyl)-3-(2-(pyridin-3-yl)ethyl)urea

Phenyl (3-iodo-4-(pyridin-4-yl)phenyl)carbamate (1.14 g, 2 mmol, 1 eq) was dissolved in THF (20 ml, 0.1 M), and then 2-(pyridin-3-yl)ethan-1-amine (0.47 ml, 4 mmol, 2 eq) and TEA (0.84 ml, 6 mmol, 3 eq) were added and stirred at 80°C for four hours. The reaction mixture was concentrated and purified by column chromatography (silica gel, MC/MeOH) to obtain the title compound (0.552, 62%).

¹H NMR (400 MHz, CDCl3) δ 8.62 (d, J = 5.9 Hz, 2H), 8.40 (s, 2H), 7.93 (d, J = 2.0 Hz, 1H), 7.62 (d, J = 7.8 Hz, 1H), 7.51 (s, 1H), 7.41 (dd, J = 8.4, 2.1 Hz, 1H), 7.31 - 7.27 (m, 1H), 7.24 (s, 1H), 7.12 (d, J = 8.3 Hz, 1H), 5.42 (t, J = 5.6 Hz, 1H), 3.58 (q, J = 6.2 Hz, 2H), 2.89 (t, J = 6.5 Hz, 2H).

### Step 4 (d) Synthesis of tert-butyl (1-(2-(4-((5-(3-(2-(pyridin-3-yl)ethyl)ureido)-2-(pyridin-4-yl)phenyl)ethynyl)benzamido)ethyl)piperidin-3-yl)carbamate

1-(3-iodo-4-(pyridin-4-yl)phenyl)-3-(2-(pyridin-3-yl)ethyl)urea (0.044 g, 0.1 mmol, 1 eq) was dissolved in ACN (1 ml, 0.1 M), and then TEA (0.03 ml, 0.22 mmol, 2.2 eq), PdCl₂(PPh₃)₂ (2.1 mg, 0.003 mmol, 3 mol%), and CuI (0.8 mg, 0.004 mmol, 4 mol%) were added and stirred at room temperature for five minutes. Tert-butyl (1-(2-(4-ethynylbenzamido)ethyl)piperidin-3-yl)carbamate (0.045 g, 0.12 mmol, 1.2 eq) was added and stirred at 90°C for two hours. The resulting mixture was cooled to room temperature, concentrated, and purified by column chromatography (silica gel, EA/HX) to obtain the title compound (0.109 g, 97.3%).

¹H NMR (400 MHz, CDCl₃) δ 8.57 (s, 4H), 8.00 (s, 1H), 7.67 (d, J = 8.1 Hz, 2H), 7.62 (t, J = 8.2 Hz, 2H), 7.45 (s, 3H), 7.31 (s, 1H), 7.25 - 7.19 (m, 4H), 5.94 (s, 1H), 5.05 (s, 1H), 3.80 (s, 1H), 3.68 - 3.50 (m, 5H), 3.03 (s, 1H), 2.91 (t, J = 6.5 Hz, 2H), 2.82 (s, 3H), 2.51 (s, 2H), 2.02 (s, 10H), 1.83 (s, 3H), 1.72 (s, 2H), 1.42 (d, J = 19.1 Hz, 12H), 1.25 (s, 2H).

### Step 5 (e) Synthesis of N-(2-(3-aminopiperidin-1-yl)ethyl)-4-((5-(3-(2-(pyridin-3-yl)ethyl)ureido)-2-(pyridin-4-yl)phenyl)ethynyl)benzamide

Tert-butyl (1-(2-(4-((5-(3-(2-(pyridin-3-yl)ethyl)ureido)-2-(pyridin-4-yl)phenyl)ethynyl)benzamido)ethyl)piperidin-3-yl)carbamate (0.03 g, 0.044 mmol, 1 eq) was dissolved in 1,4-dioxane (1 ml), and then 4N HCl in dioxane (0.1 ml, 0.436 mmol, 10 eq) was added and stirred at room temperature for two hours. The resulting mixture was washed with 1,4-dioxane and filtered under reduced pressure to obtain the title compound (0.014 g, 50%).

¹H NMR (400 MHz, MeOD) δ 8.89 (s, 2H), 8.86 (s, 1H), 8.75 (s, 1H), 8.60 (d, J = 8.1 Hz, 1H), 8.41 (d, J = 5.3 Hz, 2H), 8.08 - 8.02 (m, 1H), 7.97 (s, 1H), 7.96 - 7.94 (m, 2H), 7.68 - 7.64 (m, 1H), 7.63 - 7.59 (m, 1H), 7.59 - 7.55 (m, 2H), 3.90 (s, 1H), 3.85 (t, J = 5.6 Hz, 2H), 3.73 (s, 2H), 3.61 (t, J = 6.8 Hz, 2H), 3.49 (t, J = 5.7 Hz, 2H), 3.14 (t, J = 6.7 Hz, 3H), 2.26 - 2.13 (m, 2H), 2.09 - 1.96 (m, 1H), 1.80 - 1.66 (m, 1H), 1.44 (s, 1H).

### Example 17: Synthesis of compound 188 (synthesis reaction formula 12)

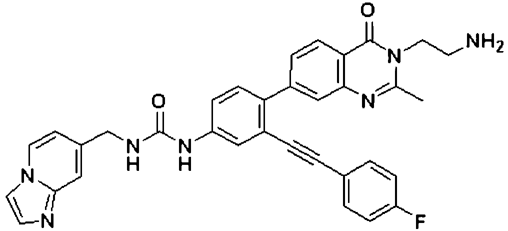

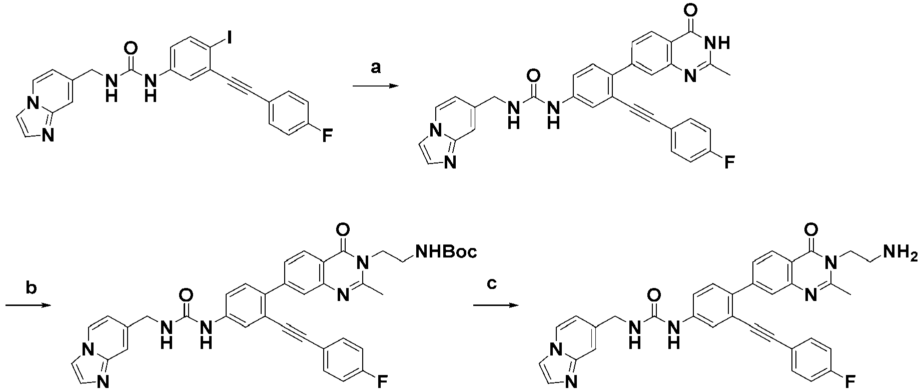

Reaction Formula 12. (a) Pd(dppf)Cl₂, K₂CO₃, 1,4-dioxane, water (H₂O), 90°C, overnight; (b) tert-butyl (2-bromoethyl)carbamate, Cs₂CO₃, DMF, 80°C, 24 hours; (c) 4N HCl in dioxane, 1,4-dioxane, RT, 24 hours

### Step 1 (a) Synthesis of 1-(3-((4-fluorophenyl)ethynyl)-4-(2-methyl-4-oxo-3,4-dihydroquinazolin-7-yl)phenyl)-3-(imidazo[1,2-a]pyridin-7-ylmethyl)urea

The title compound (0.034 g, 31.2%) was prepared in substantially the same manner as in the synthesis of step 6) of compound 14 in Example 11, except that 1-(3-((4-fluorophenyl)ethynyl)-4-iodophenyl)-3-(imidazo[1,2-a]pyridin-7-ylmethyl)urea was used instead of 1-(3-((4-fluorophenyl)ethynyl)-4-iodophenyl)-3-(2-(pyridin-3-yl)ethyl)urea in step 2) compared to the synthesis of compound 14 in Example 11.

1H NMR (400 MHz, MeOD) δ 8.43 (d, J = 7.1 Hz, 1H), 8.26 (d, J = 8.3 Hz, 1H), 7.91 (s, 1H), 7.85 (d, J = 2.0 Hz, 1H), 7.82 (s, 1H), 7.78 (d, J = 8.2 Hz, 1H), 7.57 - 7.50 (m, 3H), 7.49 - 7.45 (m, 1H), 7.36 (dd, J = 8.6, 5.5 Hz, 2H), 7.07 (t, J = 8.8 Hz, 2H), 6.97 (d, J = 7.1 Hz, 1H), 4.52 (s, 2H), 2.50 (s, J = 5.9 Hz, 3H).

### Step 2 (b) Synthesis of tert-butyl (2-(7-(2-((4-fluorophenyl)ethynyl)-4-(3-(imidazo[1,2-a]pyridin-7-ylmethyl)ureido)phenyl)-2-methyl-4-oxoquinazolin-3(4H)-yl)ethyl)carbamate

The title compound (23 mg, 56.1%) was obtained in substantially the same manner as in the synthesis of step 1) of compound 186 in Example 15, except that 1-(3-((4-fluorophenyl)ethynyl)-4-(2-methyl-4-oxo-3,4-dihydroquinazolin-7-yl)phenyl)-3-(imidazo[1,2-a]pyridin-7-ylmethyl)urea was used instead of 1-(3-((4-fluorophenyl)ethynyl)-4-(2-methyl-4-oxo-3,4-dihydroquinazolin-7-yl)phenyl)-3-(2-(pyridin-3-yl)ethyl)urea in step 1) compared to the synthesis of compound 186 in Example 15.

### Step 3 (c) Synthesis of 1-(4-(3-(2-aminoethyl)-2-methyl-4-oxo-3,4-dihydroquinazolin-7-yl)-3-((4-fluorophenyl)ethynyl)phenyl)-3-(imidazo[1,2-a]pyridin-7-ylmethyl)urea

The title compound (10 mg, 86.3%) was obtained in substantially the same manner as in the synthesis of step 2) of compound 186 in Example 15, except that tert-butyl (2-(7-(2-((4-fluorophenyl)ethynyl)-4-(3-(imidazo[1,2-a]pyridin-7-ylmethyl)ureido)phenyl)-2-methyl-4-oxoquinazolin-3(4H)-yl)ethyl)carbamate was used instead of tert-butyl (2-(7-(2-((4-fluorophenyl)ethynyl)-4-(3-(2-(pyridin-3-yl)ethyl)ureido)phenyl)-2-methyl-4-oxoquinazolin-3(4H)-yl)ethyl)carbamate in step 2) compared to the synthesis of compound 186 in Example 15.

1H NMR (400 MHz, MeOD) δ 8.79 (d, J = 7.0 Hz, 1H), 8.43 (d, J = 8.3 Hz, 1H), 8.21 (s, 1H), 8.07 - 8.00 (m, 2H), 7.98 (s, 1H), 7.92 (d, J = 1.9 Hz, 1H), 7.88 (s, 1H), 7.62 - 7.57 (m, 1H), 7.57 - 7.47 (m, 2H), 7.45 - 7.35 (m, 2H), 7.11 (t, J = 8.7 Hz, 2H), 4.67 (s, 2H), 4.60 (t, J = 6.1 Hz, 2H), 3.48 (t, J = 6.0 Hz, 2H), 2.99 (s, 3H).

### Example 18: Synthesis of compound 110 (synthesis reaction formula 13)

### Synthesis of 1-(3-(1H-pyrazol-4-yl)propyl)-3-(3-((4-fluorophenyl)ethynyl)-4-(pyridin-4-yl)phenyl)urea

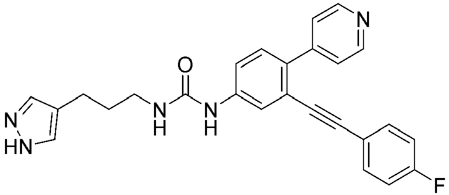

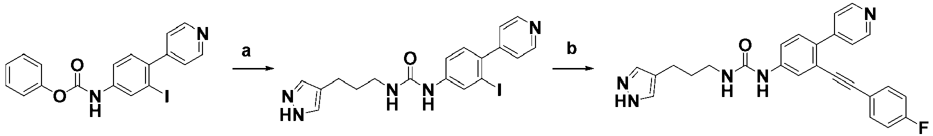

Reaction Formula 13. (a) 3-(1H-pyrazol-4-yl)propan-1-amine, TEA, THF, reflux, 3 hours; (b) 1-ethynyl-4-fluorobenzene, PdCl₂(PPh₃)₂, CuI, TEA, ACN, 90°C, 24 hours;

### Step 1 (a) Synthesis of 1-(3-(1H-pyrazol-4-yl)propyl)-3-(3-iodo-4-(pyridin-4-yl)phenyl)urea

The title compound was prepared in substantially the same manner as in the synthesis of compound 40, except that 3-(1H-pyrazol-4-yl)propan-1-amine was used instead of 2-(pyridin-3-yl)ethan-1-amine in step 3) compared to the synthesis of compound 40 in Example 16. (0.1 g, 93.5%)

¹H NMR (400 MHz, MeOD) δ 8.60 (d, J = 5.5 Hz, 2H), 8.21 (d, J = 2.1 Hz, 1H), 7.53 - 7.42 (m, 5H), 7.24 (d, J = 8.4 Hz, 1H), 3.37 (s, 1H), 3.28 - 3.26 (m, 2H), 2.61 (t, J = 7.6 Hz, 2H), 1.90 - 1.78 (m, 2H).

### Step 2 (b) Synthesis of 1-(3-(1H-pyrazol-4-yl)propyl)-3-(3-((4-fluorophenyl)ethynyl)-4-(pyridin-4-yl)phenyl)urea

1-(3-(1H-pyrazol-4-yl)propyl)-3-(3-iodo-4-(pyridin-4-yl)phenyl)urea (0.1 g, 0.22 mmol, 1 eq) was dissolved in ACN (2 ml, 0.1 M), and then TEA (0.07 ml, 0.48 mmol, 2.2 eq), PdCl₂(PPh₃)₂ (4.6 mg, 0.007 mmol, 3 mol%), and CuI (2 mg, 0.009 mmol, 4 mol%) were added and stirred at room temperature for five minutes. 1-ethynyl-4-fluorobenzene (0.030 ml, 0.27 mmol, 1.2 eq) was added and stirred at 90°C for two hours. The resulting mixture was cooled to room temperature, and then concentrated and purified by column chromatography (C18, 0.1% formic acid in H₂O/acetonitrile) to obtain the title compound (2.8 mg, 3%).

¹H NMR (400 MHz, MeOD) δ 8.62 (s, 2H), 7.82 (d, J = 2.2 Hz, 1H), 7.74 (d, J = 6.0 Hz, 2H), 7.70 - 7.63 (m, 2H), 7.61 - 7.57 (m, 1H), 7.53 - 7.49 (m, 1H), 7.47 - 7.44 (m, 1H), 7.42 - 7.38 (m, 2H), 7.12 (t, J = 8.8 Hz, 2H), 3.28 (t, J = 7.0 Hz, 2H), 2.62 (t, J = 7.5 Hz, 2H), 1.90 - 1.81 (m, 2H).

### Example 19: Synthesis of compound 57 (synthesis reaction formula 14)

### Synthesis of 1-(3-((4-fluorophenyl)ethynyl)-4-(isoquinolin-6-yl)phenyl)-3-(2-(pyridin-3-yl)ethyl)urea

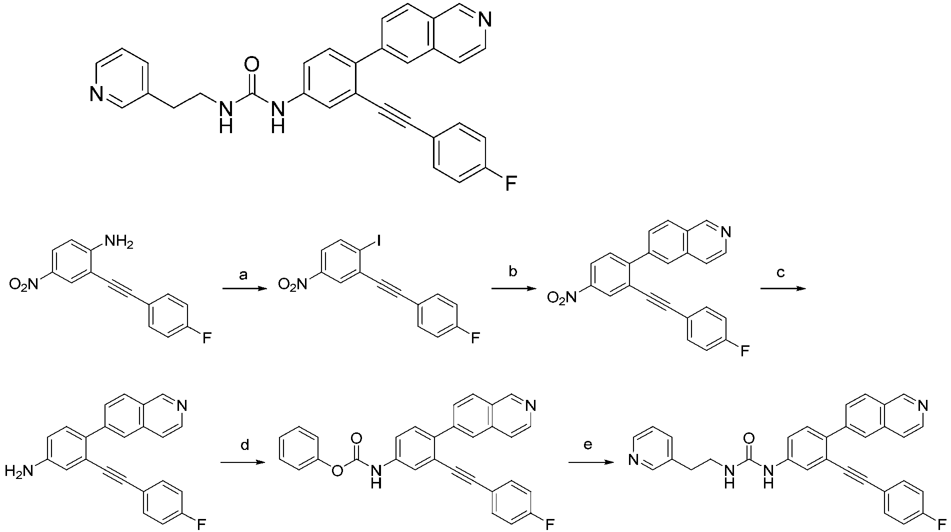

Reaction Formula 14. (a) NaCO₂, KI, pTSA, ACN, 0°C, 1 hour; (b) Pd(dppf)Cl₂, K₂CO₃, 1,4-dioxane, water (H₂O), 90°C, 3 hours; (c) Fe, NH₄Cl, EtOH, water (H₂O), reflux, 1 hour; (d) phenyl chloroformate, pyridine, THF, 0°C to RT, 2 hours; (e) 2-(pyridin-3-yl)ethan-1-amine, TEA, THF, RT, reflux

### Step 1 (a) Synthesis of 2-((4-fluorophenyl)ethynyl)-1-iodo-4-nitrobenzene

2-((4-fluorophenyl)ethynyl)-4-nitroaniline (0.1 g, 0.39 mmol, 1 eq) was dissolved in ACN (4 ml, 0.1M), and then pTSA (0.2 g, 1.17 mmol, 3 eq) was added, and then NaNO₂ (0.03 g, 0.408 mmol, 1.02 eq) dissolved in 0.15 ml of water and KI (0.13, 0.78 mmol, 2 eq) dissolved in 0.15 ml of water were added at 0°C. After reaction at room temperature for one hour, saturated sodium bicarbonate (Sat. NaHCO₃) aqueous solution was added, neutralized to pH 8 or higher, and extracted with EA. The organic layer was dried over MgSO₄, filtered and concentrated to obtain the title compound as a crude product.

¹H NMR (400 MHz, CDCl3) δ 8.31 (d, J = 2.6 Hz, 1H), 8.07 (d, J = 8.7 Hz, 1H), 7.83 (dd, J = 8.7, 2.7 Hz, 1H), 7.64 - 7.59 (m, 2H), 7.10 (t, J = 8.6 Hz, 2H)

### Step 2 (b) Synthesis of 6-(2-((4-fluorophenyl)ethynyl)-4-nitrophenyl)isoquinoline

2-((4-fluorophenyl)ethynyl)-1-iodo-4-nitrobenzene (0.14 g, 0.38 mmol, 1 eq) was dissolved in 1,4-dioxane:water (H₂O) (3:1 (v/v), 2 ml, 0.2 M), and then isoquinolin-6-ylboronic acid (0.066 g, 0.38 mmol, 1 eq), Pd(dppf)Cl₂ (0.056 g, 0.076 mmol, 0.2 eq), and K₂CO₃ (0.116 g, 0.836 mmol, 2.2 eq) were added and degassed with nitrogen (N₂) gas. The reaction solution was heated to 90°C and stirred for three hours. After completion of the reaction, the resulting mixture was filtered through a celite filter, and extracted with EA and H₂O. The organic layer was dried over MgSO₄, filtered, concentrated and then purified by column chromatography (silica gel, EA/Hx) to obtain the title compound (0.032 g, 23%).

¹H NMR (400 MHz, MeOD) δ 9.37 (s, 1H), 8.58 - 8.52 (m, 2H), 8.38 (d, J = 8.5 Hz, 1H), 8.31 (s, 2H), 8.06 (d, J = 8.5 Hz, 1H), 7.97 (d, J = 5.7 Hz, 1H), 7.87 (d, J = 8.8 Hz, 1H), 7.38 - 7.30 (m, 2H), 7.08 (t, J = 7.9 Hz, 2H). MS(ESI) m/z MH+ 369

### Step 3 (c) Synthesis of 3-((4-fluorophenyl)ethynyl)-4-(isoquinolin-6-yl)aniline

A mixture of 6-(2-((4-fluorophenyl)ethynyl)-4-nitrophenyl)isoquinoline (0.032 g, 0.087 mmol, 1.0 eq), Fe (0.024 g, 0.434 mmol, 5 eq) and NH₄Cl (0.023 g, 0.434 mmol, 5 eq) was stirred at 80°C for one hour. The catalyst was removed by celite filtration, and the filtrate was concentrated under reduced pressure. Water was added and extracted with EA, and then the organic layer was concentrated under reduced pressure. The target compound was obtained as a crude product.

### Step 4 (d) Synthesis of phenyl (3-((4-fluorophenyl)ethynyl)-4-(isoquinolin-6-yl)phenyl)carbamate

The title compound was prepared in substantially the same manner as in the synthesis of step 5) of compound 1 in Example 1, except that 6-(2-((4-fluorophenyl)ethynyl)-4-nitrophenyl)isoquinoline was used instead of 3-((4-fluorophenyl)ethynyl)-4-(pyridin-4-yl)aniline in step 5) compared to the synthesis of compound 1 in Example 1.

MS(ESI) m/z MH+ 459

### Step 5 (e) Synthesis of 1-(3-((4-fluorophenyl)ethynyl)-4-(isoquinolin-6-yl)phenyl)-3-(2-(pyridin-3-yl)ethyl)urea

The title compound was prepared in substantially the same manner as in the synthesis of step 6) of compound 1 in Example 1, except that phenyl (3-((4-fluorophenyl)ethynyl)-4-(isoquinolin-6-yl)phenyl)carbamate was used instead of phenyl (3-((4-fluorophenyl)ethynyl)-4-(pyridin-4-yl)phenyl)carbamate in step 6) compared to the synthesis of compound 1 in Example 1.

¹H NMR (400 MHz, MeOD) δ 9.29 (s, 1H), 8.53 - 8.46 (m, 2H), 8.43 (s, 1H), 8.23 - 8.16 (m, 2H), 8.02 (d, J = 8.4 Hz, 1H), 7.90 (d, J = 5.3 Hz, 1H), 7.86 - 7.79 (m, 2H), 7.54 - 7.47 (m, 2H), 7.47 - 7.40 (m, 1H), 7.34 - 7.26 (m, 2H), 7.05 (t, J = 8.2 Hz, 2H), 3.54 (t, J = 6.8 Hz, 2H), 2.95 (t, J = 6.4 Hz, 2H). MS(ESI) m/z MH+ 487

### Example 20: Synthesis of compound 182 (synthesis reaction formula 15)

### Synthesis of 1-(3-((4-fluorophenyl)ethynyl)-4-(pyridin-4-yl)phenyl)-3-(2-(6-nitropyridin-3-yl)ethyl)urea

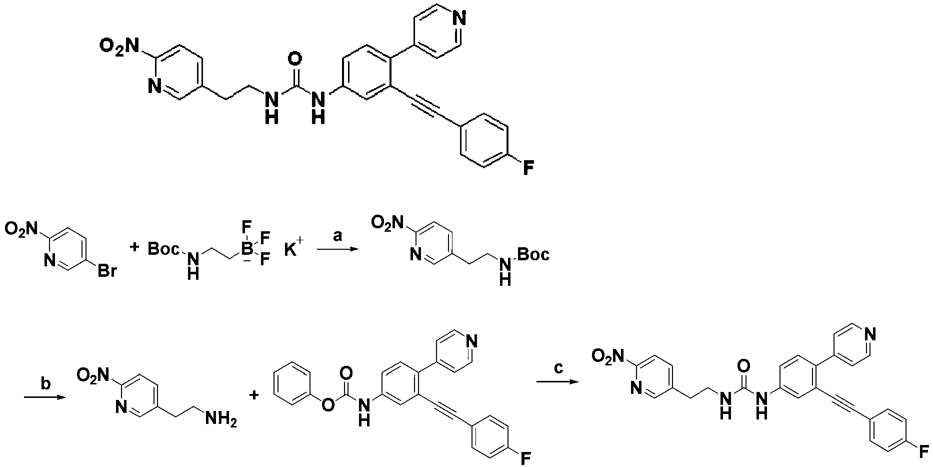

Reaction Formula 15. Reagents and conditions: (a) Pd(dppf)Cl₂, Cs₂CO₃, toluene, water (H₂O), 80°C, 16 hours; (b) 4N HCl in dioxane, 1,4-dioxane, RT, 4 hours; (c) triethylamine (TEA), THF, RT, overnight

### Step 1 (a) Synthesis of tert-butyl (2-(6-nitropyridin-3-yl)ethyl)carbamate

5-bromo-2-nitro-pyridine (1 g, 4.93 mmol, 1.0 eq) was dissolved in toluene (20 ml, 0.25 M) and water (H₂O, 5 ml, 1 M), and then potassium 2-(Boc-aminoethyl)trifluoroborate (1.36 g, 5.41 mmol, 1.1 eq), Pd(dppf)Cl₂ (0.302 g, 0.369 mmol, 0.07 eq), and Cs₂CO₃ (4.82 g, 14.8 mmol, 3 eq) were added. The reactant was heated to 80°C and stirred for 16 hours. After completion of the reaction, the resulting mixture was cooled to room temperature and extracted with EA and H₂O. The organic layer was dried over MgSO₄, filtered, concentrated and then purified by column chromatography (silica gel, EA/HX) to obtain the title compound (0.842 g, 64%).

### Step 2 (b) Synthesis of 2-(6-nitropyridin-3-yl)ethan-1-amine

Tert-butyl (2-(6-nitropyridin-3-yl)ethyl)carbamate (0.84 g, 3.15 mmol, 1 eq) was dissolved in 1,4-dioxane (6 ml, 0.5 M), and then 4N HCl in dioxane (8 ml, 31.5 mmol, 10 eq) was added and stirred at room temperature for 16 hours. After completion of the reaction, the resulting mixture was concentrated and added with diethyl ether. The resulting crystal was washed with ether and filtered under reduced pressure to obtain the title compound (0.507 g, 79%).

### Step 3 (c) Synthesis of 1-(3-((4-fluorophenyl)ethynyl)-4-(pyridin-4-yl)phenyl)-3-(2-(6-nitropyridin-3-yl)ethyl)urea

Phenyl (3-((4-fluorophenyl)ethynyl)-4-(pyridin-4-yl)phenyl)carbamate (0.081 g, 0.20 mmol, 1 eq) was dissolved in THF (1 ml, 0.2 M), and then 2-(6-nitropyridin-3-yl)ethan-1-amine (0.082 mg, 0.4 mmol, 2 eq) and TEA (0.84 ml, 0.6 mmol, 3 eq) were added and stirred at room temperature for 16 hours. After completion of the reaction, the resulting mixture was concentrated and purified by column chromatography (silica gel, MeOH/DCM) to obtain the title compound (0.113 g, 23%).

¹H NMR (400 MHz, CDCl3) δ 8.67 (d, *J=* 4.2 Hz, 2H), 8.49 (s, 1H), 8.17 (d, *J =* 8.2 Hz, 1H), 7.93 (d, *J=* 8.2 Hz, 1H), 7.74 (s, 1H), 7.64 (d, *J=* 5.1 Hz, 3H), 7.39 (d, *J=* 8.5 Hz, 1H), 7.31 (d, *J =* 8.2 Hz, 2H), 6.99 (t, *J =* 8.2 Hz, 2H), 5.77 (s, 1H), 3.65 (d, *J =* 6.1 Hz, 2H), 3.08 (t, *J =* 6.3 Hz, 2H); MS(ESI) MH⁺ 482.4

### Example 21: Synthesis of compound 10 (synthesis reaction formula 16)

### Synthesis of 1-(2-(6-aminopyridin-3-yl)ethyl)-3-(3-((4-fluorophenyl)ethynyl)-4-(pyridin-4-yl)phenyl)urea

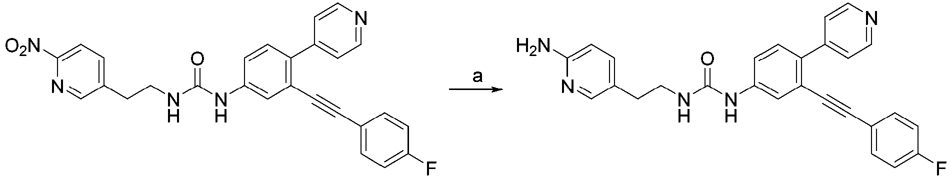

### Reaction Formula 16. Reagents and conditions: (a) Zn, NH₄Cl, 1,4-dioxane, water (H₂O), RT, overnight

1-(3-((4-fluorophenyl)ethynyl)-4-(pyridin-4-yl)phenyl)-3-(2-(6-nitropyridin-3-yl)ethyl)urea (0.048 g, 0.1 mmol, 1 eq) was dissolved in 1,4-dioxane:water (H₂O) (3:1 (v/v), 0.5 ml, 0.2 M), and then Zn (0.065 g, 1 mmol, 10 eq) and NH₄Cl (0.053 g, 1 mmol, 10 eq) were added and stirred at room temperature overnight. The resulting mixture was washed with MeOH, filtered under reduced pressure, concentrated and then purified by reverse-phase column chromatography (0.1% formic acid in H₂O/ACN) to obtain the title compound (0.001 g, 2.4%).

¹H NMR (400 MHz, MeOD) δ 8.62 (s, 2H), 7.80 (s, 2H), 7.73 (d, J = 4.5 Hz, 2H), 7.61 (d, J = 8.7 Hz, 1H), 7.46 (dd, J = 18.2, 8.6 Hz, 2H), 7.42 - 7.37 (m, 2H), 7.12 (t, J = 8.3 Hz, 2H), 6.74 (d, J = 8.5 Hz, 1H), 3.45 (t, J = 6.8 Hz, 2H), 2.75 (t, J = 6.8 Hz, 2H); MS MH⁻ 450.39

### Example 22: Synthesis of compound 47 (synthesis reaction formula 17)

### Synthesis of N-(2-(piperazin-1-yl)ethyl)-4-((5-(3-(2-(pyridin-3-yl)ethyl)ureido)-2-(pyridin-4-yl)phenyl)ethynyl)benzamide

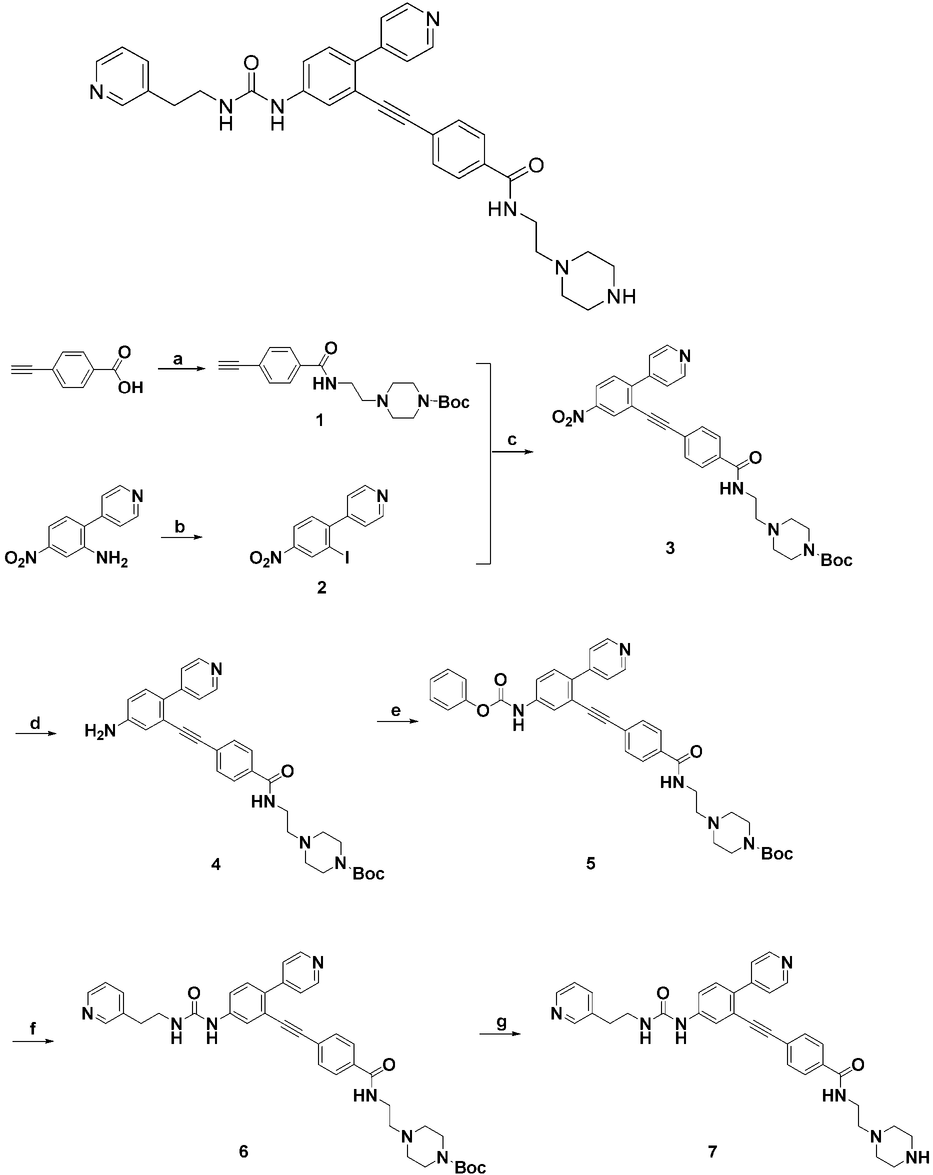

### Reaction Formula 17. Reagents and conditions: (a) tert-butyl 4-(2-aminoethyl)piperazine-1-carboxylate, HATU, DIPEA, DMF, RT, overnight; (b) NaNO₂, KI, HCl, H₂O, 5°C, 1 hour; (c) PdCl₂(PPh₃)₂, CuI, TEA, ACN, 60°C, 5 hours; (d) Zn, NH₄Cl, 1,4-dioxane, water (H₂O), RT, overnight; (e) phenyl chloroformate, pyridine, THF, 0°C to RT, overnight; (f) 2-(pyridin-3-yl)ethan-1-amine, TEA, THF, RT, overnight; (g) TFA, DCM, RT, overnight

### Step 1 (a) Synthesis of tert-butyl 4-(2-(4-ethynylbenzamido)ethyl)piperazine-1-carboxylate

4-ethynylbenzoic acid (0.25 g, 1.71 mmol, 1 eq) was dissolved in DMF (11 ml, 0.3 M), and then tert-butyl 4-(2-aminoethyl)piperazin-1-carboxylate (0.589 g, 2.57 eq, 1.5 eq), HATU (0.977 g, 2.57 mmol, 1.5 eq) and DIPEA (0.663 g, 5.13 mmol, 3 eq) were added and stirred at room temperature overnight. After extracting with EA and H₂O, the organic layer was dried over MgSO₄, filtered, concentrated and then purified by column chromatography (silica gel, MeOH/DCM) to obtain the title compound (0.382 g, 63%).

### Step 2 (b) Synthesis of 4-(2-iodo-4-nitrophenyl)pyridine

4.5 ml of water and 4.5 ml of concentrated HCl were added to 5-nitro-2-(pyridin-4-yl)aniline (1.00 g, 4.64 mmol, 1 eq), and then NaNO₂ (0.326 g, 4.73 mmol, 1.0 eq) dissolved in 4.5 ml of water at 5°C was slowly added dropwise and stirred. The resulting mixture was stirred for 10 minutes after the addition, and KI (1.464 g, 8.82 mmol, 2 eq) dissolved in 4.5 ml of water was added, warmed to room temperature, and stirred for 30 minutes. K₂CO₃ aqueous solution was added, neutralized to pH 8 or higher, and extracted with DCM. The organic layer was dried over MgSO₄, filtered, concentrated and then purified by column chromatography (silica gel, Hex/EA) to obtain the title compound (0.952 g, 62.9%).

### Step 3 (c) Synthesis of tert-butyl 4-(2-(4-((5-nitro-2-(pyridin-4-yl)phenyl)ethynyl)benzamido)ethyl)piperazin-1-carboxylate

4-(2-iodo-4-nitrophenyl)pyridine (0.232 g, 0.71 mmol, 1 eq), tert-butyl 4-(2-(4-ethynylbenzamido)ethyl)piperazin-1-carboxylate (0.382 g, 1.07 mmol, 1.5 eq), PdCl₂(PPh₃)₂ (0.014 g, 0.02 mmol, 0.03 eq), CuI (0.0038 g, 0.02 mmol, 0.03 eq), and TEA (0.144 g, 1.42 mmol, 2 eq) were dissolved in ACN (2 ml, 0.3 M) and degassed with nitrogen (N₂) gas. The reactant was heated to 60°C and stirred for five hours. After completion of the reaction, the resulting mixture was extracted with EA and H₂O. The organic layer was dried over MgSO₄, filtered, concentrated and then purified by column chromatography (silica gel, DCM/MeOH) to obtain the title compound (0.25 g, 65%).

### Step 4 (d) Synthesis of tert-butyl 4-(2-(4-((5-amino-2-(pyridin-4-yl)phenyl)ethynyl)benzamido)ethyl)piperazin-1-carboxylate

Tert-butyl 4-(2-(4-((5-nitro-2-(pyridin-4-yl)phenyl)ethynyl)benzamido)ethyl)piperazin-1-carboxylate (0.255 g, 0.46 mmol, 1 eq) was dissolved in 1,4-dioxane:water (H₂O) (3:1 (v/v), 4.6 ml, 0.1 M), and then Zn (0.3 g, 4.6 mmol, 10 eq) and NH₄Cl (0.246 g, 4.6 mmol, 10 eq) were added and stirred at room temperature overnight. The resulting mixture was washed with MeOH and filtered under reduced pressure. The filtrate was concentrated and then subjected to the following reaction of step 5) without purification.

### Step 5 (e) Synthesis of tert-butyl 4-(2-(4-((5-((phenoxycarbonyl)amino)-2-(pyridin-4-yl)phenyl)ethynyl)benzamido)ethyl)piperazine-1-carboxylate

Tert-butyl 4-(2-(4-((5-amino-2-(pyridin-4-yl)phenyl)ethynyl)benzamido)ethyl)piperazin-1-carboxylate (0.023 g, 0.043 mmol, 1 eq) was dissolved in DMF (0.3 ml, 0.1 M), and then pyridine (0.01 g, 0.129 mmol, 3 eq) was added. After cooling to 0°C, phenyl chloroformate (0.007 g, 0.0473 mmol, 1.1 eq) was added dropwise. After slowly heating to room temperature, the resulting mixture was stirred for two hours. After completion of the reaction, the resulting mixture was concentrated, neutralized with 1N NaOH, and then extracted with EA and H₂O. The organic layer was dried over MgSO₄, filtered, concentrated and then purified by column chromatography (silica gel, DCM/MeOH) to obtain the title compound (0.020 g, 72%).

### Step 6 (f) Synthesis of tert-butyl 4-(2-(4-((5-(3-(2-(pyridin-3-yl)ethyl)ureido)-2-(pyridin-4-yl)phenyl)ethynyl)benzamido)ethyl)piperazine-1-carboxylate

Tert-butyl 4-(2-(4-((5-((phenoxycarbonyl)amino)-2-(pyridin-4-yl)phenyl)ethynyl)benzamido)ethyl)piperazine-1-carboxylate (0.030 g, 0.046 mmol, 1 eq) was dissolved in THF (1 ml, 0.05 M), and then 2-(pyridin-3-yl)ethan-1-amine (0.011 g, 0.092 mmol, 2 eq) and TEA (0.014 g, 0.138 mmol, 3 eq) were added and stirred at room temperature overnight. After completion of the reaction, the resulting mixture was concentrated and purified by column chromatography (silica gel, DCM/MeOH) to obtain the title compound (0.021 g, 70%).

### Step 7 (g) Synthesis of N-(2-(piperazin-1-yl)ethyl)-4-((5-(3-(2-(pyridin-3-yl)ethyl)ureido)-2-(pyridin-4-yl)phenyl)ethynyl)benzamide

Tert-butyl 4-(2-(4-((5-(3-(2-(pyridin-3-yl)ethyl)ureido)-2-(pyridin-4-yl)phenyl)ethynyl)benzamido)ethyl)piperazin-1-carboxylate (0.064 g, 0.094 mmol, 1 eq) was dissolved in DCM (1 ml, 0.1 M), and then TFA (0.107 g, 0.94 mmol, 10 eq) was added and stirred at room temperature overnight. After completion of the reaction, the resulting mixture was concentrated and purified by reverse-phase column chromatography (0.1% formic acid in H₂O/ACN) to obtain the title compound (0.021 g, 40%).

¹H NMR (400 MHz, MeOD) δ 8.63 (d, *J* = 4.4 Hz, 2H), 8.49 (s, 1H), 8.43 (s, 1H), 7.88 (s, 1H), 7.82 (d, *J =* 7.9 Hz, 4H), 7.74 (d, *J =* 4.7 Hz, 2H), 7.45 (d, *J =* 7.3 Hz, 5H), 3.55 (d, *J* = 10.7 Hz, 4H), 3.23 (s, 4H), 2.94 (t, *J =* 6.8 Hz, 2H), 2.79 (s, 4H), 2.69 (t, *J =* 6.5 Hz, 2H).

### Example 23: Synthesis of compound 95 (synthesis reaction formula 18)

### Synthesis of N-phenyl-2-((4-((2-(piperidin-1-yl)ethyl)carbamoyl)phenyl)ethynyl)-4-(3-(2-(pyridin-3-yl)ethyl)ureido)benzamide

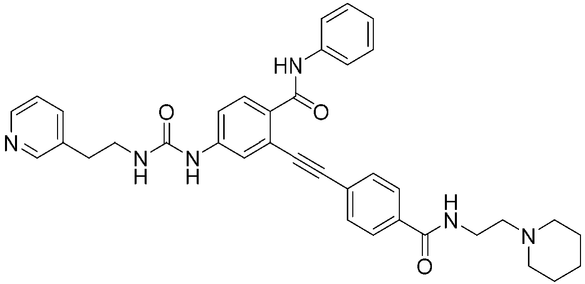

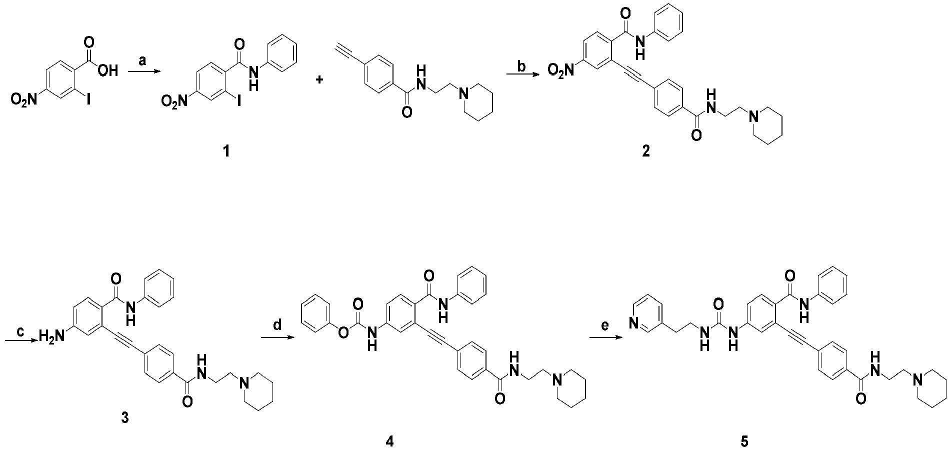

Reaction Formula 18. Reagents and conditions: (a) aniline, EDC, DMAP, DMF, overnight; (b) PdCl₂(PPh₃)₂, CuI, TEA, ACN, 60°C, 5 hours; (c) Zn, NH₄Cl, 1,4-dioxane, water (H₂O), RT, overnight; (d) phenyl chloroformate, pyridine, THF, 0°C to RT, overnight; (e) 2-(pyridin-3-yl)ethan-1-amine, TEA, THF, RT, overnight

### Step 1 (a) Synthesis of 2-iodo-4-nitro-N-phenylbenzamide

2-iodo-4-nitrobenzoic acid (0.5 g, 1.71 mmol, 1 eq) was dissolved in DMF (7 ml, 0.25 M), and then aniline (0.159 g, 1.71 mmol, 1 eq), EDC (0.392 g, 2.05 mmol, 1.2 eq), and DMAP (0.417 g, 3.42 mmol, 2 eq) were added under nitrogen (N₂) gas and stirred at room temperature for 16 hours. After completion of the reaction, 5% LiCl aqueous solution (100 ml) was added, stirred and extracted with DCM. The organic layer was dried over MgSO₄, filtered, concentrated and then purified by column chromatography (silica gel, Hex/EA) to obtain the title compound (0.341 g, 54%).

### Step 2 (b) Synthesis of 4-nitro-N-phenyl-2-((4-((2-(piperidin-1-yl)ethyl)carbamoyl)phenyl)ethynyl)benzamide

2-iodo-4-nitro-N-phenylbenzamide (0.34 g, 0.92 mmol, 1 eq), 4-ethynyl-N-(2-(piperidin-1-yl)ethyl)benzamide (0.353 g, 1.38 mmol, 1.5 eq), PdCl₂(PPh₃)₂ (0.019 g, 0.03 mmol, 0.03 eq), CuI (0.005 g, 0.03 mmol, 0.03 eq), and TEA (0.186 g, 1.84 mmol, 2 eq) were dissolved in ACN (4 ml, 0.25 M), and then degassed with nitrogen (N₂) gas. The reactant was heated to 60°C and stirred for five hours. After completion of the reaction, the resulting mixture was extracted with EA and H₂O. The organic layer was dried over MgSO₄, filtered, concentrated and then purified by column chromatography (silica gel, MeOH/DCM) to obtain the title compound (0.276 g, 60%).

### Step 3 (c) Synthesis of 4-amino-N-phenyl-2-((4-((2-(piperidin-1-yl)ethyl)carbamoyl)phenyl)ethynyl)benzamide

Tert-butyl 4-(2-(4-((5-nitro-2-(pyridin-4-yl)phenyl)ethynyl)benzamido)ethyl)piperazin-1-carboxylate (0.1 g, 0.20 mmol, 1 eq) was dissolved in 1,4-dioxane:water (H₂O) (3:1 (v/v), 2 ml, 0.1 M), and then Zn (0.13 g, 2 mmol, 10 eq) and NH₄Cl (0.106 g, 2 mmol, 10 eq) were added and stirred at room temperature overnight. The resulting mixture was washed with MeOH and filtered under reduced pressure. The filtrate was concentrated and then subjected to the following reaction of step 4) without purification.

### Step 4 (d) Synthesis of phenyl (4-(phenylcarbamoyl)-3-((4-((2-(piperidin-1-yl)ethyl)carbamoyl)phenyl)ethynyl)phenyl)carbamate

4-amino-N-phenyl-2-((4-((2-(piperidin-1-yl)ethyl)carbamoyl)phenyl)ethynyl)benzamide (0.093 g, 0.2 mmol, 1 eq) was dissolved in THF (2 ml, 0.1 M), and then pyridine (0.02 g, 0.25 mmol, 1.25 eq) was added. After cooling to 0°C, phenyl chloroformate (0.056 g, 0.36 mmol, 1.8 eq) was added dropwise. After slowly heating to room temperature, the resulting mixture was stirred for two hours. After completion of the reaction, the resulting mixture was concentrated, neutralized with 1N NaOH, and then extracted with EA and H₂O. The organic layer was dried over MgSO₄, filtered, concentrated and then purified by column chromatography (silica gel, MeOH/DCM) to obtain the title compound (0.052 g, 44%).

### Step 5 (e) Synthesis of N-phenyl-2-((4-((2-(piperidin-1-yl)ethyl)carbamoyl)phenyl)ethynyl)-4-(3-(2-(pyridin-3-yl)ethyl)ureido)benzamide

Phenyl (4-(phenylcarbamoyl)-3-((4-((2-(piperidin-1-yl)ethyl)carbamoyl)phenyl)ethynyl)phenyl)carbamate (0.052 g, 0.089 mmol, 1 eq) was dissolved in THF (1 ml, 0.1 M), and then 2-(pyridin-3-yl)ethan-1-amine (0.022 g, 0.178 mmol, 2 eq) and TEA (0.027 g, 0.267 mmol, 3 eq) were added and stirred at room temperature overnight. After completion of the reaction, the resulting mixture was concentrated and purified by column chromatography (silica gel, MeOH/DCM) to obtain the title compound (0.033 g, 60%).

¹H NMR (400 MHz, MeOD) δ 8.45 (d, J = 22.7 Hz, 2H), 8.18 (s, 1H), 7.96 (d, J = 8.0 Hz, 2H), 7.92 (s, 1H), 7.81 (d, J = 7.6 Hz, 1H), 7.62 (t, J = 7.4 Hz, 2H), 7.56 (d, J = 7.0 Hz, 3H), 7.46 (d, J = 6.5 Hz, 4H), 7.33 (d, J = 8.9 Hz, 1H), 6.23 (s, 1H), 3.83 (s, 2H), 3.51 (t, J = 6.5 Hz, 2H), 3.37 (s, 4H), 2.92 (t, J = 6.5 Hz, 2H), 1.93 (s, 4H), 1.72 (s, 2H)

The reactants may be changed in substantially the same manner as in the synthesis method of above Example 23, so as to synthesize compounds 87, 88, 90, 127, 128, 168, and 176.

### Example 24: Synthesis of compound 184 (synthesis reaction formula 19)

### Synthesis of 4-((2-(3-aminopiperidin-1-yl)-5-(3-(2-(pyridin-3-yl)ethyl)ureido)phenyl)ethynyl)-N-(2-(piperidin-1-yl)ethyl)benzamide

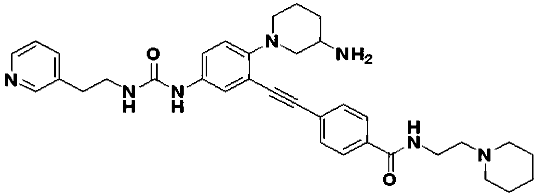

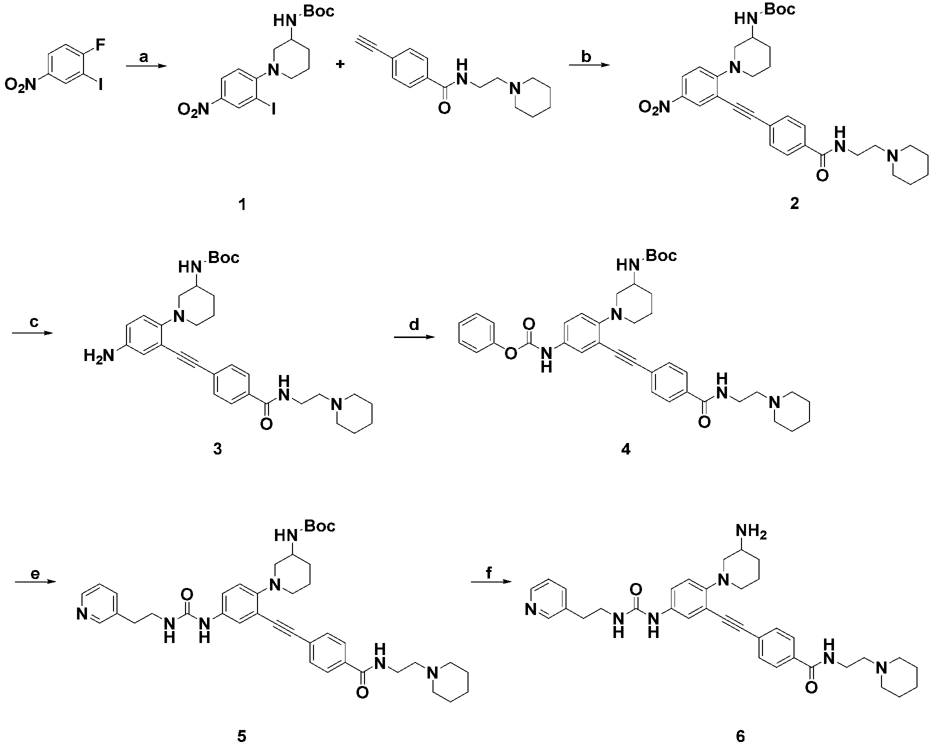

Reaction Formula 19. Reagents and conditions: (a) tert-butyl piperidin-3-ylcarbamate, Cs₂CO₃, DMF, 80°C, overnight; (b) PdCl₂(PPh₃)₂, CuI, TEA, ACN, 60°C, 5 hours; (c) Zn, NH₄Cl, 1,4-dioxane, water (H₂O), RT, overnight; (d) phenyl chloroformate, pyridine, THF, 0°C to RT, overnight; (e) 2-(pyridin-3-yl)ethan-1-amine, TEA, THF, 55°C, overnight; (f) TFA, DCM, RT, overnight

### Step 1 (a) Synthesis of tert-butyl (1-(2-iodo-4-nitrophenyl)piperidin-3-yl)carbamate

1-fluoro-2-iodo-4-nitrobenzene (0.5 g, 1.87 mmol, 1 eq) was dissolved in DMF (3.74 ml, 0.5 M), and then tert-butyl piperidin-3-ylcarbamate (0.412 g, 2.057 mmol, 1.1 eq) and Cs₂CO₃ (0.670 g, 2.057 mmol, 1.1 eq) were added and heated to 80°C for 16 hours while stirring. After completion of the reaction, the resulting mixture was slowly cooled to room temperature and then extracted with EA and H₂O. The organic layer was dried over MgSO₄, filtered, concentrated and then purified by column chromatography (silica gel, Hex/EA) to obtain the title compound (0.614 g, 73%).

### Step 2 (b) Synthesis of tert-butyl (1-(4-nitro-2-((4-((2-(piperidin-1-yl)ethyl)carbamoyl)phenyl)ethynyl)phenyl)piperidin-3-yl)carbamate

Tert-butyl (1-(2-iodo-4-nitrophenyl)piperidin-3-yl)carbamate (0.614 g, 1.37 mmol, 1 eq), 4-ethynyl-N-(2-(piperidin-1-yl)ethyl)benzamide (0.527 g, 2.06 mmol, 1.5 eq), PdCl₂(PPh₃)₂ (0.028 g, 0.04 mmol, 0.03 eq), CuI (0.008 g, 0.04 mmol, 0.03 eq), and TEA (0.38 g, 2.74 mmol, 2 eq) were dissolved in ACN (13.7 ml, 0.1 M), and then degassed with nitrogen (N₂) gas. The reactant was heated to 60°C and stirred for five hours. After completion of the reaction, the resulting mixture was extracted with EA and H₂O. The organic layer was dried over MgSO₄, filtered, concentrated and then purified by column chromatography (silica gel, MeOH/DCM) to obtain the title compound (0.277 g, 35%).

### Step 3 (c) Synthesis of tert-butyl (1-(4-amino-2-((4-((2-(piperidin-1-yl)ethyl)carbamoyl)phenyl)ethynyl)phenyl)piperidin-3-yl)carbamate

Tert-butyl (1-(4-nitro-2-((4-((2-(piperidin-1-yl)ethyl)carbamoyl)phenyl)ethynyl)phenyl)piperidin-3-yl)carbamate (0.277 g, 0.48 mmol, 1 eq) was dissolved in 1,4-dioxane:H₂O (3:1, 4.8 ml, 0.1 M), and then Zn (0.314 g, 4.8 mmol, 10 eq) and NH₄Cl (0.257 g, 4.8 mmol, 10 eq) were added and stirred at room temperature overnight. The resulting mixture was washed with MeOH and filtered under reduced pressure. The filtrate was concentrated and then subjected to the following reaction without purification.

### Step 4 (d) Synthesis of tert-butyl (1-(4-((phenoxycarbonyl)amino)-2-((4-((2-(piperidin-1-yl)ethyl)carbamoyl)phenyl)ethynyl)phenyl)piperidin-3-yl)carbamate

Tert-butyl (1-(4-amino-2-((4-((2-(piperidin-1-yl)ethyl)carbamoyl)phenyl)ethynyl)phenyl)piperidin-3-yl)carbamate (0.209 g, 0.38 mmol, 1 eq) was dissolved in THF (3.8 ml, 0.1 M), and then pyridine (0.09 g, 1.14 mmol, 3 eq) was added. After cooling to 0°C, phenyl chloroformate (0.107 g, 0.68 mmol, 1.8 eq) was added dropwise. After slowly heating to room temperature, the resulting mixture was stirred for two hours. After completion of the reaction, the resulting mixture was concentrated, neutralized with 1N NaOH, and then extracted with EA and H₂O. The organic layer was dried over MgSO₄, filtered, concentrated and then purified by column chromatography (silica gel, MeOH/DCM) to obtain the title compound (0.052 g, 44%).

### Step 5 (e) Synthesis of tert-butyl (1-(2-((4-((2-(piperidin-1-yl)ethyl)carbamoyl)phenyl)ethynyl)-4-(3-(2-(pyridin-3-yl)ethyl)ureido)phenyl)piperidin-3-yl)carbamate

Tert-butyl (1-(4-((phenoxycarbonyl)amino)-2-((4-((2-(piperidin-1-yl)ethyl)carbamoyl)phenyl)ethynyl)phenyl)piperidin-3-yl)carbamate (0.1 g, 0.15 mmol, 1 eq) was dissolved in THF (3 ml, 0.05 M), and then 2-(pyridin-3-yl)ethan-1-amine (0.054 g, 0.45 mmol, 3 eq) and TEA (0.045 g, 0.45 mmol, 3 eq) were added and stirred at 55°C overnight. After completion of the reaction, the resulting mixture was concentrated and purified by column chromatography (silica gel, MeOH/DCM) to obtain the title compound (0.070 g, 67%).

### Step 6 (f) Synthesis of 4-((2-(3-aminopiperidin-1-yl)-5-(3-(2-(pyridin-3-yl)ethyl)ureido)phenyl)ethynyl)-N-(2-(piperidin-1-yl)ethyl)benzamide

Tert-butyl (1-(2-((4-((2-(piperidin-1-yl)ethyl)carbamoyl)phenyl)ethynyl)-4-(3-(2-(pyridin-3-yl)ethyl)ureido)phenyl)piperidin-3-yl)carbamate (0.030 g, 0.043 mmol, 1 eq) was dissolved in DCM (1 ml, 0.05 M), and then TFA (0.025 g, 0.215 mmol, 5 eq) was added and stirred at room temperature overnight. After completion of the reaction, the resulting mixture was concentrated and purified by reverse-phase column chromatography (0.1% formic acid in H₂O/ACN) to obtain the title compound (0.0947 g, 37%).

¹H NMR (400 MHz, MeOD) δ 8.46 (s, 2H), 7.91 (d, J = 6.9 Hz, 2H), 7.78 (d, J = 7.5 Hz, 1H), 7.65 (d, J = 6.9 Hz, 2H), 7.59 (s, 1H), 7.41 (s, 1H), 7.28 (d, J = 8.4 Hz, 1H), 7.01 (d, J = 8.8 Hz, 1H), 3.75 (s, 2H), 3.49 (dd, J = 19.1, 12.4 Hz, 4H), 3.32 (s, 2H), 3.21 (s, 5H), 2.89 (t, J = 6.7 Hz, 4H), 2.66 (s, 1H), 2.10 (s, 1H), 1.99 (s, 1H), 1.85 (s, 5H), 1.66 (s, 3H).

### Example 25: Synthesis of compound 43 (synthesis reaction formula 20)

### Synthesis of 4-((3'-(3-aminopyrrolidine-1-carbonyl)-4-(3-(2-(pyridin-3-yl)ethyl)ureido)-[1,1'-biphenyl]-2-yl)ethynyl)-N-(2-(piperidin-1-yl)ethyl)benzamide

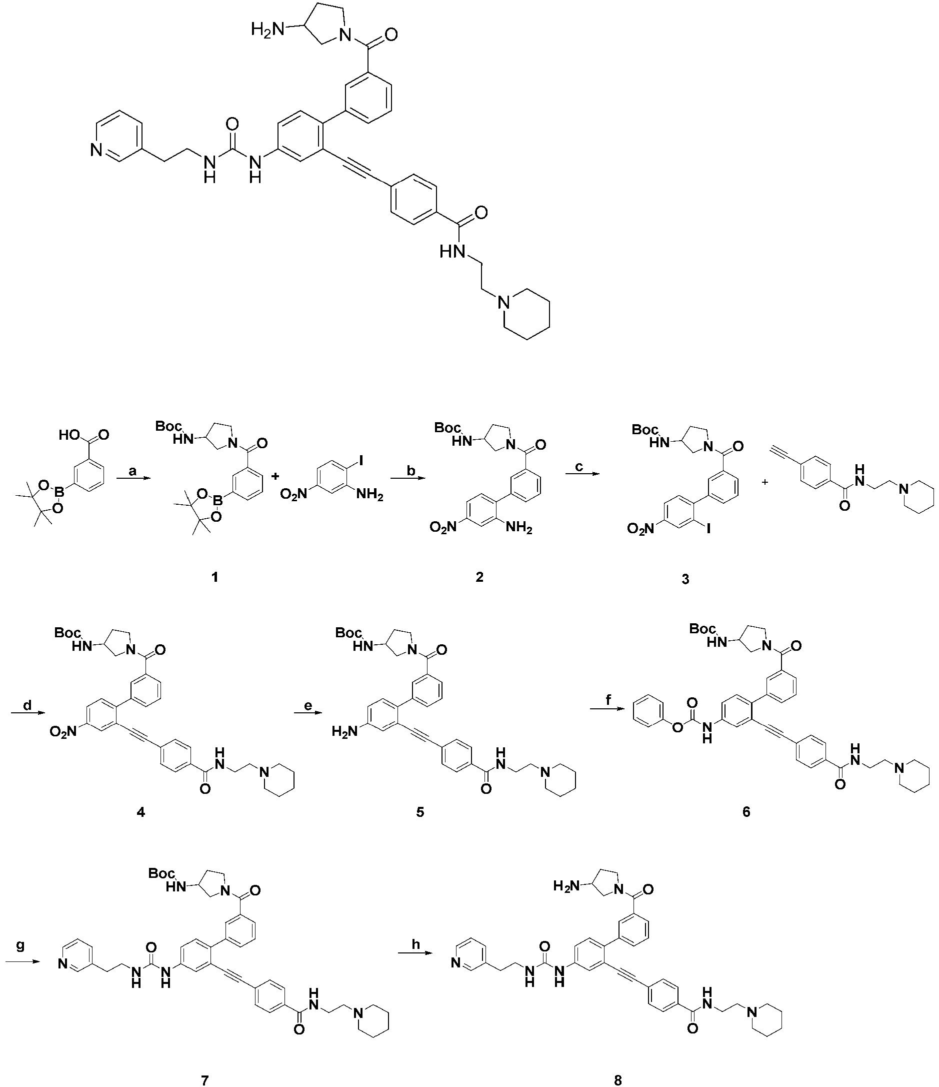

Reaction Formula 20. Reagents and conditions: (a) tert-butyl pyrrolidin-3-ylcarbamate, EDC, HOBT, TEA, DCM, RT, overnight; (b) Pd(dppf)Cl₂, K₂CO₃, 1,4-dioxane, water (H₂O), 90°C, overnight; (c) 10% P-TSA aqueous solution, NaNO₂, KI, H₂O, ACN, 0°C, 1 hour; (d) PdCl₂(PPh₃)₂, CuI, TEA, ACN, 60°C, 5 hours; (e) Zn, NH₄Cl, 1,4-dioxane, water (H₂O), RT, overnight; (f); (g) 2-(pyridin-3-yl)ethan-1-amine, TEA, THF, RT, overnight; (h) TFA, DCM, RT, overnight

### Step 1 (a) Synthesis of tert-butyl (1-(3-(4,4,5,5-tetramethyl-1,3,2-dioxaborolan-2-yl)benzoyl)pyrrolidin-3-yl)carbamate

3-(4,4,5,5-tetramethyl-1,3,2-dioxaborolan-2-yl)benzoic acid (2 g, 8.06 mmol, 1 eq) was dissolved in DCM (80 ml, 0.1 M), and then tert-butyl pyrrolidin-3-ylcarbamate (1.8 g, 9.67 mmol, 1.2 eq), EDC (1.853 g, 9.67 mmol, 1.2 eq), HOBT (1.306 g, 9.67 mmol, 1.2 eq), and TEA (1.63 g, 16.12 mmol, 2 eq) were added and stirred at room temperature overnight. After completion of the reaction, the resulting mixture was extracted with MC and H₂O. The organic layer was dried over MgSO₄, filtered, concentrated, and then purified by column chromatography (silica gel. EA/Hex) to obtain the title compound (2.1 g, 98%).

### Step 2 (b) Synthesis of tert-butyl (1-(2'-amino-4'-nitro-[1,1'-biphenyl]-3-carbonyl)pyrrolidin-3-yl)carbamate

2-iodo-5-nitroaniline (0.7 g, 2.65 mmol, 1 eq) was dissolved in 1,4-dioxane:water (H₂O)(3:1 (v/v), 13 ml, 0.14 M), and then tert-butyl (1-(3-(4,4,5,5-tetramethyl-1,3,2-dioxaborolan-2-yl)benzoyl)pyrrolidin-3-yl)carbamate (1.65 g, 3.97 mmol, 1.5 eq), Pd(dppf)Cl₂ (0.387 g, 0.53 mmol, 0.2 eq), and K₂CO₃ (0.805 g, 5.83 mmol, 2.2 eq) were added and degassed with nitrogen (N₂) gas. The reaction solution was heated to 90°C and stirred overnight. After completion of the reaction, the resulting mixture was filtered through a celite filter, and extracted with EA and H₂O. The organic layer was dried over MgSO₄, filtered, concentrated and then purified by column chromatography (silica gel, EA/Hex) to obtain the title compound (1.1 g, 97%).

### Step 3 (c) Synthesis of tert-butyl (1-(2'-iodo-4'-nitro-[1,1'-biphenyl]-3-carbonyl)pyrrolidin-3-yl)carbamate

Tert-butyl (1-(2'-amino-4'-nitro-[1,1'-biphenyl]-3-carbonyl)pyrrolidin-3-yl)carbamate (0.6 g, 1.4 mmol, 1 eq) was dissolved in ACN (14 ml, 0.1 M), and then 10% p-TSA (1.687 g, 9.8 mmol, 7 eq) aqueous solution was added, and 0.1 M NaNO₂ (0.106 g, 1.54 mmol, 1.1 eq) aqueous solution was added dropwise for 0.5 hours. 0.1 M NaI (0.419 g, 2.8 mmol, 2 eq) was added at once to the reaction solution at 0°C, and then stirred for 0.5 hours. After completion of the reaction, water (H₂O) was added to the reaction solution and extracted with EA and H₂O. The organic layer was dried over MgSO₄, filtered, concentrated and then purified by column chromatography (silica gel, EA/Hex) to obtain the title compound (0.671 g, 89%).

### Step 4 (d) Synthesis of tert-butyl (1-(4'-nitro-2'-((4-((2-(piperidin-1-yl)ethyl)carbamoyl)phenyl)ethynyl)-[1,1'-biphenyl]-3-carbonyl)pyrrolidin-3-yl)carbamate

Tert-butyl (1-(2'-iodo-4'-nitro-[1,1'-biphenyl]-3-carbonyl)pyrrolidin-3-yl)carbamate (0.3 g, 0.56 mmol, 1 eq), 4-ethynyl-N-(2-(piperidin-1-yl)ethyl)benzamide (0.215 g, 0.84 mmol, 1.5 eq), PdCl₂(PPh₃)₂ (0.012 g, 0.017 mmol, 0.03 eq), CuI (0.003 g, 0.017 mmol, 0.03 eq), and TEA (0.113 g, 1.12 mmol, 2 eq) were dissolved in ACN (5.6 ml, 0.1 M), and then degassed with nitrogen (N₂) gas. The reactant was heated to 60°C and stirred for five hours. After completion of the reaction, the resulting mixture was extracted with EA and H₂O. The organic layer was dried over MgSO₄, filtered, concentrated and then purified by column chromatography (silica gel, MeOH/DCM) to obtain the title compound (0.316 g, 85%).

### Step 5 (e) Synthesis of tert-butyl (1-(4'-amino-2'-((4-((2-(piperidin-1-yl)ethyl)carbamoyl)phenyl)ethynyl)-[1,1'-biphenyl]-3-carbonyl)pyrrolidin-3-yl)carbamate

Tert-butyl (1-(4'-nitro-2'-((4-((2-(piperidin-1-yl)ethyl)carbamoyl)phenyl)ethynyl)-[1,1'-biphenyl]-3-carbonyl)pyrrolidin-3-yl)carbamate (0.2 g, 0.3 mmol, 1 eq) was dissolved in 1,4-dioxane:water (H₂O) (3:1 (v/v), 3 ml, 0.1 M), and then Zn (0.196 g, 3 mmol, 10 eq) and NH₄Cl (0.16 g, 3 mmol, 10 eq) were added and stirred at room temperature overnight. The resulting mixture was washed with MeOH and filtered under reduced pressure. The filtrate was concentrated and then subjected to the following reaction of step 6) without purification.

### Step 6 (f) Synthesis of tert-butyl (1-(4'-((phenoxycarbonyl)amino)-2'-((4-((2-(piperidin-1-yl)ethyl)carbamoyl)phenyl)ethynyl)-[1,1'-biphenyl]-3-carbonyl)pyrrolidin-3-yl)carbamate

Tert-butyl (1-(4'-amino-2'-((4-((2-(piperidin-1-yl)ethyl)carbamoyl)phenyl)ethynyl)-[1,1'-biphenyl]-3-carbonyl)pyrrolidin-3-yl)carbamate (0.19 g, 0.3 mmol, 1 eq) was dissolved in THF (3 ml, 0.1 M), and then pyridine (0.07 g, 0.9 mmol, 3 eq) was added. After cooling to 0°C, phenyl chloroformate (0.084 g, 0.54 mmol, 1.8 eq) was added dropwise. After slowly heating to room temperature, the resulting mixture was stirred for two hours. After completion of the reaction, the resulting mixture was concentrated, neutralized with 1N NaOH, and then extracted with EA and H₂O. The organic layer was dried over MgSO₄, filtered, concentrated, and then purified by column chromatography (silica gel, MeOH/DCM) to obtain the title compound (0.083 g, 33%).

### Step 7 (g) Synthesis of tert-butyl (1-(2'-((4-((2-(piperidin-1-yl)ethyl)carbamoyl)phenyl)ethynyl)-4'-(3-(2-(pyridin-3-yl)ethyl)ureido)-[1,1'-biphenyl]-3-carbonyl)pyrrolidin-3-yl)carbamate

Tert-butyl (1-(4'-((phenoxycarbonyl)amino)-2'-((4-((2-(piperidin-1-yl)ethyl)carbamoyl)phenyl)ethynyl)-[1,1'-biphenyl]-3-carbonyl)pyrrolidin-3-yl)carbamate (0.083 g, 0.1 mmol, 1 eq) was dissolved in THF (1 ml, 0.1 M), and then 2-(pyridin-3-yl)ethan-1-amine (0.024 g, 0.2 mmol, 2 eq) and TEA (0.030 g, 0.3 mmol, 3 eq) were added and stirred at 55°C overnight. After completion of the reaction, the resulting mixture was concentrated and purified by column chromatography (silica gel, MeOH/DCM) to obtain the title compound (0.049 g, 62%).

### Step 8 (h) Synthesis of 4-((3'-(3-aminopyrrolidin-1-carbonyl)-4-(3-(2-(pyridin-3-yl)ethyl)ureido)-[1,1'-biphenyl]-2-yl)ethynyl)-N-(2-(piperidin-1-yl)ethyl)benzamide

Tert-butyl (1-(2'-((4-((2-(piperidin-1-yl)ethyl)carbamoyl)phenyl)ethynyl)-4'-(3-(2-(pyridin-3-yl)ethyl)ureido)-[1,1'-biphenyl]-3-carbonyl)pyrrolidin-3-yl)carbamate (0.049 g, 0.062 mmol, 1 eq) was dissolved in DCM (1 ml, 0.05 M), and then TFA (0.035 g, 0.31 mmol, 5 eq) was added and stirred at room temperature overnight. After completion of the reaction, the resulting mixture was concentrated and purified by reverse-phase column chromatography (0.1% formic acid in H₂O/ACN) to obtain the title compound (0.014 g, 33%).

¹H NMR (400 MHz, MeOD) δ 8.39 (s, 2H), 8.28 (s, 1H), 7.94 (d, J = 5.5 Hz, 2H), 7.85 (d, J = 7.8 Hz, 1H), 7.79 - 7.71 (m, 1H), 7.65 - 7.45 (m, 6H), 7.38 (s, 1H), 7.33 (t, J = 7.6 Hz, 1H), 7.30 - 7.22 (m, 1H), 4.05 - 3.88 (m, 2H), 3.78 (d, J = 22.7 Hz, 5H), 3.63 (s, 1H), 3.53 - 3.33 (m, 6H), 3.21 (dd, J = 14.6, 7.3 Hz, 1H), 2.87 - 2.77 (m, 2H), 2.66 (s, 1H), 2.40 (s, 1H), 2.16 (s, 1H), 1.95 - 1.86 (m, 4H), 1.69 (s, 2H), 1.33 (dd, J = 16.2, 8.9 Hz, 1H).

### Example 26: Synthesis of compound 185 (synthesis reaction formula 21)

### Synthesis of 1-(2-((4-fluorophenyl)ethynyl)-4'-(piperazin-1-yl)-[1,1'-biphenyl]-4-yl)-3-(imidazo[1,2-a]pyridin-7-ylmethyl)urea

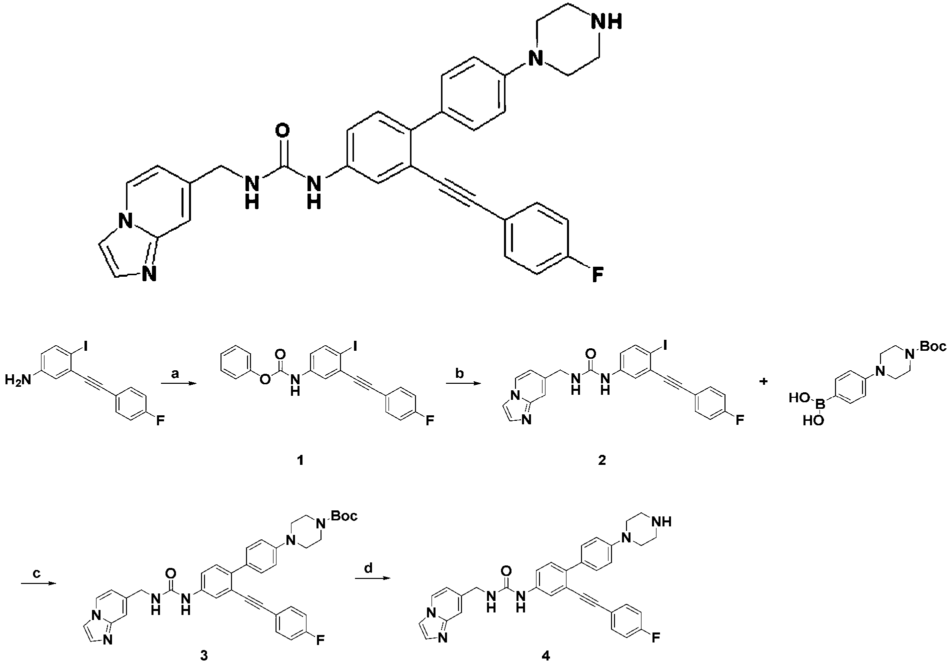

Reaction Formula 21. Reagents and conditions: (a) phenyl chloroformate, pyridine, THF, 0°C to RT, overnight; (b) imidazo[1,2-a]pyridin-7-ylmethanamine*HCl, TEA, THF, reflux, overnight; (c) Pd(dppf)Cl₂, K₂CO₃, 1,4-dioxane, water (H₂O), 90°C, overnight; (d) TFA, DCM, RT, overnight

### Step 1 (a) Synthesis of phenyl (3-((4-fluorophenyl)ethynyl)-4-iodophenyl)carbamate

3-((4-fluorophenyl)ethynyl)-4-iodoaniline (1 g, 2.97 mmol, 1 eq) was dissolved in THF (10 ml, 0.3 M), and then pyridine (0.329 g, 41.58 mmol, 14 eq) and phenyl chloroformate (0.558 g, 3.564 mmol, 1.2 eq) were added dropwise at 0°C and stirred. After completion of the reaction, the resulting mixture was concentrated and extracted with EA and water (H₂O). The organic layer was dried over MgSO₄, filtered, concentrated and then purified by column chromatography (silica gel, EA/Hex) to obtain the title compound (1.3 g, 96%).

### Step 2 (b) Synthesis of 1-(3-((4-fluorophenyl)ethynyl)-4-iodophenyl)-3-(imidazo[1,2-a]pyridin-7-ylmethyl)urea

Phenyl (3-((4-fluorophenyl)ethynyl)-4-iodophenyl)carbamate (1 g, 2.18 mmol, 1 eq) was dissolved in THF (21.8 ml, 0.1 M), and then imidazo[1,2-a]pyridin-7-ylmethanamine*HCl salt (0.8 g, 4.36 mmol, 2 eq) and TEA (0.662 g, 6.54 mmol, 3 eq) were added, and then heated under reflux and stirred overnight. After completion of the reaction, the resulting mixture was subjected to crystallization with Hex and MC, and the crystal was washed three times with H₂O and filtered to obtain the title compound (0.85 g, 76%).

### Step 3 (c) Synthesis of tert-butyl 4-(2'-((4-fluorophenyl)ethynyl)-4'-(3-(imidazo[1,2-a]pyridin-7-ylmethyl)ureido)-[1,1'-biphenyl]-4-yl)piperazin-1-carboxylate

1-(3-((4-fluorophenyl)ethynyl)-4-iodophenyl)-3-(imidazo[1,2-a]pyridin-7-ylmethyl)urea (0.1 g, 0.19 mmol, 1 eq) was dissolved in 1,4-dioxane:water (H₂O)(3:1 (v/v), 0.8 ml, 0.25 M), and then (4-(4-(tert-butoxycarbonyl)piperazin-1-yl)phenyl)boronic acid (0.11 g, 0.38 mmol, 2 eq), Pd(dppf)Cl₂ (0.027 g, 0.038 mmol, 0.2 eq), and K₂CO₃ (0.058 g, 0.418 mmol, 2.2 eq) were added and degassed with nitrogen (N₂) gas. The reaction solution was heated to 90°C and stirred overnight. After completion of the reaction, the resulting mixture was filtered through a celite filter, and extracted with EA and H₂O. The organic layer was dried over MgSO₄, filtered, concentrated and then purified by column chromatography (silica gel, MeOH/DCM) to obtain the title compound (0.024 g, 19%).

### Step 4 (d) Synthesis of 1-(2-((4-fluorophenyl)ethynyl)-4'-(piperazin-1-yl)-[1,1'-biphenyl]-4-yl)-3-(imidazo[1,2-a]pyridin-7-ylmethyl)urea

Tert-butyl 4-(2'-((4-fluorophenyl)ethynyl)-4'-(3-(imidazo[1,2-a]pyridin-7-ylmethyl)ureido)-[1,1'-biphenyl]-4-yl)piperazin-1-carboxylate (0.024 g, 0.037 mmol, 1 eq) was dissolved in DCM (1 ml, 0.03 M), and then TFA (0.021 g, 0.185 mmol, 5 eq) was added and stirred at room temperature overnight. After completion of the reaction, the resulting mixture was concentrated and purified by reverse-phase column chromatography (0.1% formic acid in H₂O/ACN) to obtain the title compound (0.007 g, 35%).

¹H NMR (400 MHz, MeOD) δ 8.42 (s, 1H), 7.83 (s, 1H), 7.72 (s, 1H), 7.61 - 7.49 (m, 4H), 7.45 - 7.31 (m, 4H), 7.08 (d, J = 6.9 Hz, 4H), 6.97 (d, J = 6.6 Hz, 1H), 4.51 (s, 2H), 3.62 (s, 4H), 3.21 (s, 4H).

### Example 27: Synthesis of compound 187 (synthesis reaction formula 22)

### Synthesis of 1-(imidazo[1,2-a]pyridin-7-ylmethyl)-3-(3-((4-(piperidin-4-yl)phenyl)ethynyl)-4-(pyridin-4-yl)phenyl)urea

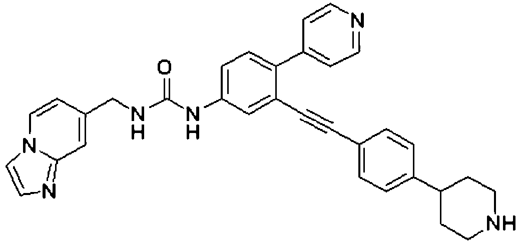

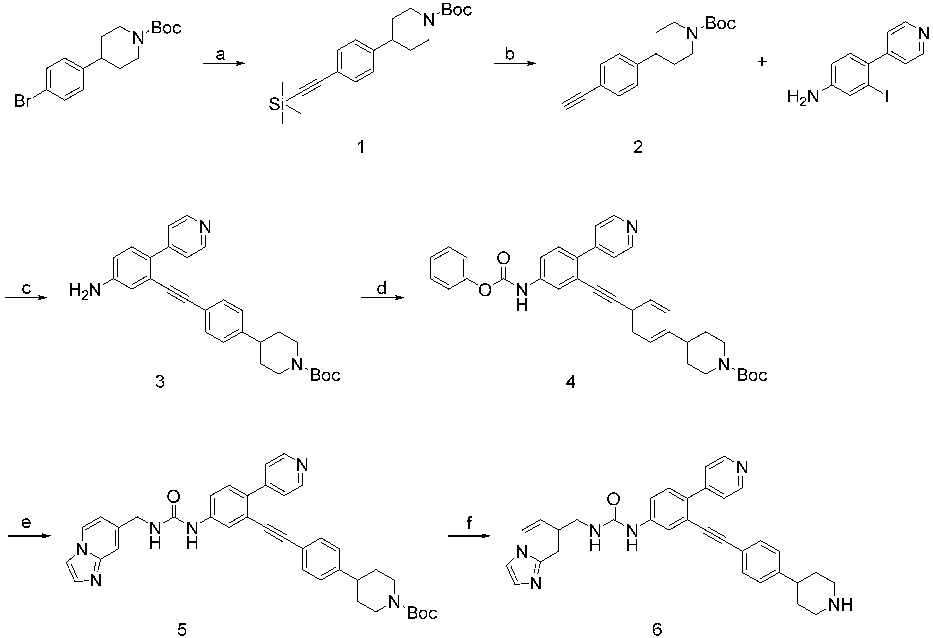

Reaction Formula 22. Reagents and conditions: (a) trimethylsilylacetylene, PdCl₂(PPh₃)₂, CuI, TEA, toluene, 100°C, 12 hours; (b) K₂CO₃, MeOH, RT, 2 hours; (c) PdCl₂(PPh₃)₂, CuI, TEA, ACN, 60°C, 1 hour; (d) phenyl chloroformate, pyridine, THF, 0°C to RT, overnight; (e) imidazo[1,2-a]pyridin-7-ylmethanamine, TEA, THF, 80°C, overnight; (f) TFA, DCM, RT, overnight,

### Step 1 (a) Synthesis of tert-butyl 4-(4-((trimethylsilyl)ethynyl)phenyl)piperidine-1-carboxylate

Tert-butyl 4-(4-bromophenyl)piperidine-1-carboxylate (0.5 g, 1.47 mmol, 1 eq) was dissolved in toluene (7.4 ml, 0.2 M), and then trimethylsilylacetylene (0.216 g, 2.2 mmol, 1.5 eq), PdCl₂(PPh₃)₂ (0.103 g, 0.147 mmol, 0.1 eq), CuI (0.027 g, 0.147 mmol, 0.1 eq), and TEA (0.594 g, 5.88 mmol, 4 eq) were added and degassed with nitrogen (N₂) gas. The reactant was heated to 100°C and stirred for 12 hours. After completion of the reaction, the resulting mixture was extracted with DCM and H₂O, and the organic layer was dried over MgSO₄, filtered, concentrated and then purified by column chromatography (silica gel, EA/Hex) to obtain the title compound (0.196 g, 37%).

### Step 2 (b) Synthesis of tert-butyl 4-(4-ethynylphenyl)piperidine-1-carboxylate

Tert-butyl 4-(4-((trimethylsilyl)ethynyl)phenyl)piperidine-1-carboxylate (0.196 g, 0.55 mmol, 1 eq) was dissolved in MeOH (5.5 ml, 0.1 M), and then K₂CO₃ (0.38 g, 2.75 mmol, 5 eq) was added and stirred at room temperature for two hours. After completion of the reaction, the resulting mixture was filtered, concentrated and then purified by column chromatography (silica gel, EA/Hex) to obtain the title compound (0.156 g, 100%).

### Step 3 (c) Synthesis of tert-butyl 4-(4-((5-amino-2-(pyridin-4-yl)phenyl)ethynyl)phenyl)piperidine-1-carboxylate

3-iodo-4-(pyridin-4-yl)aniline (0.1 g, 0.37 mmol, 1 eq), tert-butyl 4-(4-ethynylphenyl)piperidine-1-carboxylate (0.157 g, 0.55 mmol, 1.5 eq), PdCl₂(PPh₃)₂ (0.007 g, 0.01 mmol, 0.03 eq), CuI (0.002 g, 0.01 mmol, 0.03 eq), and TEA (0.075 g, 0.74 mmol, 2 eq) were dissolved in ACN (3.7 ml, 0.1 M) and degassed with nitrogen (N₂) gas. The reactant was heated to 60°C and stirred for one hour. After completion of the reaction, the resulting mixture was extracted with EA and H₂O. The organic layer was dried over MgSO₄, filtered, concentrated and then purified by column chromatography (silica gel, MeOH/DCM) to obtain the title compound (0.075 g, 43%).

### Step 4 (d) Synthesis of tert-butyl 4-(4-((5-((phenoxycarbonyl)amino)-2-(pyridin-4-yl)phenyl)ethynyl)phenyl)piperidine-1-carboxylate

Tert-butyl 4-(4-((5-amino-2-(pyridin-4-yl)phenyl)ethynyl)phenyl)piperidine-1-carboxylate (0.075 g, 0.16 mmol, 1 eq) was dissolved in THF (1.6 ml, 0.1 M), and then pyridine (0.177 g, 2.24 mmol, 14 eq) and phenyl chloroformate (0.030 g, 0.192 mmol, 1.2 eq) were added dropwise at 0°C and stirred. After completion of the reaction, the resulting mixture was concentrated and extracted with EA and H₂O. The organic layer was dried over MgSO₄, filtered, concentrated and then purified by column chromatography (silica gel, MeOH/DCM) to obtain the title compound (0.06 g, 63%).

### Step 5 (e) Synthesis of tert-butyl 4-(4-((5-(3-(imidazo[1,2-a]pyridin-7-ylmethyl)ureido)-2-(pyridin-4-yl)phenyl)ethynyl)phenyl)piperidine-1-carboxylate

Tert-butyl 4-(4-((5-((phenoxycarbonyl)amino)-2-(pyridin-4-yl)phenyl)ethynyl)phenyl)piperidine-1-carboxylate (0.060 g, 0.1 mmol, 1 eq) was dissolved in THF (1 ml, 0.1 M), and then imidazo[1,2-a]pyridin-7-ylmethanamine*HCl salt (0.037 g, 0.2 mmol, 2 eq) and TEA (0.030 g, 0.3 mmol, 3 eq) were added, heated under reflux, and stirred overnight. After completion of the reaction, the resulting mixture was concentrated and subjected to the following reaction of step 6) without purification.

### Step 6 (f) Synthesis of 1-(imidazo[1,2-a]pyridin-7-ylmethyl)-3-(3-((4-(piperidin-4-yl)phenyl)ethynyl)-4-(pyridin-4-yl)phenyl)urea

Tert-butyl 4-(4-((5-(3-(imidazo[1,2-a]pyridin-7-ylmethyl)ureido)-2-(pyridin-4-yl)phenyl)ethynyl)phenyl)piperidine-1-carboxylate (0.063 g, 0.1 mmol, 1 eq) was dissolved in DCM (1 ml, 0.1 M), and then TFA (0.057 g, 0.5 mmol, 5 eq) was added and stirred at room temperature overnight. After completion of the reaction, the resulting mixture was concentrated and purified by reverse-phase column chromatography (0.1% formic acid in H₂O/ACN) to obtain the title compound (0.021 g, 40%).

¹H NMR (400 MHz, MeOD) δ 8.60 (d, J = 5.4 Hz, 2H), 8.56 (d, J = 6.9 Hz, 1H), 8.21 (s, 1H), 7.97 (s, 1H), 7.88 (d, J = 1.7 Hz, 1H), 7.76 - 7.71 (m, 3H), 7.68 (s, 1H), 7.53 (dd, J = 8.4, 1.9 Hz, 1H), 7.44 (d, J = 8.5 Hz, 1H), 7.32 (d, J = 8.1 Hz, 2H), 7.27 (d, J = 8.2 Hz, 2H), 7.20 (d, J = 6.8 Hz, 1H), 4.58 (s, 2H), 3.51 (d, J = 12.6 Hz, 2H), 3.14 (t, J = 11.8 Hz, 2H), 2.92 (t, J = 12.2 Hz, 1H), 2.07 (d, J = 13.6 Hz, 2H), 1.92 (t, J = 11.8 Hz, 2H); MS MH⁺ 527.25

### Example 28: Synthesis of compound 35 (synthesis reaction formula 23)

### Synthesis of N-(2-(2,8-diazaspiro[4.5]decan-8-yl)ethyl)-4-((5-(3-(2-(pyridin-3-yl)ethyl)ureido)-2-(pyridin-4-yl)phenyl)ethynyl)benzamide

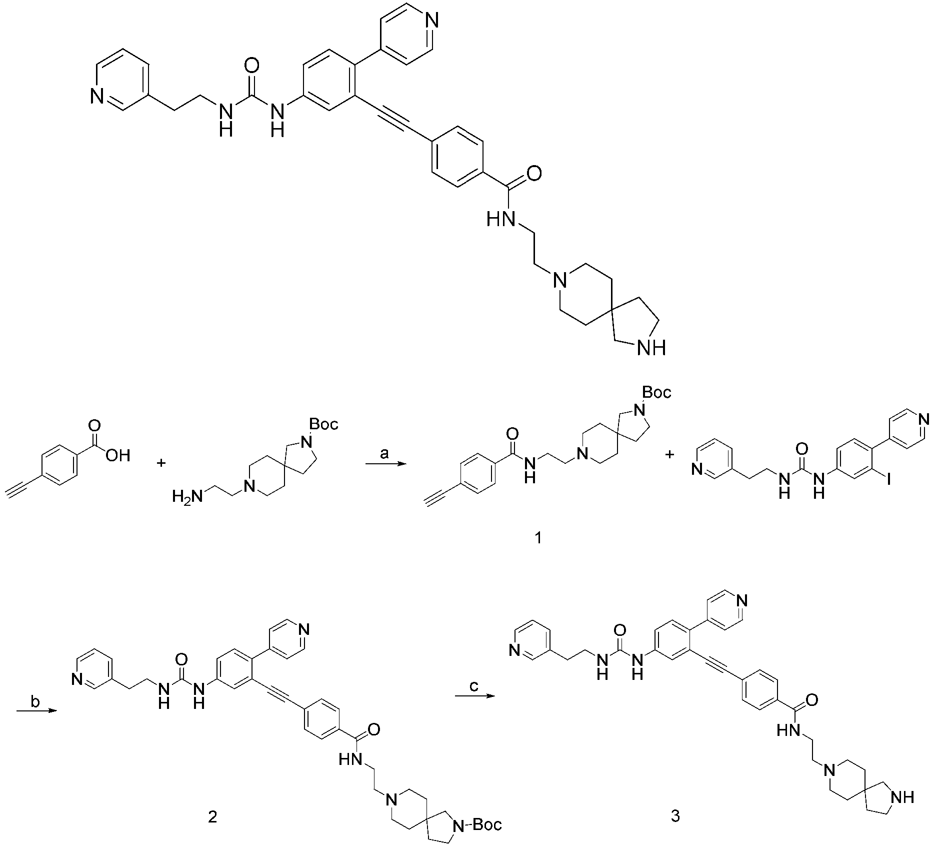

Reaction Formula 23. Reagents and conditions: (a) HATU, DIPEA, DMF, RT, overnight; (b) PdCl₂(PPh₃)₂, CuI, TEA, ACN, 60°C, 5 hours; (c) TFA, DCM, RT, overnight.

### Step 1 (a) Synthesis of tert-butyl 8-(2-(4-ethynylbenzamido)ethyl)-2,8-diazaspiro [4.5] decane-2-carboxylate

4-ethynylbenzoic acid (0.05 g, 0.34 mmol, 1 eq) was dissolved in DMF (3.4 ml, 0.1 M), and then HATU (0.193 g, 0.51 mmol, 1.5 eq) was added and stirred at room temperature for 10 minutes. Tert-butyl 8-(2-aminoethyl)-2,8-diazaspiro[4.5]decan-2-carboxylate (0.149 g, 0.51 mmol, 1.5 eq) and DIPEA (0.131 g, 1.02 mmol, 3 eq) were added to the reaction solution and stirred at room temperature overnight. After completion of the reaction, the resulting mixture was extracted with EA and H₂O. The organic layer was dried over MgSO₄, filtered, concentrated and then purified by column chromatography (silica gel, MeOH/DCM) to obtain the title compound (0.118 g, 84%).

### Step 2 (b) Synthesis of tert-butyl 8-(2-(4-((5-(3-(2-(pyridin-3-yl)ethyl)ureido)-2-(pyridin-4-yl)phenyl)ethynyl)benzamido)ethyl)-2,8-diazaspiro[4.5]decane-2-carboxylate

1-(3-iodo-4-(pyridin-4-yl)phenyl)-3-(2-(pyridin-3-yl)ethyl)urea (0.05 g, 0.11 mmol, 1 eq), tert-butyl 8-(2-(4-ethynylbenzamido)ethyl)-2,8-diazaspiro[4.5]decan-2-carboxylate (0.068 g, 0.165 mmol, 1.5 eq), PdCl₂(PPh₃)₂ (0.002 g, 0.0033 mmol, 0.03 eq), CuI (0.00063 g, 0.0033 mmol, 0.03 eq), and TEA (0.022 g, 0.22 mmol, 2 eq) were dissolved in ACN (1.1 ml, 0.1 M) and degassed with nitrogen (N₂) gas. The reactant was heated to 60°C and stirred for five hours. After completion of the reaction, the resulting mixture was extracted with DCM:MeOH (9:1 (v/v)) and water (H₂O). The organic layer was dried over MgSO₄, filtered, concentrated and then purified by column chromatography (silica gel, MeOH/DCM) to obtain the title compound (0.052 g, 65%).

### Step 3 (c) Synthesis of N-(2-(2,8-diazaspiro[4.5]decane-8-yl)ethyl)-4-((5-(3-(2-(pyridin-3-yl)ethyl)ureido)-2-(pyridin-4-yl)phenyl)ethynyl)benzamide

Tert-butyl 8-(2-(4-((5-(3-(2-(pyridin-3-yl)ethyl)ureido)-2-(pyridin-4-yl)phenyl)ethynyl)benzamido)ethyl)-2,8-diazaspiro[4.5]decan-2-carboxylate (0.052 g, 0.071 mmol, 1 eq) was dissolved in DCM (1 ml, 0.1 M), and then TFA (0.04 g, 0.36 mmol, 5 eq) was added and stirred at room temperature overnight. After completion of the reaction, the resulting mixture was concentrated and purified by reverse-phase column chromatography (0.1% formic acid in H₂O/ACN) to obtain the title compound (0.015 g, 34%).

¹H NMR (400 MHz, MeOD) δ 8.59 (d, J = 3.9 Hz, 2H), 8.48 (s, 1H), 8.40 (s, 1H), 8.33 (s, 1H), 7.84 (dd, J = 21.1, 10.8 Hz, 4H), 7.70 (d, J = 4.5 Hz, 2H), 7.47 (d, J = 8.5 Hz, 1H), 7.42 (d, J = 7.1 Hz, 3H), 3.73 (s, 2H), 3.51 (t, J = 6.5 Hz, 2H), 3.41 (s, 2H), 3.15 (d, J = 29.0 Hz, 8H), 2.92 (t, J = 6.5 Hz, 2H), 2.05 - 1.79 (m, 6H); MS MH⁺ 628.5

### Example 29: Synthesis of compound 39 (synthesis reaction formula 24)

### Synthesis of N-(2-(3-aminopyrrolidin-1-yl)ethyl)-4-((5-(3-(2-(pyridin-3-yl)ethyl)ureido)-2-(pyridin-4-yl)phenyl)ethynyl)benzamide

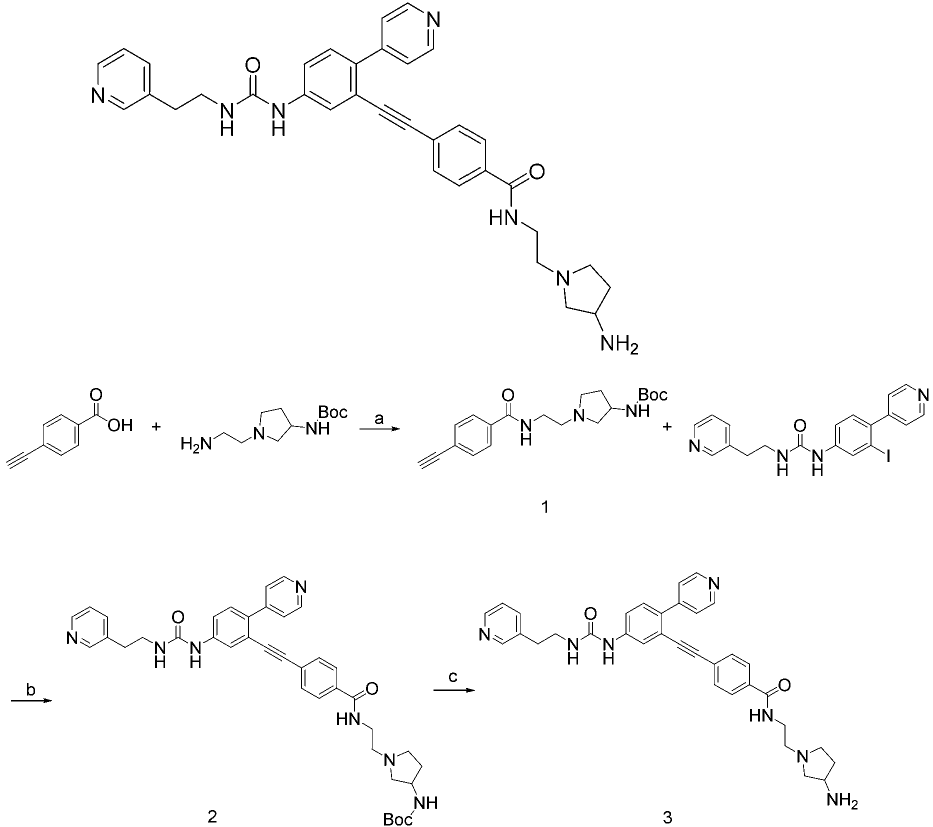

Reaction Formula 24. Reagents and conditions: (a) HATU, DIPEA, DMF, RT, overnight; (b) PdCl₂(PPh₃)₂, CuI, TEA, ACN, 60°C, 5 hours; (c) TFA, DCM, RT, overnight.

### Step 1 (a) Synthesis of tert-butyl (1-(2-(4-ethynylbenzamido)ethyl)pyrrolidin-3-yl)carbamate

4-ethynylbenzoic acid (0.05 g, 0.34 mmol, 1 eq) was dissolved in DMF (3.4 ml, 0.1 M), and then HATU (0.193 g, 0.51 mmol, 1.5 eq) was added and stirred at room temperature for 10 minutes. Tert-butyl (1-(2-aminoethyl)pyrrolidin-3-yl)carbamate (0.117 g, 0.51 mmol, 1.5 eq) and DIPEA (0.131 g, 1.02 mmol, 3 eq) were added to the reaction solution and stirred at room temperature overnight. After completion of the reaction, the resulting mixture was extracted with EA and H₂O. The organic layer was dried over MgSO₄, filtered, concentrated and then purified by column chromatography (silica gel, MeOH/DCM) to obtain the title compound (0.063 g, 26%).

### Step 2 (b) Synthesis of tert-butyl (1-(2-(4-((5-(3-(2-(pyridin-3-yl)ethyl)ureido)-2-(pyridin-4-yl)phenyl)ethynyl)benzamido)ethyl)pyrrolidin-3-yl)carbamate

1-(3-iodo-4-(pyridin-4-yl)phenyl)-3-(2-(pyridin-3-yl)ethyl)urea (0.053 g, 0.12 mmol, 1 eq), tert-butyl (1-(2-(4-ethynylbenzamido)ethyl)pyrrolidin-3-yl)carbamate (0.063 g, 0.176 mmol, 1.5 eq), PdCl₂(PPh₃)₂ (0.003 g, 0.0036 mmol, 0.03 eq), CuI (0.00069 g, 0.0036 mmol, 0.03 eq) and TEA (0.024 g, 0.24 mmol, 2 eq) were dissolved in ACN (1.2 ml, 0.1 M) and degassed with nitrogen (N₂) gas. The reactant was heated to 60°C and stirred for five hours. After completion of the reaction, the resulting mixture was extracted with DCM:MeOH (9:1 (v/v)) and water (H₂O). The organic layer was dried over MgSO₄, filtered, concentrated and then purified by column chromatography (silica gel, MeOH/DCM) to obtain the title compound (0.036 g, 44%).

### Step 3 (c) Synthesis of N-(2-(3-aminopyrrolidin-1-yl)ethyl)-4-((5-(3-(2-(pyridin-3-yl)ethyl)ureido)-2-(pyridin-4-yl)phenyl)ethynyl)benzamide

Tert-butyl (1-(2-(4-((5-(3-(2-(pyridin-3-yl)ethyl)ureido)-2-(pyridin-4-yl)phenyl)ethynyl)benzamido)ethyl)pyrrolidin-3-yl)carbamate (0.036 g, 0.053 mmol, 1 eq) was dissolved in DCM (1 ml, 0.05 M), and then TFA (0.06 g, 0.53 mmol, 10 eq) was added and stirred at room temperature overnight. After completion of the reaction, the resulting mixture was concentrated and purified by reverse-phase column chromatography (0.1% formic acid in H₂O/ACN) to obtain the title compound (0.020 g, 66%).

¹H NMR (400 MHz, MeOD) δ 8.61 (d, J = 5.9 Hz, 1H), 8.48 (s, 1H), 8.41 (d, J = 4.4 Hz, 1H), 8.12 (s, 2H), 7.85 (dt, J = 22.1, 7.8 Hz, 4H), 7.73 (d, J = 5.9 Hz, 1H), 7.64 (d, J = 8.1 Hz, 1H), 7.46 - 7.39 (m, 4H), 3.83 (s, 1H), 3.65 - 3.55 (m, 2H), 3.51 (t, J = 7.0 Hz, 2H), 3.19 (s, 1H), 3.12 - 3.01 (m, 1H), 2.96 - 2.84 (m, 5H), 2.62 (dd, J = 17.1, 9.1 Hz, 2H), 2.38 (s, 1H), 1.90 (s, 1H).

### Example 30: Synthesis of compound 139 (synthesis reaction formula 25)

### Synthesis of 1-(3-((3-aminophenyl)ethynyl)-4-(pyridin-4-yl)phenyl)-3-(2-(pyridin-3-yl)ethyl)urea

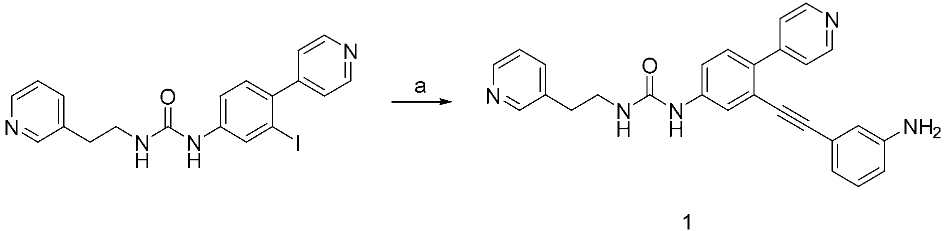

### Reaction Formula 25. Reagents and conditions: (a) 3-ethynylaniline, PdCl₂(PPh₃)₂, CuI, TEA, ACN, 80°C, 3 hours (3h)

1-(3-iodo-4-(pyridin-4-yl)phenyl)-3-(2-(pyridin-3-yl)ethyl)urea (0.050 g, 0.11 mmol, 1 eq), 3-ethynylaniline (0.019 g, 0.165 mmol, 1.5 eq), PdCl₂(PPh₃)₂ (0.007 g, 0.011 mmol, 0.1 eq), CuI (0.002 g, 0.011 mmol, 0.1 eq), and TEA (0.044 g, 0.44 mmol, 4 eq) were dissolved in ACN (1.1 ml, 0.1 M) and degassed with nitrogen (N₂) gas. The reactant was heated to 80°C and stirred for three hours. After completion of the reaction, the resulting mixture was extracted with DCM:MeOH (9:1 (v/v)) and (H₂O). The organic layer was dried over MgSO₄, filtered, concentrated and then purified by column chromatography (silica gel, MeOH/DCM) to obtain the title compound (15 mg, 32%).

¹H NMR (400 MHz, DMSO) δ 8.79 (s, 1H), 8.66 (s, 2H), 8.46 (d, J = 13.7 Hz, 2H), 7.83 (s, 1H), 7.68 (d, J = 7.8 Hz, 1H), 7.64 (d, J = 5.4 Hz, 2H), 7.43 (d, J = 1.1 Hz, 2H), 7.35 (dd, J = 7.6, 4.8 Hz, 1H), 7.02 (dd, J = 8.4, 7.5 Hz, 1H), 6.58 (dd, J = 8.6, 1.1 Hz, 2H), 6.53 (d, J = 7.5 Hz, 1H), 6.31 (t, J = 5.7 Hz, 1H), 5.26 (s, 2H), 3.39 (dd, J = 12.8, 6.8 Hz, 2H), 2.80 (t, J = 7.0 Hz, 2H).

The reactants may be changed in substantially the same manner as in the synthesis method of above Example 30, so as to synthesize compounds 15, 16, 17, 18, 21, 37, 38, 53, 55, 129, 133, 135, 137, 140, 141, 156, 157, 158, 160, 162, and 172.

### Example 31: Synthesis of compound 89 (synthesis reaction formula 26)

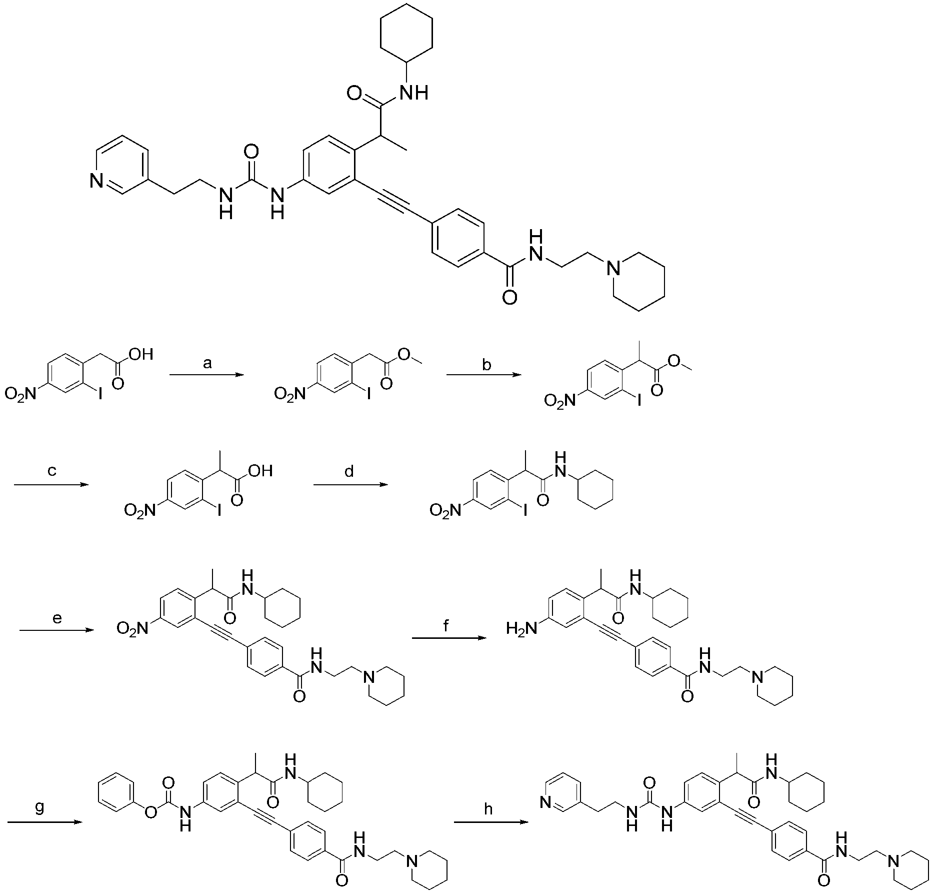

Reaction formula 26 (a) SOCl₂, MeOH, RT; (b) NaH, MeI, DMF, 4 hours; (c) NaOH, H₂O, MeOH, reflux, 4 hours; (d) TBTU, TEA, cyclohexanamine, DCM, 24 hours; (e) Pd[PPh₃]₄, CuI, TEA, 4-ethynyl-N-(2-(piperidin-1-yl)ethyl)benzamide, ACN, RT, 1 hour; (f) Fe, NH₄Cl, EtOH, H₂O, 90°C, 2 hours; (g) phenyl chloroformate, pyridine, THF, RT, 2 hours; (h) 2-(pyridin-3-yl)ethan-1-amine, TEA, THF, reflux, 24 hours

### Step 1 (a) Synthesis of methyl 2-(2-iodo-4-nitrophenyl)acetate

2-(2-iodo-4-nitrophenyl)acetic acid (1 g, 3.26 mmol, 1 eq) was dissolved in MeOH (0.25 M), and the resulting reactant was cooled with ice, and SOCl₂ (7.8 ml) was slowly added dropwise. The resulting mixture was stirred at room temperature, and when the reaction was completed, the reactant was concentrated under reduced pressure to obtain the title target compound.

### Step 2 (b) Synthesis of methyl 2-(2-iodo-4-nitrophenyl)propanoate

Methyl 2-(2-iodo-4-nitrophenyl)acetate (3.25 mmol) was dissolved in DMF (6 ml), and then MeI (0.24 ml, 1.2 eq) was added. The reaction solution was cooled with ice, and 60% NaH (0.14 g, 3.58 mmol, 1.1 eq) was added in portions and stirred for four hours. Water was added to the resulting reactant, which was extracted with EA, and the organic layer was washed with saturated ammonium chloride aqueous solution, dried over magnesium sulfate, and concentrated under reduced pressure to obtain the title target compound (1.2 g).

### Step 3 (c) Synthesis of 2-(2-iodo-4-nitrophenyl)propanoic acid

A mixture of methyl 2-(2-iodo-4-nitrophenyl)propanoate (0.36 g, 2.98 mmol, 1 eq) and NaOH (0.3 g, 7.5 mmol, 2.5 eq) dissolved in water (0.2 M) and MeOH (0.1 M) was stirred at 60°C for two to four hours. When the reaction was completed by confirming the reaction through TLC, the pH of the reactant was adjusted to 1 with 1N HCl aqueous solution and extracted with EA. The organic layer was washed with water, dried over magnesium sulfate, and concentrated under reduced pressure to obtain the title target compound (1 g).

### Step 4 (d) Synthesis of N-cyclohexyl-2-(2-iodo-4-nitrophenyl)propenamide

2-(2-iodo-4-nitrophenyl)propanoic acid (1 g, 3 mmol) and TBTU (1.4 g, 4.5 mmol, 1.5 eq) were added to 30 mL of DCM and stirred at room temperature for 50 minutes. TEA (0.83 ml, 6 mmol, 2 eq) and cyclohexanamine (0.41 ml, 3.6 mmol, 1.2 eq) were added to the reactant and stirred at room temperature for 24 hours. Water was added to the reactant, which was extracted with EA, and the organic layer was dried over magnesium sulfate and concentrated under reduced pressure, and the resulting residue was purified by column chromatography to obtain the title target compound (1.4 g).

### Step 5 (e) Synthesis of 4-((2-(1-(cyclohexylamino)-1-oxopropan-2-yl)-5-nitrophenyl)ethynyl)-N-(2-(piperidin-1-yl)ethyl)benzamide

TEA (0.1 mL, 3 eq), CuI (1.4 mg, 3 mol%), and Pd[PPh₃]₄ (5.3 mg, 3 mol%) were added to a solution of N-cyclohexyl-2-(2-iodo-4-nitrophenyl)propenamide dissolved in MeCN (2 mL). The mixture was stirred at room temperature for five minutes under a nitrogen atmosphere. Then, 4-ethynyl-N-(2-(piperidin-1-yl)ethyl)benzamide (7 mg, 0.3 mmol, 1.0 eq) was added. The reaction mixture was stirred at room temperature for one hour and concentrated under reduced pressure. The obtained residue was purified by column chromatography to obtain the target compound (0.15 g).

### Step 6 (f) Synthesis of 4-((5-amino-2-(1-(cyclohexylamino)-1-oxopropan-2-yl)phenyl)ethynyl)-N-(2-(piperidin-1-yl)ethyl)benzamide

4-((2-(1-(cyclohexylamino)-1-oxopropan-2-yl)-5-nitrophenyl)ethynyl)-N-(2-(piperidin-1-yl)ethyl)benzamide (0.26 g, 0.49 mmol, 1.0 eq), Fe (0.27 g, 10 eq), NH₄Cl (0.24 g, 10 eq), EtOH (0.1 M), and H₂O (0.1 M) were added and stirred at 90°C for two hours. When the reactant became a black suspension and the reaction was completed, the reaction mixture was washed with water and extracted with DCM. The organic layer was dried over magnesium sulfate and concentrated under reduced pressure to obtain the target compound as a crude product.

### Step 7 (g) Synthesis of phenyl (4-(1-(cyclohexylamino)-1-oxopropan-2-yl)-3-((4-((2-(piperidin-1-yl)ethyl)carbamoyl)phenyl)ethynyl)phenyl)carbamate

4-((5-amino-2-(1-(cyclohexylamino)-1-oxopropan-2-yl)phenyl)ethynyl)-N-(2-(piperidin-1-yl)ethyl)benzamide (0.24 g, 0.48 mmol, 1.0 eq) and pyridine (0.087 ml, 2 eq) were dissolved in THF (10 mL), and then the reaction solution was cooled with ice, phenyl chloroformate (0.091 ml, 1.5 eq) was added, and the mixture was stirred at room temperature for two hours. After the reaction was completed, water (0.2 mL) was added to the reaction mixture, and the reaction mixture was concentrated under reduced pressure. The obtained residue was diluted with water and EA. The organic layer was washed with water, dried over magnesium sulfate, and concentrated under reduced pressure, and the obtained residue was purified by column chromatography (silica gel, 0-5% MeOH/DCM) to obtain the target compound.

### Step 8 (h) Synthesis of 4-((2-(1-(cyclohexylamino)-1-oxopropan-2-yl)-5-(3-(2-(pyridin-3-yl)ethyl)ureido)phenyl)ethynyl)-N-(2-(piperidin-1-yl)ethyl)benzamide

A mixture of phenyl (4-(1-(cyclohexylamino)-1-oxopropan-2-yl)-3-((4-((2-(piperidin-1-yl)ethyl)carbamoyl)phenyl)ethynyl)phenyl)carbamate (0.08 g, 0.13 mmol, 1.0 eq), 3-(2-aminoethyl)pyridine (0.031 g, 0.25 mmol, 2 eq) and TEA (0.054 ml, 3 eq) dissolved in THF (2 mL) was heated at 80°C for 24 hours. The reaction mixture was concentrated under reduced pressure, and the obtained residue was purified by column chromatography (silica gel, 0-5% MeOH/DCM) to obtain the target compound.

¹H NMR (400 MHz, MeOD) δ 8.47 (d, *J=* 1.4 Hz, 1H), 8.42 (dd, *J=* 4.8, 1.2 Hz, 1H), 7.89 (d, *J =* 8.3 Hz, 2H), 7.81 - 7.79 (m, 1H), 7.69 - 7.67 (m, 3H), 7.50 (d, *J =* 7.8 Hz, 1H), 7.42 (dd, *J* = 7.6, 4.9 Hz, 1H), 7.36 - 7.34 (m, 1H), 7.28 - 7.27 (m, 1H), 4.13 (q, *J* = 7.2 Hz, 1H), 3.64 - 3.61 (m, 3H), 3.50 (t, *J =* 7.0 Hz, 2H), 3.37 (s, 1H), 2.92 (t, *J =* 6.9 Hz, 2H), 2.76 - 2.71 (m, 6H), 1.86 - 1.83 (m, 1H), 1.71 - 1.65 (m, 10H), 1.51 (d, *J=* 7.1 Hz, 3H), 1.39 - 1.00 (m, 6H).

The reactants may be changed in substantially the same manner as in the synthesis method of above Example 31, so as to synthesize compound 101.

### Example 32: Synthesis of compound 69 (synthesis reaction formula 27)

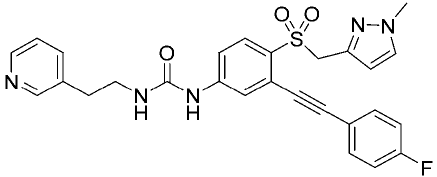

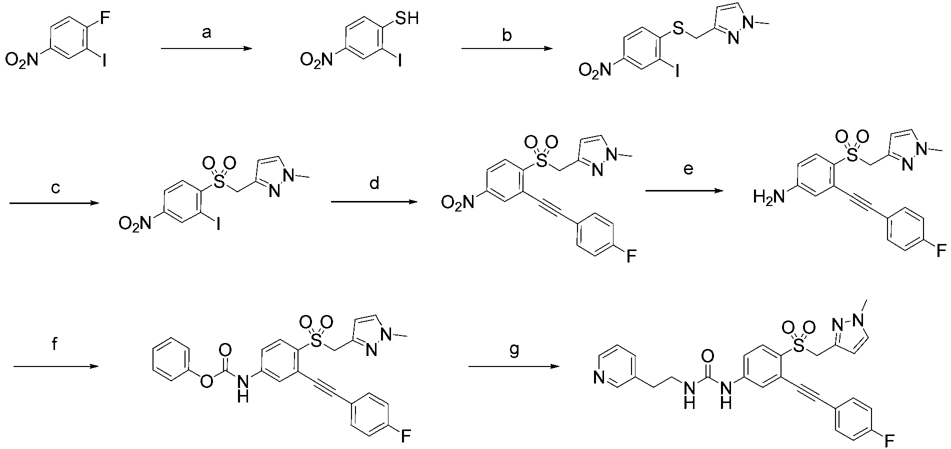

Reaction Formula 27. (a) Na₂S, DMF, r.t (b) 3-(chloromethyl)-1-methyl-1H-pyrazole, K₂CO₃, DMF, RT, 16 hours (c) mCPBA, DCM, RT, 16 hours (d) PdCl₂[PPh₃]₂, CuI, TEA, ACN, RT, 16 hours (e) Fe, AcOH, 80°C, 2 hours (2h); (f) phenyl chloroformate, pyridine, THF, RT, 3 hours; (g) 2-(pyridin-3-yl)ethan-1-amine, TEA, THF, reflux, 24 hours

### Step 1 (a) Synthesis of 2-iodo-4-nitrobenzenethiol

1-fluoro-2-iodo-4-nitrobenzene (5 g, 0.019 mol, 1 eq) was dissolved in DMF (25 ml), and sodium disulfide (1.6 g, 0.021 mol, 1.1 eq) was added and stirred at room temperature for 16 hours. When the reaction was completed, 200 ml of water was added to the reactant, and the pH was adjusted to 5 with 1N HCl, and extracted several times with DCM. The organic layer was dried over magnesium sulfate and concentrated under reduced pressure, and the obtained residue was purified by column chromatography (silica gel, 25% EA/HX) to obtain the target compound (4 g, 0.014 mol, 73%).

### Step 2 (b) Synthesis of 3-(((2-iodo-4-nitrophenyl)thio)methyl)-1-methyl-1H-pyrazole

2-iodo-4-nitrobenzenethiol (4 g, 0.014 mol, 1 eq) was dissolved in DMF (40 ml) and K₂CO₃ (3.9 g, 0.028 mol, 2 eq) was added and stirred. 3-(chloromethyl)-1-methyl-1H-pyrazole (1.85 g, 0.014 mol, 1 eq) was added to the reactant and stirred for 16 hours. When the reaction was completed by confirming the reaction through TLC, water was added to the reactant and the resulting precipitate was filtered to obtain the target compound (4.9 g, 0.013 mol, 93%).

### Step 3 (c) Synthesis of 3-(((2-iodo-4-nitrophenyl)sulfonyl)methyl)-1-methyl-1H-pyrazole

3-(((2-iodo-4-nitrophenyl)thio)methyl)-1-methyl-1H-pyrazole (4.5 g, 0.012 mmol, 1 eq) was dissolved in DCM (200 ml), and meta-chloroperoxybenzoic acid (mCPBA, 8.3 g, 0.048 mol, 4 eq) was added and stirred for two days. The reaction was confirmed through TLC, and mCPBA was further added or, when the reaction was completed, water was added to dilute, neutralized with saturated NaHCO₃ aqueous solution, and extracted several times with DCM. The organic layer was dried over magnesium sulfate, concentrated under reduced pressure, and subjected to recrystallization (MeOH/MC) to obtain the target compound (3 g, 0.0074 mol, 62%).

### Step 4 (d) Synthesis of 3-(((2-((4-fluorophenyl)ethynyl)-4-nitrophenyl)sulfonyl)methyl)-1-methyl-1H-pyrazole

MeCN (30 ml) was added to 3-(((2-iodo-4-nitrophenyl)sulfonyl)methyl)-1-methyl-1H-pyrazole (3 g, 0.0074 mol, 1 eq), and TEA (3 ml, 0.022 mol, 3 eq) was added. PdCl₂[PPh₃]₂ (155 mg, 3 mol%) and CuI (42 mg, 3 mol%) were added to the reactant and stirred at room temperature for five minutes under a nitrogen atmosphere. Then, 4-fluorophenyl-acetylene (1 g, 0.0088 mol, 1.2 eq) was added. The reaction mixture was stirred at room temperature for 16 hours and concentrated under reduced pressure. The obtained residue was purified by column chromatography (silica gel, 4% MeOH/DCM) to obtain the target compound (2 g, 0.005 mmol, 68%).

### Step 5 (e) Synthesis of 3-((4-fluorophenyl)ethynyl)-4-(((1-methyl-1H-pyrazol-3-yl)methyl)sulfonyl)aniline

AcOH (30 ml) and iron (2.8 g, 0.05 mol, 10 eq) were added to 3-(((2-((4-fluorophenyl)ethynyl)-4-nitrophenyl)sulfonyl)methyl)-1-methyl-1H-pyrazole (2 g, 0.005 mol, 1 eq)and the resulting mixture was stirred at 85°C for two to three hours. The catalyst was removed by celite filtration, and the filtrate was concentrated under reduced pressure. The residue was basified with potassium carbonate aqueous solution and extracted with EA, and then the organic layer was concentrated under reduced pressure to obtain the target compound as a crude product.

### Step 6 (f) Synthesis of phenyl (3-((4-fluorophenyl)ethynyl)-4-(((1-methyl-1H-pyrazol-3-yl)methyl)sulfonyl)phenyl)carbamate

3-((4-fluorophenyl)ethynyl)-4-(((1-methyl-1H-pyrazol-3-yl)methyl)sulfonyl)aniline (1.3 g, 0.0035 mol, 1 eq) and pyridine (0.56 ml, 0.0053 mol, 2 eq) were dissolved in THF, and then the reaction solution was cooled with ice, phenyl chloroformate (0.66 ml, 0.0053 mol, 1.5 eq) was added, and the mixture was stirred at room temperature for three to four hours. After the reaction was completed, water (0.1 mL) was added to the reaction mixture, and the reaction mixture was concentrated under reduced pressure. Water was added to the obtained residue and extracted with EA. The organic layer was dried over magnesium sulfate and concentrated under reduced pressure, and the obtained residue was purified by column chromatography (silica gel, 4-6% MeOH/DCM) to obtain the target compound (0.8 g, 0.0016 mol, 46%).

### Step 7 (g) Synthesis of 1-(3-((4-fluorophenyl)ethynyl)-4-(((1-methyl-1H-pyrazol-3-yl)methyl)sulfonyl)phenyl)-3-(2-(pyridin-3-yl)ethyl)urea (compound 69)

A mixture of phenyl (3-((4-fluorophenyl)ethynyl)-4-(((1-methyl-1H-pyrazol-3-yl)methyl)sulfonyl)phenyl)carbamate (0.1 g, 0.2 mmol, 1.0 eq), 3-(2-aminoethyl)pyridine (2 eq) and TEA (3 eq) dissolved in THF (4 mL) was heated at 80°C for 24 hours. The reaction mixture was concentrated under reduced pressure, and the obtained residue was purified by column chromatography (silica gel, 0-5% MeOH/DCM) to obtain the target compound.

¹H NMR (400 MHz, MeOD) δ 8.48 (s, 1H), 8.42 (m, 1H), 7.90 (s, 1H), 7.81 (d, *J* = 7.7 Hz, 1H), 7.74 -7.71 (m, 3H), 7.47 - 7.38 (m, 3H), 7.22 (t, *J* = 8.1 Hz, 2H), 6.13 (s, 1H), 4.75 (s, 2H), 3.77 (s, 3H), 3.52 (t, *J=* 6.7 Hz, 2H), 2.93 (t, *J =* 6.5 Hz, 2H).

MS(ESI) m/e MH⁺ 518.4

The reactants may be changed in substantially the same manner as in the synthesis method of above Example 32, so as to synthesize compounds 23, 24, 70, 71, 77, 78, 79, 80, 81, 93, 98, 100, and 150.

### Example 33: Synthesis of compound 183

### Synthesis of 1-(3-((4-fluorophenyl)ethynyl)-4-(((1-methyl-1H-pyrazol-3-yl)methyl)sulfonyl)phenyl)-3-(imidazo[1,2-a]pyridin-7-ylmethyl)urea

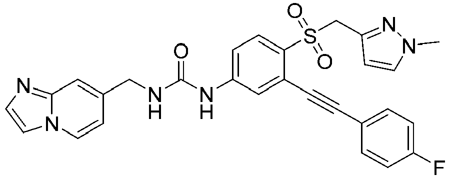

Compound 183 was prepared in substantially the same manner as in the synthesis of compound 69, except that 1-{imidazo[1,2-a]pyridin-7-yl}methanamine dihydrochloride was used instead of 3-(2-aminoethyl)pyridine in step 7) compared to the synthesis of compound 69 in Example 32.

¹H NMR (400 MHz, DMSO) δ 9.41 (s, 1H), 8.50 (d, J = 6.9 Hz, 1H), 8.17 (s, 1H), 8.00 (s, 1H), 7.89 (s, 1H), 7.75 (m, 2H), 7.67 (d, J = 8.8 Hz, 1H), 7.58 (s, 1H), 7.52 (s, 1H), 7.46 (d, J = 8.7 Hz, 1H), 7.41 (s, 1H), 7.35 (t, J = 8.5 Hz, 2H), 7.12 (m, 1H), 6.86 (d, J = 6.9 Hz, 1H), 6.03 (s, 1H), 4.72 (s, 2H), 4.36 (d, J = 4.8 Hz, 2H), 3.73 (s, 3H).

MS(ESI) m/e MH⁺ 543.4

### Example 34: Synthesis of compound 177 (synthesis reaction formula 28)

### Synthesis of 3-amino-N-(2-((4-fluorophenyl)ethynyl)-4-(3-(2-(pyridin-3-yl)ethyl)ureido)phenyl)benzamide

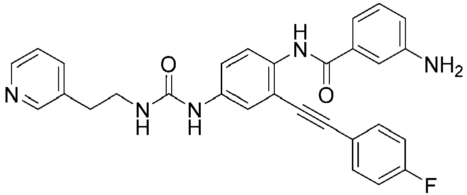

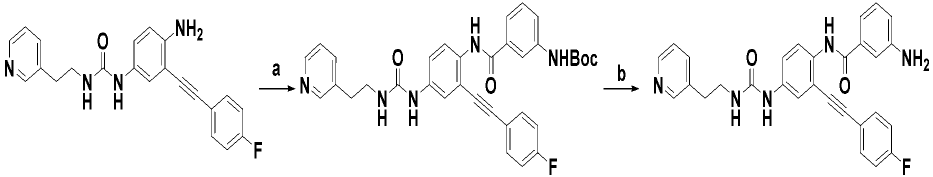

Reaction Formula 28. Reagents and conditions: (a) 3-((tert-butoxycarbonyl)amino)benzoic acid, DIPEA, HATU, DCM, room temperature (rt), 24 hours (24h); (b) 4N HCl in dioxane, 1,4-dioxane, room temperature (rt), 24 hours (24h)

### Step 1 (a) Synthesis of tert-butyl (3-((2-((4-fluorophenyl)ethynyl)-4-(3-(2-(pyridin-3-yl)ethyl)ureido)phenyl)carbamoyl)phenyl)carbamate

1-(4-amino-3-((4-fluorophenyl)ethynyl)phenyl)-3-(2-(pyridin-3-yl)ethyl)urea (0.05 g, 0.134 mmol, 1 eq) was dissolved in DCM (0.5 ml), and then 3-((tert-butoxycarbonyl)amino)benzoic acid (0.032g, 0.134 mmol, 1 eq), DIPEA (0.07 ml, 3 eq), and HATU (0.061 g, 1.2 eq) were added and stirred at room temperature for 24 hours. The reaction mixture was concentrated and then purified by reverse-phase column chromatography to obtain the title compound (0.047 g, 59.5%).

### Step 2 (b) Synthesis of 3-amino-N-(2-((4-fluorophenyl)ethynyl)-4-(3-(2-(pyridin-3-yl)ethyl)ureido)phenyl)benzamide

Tert-butyl (3-((2-((4-fluorophenyl)ethynyl)-4-(3-(2-(pyridin-3-yl)ethyl)ureido)phenyl)carbamoyl)phenyl)carbamate (0.047 g, 0.079 mmol, 1 eq) was dissolved in 1,4-dioxane (0.9 ml, 0.2 M), and then 4N HCl in dioxane (0.2 ml, 0.79 mmol, 10 eq) was added and stirred at room temperature for 24 hours. The reaction mixture was concentrated and then purified by reverse-phase column chromatography to obtain the title compound (2.62 mg, 7%).

¹H NMR (400 MHz, MeOD) δ 8.44 (d, *J* = 21.9 Hz, 2H), 7.82 - 7.74 (m, 2H), 7.68 (d, *J= 2.3* Hz, 1H), 7.54 - 7.46 (m, 2H), 7.44 - 7.37 (m, 1H), 7.32 (dd, *J =* 8.8, 2.3 Hz, 1H), 7.29 - 7.19 (m, 3H), 7.11 (t, *J =* 8.7 Hz, 2H), 6.94 - 6.88 (m, 1H), 3.49 (t, *J=* 7.0 Hz, 2H), 2.91 (t, *J* = 6.9 Hz, 2H).

The reactants may be changed in substantially the same manner as in the synthesis method of above Example 34, so as to synthesize compounds 123, 125, and 166.

### Example 35: Synthesis of compound 124 (synthesis reaction formula 29)

### Synthesis of 4-fluoro-N-(2-((4-fluorophenyl)ethynyl)-4-(3-(2-(pyridin-3-yl)ethyl)ureido)phenyl)benzamide

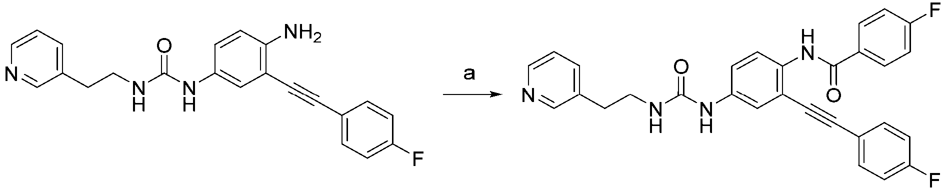

### Reaction Formula 29. Reagents and conditions: (a) 4-fluorobenzoic acid, DIPEA, HATU, DCM, RT, 24 hours;

1-(4-amino-3-((4-fluorophenyl)ethynyl)phenyl)-3-(2-(pyridin-3-yl)ethyl)urea (0.05 g, 0.134 mmol, 1 eq) was dissolved in DCM (0.5 ml), and then 4-fluorobenzoic acid (0.019 g, 0.134 mmol, 1 eq), DIPEA (0.07 ml, 3 eq), and HATU (0.061 g, 1.2 eq) were added and stirred at room temperature for 24 hours. The reaction mixture was concentrated and then purified by reverse-phase column chromatography to obtain the title compound (0.047 g, 59.5%).

¹H NMR (400 MHz, MeOD) δ 8.46 (d, *J =* 24.0 Hz, 2H), 8.12 - 8.03 (m, 2H), 7.81 (d, *J =* 7.8 Hz, 1H), 7.72 (d, *J* = 2.4 Hz, 1H), 7.67 (d, *J* = 8.8 Hz, 1H), 7.55 - 7.46 (m, 2H), 7.45 - 7.39 (m, 1H), 7.36 (dd, *J* = 8.8, 2.5 Hz, 1H), 7.28 (t, *J* = 8.7 Hz, 2H), 7.12 (t, *J* = 8.8 Hz, 2H), 3.51 (t, *J =* 7.0 Hz, 2H), 2.93 (t, *J=* 6.9 Hz, 2H).

### Example 36: Synthesis of compound 122 (synthesis reaction formula 30)

### Synthesis of N-(2-((4-fluorophenyl)ethynyl)-4-(3-(2-(pyridin-3-yl)ethyl)ureido)phenyl)propionamide

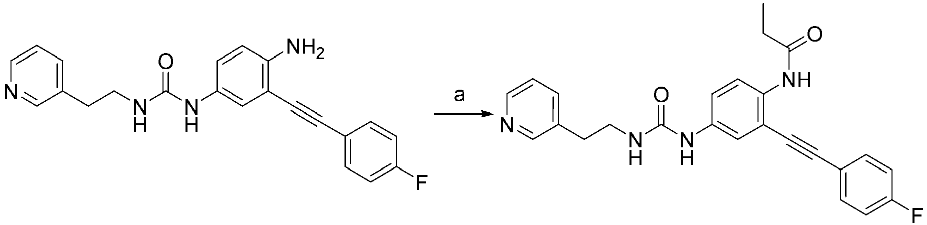

Reaction Formula 30. Reagents and conditions: (a) propionic acid, DIPEA, HATU, DCM, RT, 24 hours;

1-(4-amino-3-((4-fluorophenyl)ethynyl)phenyl)-3-(2-(pyridin-3-yl)ethyl)urea (0.05 g, 0.134 mmol, 1 eq) was dissolved in DCM (0.5 ml), and then propionic acid (0.01 ml, 0.134 mmol, 1 eq), DIPEA (0.07 ml, 3 eq), and HATU (0.061 g, 1.2 eq) were added and stirred at room temperature for 24 hours. The reaction mixture was concentrated and then purified by column chromatography (silica gel, DCM/MeOH) to obtain the title compound (0.039 g, 67%).

¹H NMR (400 MHz, DMSO) δ 9.30 (s, 1H), 8.58 (s, 1H), 8.47 (d, *J* = 1.7 Hz, 1H), 8.44 (dd, *J* = 4.7, 1.4 Hz, 1H), 7.73 (d, *J =* 2.4 Hz, 1H), 7.70 - 7.62 (m, 3H), 7.52 (d, *J* = 8.7 Hz, 1H), 7.37 - 7.28 (m, 3H), 7.22 (dd, *J =* 8.8, 2.4 Hz, 1H), 6.23 (t, *J =* 5.7 Hz, 1H), 3.37 (dd, *J=* 13.0, 6.9 Hz, 2H), 2.79 (t, *J=* 7.0 Hz, 2H), 2.42 - 2.35 (m, 2H), 1.11 (t, *J =* 7.5 Hz, 3H).

### Example 37: Compound 74 (synthesis reaction formula 31)

### Synthesis of 1-(3-((4-fluorophenyl)ethynyl)-4-(2-oxo-2-(piperidin-1-yl)ethyl)phenyl)-3-(2-(pyridin-3-yl)ethyl)urea

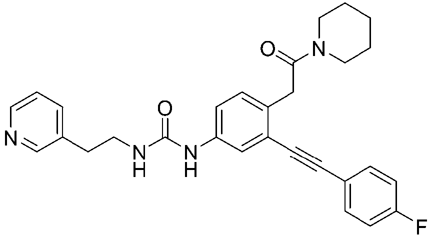

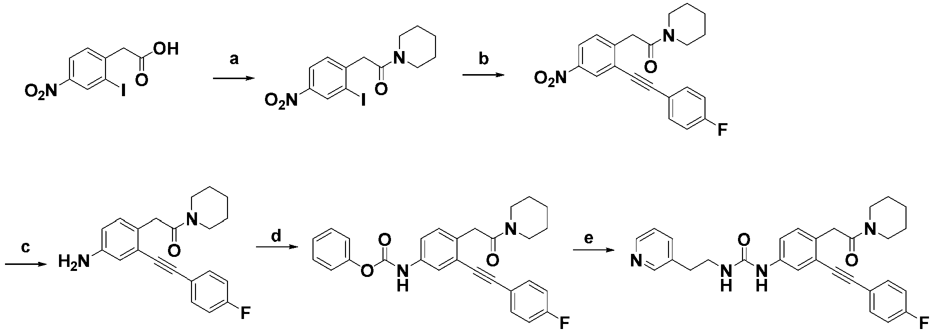

Reaction Formula 31 (a) TBTU, piperidine, TEA, DCM (b) Pd[PPh₃]₄, CuI, TEA, 1-ethynyl-4-fluorobenzene, ACN, RT, 1 hour; (c) Zn, NH₄Cl, 1,4-dioxane, water (H₂O); (d) phenyl chloroformate, pyridine, THF, RT, 2 hours; (e) 2-(pyridin-3-yl)ethan-1-amine, TEA, THF, reflux, 24 hours

### Step 1 (a): Synthesis of 2-(2-iodo-4-nitrophenyl)-1-(piperidin-1-yl)ethan-1-one

To 2-(2-iodo-4-nitrophenyl)acetic acid (1 g, 3.26 mmol), DCM 20 ml and TBTU (1.57 g, 1.5 eq) were added and stirred at room temperature for 50 minutes. TEA (0.9 ml, 2 eq) and piperidine (0.38 ml, 1.2 eq) were added and stirred for 24 hours. Water was added to the reactant and extracted with ethyl acetate, and the organic layer was dried over magnesium sulfate, and concentrated under reduced pressure to obtain the target compound (1.1 g, 90%).

### Step 2 (b): Synthesis of 2-(2-((4-fluorophenyl)ethynyl)-4-nitrophenyl)-1-(piperidin-1-yl)ethan-1-one

Triethylamine (1.22 ml, 3 eq), CuI (17 mg, 3 mol%) and Pd[PPh₃]₄ (62 mg, 3 mol%) were added to a solution of 2-(2-iodo-4-nitrophenyl)-1-(piperidin-1-yl)ethan-1-one (1.1 g, 2.94 mmol) dissolved in ACN (30 mL). The mixture was stirred at room temperature for five minutes under a nitrogen atmosphere. Then, 1-ethynyl-4-fluorobenzene (0.42 mg, 1.2 eq) was added. The reaction mixture was stirred at room temperature for one hour and concentrated under reduced pressure. The obtained residue was purified by column chromatography (30% EA/HX) to obtain the target compound (0.84 g, 78%).

### Step 3 (c): Synthesis of 2-(4-amino-2-((4-fluorophenyl)ethynyl)phenyl)-1-(piperidin-1-yl)ethan-1-one

2-(2-((4-fluorophenyl)ethynyl)-4-nitrophenyl)-1-(piperidin-1-yl)ethan-1-one (0.84 g, 2.3 mmol, 1.0 eq), Zn (1.5 g, 10 eq), NH₄Cl (1.37 g, 10 eq), and dioxane:water (H₂O) (12 ml, 3:1 (v:v), 0.02 M) were added and stirred at room temperature for 24 hours. When the reaction was completed, the resulting mixture was concentrated under reduced pressure to remove dioxane therefrom, and the reaction mixture was washed with water and saturated NaHCO₃ solution, and then extracted with ethyl acetate. The organic layer was dried over magnesium sulfate and concentrated under reduced pressure to obtain the title target compound as a crude product.

### Step 4 (d): Synthesis of phenyl (3-((4-fluorophenyl)ethynyl)-4-(2-oxo-2-(piperidin-1-yl)ethyl)phenyl)carbamate

2-(4-amino-2-((4-fluorophenyl)ethynyl)phenyl)-1-(piperidin-1-yl)ethan-1-one (1.0 eq) and pyridine (0.42 ml, 2 eq) were dissolved in tetrahydrofuran (10 mL), and then the reaction solution was cooled with ice, phenyl chloroformate (0.44 ml, 1.5 eq) was added, and the mixture was stirred at room temperature for two hours. After the reaction was completed, water (0.2 mL) was added to the reaction mixture, and the reaction mixture was concentrated under reduced pressure. The obtained residue was diluted with water and ethyl acetate. The organic layer was washed with water, dried over magnesium sulfate, and concentrated under reduced pressure, and the obtained residue was purified by column chromatography (silica gel, 0-5% methanol/dichloromethane) to obtain the target compound.

### Step 5 (e): Synthesis of 1-(3-((4-fluorophenyl)ethynyl)-4-(2-oxo-2-(piperidin-1-yl)ethyl)phenyl)-3-(2-(pyridin-3-yl)ethyl)urea

A mixture of phenyl (3-((4-fluorophenyl)ethynyl)-4-(2-oxo-2-(piperidin-1-yl)ethyl)phenyl)carbamate (0.2 g, 0.44 mmol, 1.0 eq), 3-(2-aminoethyl)pyridine (0.1 g, 2 eq) and triethylamine (0.18 ml, 3 eq) dissolved in THF (8 mL) was heated at 80°C for 24 hours. The reaction mixture was concentrated under reduced pressure, and the obtained residue was purified by column chromatography (silica gel, 0-5% methanol/dichloromethane) to obtain the target compound.

¹H NMR (400 MHz, DMSO) δ 8.59 (s, 1H), 8.47 (d, *J=* 1.8 Hz, 1H), 8.44 (dd, *J =* 4.7, 1.5 Hz, 1H), 7.71 (d, *J* = 2.2 Hz, 1H), 7.68 (dt, *J* = 7.6, 1.7 Hz, 1H), 7.64 - 7.57 (m, 2H), 7.37 - 7.27 (m, 3H), 7.23 (dd, *J* = 8.4, 2.3 Hz, 1H), 7.13 (d, *J =* 8.4 Hz, 1H), 6.25 (t, *J* = 5.6 Hz, 1H), 3.80 (s, 2H), 3.47 - 3.42 (m, 4H), 3.37 (dd, *J* = 12.9, 6.8 Hz, 2H), 2.79 (t, *J* = 7.0 Hz, 2H), 1.54 - 1.52 (m, *J =* 5.1 Hz, 2H), 1.39 (s, 4H).

The reactants may be changed in substantially the same manner as in the synthesis method of above Example 37, so as to synthesize compounds 99, 75, and 76.

### Example 38: Compound 66 (synthesis reaction formula 32)

### Synthesis of N-(piperidin-4-yl)-4-((5-(3-(2-(pyridin-3-yl)ethyl)ureido)-2-(pyridin-4-yl)phenyl)ethynyl)benzamide

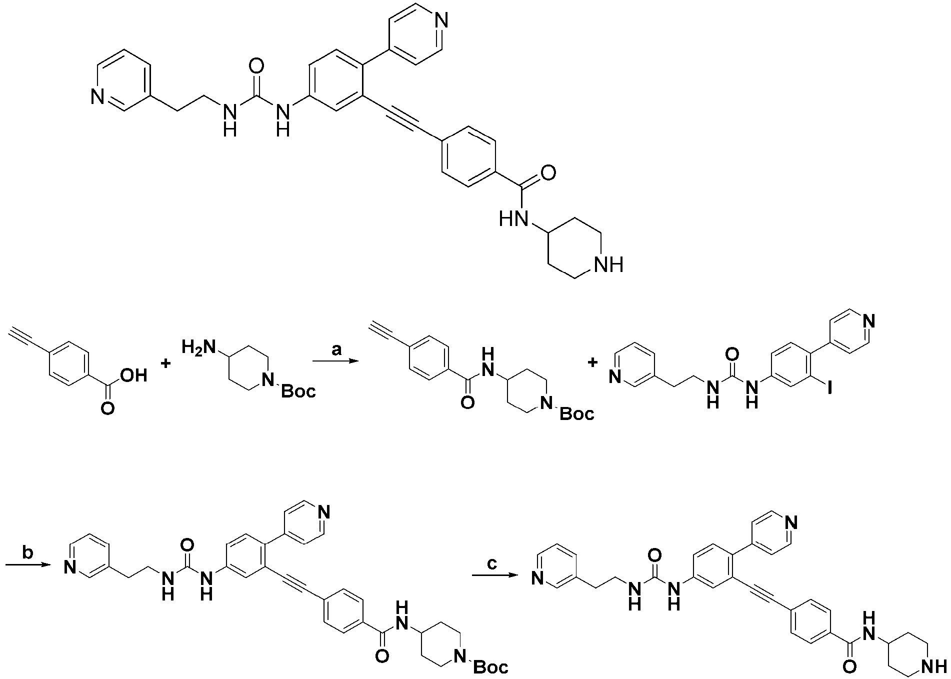

Reaction Formula 32. Reagents and conditions: (a) HATU, DIPEA, DCM, 0°C to RT, overnight; (b) PdCl₂(PPh₃)₂, CuI, TEA, ACN, 60°C, overnight; (c) TFA, DCM, RT, overnight

### Step 1 (a): Synthesis of tert-butyl 4-(4-ethynylbenzamido)piperidine-1-carboxylate

4-ethynylbenzoic acid (0.5 g, 3.42 mmol, 1 eq) was dissolved in DCM (34.2 ml, 0.1 M), and then HATU (1.55 g, 4.1 mmol, 1.5 eq) was added and stirred at room temperature for 10 minutes. Tert-butyl 4-aminopiperidin-1-carboxylate (0.821 g, 4.1 mmol, 1.5 eq) and DIPEA (1.326 g, 10.26 mmol, 3 eq) were added to the reaction solution and stirred at room temperature overnight. After completion of the reaction, the resulting mixture was extracted with EA and water (H₂O). The organic layer was dried over MgSO₄, filtered, concentrated and then purified by column chromatography (silica gel, MeOH/DCM) to obtain the title compound (0.769 g, 68.4%).

### Step 2 (b) Synthesis of tert-butyl 4-(4-((5-(3-(2-(pyridin-3-yl)ethyl)ureido)-2-(pyridin-4-yl)phenyl)ethynyl)benzamido)piperidine-1-carboxylate

1-(3-iodo-4-(pyridin-4-yl)phenyl)-3-(2-(pyridin-3-yl)ethyl)urea (0.05 g, 0.11 mmol, 1 eq), tert-butyl 4-(4-ethynylbenzamido)piperidin-1-carboxylate (0.054 g, 0.165 mmol, 1.5 eq), PdCl₂(PPh₃)₂ (0.002 g, 0.0033 mmol, 0.03 eq), CuI (0.00063 g, 0.0033 mmol, 0.03 eq), and TEA (0.022 g, 0.22 mmol, 2 eq) were dissolved in ACN (1.1 ml, 0.1 M) and degassed with nitrogen (N₂) gas. The reactant was heated to 60°C and stirred overnight. After completion of the reaction, the resulting mixture was extracted with DCM and water (H₂O). The organic layer was dried over MgSO₄, filtered, concentrated and then purified by column chromatography (silica gel, MeOH/DCM) to obtain the title compound (0.021 g, 29.6%).

### Step 3 (c) Synthesis of N-(piperidin-4-yl)-4-((5-(3-(2-(pyridin-3-yl)ethyl)ureido)-2-(pyridin-4-yl)phenyl)ethynyl)benzamide

Tert-butyl 4-(4-((5-(3-(2-(pyridin-3-yl)ethyl)ureido)-2-(pyridin-4-yl)phenyl)ethynyl)benzamido)piperidin-1-carboxylate (0.021g, 0.032 mmol, 1 eq) was dissolved in DCM (1 ml, 0.07 M), and then TFA (0.036 g, 0.32 mmol, 10 eq) was added and stirred at room temperature overnight. After completion of the reaction, the resulting mixture was concentrated and purified by reverse-phase column chromatography (0.1% formic acid in H₂O/ACN). (0.008 g, 49.3%)

¹H NMR (400 MHz, DMSO) δ 9.22 (s, 1H), 8.66 (d, J = 5.6 Hz, 2H), 8.45 (dd, J = 11.1, 6.4 Hz, 3H), 8.33 (s, 1H), 7.95 (s, 1H), 7.87 (d, J = 8.2 Hz, 2H), 7.67 (dd, J = 12.2, 6.7 Hz, 3H), 7.46 (dd, J = 14.7, 8.4 Hz, 4H), 7.38 - 7.30 (m, 1H), 6.76 (s, 1H), 3.96 (s, 1H), 3.44 - 3.34 (m, 5H), 2.85 - 2.77 (m, 3H), 1.93-1.85 (m, 2H), 1.68-1.58 (m, 2H).

### Example 39: Compound 65 (synthesis reaction formula 33)

### Synthesis of 1-(3-((4-(4-aminopiperidin-1-carbonyl)phenyl)ethynyl)-4-(pyridin-4-yl)phenyl)-3-(2-(pyridin-3-yl)ethyl)urea

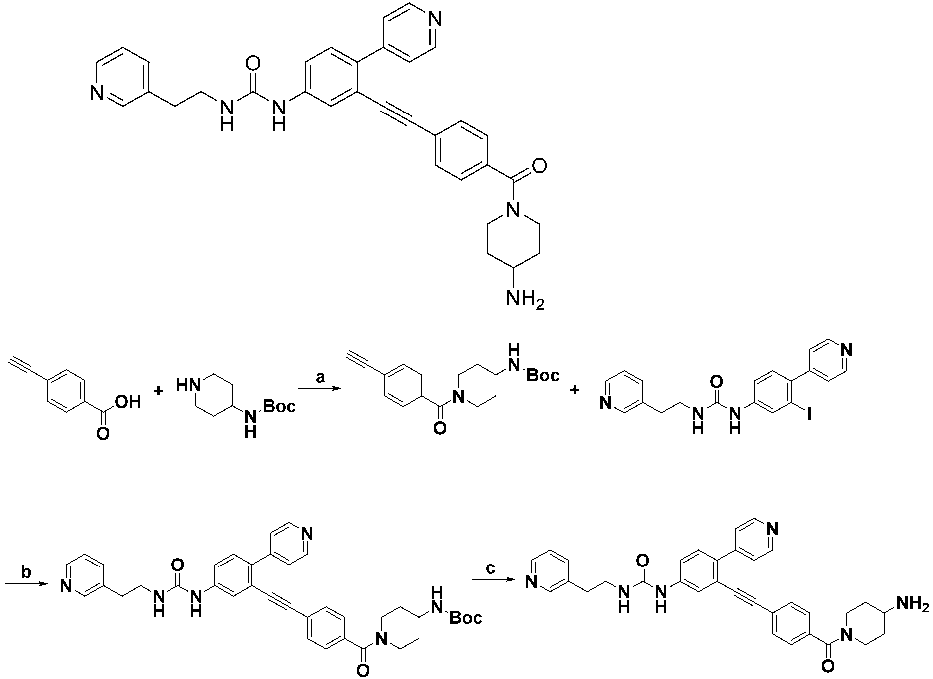

Reaction Formula 33. Reagents and conditions: (a) HATU, DIPEA, DCM, 0°C to RT, overnight; (b) PdCl₂(PPh₃)₂, CuI, TEA, ACN, 60°C, overnight; (c) TFA, DCM, RT, overnight

### Step 1 (a) Synthesis of tert-butyl (1-(4-ethynylbenzoyl)piperidin-4-yl)carbamate

4-ethynylbenzoic acid (0.5 g, 3.42 mmol, 1 eq) was dissolved in DCM (34.2 ml, 0.1 M), and then HATU (1.55 g, 4.1 mmol, 1.5 eq) was added and stirred at room temperature for 10 minutes. Tert-butyl piperidin-4-ylcarbamate (0.821 g, 4.1 mmol, 1.5 eq) and DIPEA (1.326 g, 10.26 mmol, 3 eq) were added to the reaction solution and stirred at room temperature overnight. After completion of the reaction, the resulting mixture was extracted with EA and water (H₂O). The organic layer was dried over MgSO₄, filtered, concentrated and then purified by column chromatography (silica gel, MeOH/DCM) to obtain the title compound (0.640 g, 57%).

### Step 2 (b) Synthesis of tert-butyl (1-(4-((5-(3-(2-(pyridin-3-yl)ethyl)ureido)-2-(pyridin-4-yl)phenyl)ethynyl)benzoyl)piperidin-4-yl)carbamate

1-(3-iodo-4-(pyridin-4-yl)phenyl)-3-(2-(pyridin-3-yl)ethyl)urea (0.1 g, 0.23 mmol, 1 eq), tert-butyl (1-(4-ethynylbenzoyl)piperidin-4-yl)carbamate (0.113 g, 0.345 mmol, 1.5 eq), PdCl₂(PPh₃)₂ (0.005 g, 0.0069 mmol, 0.03 eq), CuI (0.0013 g, 0.0069 mmol, 0.03 eq), and TEA (0.047 g, 0.46 mmol, 2 eq) were dissolved in ACN (2.3 ml, 0.1 M) and degassed with nitrogen (N₂) gas. The reactant was heated to 60°C and stirred overnight. After completion of the reaction, the resulting mixture was extracted with DCM and water (H₂O). The organic layer was dried over MgSO₄, filtered, concentrated and then purified by column chromatography (silica gel, MeOH/DCM) to obtain the title compound (0.075 g, 50.6%).

### Step 3 (c) Synthesis of 1-(3-((4-(4-aminopiperidin-1-carbonyl)phenyl)ethynyl)-4-(pyridin-4-yl)phenyl)-3-(2-(pyridin-3-yl)ethyl)urea

Tert-butyl (1-(4-((5-(3-(2-(pyridin-3-yl)ethyl)ureido)-2-(pyridin-4-yl)phenyl)ethynyl)benzoyl)piperidin-4-yl)carbamate (0.068 g, 0.1 mmol, 1 eq) was dissolved in DCM (1 ml, 0.1 M), and then TFA (0.114 g, 1 mmol, 10 eq) was added and stirred at room temperature overnight. After completion of the reaction, the resulting mixture was concentrated and purified by reverse-phase column chromatography (0.1% formic acid in H₂O/ACN) to obtain the title compound (0.0337 g, 61.9%).

¹H NMR (400 MHz, DMSO) δ 8.94 (s, 1H), 8.68 (s, 2H), 8.48 (d, J = 13.5 Hz, 2H), 7.97 - 7.90 (m, 3H), 7.73 (d, J = 7.8 Hz, 1H), 7.68 (d, J = 5.3 Hz, 2H), 7.50 - 7.44 (m, 4H), 7.41 - 7.36 (m, 3H), 6.46 (t, J = 5.6 Hz, 1H), 4.57-4.27 (m, 1H), 3.58 (s, 2H), 3.39 (dd, J = 12.7, 6.7 Hz, 3H), 3.13 (s, 1H), 2.81 (t, J = 6.9 Hz, 2H), 2.02-1.81 (m, 2H), 1.43 (s, 2H); MS MH⁺ 545.13

### Example 40: Compound 126 (synthesis reaction formula 34)

### Synthesis of N-(4-((5-(3-(2-(pyridin-3-yl)ethyl)ureido)-2-(pyridin-4-yl)phenyl)ethynyl)phenyl)acetamide

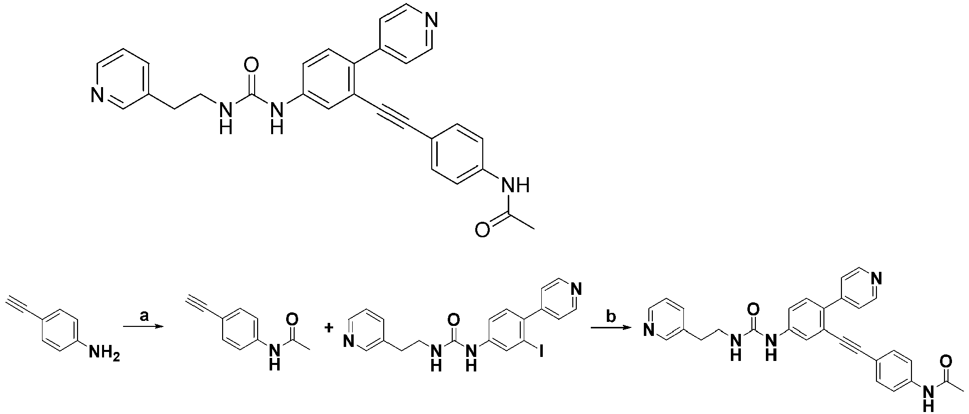

Reaction Formula 34. Reagents and conditions: (a) acetic anhydride, DCM, RT, overnight; (b) PdCl₂(PPh₃)₂, CuI, TEA, ACN, 60°C, overnight

### Step 1 (a) Synthesis of N-(4-ethynylphenyl)acetamide

4-ethynylaniline (0.5 g, 4.26 mmol, 1 eq) was dissolved in DCM (12.78 ml, 0.3 M), and then acetic anhydride (0.477 g, 4.68 mmol, 1.1 eq) was added and stirred at room temperature. After completion of the reaction, the resulting mixture was extracted with DCM and water (H₂O). The organic layer was dried over MgSO₄, filtered, concentrated and then purified by column chromatography (silica gel, EA/HEX) to obtain the title compound.

### Step 2 (b) Synthesis of N-(4-((5-(3-(2-(pyridin-3-yl)ethyl)ureido)-2-(pyridin-4-yl)phenyl)ethynyl)phenyl)acetamide

1-(3-iodo-4-(pyridin-4-yl)phenyl)-3-(2-(pyridin-3-yl)ethyl)urea (0.1 g, 0.23 mmol, 1 eq), N-(4-ethynylphenyl)acetamide (0.055 g, 0.345 mmol, 1.5 eq), PdCl₂(PPh₃)₂ (0.005 g, 0.0069 mmol, 0.03 eq), CuI (0.0013 g, 0.0069 mmol, 0.03 eq), and TEA (0.047 g, 0.46 mmol, 2 eq) were dissolved in ACN (2.3 ml, 0.1 M) and degassed with nitrogen (N₂) gas. The reactant was heated to 60°C and stirred overnight. After completion of the reaction, the resulting mixture was extracted with DCM and water (H₂O). The organic layer was dried over MgSO₄, filtered, concentrated and then purified by column chromatography (silica gel, MeOH/DCM) to obtain the title compound (0.047 mg, 43%).

¹H NMR (400 MHz, DMSO) δ 10.12 (s, 1H), 8.81 (s, 1H), 8.66 (d, J = 6.0 Hz, 2H), 8.50 - 8.41 (m, 2H), 7.85 (s, 1H), 7.64 (ddd, J = 14.3, 13.3, 8.2 Hz, 5H), 7.42 (s, 2H), 7.37 - 7.31 (m, 3H), 6.34 (t, J = 5.6 Hz, 1H), 3.39 (dd, J = 12.8, 6.7 Hz, 2H), 2.80 (t, J = 7.0 Hz, 2H), 2.06 (s, 3H); MS MH⁺ 476.2

The reactants may be changed in substantially the same manner as in the synthesis method of above Example 40, so as to synthesize compound 60.

### Example 41: Compound 189 (synthesis reaction formula 35)

### Synthesis of 1-(4-(6-aminopyridin-3-yl)-3-((4-fluorophenyl)ethynyl)phenyl)-3-(2-(pyridin-3-yl)ethyl)urea

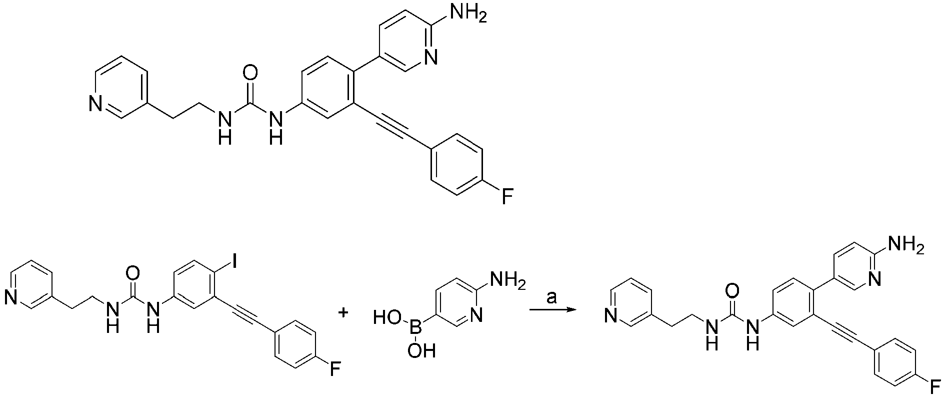

Reaction Formula 35. Reagents and conditions: (a) PdCl₂(dppf)₂, K₂CO₃, 1,4-dioxane, H₂O, 90°C, 1 hour

1-(3-((4-fluorophenyl)ethynyl)-4-iodophenyl)-3-(2-(pyridin-3-yl)ethyl)urea (0.1 g, 0.20 mmol, 1 eq) was dissolved in 1,4-dioxane:water (H₂O) (3:1 (v:v), 2 ml, 0.1 M), and then (6-aminopyridin-3-yl)boronic acid (0.055 g, 0.4 mmol, 2 eq), PdCl₂(dppf)₂ (0.029 g, 0.04 mmol, 0.2 eq), and K₂CO₃ (0.061 g, 0.44 mmol, 2.2 eq) were added and degassed with nitrogen (N₂) gas. The reaction solution was heated to 90°C and stirred for one hour. After completion of the reaction, the resulting mixture was filtered through a celite filter, and extracted with EA and water (H₂O). The organic layer was dried over MgSO₄, filtered, concentrated and then purified by column chromatography (silica gel, MeOH/DCM) to obtain the title compound (0.053 g, 58.8%).

¹H NMR (400 MHz, MeOD) δ 8.47 (d, J = 1.5 Hz, 1H), 8.42 - 8.38 (m, 1H), 8.16 (d, J = 2.1 Hz, 1H), 7.79 (d, J = 7.9 Hz, 1H), 7.74 (dd, J = 8.6, 2.3 Hz, 1H), 7.68 (d, J = 2.2 Hz, 1H), 7.42 - 7.34 (m, 4H), 7.28 (d, J = 8.5 Hz, 1H), 7.08 (t, J = 8.8 Hz, 2H), 6.67 (d, J = 8.6 Hz, 1H), 3.50 (t, J = 7.0 Hz, 2H), 2.91 (t, J = 7.0 Hz, 2H); MS MH⁺ 452.08

### Example 42: Compound 138 (synthesis reaction formula 36)

### Synthesis of 1-(3-((4-aminophenyl)ethynyl)-4-(pyridin-4-yl)phenyl)-3-(2-(pyridin-3-yl)ethyl)urea

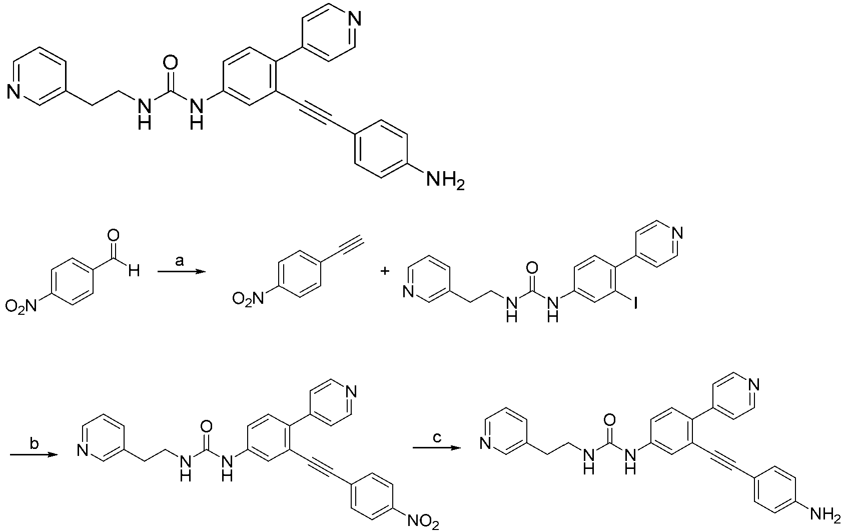

Reaction Formula 36. Reagents and conditions: (a) dimethyl 1-diazo-2-oxopropylphosphate, K₂CO₃, methanol (MeOH), RT, 3 hours; (b) PdCl₂(PPh₃)₂, CuI, Et₃N, ACN, 90°C, 2 hours; (c) Zn, NH₄Cl, 1,4-dioxane, H₂O, RT, overnight.

### Step 1 (a) Synthesis of 1-ethynyl-4-nitrobenzene

4-nitrobenzaldehyde (0.3 g, 1.99 mmol, 1 eq) was dissolved in methanol (MeOH) (8 ml, 0.25 M), and then dimethyl 1-diazo-2-oxopropylphosphate (0.458 g, 2.388 mmol, 1.2 ,eq) and K₂CO₃ (0.55 g, 3.98 mmol, 2 eq) were added and stirred at room temperature for three hours. After completion of the reaction, the resulting mixture was concentrated, extracted with ethyl acetate (EA), and sequentially extracted with brine and water. The organic layer was dried over MgSO₄, filtered, concentrated and then purified by column chromatography (silica gel, EA/HEX) to obtain the title compound (0.25 g, 85%).

### Step 2 (b) Synthesis of 1-(3-((4-nitrophenyl)ethynyl)-4-(pyridin-4-yl)phenyl)-3-(2-(pyridin-3-yl)ethyl)urea

1-(3-iodo-4-(pyridin-4-yl)phenyl)-3-(2-(pyridin-3-yl)ethyl)urea (0.2 g, 0.45 mmol, 1 eq) was dissolved in acetonitrile (ACN , 4.5 ml, 0.1 M), and then 1-ethynyl-4-nitrobenzene (0.099 g, 0.675 mmol, 1.5 eq), PdCl₂(PPh₃)₂ (0.009 g, 0.0135 mmol, 0.03 eq), CuI (0.002 g, 0.0135 mmol, 0.03 eq), and Et₃N (0.091 g, 0.9 mmol, 2 eq) were added and degassed with nitrogen (N₂) gas. The reaction solution was heated to 90°C and stirred for two hours. After completion of the reaction, the resulting mixture was filtered through a celite filter and extracted with EA and H₂O. The organic layer was dried over MgSO₄, filtered, concentrated and then purified by column chromatography (silica gel, MeOH/DCM) to obtain the title compound (0.109 g, 52%).

### Step 3 (c) Synthesis of 1-(3-((4-aminophenyl)ethynyl)-4-(pyridin-4-yl)phenyl)-3-(2-(pyridin-3-yl)ethyl)urea

1-(3-((4-nitrophenyl)ethynyl)-4-(pyridin-4-yl)phenyl)-3-(2-(pyridin-3-yl)ethyl)urea (0.020 g, 0.043 mmol, 1 eq) was dissolved in 1,4-dioxane:water (H₂O) (3:1 (v/v), 2 ml, 0.02 M), and then Zn (0.028 g, 0.43 mmol, 10 eq) and NH₄Cl (0.023 g, 0.43 mmol, 10 eq) were added and stirred at room temperature overnight. The resulting mixture was washed with methanol and filtered under reduced pressure. The filtrate was concentrated and extracted with DCM:MeOH (9:1 (v/v)) and H₂O. The organic layer was dried over MgSO₄, filtered, concentrated and then purified by column chromatography (silica gel, MeOH/DCM) to obtain the title compound (0.013 g, 69%).

¹H NMR (400 MHz, MeOD) δ 8.56 (s, 2H), 8.43 (d, J = 25.2 Hz, 2H), 7.78 (d, J = 7.8 Hz, 1H), 7.70 (d, J = 4.3 Hz, 2H), 7.67 (d, J = 2.0 Hz, 1H), 7.39 (dt, J = 16.2, 5.2 Hz, 3H), 7.06 (d, J = 8.4 Hz, 2H), 6.61 (d, J = 8.4 Hz, 2H), 3.49 (t, J = 7.0 Hz, 2H), 2.90 (t, J = 7.0 Hz, 2H).

### <Experimental Example 1> NAMPT enzyme inhibition assay

The NAMPT enzyme activity inhibition ability (IC₅₀) of the compound was evaluated using the NAMPT Inhibitor Screening Assay Kit (# 7176-1) from BPS bioscience. Recombinant NAMPT protein was added to each well of a black 96-well plate, and dilution buffer was added to a blank well instead of NAMPT protein. A 5x concentrated compound solution was added and pre-incubated at room temperature (approximately 22°C) for 30 minutes before the start of the reaction so that the compound and protein might bind before the enzyme reaction. At this time, the concentration of NAMPT protein was adjusted and used according to the enzyme activity of each protein lot within the final concentration range of 4-10 ng/µl, and the test compound was serially diluted at a ratio of 1:3 from 1000 nM to 1 nM, and experiments were conducted with n=2 for about seven concentrations. A 2x concentrated test buffer containing ATP (final concentration of 20 µM), nicotinamide (final concentration of 20 µM), phosphoribosyl pyrophosphate (PRPP, final concentration of 40 µM), and ethanol (final concentration of 1.5%) was added, and the plate was incubated at 30°C for two hours, and the fluorescence of the generated reactant was measured with excitation of 340 nm and emission of 460 nM. The NAMPT enzyme activity (%) was calculated by subtracting the measurement value of the blank well without NAMPT protein from the measurement value of all wells, and then taking the measurement value of the control well without the compound as 100%. IC₅₀ was calculated using GraphPad Prism 9 software, and the results are shown in Table 5 below.

In following Table 5, A, B and C may be as follows.
A: **NAMPT IC₅₀** ≤0.5 µM (0.5 µM or less)
B: 0.5 µM **<NAMPT IC₅₀**≤1 µM (more than 0.5 µM and 1 µM or less)
C: **NAMPT IC₅₀**>1 µM (more than 1 µM)

**Table 5 NAMPT enzyme inhibition assay (IC 50)**

| **Compound** | | **NAMPT IC₅₀** |
|---|---|---|
| **1** | 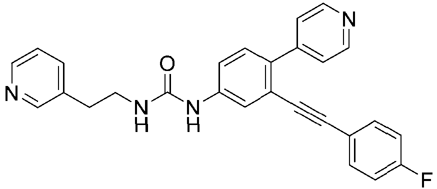 | **A** |
| **2** | 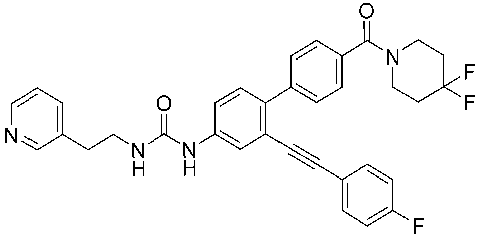 | **A** |
| **3** | 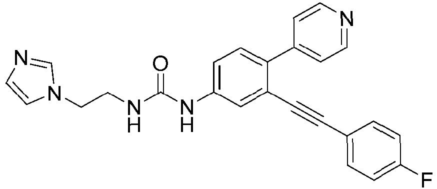 | **B** |
| **4** | 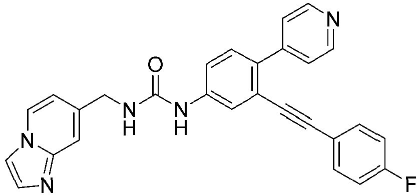 | **A** |
| **5** | 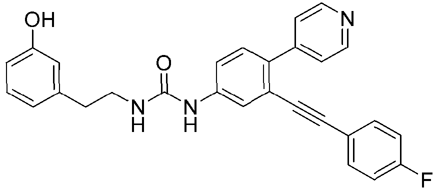 | **C** |
| **6** | 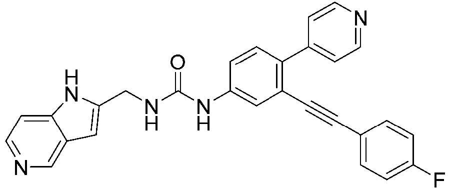 | **A** |
| **7** | 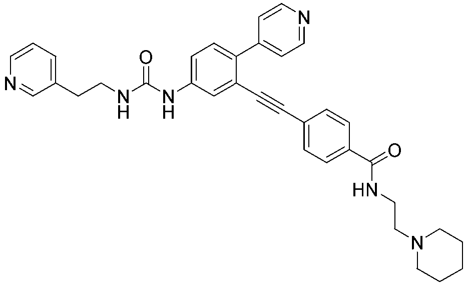 | **A** |
| **8** | 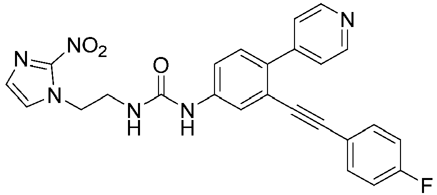 | **C** |
| **9** | 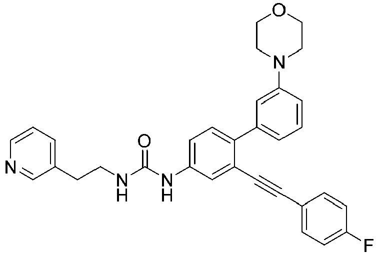 | **B** |
| **10** | 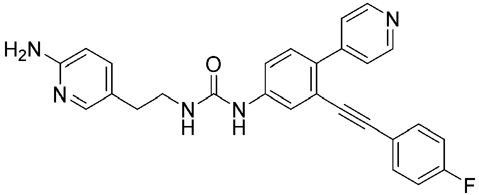 | **C** |
| **11** | 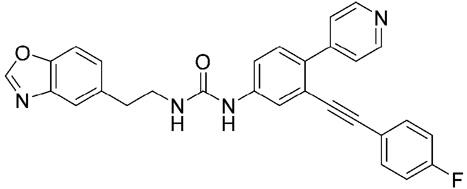 | **C** |
| **13** | 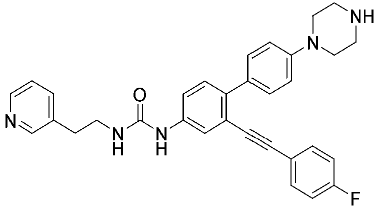 | **A** |
| **14** | 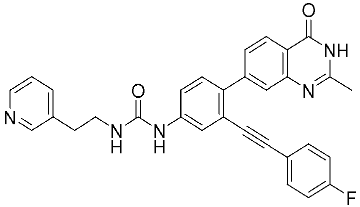 | **A** |
| **35** | 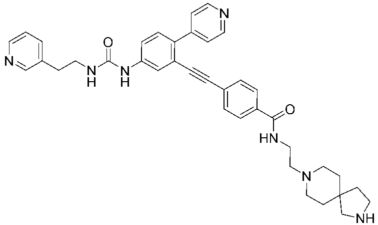 | **A** |
| **39** | 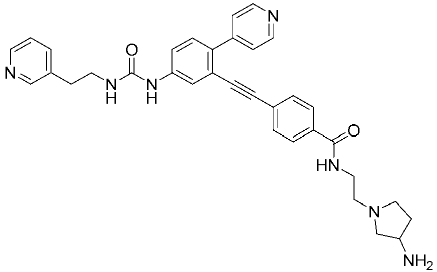 | **A** |
| **40** | 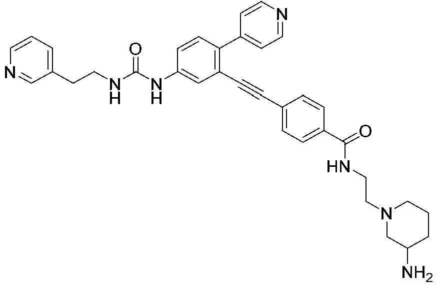 | **A** |
| **43** | 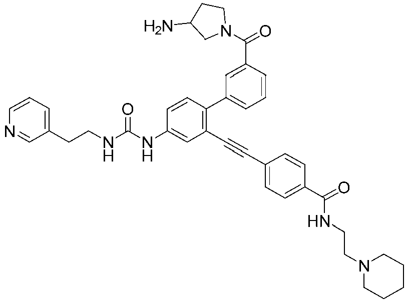 | **C** |
| **47** | 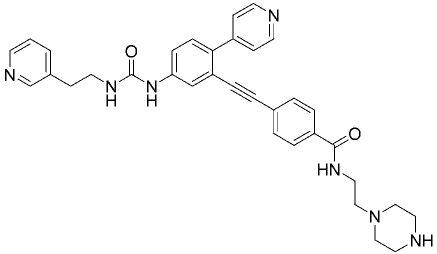 | **A** |
| **52** | 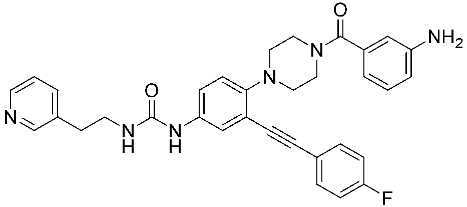 | **A** |
| **57** | 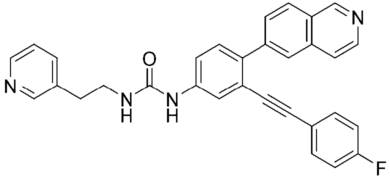 | **A** |
| **65** | 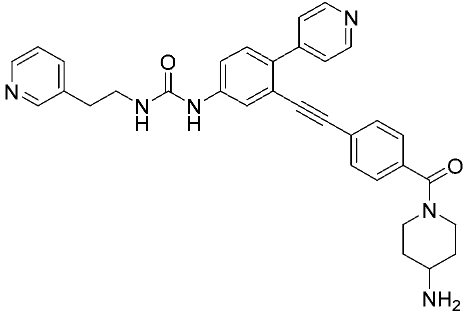 | **A** |
| **66** | 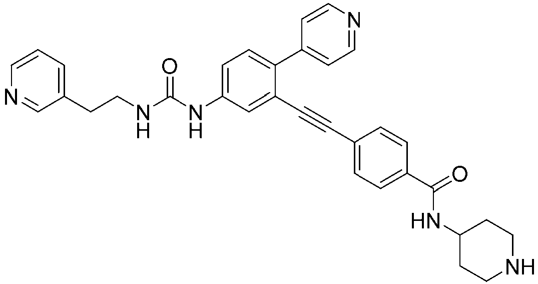 | **A** |
| **69** | 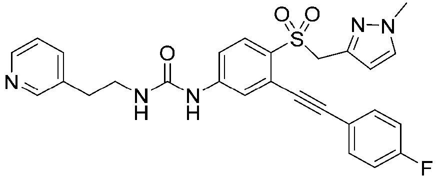 | **A** |
| **74** | 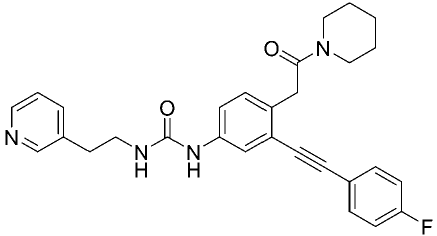 | **A** |
| **89** | 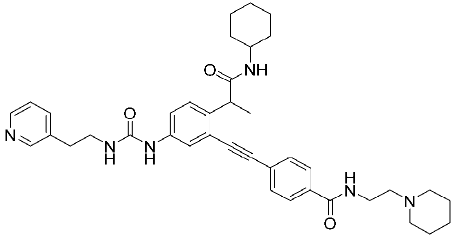 | **A** |
| **95** | 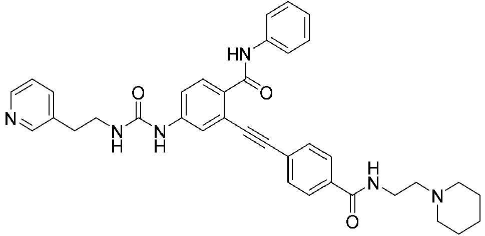 | **A** |
| **110** | 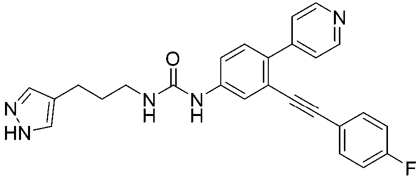 | **A** |
| **118** | 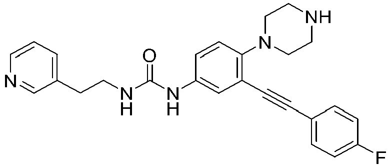 | **C** |
| **122** | 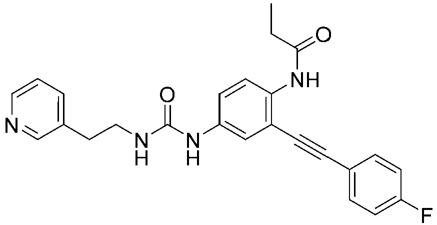 | **A** |
| **124** | 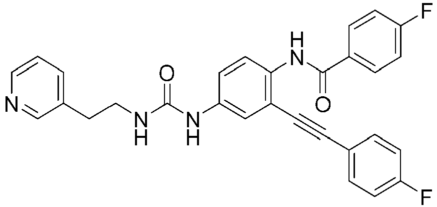 | **A** |
| **126** | 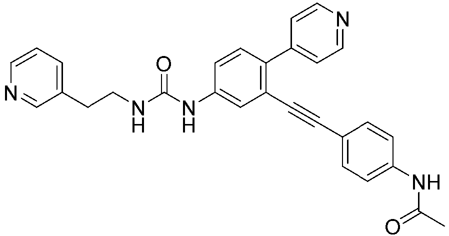 | **A** |
| **139** | 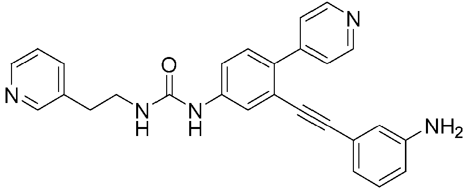 | **A** |
| **177** | 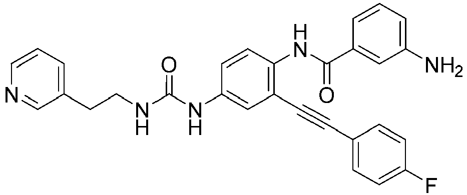 | **A** |
| **182** | 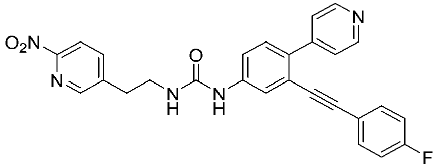 | **C** |
| **183** | 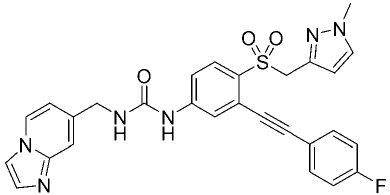 | **A** |
| **184** | 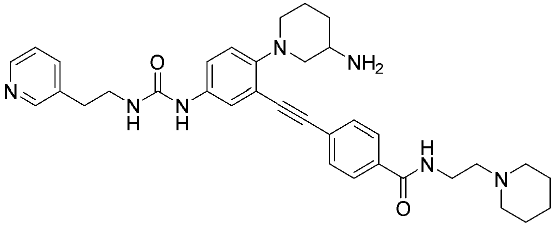 | **B** |
| **185** | 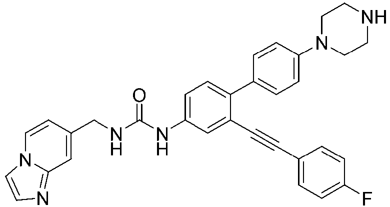 | **B** |
| **186** | 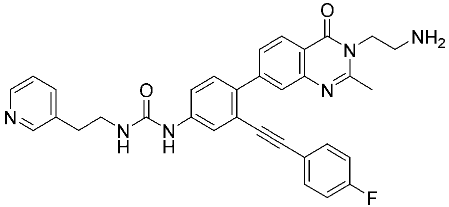 | **A** |
| **187** | 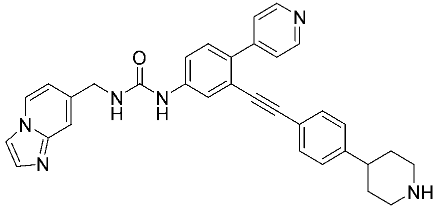 | **A** |
| **188** | 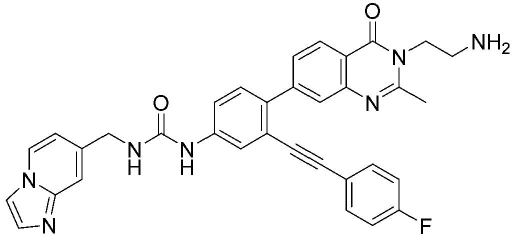 | **A** |

As can be seen from the above Table, it can be found that the compounds of the present invention exhibit excellent inhibitory activity against NAMPT and show sufficient inhibitory activity even at low concentrations.

Thus, the compounds of the present invention may have excellent preventive or therapeutic effects on NAMPT-related diseases.

### <Experimental Example 2> Cellular NAD measurement assay

With regard to HCT-116 cell lines, the decrease in intracellular NAD+ and NADH levels due to the inhibition of NAMPT enzyme activity by compounds was evaluated using NAD/NADH-Glo^{™} Assay (Promega, G9072) from Promega. The cells were incubated using DMEM (Gibco, 11995-065) medium containing 10% FBS. The HCT-116 cells were incubated for 24 hours after being distributed at a density of 2,500 cells per well in a 96-well plate, and were treated with the drugs using serum-free DMEM. First, the compounds were dissolved in 100% dimethyl sulfoxide (DMSO), and then diluted in the culture medium so that the final concentration of DMSO might reach 0.5%, and treated with the drugs. The final concentration range of the compounds was 0.1 nM to 100 nM. After that, they were further incubated for 24 hours under conditions of 37°C and CO₂ 5%. To conduct the NAD/NADH-Glo^{™} Assay, the culture medium of drug-treated cells was removed, 50 µL of DPBS was treated per well, and left at room temperature for five minutes. The required amount of NAD/NADH-Glo^{™} Detection Reagent (Reconstituted Luciferin Detection Reagent 1 mL, Reductase 5 µL, Reductase Substrate 5 µL, NAD Cycling Enzyme 5 µL, NAD Cycling Substrate 25 µL) was prepared according to the manufacturer's manual and treated in an amount of 50 µL per well. After incubating for two minutes at 400 rpm in a plate shaker for cell lysis, the cells were incubated for 30 minutes at room temperature, and luminescence was measured. NAD+/NADH levels were calculated by subtracting the measurement value of the blank well without cells from the measurement value of all wells and taking the 0.5% DMSO-treated control as 100%. IC₅₀ was calculated using GraphPad Prism 9 software and the results are shown in Table 6 below.

In Table 6 below, A, B and C may be as follows.
A: **NAD IC₅₀**≤10 nM (10 nM or less)
B: 10 nM **< NAD IC₅₀** ≤1 µM µM (more than 10 nM, 1 µM or less)
C: **NAD IC₅₀**>1 µM (more than 1 µM)

**Table 6 Cellular NAD measurement analysis (NAD, IC₅₀)**

| **Compound** | | **NAD, IC₅₀** |
|---|---|---|
| | | **HCT-116** |
| **1** | 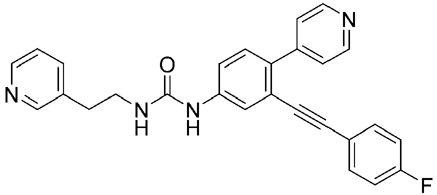 | **B** |
| **4** | 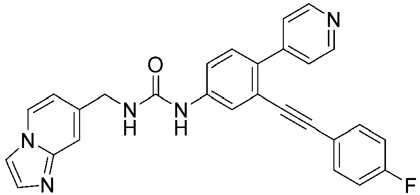 | **A** |
| **13** | 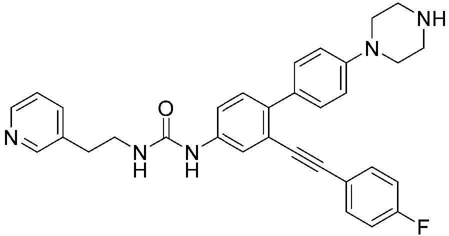 | **A** |
| **14** | 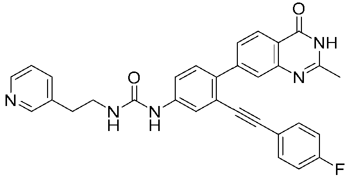 | **A** |
| **47** | 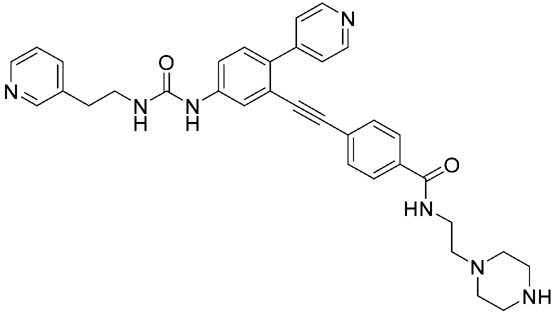 | **B** |
| **183** | 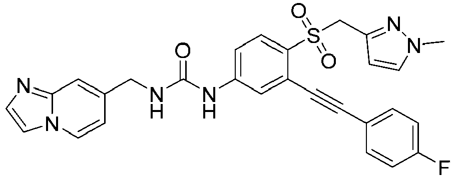 | **A** |
| **186** | 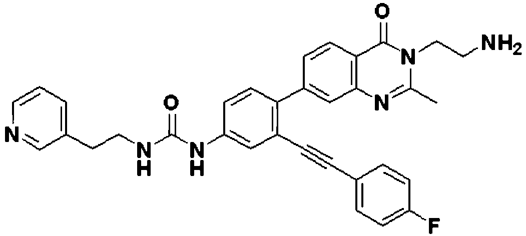 | **A** |
| **187** | 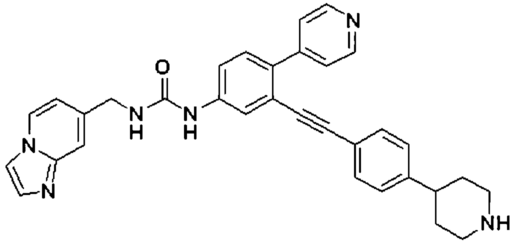 | **A** |

As can be seen from the above Table, it was found that the compounds of the present invention remarkably inhibit NAD production in HCT-116 cell lines and sufficiently inhibit the concentration of intracellular NAD even at low concentrations.

Thus, the compounds of the present invention may inhibit NAMPT and sufficiently lower the concentration of NAD in cells even at low concentrations, and thus may have excellent effects of preventing or treating diseases (e.g., cancer) which may be treated by inhibiting NAMPT.

### <Experimental Example 3> Tumor cytotoxicity assay

The anticancer efficacy of the compound was evaluated using the MTT analysis method in two cell lines, HCT-116 (colorectal cancer) and NCI-N87 (gastric cancer). The HCT-116 cell lines were incubated in DMEM (Gibco, 11995-065) containing 10% FBS, and the NCI-N87 cell lines were incubated in RPMI Medium 1640 (Gibco, 11875-093) containing 10% FBS and 25 mM HEPES. The HCT-116 and NCI-N87 cell lines were distributed in a 96-well plate at a density of 2,500 cells per well and 10,000 cells per well, respectively, and each cell was incubated for 24 hours, and then treated with the drug by using a medium not containing FBS. First, the compounds were prepared in 100% dimethyl sulfoxide (DMSO) at concentrations of 0.02 µM to 200 µM, and then diluted in the culture medium so that the final concentration of DMSO might reach 0.5%, and treated with the drugs. The final concentration range of the compounds was 0.1 nM to 1000 nM. After that, they were further incubated for 72 hours under conditions of 37°C and CO₂ 5%. To confirm cytotoxicity, MTT (Sigma-Aldrich, M2128) was treated for two or four hours, and formazan produced by reduction by enzymes in the mitochondria of living cells was measured using Spark^{®} Multimode Microplate Reader TECAN equipment. The cell viability (%) was relatively determined by subtracting the reference 650 nm optical density (O.D.) from the 570 nm O.D., and the 0.5% DMSO-treated control was set as 100% as the standard. The results were plotted as a function of compound concentration using GraphPad Prism 9 software to calculate the CC₅₀ (50% cytotoxic concentration) value, and the results are shown in Table 7 below.

In Table 7 below, A, B and C may be as follows.
A: CC₅₀ ≤30 nM (30 nM or less)
B: 30 nM < CC₅₀≤100 nM (more than 30 nM and 100 nM or less)
C: CC₅₀>1 µM (more than 1 µM)

**Table 7 Cytotoxicity evaluation**

| **Compound** | | **Cytotoxicity, CC₅₀** | |
|---|---|---|---|
| | | **HCT-116** | **NCI-N87** |
| **1** | 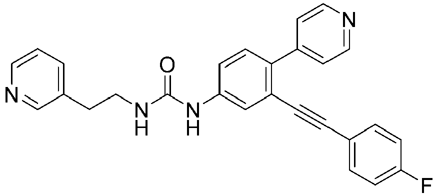 | **B** | **B** |
| **4** | 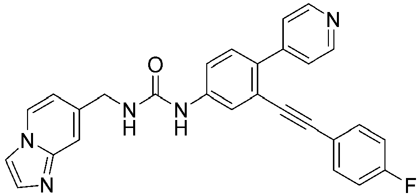 | **A** | **A** |
| **6** | 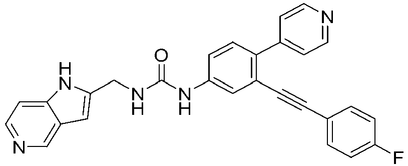 | **B** | |
| **7** | 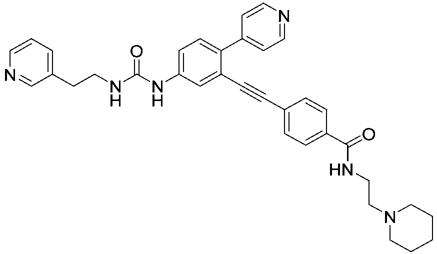 | **A** | |
| **13** | 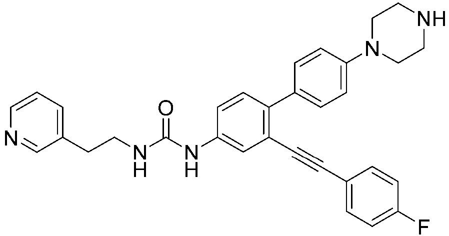 | **A** | **A** |
| **14** | 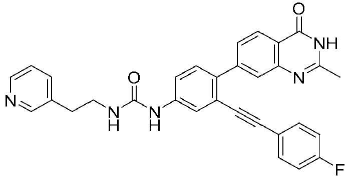 | **A** | **A** |
| **35** | 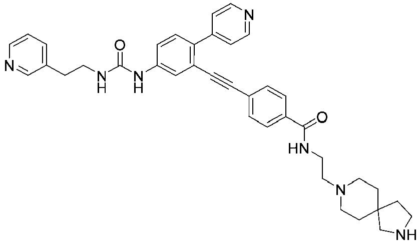 | **B** | **B** |
| **39** | 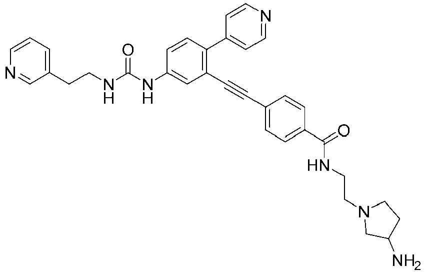 | **C** | **B** |
| **40** | 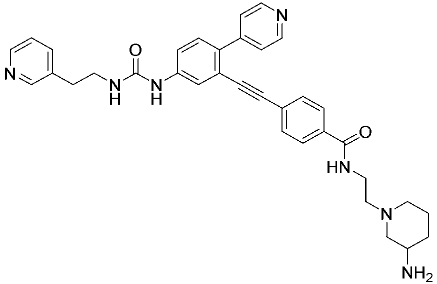 | **B** | **B** |
| **47** | 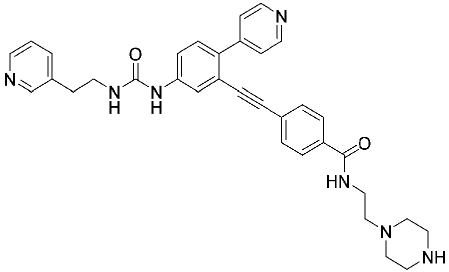 | **A** | **B** |
| **65** | 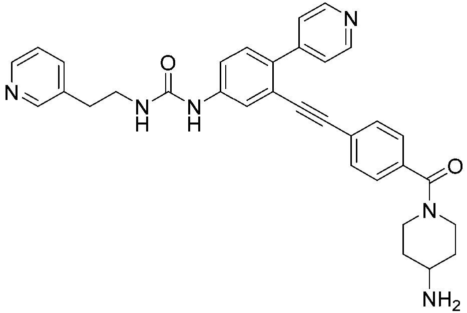 | **B** | |
| **66** | 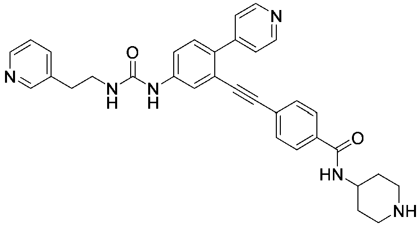 | **B** | **A** |
| **74** | 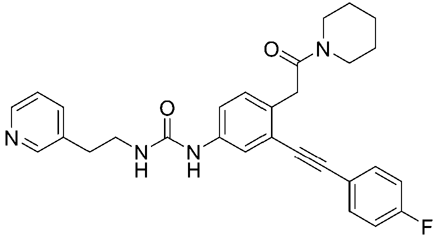 | | **A** |
| **89** | 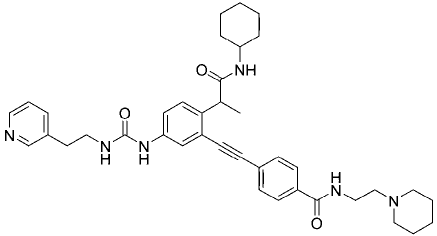 | **B** | |
| **126** | 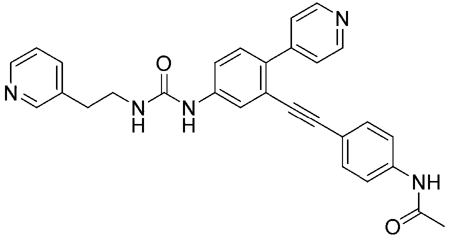 | | **A** |
| **183** | 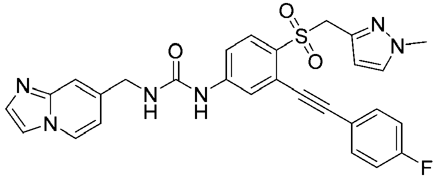 | **A** | |
| **186** | 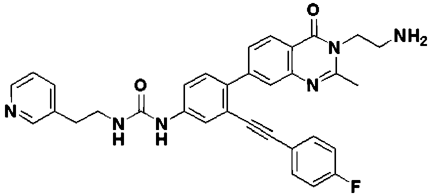 | **A** | **B** |
| **187** | 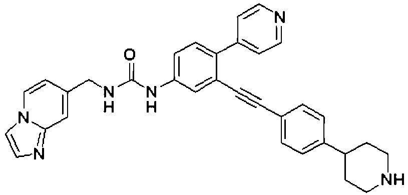 | **A** | **A** |
| **188** | 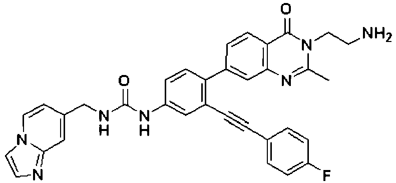 | **A** | **A** |

As can be seen from the above Table, it can be found that the compounds of the present invention exhibit excellent cytotoxicity even at low concentrations against colon cancer cell lines and gastric cancer cell lines.

Thus, it can be seen that the compounds of the present invention have excellent preventive or therapeutic effects against various diseases, such as cancer, by inhibiting NAMPT.

The present invention has been described with reference to one exemplary embodiment of the present invention, but it will be understood by those skilled in the art that the present invention may be variously changed and modified without departing from the spirit and field of the present invention, as described in the following scope of patent claims.

## Claims

1. A urea compound represented by formula I below, an optical isomer thereof, a pharmaceutically acceptable salt of them, or a hydrate or solvate of them: 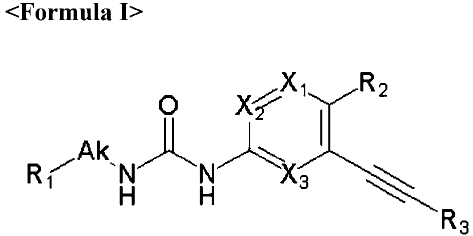
R₁ is 6- to 12-membered aryl (e.g., C₆₋₁₂ aryl); or 5- to 12-membered heteroaryl comprising at least one heteroatom selected from the group consisting of N, O and S (in which -H of aryl or heteroaryl, which is the R₁, is each independently unsubstituted or at least one -H may be each independently substituted with -NH₂, -(C₁₋₅ alkyl)NH₂, -NH-(C₁₋₅ alkyl), -N(C₁₋₅ alkyl)₂, -OH, -NO₂, C₁₋₅ alkyl, -F, -Cl, -Br, or -I;
R₂ is 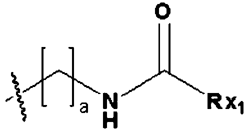 (the Rx₁ is C₁₋₅ alkyl; C₂₋₅ alkenyl; 6- to 12-membered aryl; 3- to 10-membered cycloalkyl; 3- to 10-membered heterocycloalkyl comprising at least one heteroatom selected from the group consisting of N, O and S; or 5- to 12-membered heteroaryl comprising at least one heteroatom selected from the group consisting of N, O and S); -NRx₂Rx₃ (Rx₂ or Rx₃ is each independently H or C₁₋₆ alkyl); 6- to 12-membered aryl; 5- to 12-membered heteroaryl comprising at least one heteroatom selected from the group consisting of N, O and S; -ORx₄ (Rx₄ is 3- to 7-membered aryl); 3- to 10-membered heterocycloalkyl comprising at least one heteroatom selected from the group consisting of N, O and S; 3- to 10-membered cycloalkyl; 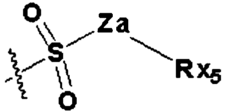 (Za is a single bond, -NH or -CH₂-, and Rx₅ is 5- to 12-membered heteroaryl comprising at least one heteroatom selected from the group consisting of N, O and S, 6- to 12-membered aryl, 3- to 10-membered cycloalkyl, or 3- to 10-membered heterocycloalkyl comprising at least one heteroatom selected from the group consisting of N, O and S, or -NYaYb {Ya or Yb is each independently H or C₁₋₆ alkyl}); 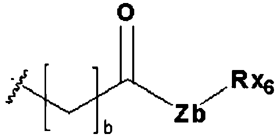 (Zb may be a single bond, -NH, -NHCH₂-, -NH(CH₂)₂ -CH₂-, -(CH₂)₂-, -(CH₂)₃-, or -CH₂NH-, Rx₆ is 5- to 12-membered heteroaryl comprising at least one heteroatom selected from the group consisting of N, O and S, 6- to 12-membered aryl, 3- to 10-membered heterocycloalkyl comprising at least one heteroatom selected from the group consisting of N, O and S, -NYcYd {Yc or Yd is each independently -H, -C₁₋₆ alkyl or C₃₋₆ cycloalkyl} or 3- to 10-membered cycloalkyl, and b is an integer of 0 to 4); 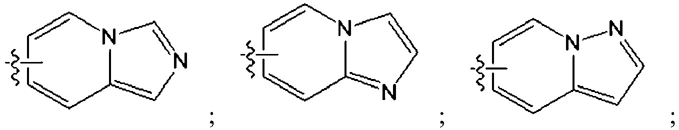 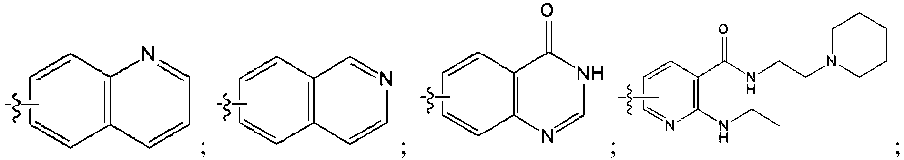 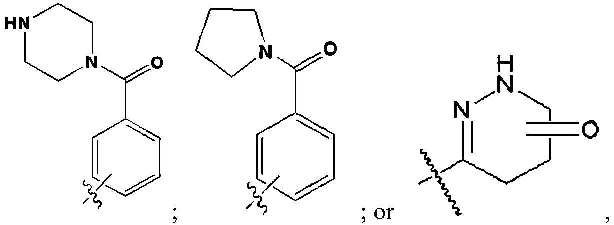
in which -H of each group of the R₂ may be each independently unsubstituted or at least one -H may be each independently substituted with one selected from the group consisting of C₁₋₅ alkyl (-H of the alkyl may be unsubstituted or at least one -H may be each independently substituted with one selected from the group consisting of phenyl, pyridinyl, morpholinyl, piperazine and piperidine); -(C₁₋₅ alkyl)NH₂; -NH₂; -NH(C₁₋₅ alkyl); -N(C₁₋₅ alkyl)₂; -NHCH₃; -N(CH₃)₂; -NHCH₂CH₃; -OH; -NO₂; -F; -Cl; -Br; -I; -CHF₂; -CF₃; -C₁₋₅alkoxy; -C(=O)N(CH₃)₂; -C(=O)NHCH₃; -C(=O)NH₂; -NHC(=O)CH₃; -C(=O)CH₂CH₃; 6- to 12-membered aryl (-H of the aryl may be unsubstituted or at least one -H may be substituted with -C₁₋₃ alkyl and -CF₃); 5- to 12-membered heteroaryl comprising at least one heteroatom selected from the group consisting of N, O and S (-H of the heteroaryl may be unsubstituted or at least one -H may be substituted with -C₁₋₃ alkyl and -CF₃); 3- to 10-membered cycloalkyl; 3- to 10-membered heterocycloalkyl comprising at least one heteroatom selected from the group consisting of N, O and S (-H of the heterocycloalkyl may be unsubstituted or at least one -H may be substituted with -C₁₋₃ alkyl and -CF₃); 3- to 10-membered cycloalkoxy; -S(=O)₂NHCH₃; -S(=O)₂, -S(=O)₂-CH₃, -C(=O)NH₂; 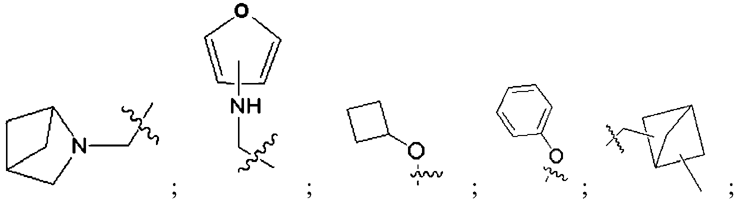 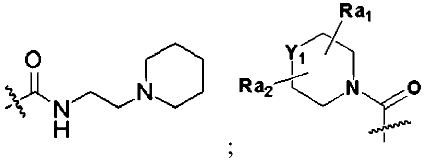 (the Ra₁ and Ra₂ may be each independently H, F, Cl, Br-, I or -C₁₋₅ alkyl, and Y₁ may be one selected from -CH₂, -NH, - and -O-), 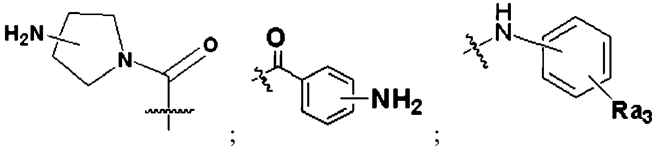 (the Ra₃ may be -H, -C₁₋₅ alkyl or -CF₃); 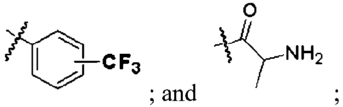
R₃ is 6- to 12-membered aryl; 5- to 12-membered heteroaryl comprising at least one heteroatom selected from the group consisting of N, O and S; 3- to 10-membered heterocycloalkyl comprising at least one heteroatom selected from the group consisting of N, O and S; or 3- to 10-membered cycloalkyl,
in which -H of R₃ may be each independently unsubstituted or at least one -H may be each independently substituted with one selected from the group consisting of 1) to 18) below:
1) C₁₋₅ alkyl;
2) -F, -Cl, -Br, or -I
3) 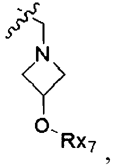 in which Rx₇ may be C₁₋₃ alkyl or -CF₃;
4) 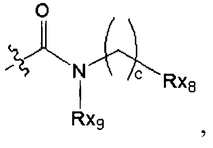 in which c is 0, 1, 2 or 3, Rx₈ may be C₁₋₅ alkyl, -NH₂, - NHCH₃, -N(CH₃)₂, piperidinyl, piperazinyl, morpholinyl, 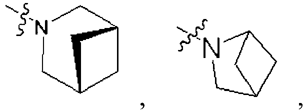 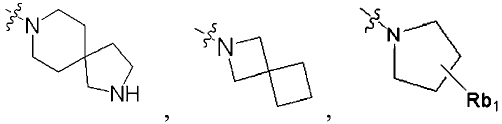 (the Rb₁ may be -NH₂ or -CH₂OH), 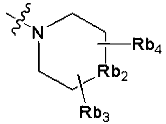 (the Rb₂ may be -CH₂-, -NH- or -O-, and Rb₃ and Rb₄ may be each independently -H, -F, -Cl, -Br, or -I), pyridinyl, pyrimidinyl or pyrrolyl, and Rx₉ may be -H or C₁₋₅ alkyl;
5) -COOH;
6) -NRx₁₀Rx₁₁, in which Rx₁₀ or Rx₁₁ may be each independently -H or -CH₃;
7) -CF₃;
8) -CN;
9) Morpholinyl or morpholidinyl in which at least one -H is each independently substituted with C₁₋₅ alkyl, -F, -Cl, -Br, I, -C(=O)NHCH₃, -C(=O)CH₃ or -C(=O)CH=CH₂;
10) Piperidinyl or piperidinyl in which at least one -H is each independently substituted with C₁₋₅ alkyl, -F, -Cl, -Br, I, -C(=O)NHCH₃, -C(=O)CH₃ or -C(=O)CH=CH₂;
11) Piperaziny or piperazinyl in which at least one -H is each independently substituted with C₁₋₅ alkyl, -F, -Cl, -Br, I, -C(=O)NHCH₃, -C(=O)CH₃ or -C(=O)CH=CH₂;
12) -ORx₁₂, in which R₁₂Rx₁₂ may be -CF₃; C₁₋₃ alkyl;
13) -OH;
14) 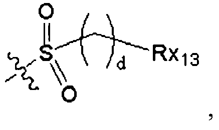 in which d may be 0, 1, 2 or 3, and Rx₁₃ may be -NH₂, - NH(CH₃), -N(CH₃)₂pyrazolyl, methylpyrazolyl, pyrazolyl, piperazinyl, piperidinyl, or morpholinyl;
15) 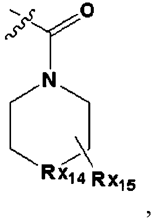 in which Rx₁₄ may be -CH₂-, -NH or -O-, and Rx₁₅ may be -H, C₁₋₅ alkyl, -NH₂, -F, -Cl, -Br, or -I;
16) 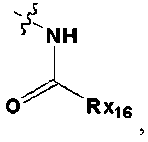 in which Rx₁₆ may be C₁₋₅ alkyl or -CH₂CH₂N(CH₃)₂,
17) 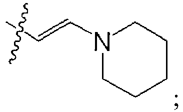 or
18) -(C₁₋₃ alkyl)-NH₂,
Ak is -(CH₂)n- or 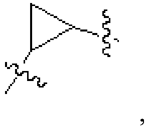 in which -H of the -(CH₂)n- or 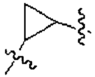 may be each independently unsubstituted or at least one -H is each independently substituted with NH₂, -OH, -NO₂, -C₁₋₅ alkyl, -CH₂NH₂, -CF₃, OCF₃, -CN, -F, -Cl, -Br, or -I, and n is 1, 2 or 3, and
X₁ to X₃ are each independently -CH- or -N-.

2. The urea compound, the optical isomer thereof, the pharmaceutically acceptable salt of them, or the hydrate or solvate of them of claim 1, wherein the R₃ is 3- to 8-membered aryl.

3. The urea compound, the optical isomer thereof, the pharmaceutically acceptable salt of them, or the hydrate or solvate of them of claim 1 or 2, wherein the R₁ is 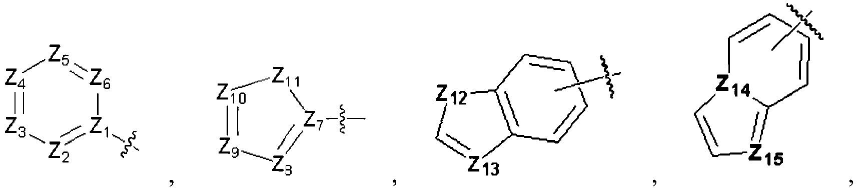 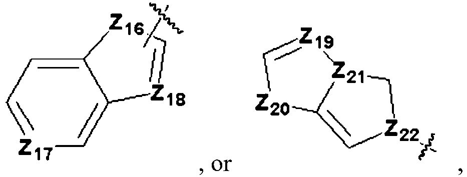 in which the Z₁ to Z₂₂ are each independently -CH-, -CH₂, -N-, -NH, -O- or -S-, in which -H of the R₁ may be each independently unsubstituted or at least one -H is each independently substituted with -NH₂, -(C₁₋₅ alkyl)NH₂, -NH-(C₁₋₅ alkyl), -N(C₁₋₅ alkyl)₂ -OH, -NO₂, C₁₋₅ alkyl, -F, -Cl, -Br, or -I.

4. The urea compound, the optical isomer thereof, the pharmaceutically acceptable salt of them, or the hydrate or solvate of them of claim 3, wherein the R₁ may be 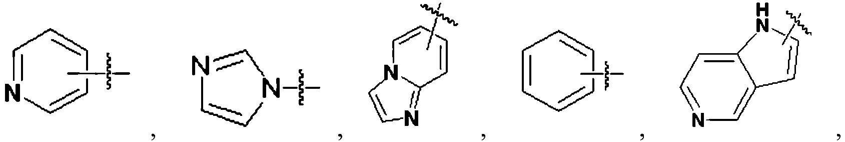 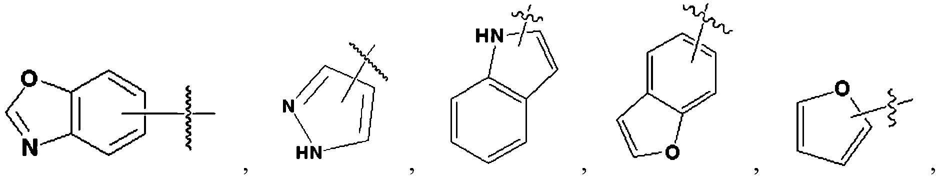 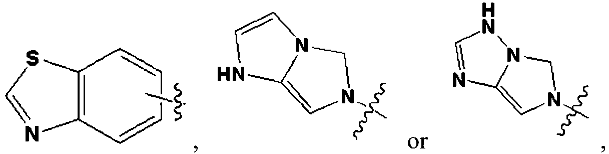 in which -H of the R₁ may be each independently unsubstituted or at least one -H may be each independently substituted with -NH₂, -(C₁₋₅ alkyl)NH₂, -NH-(C₁₋₅ alkyl), -N(C₁₋₅ alkyl)₂, -OH, -NO₂, C₁₋₅ alkyl, -F, -Cl, -Br, or -I.

5. The urea compound, the optical isomer thereof, the pharmaceutically acceptable salt of them, or the hydrate or solvate of them of claim 1, wherein the R₂ is 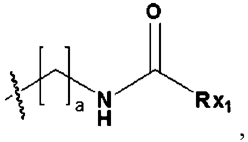
in which Rx₁ may be -C₁₋₅ alkyl; -C₂₋₅ alkenyl; 6- to 12-membered aryl; 3- to 10-membered cycloalkyl; 3- to 10-membered heterocycloalkyl comprising at least one heteroatom selected from the group consisting of N, O and S; or 5- to 12-membered heteroaryl comprising at least one heteroatom selected from the group consisting of N, O and S,
in which -H of the 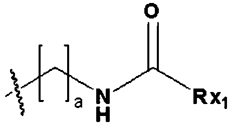 may be unsubstituted or at least one -H may be each independently substituted with -C₁₋₃ alkyl (-H of the alkyl is unsubstituted or at least one -H may be each independently substituted with one selected from the group consisting of phenyl, pyridinyl, morpholinyl, piperazine and piperidine), -morpholinyl, piperazinyl, piperidinyl, phenyl (-H of the -morpholinyl, -piperazinyl, -piperidinyl, and -phenyl may be unsubstituted or at least one -H may be each independently substituted with one selected from the group consisting of -C₁₋₃ alkyl and -CF₃), -NH₂, -F, -Cl, -Br or -I, and a is 0, 1, 2, 3 or 4.

6. The urea compound, the optical isomer thereof, the pharmaceutically acceptable salt of them, or the hydrate or solvate of them of claim 1, wherein the R₂ is 6- to 12-membered aryl, and
-H of the aryl may be unsubstituted or at least one -H may be each independently substituted with one selected from the group consisting of C₁₋₅ alkyl (-H of the alkyl may be unsubstituted or at least one -H may be each independently substituted with one selected from the group consisting of phenyl, pyridinyl, morpholinyl, piperazine and piperidine), F, -Cl, -Br, -I, -morpholinyl, -piperazinyl, -piperidinyl (-H of the -morpholinyl, -piperazinyl and - piperidinyl may be unsubstituted or at least one -H may be each independently substituted with one selected from the group consisting of -C₁₋₃ alkyl and -CF₃), -cyclobutoxy, -cyclopropoxy, -cyclopentoxy, 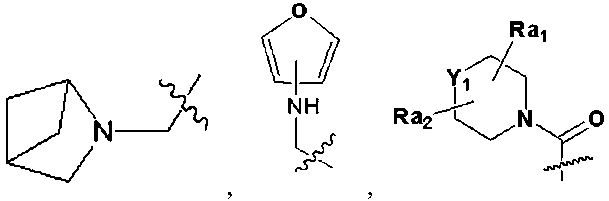 (the Ra₁ and Ra₂ may be each independently -H, -F, -Cl, -Br-, -I or -C₁₋₅ alkyl, and Y₁ may be one selected from -CH₂, -NH, - and -O-), 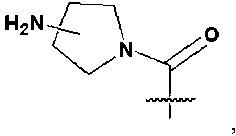 -C₁₋₃ alkoxy, -C(=O)-N(CH₃)₂ and - C(=O)NH₂.

7. The urea compound, the optical isomer thereof, the pharmaceutically acceptable salt of them, or the hydrate or solvate of them of claim 1, wherein the R₂ is -NRx₂Rx₃, the Rx₂ or Rx₃ is each independently -H or linear or branched C₁₋₆ alkyl, and if the Rx₂ or Rx₃ is alkyl, - H of the Rx₂ and Rx₃ may be unsubstituted or at least one -H may be each independently substituted with C₁₋₆ alkyl, -F, -Cl, -Br or -I.

8. The urea compound, the optical isomer thereof, the pharmaceutically acceptable salt of them, or the hydrate or solvate of them of claim 1, wherein the R₂ is -ORx₄,
in which Rx₄ may be 6- to 7-membered aryl,
in which -H of the -ORx may be unsubstituted or at least one -H may be each independently substituted with one selected from the group consisting of -C₁₋₅ alkyl, -F, -Cl, - Br, -I, -NH₂, -NH(C₁₋₅ alkyl), -N(C₁₋₅ alkyl)₂, -NHCH₃, -N(CH₃)₂, -NHCH₂CH₃, -C(=O)NH₂, -C(=O)N(CH₃)₂ and -C(=O)NH(CH₃).

9. The urea compound, the optical isomer thereof, the pharmaceutically acceptable salt of them, or the hydrate or solvate of them of claim 1, wherein the R₂ is 5- to 12-membered heteroaryl comprising at least one heteroatom selected from the group consisting of N, O and S, and
-H of the heteroaryl may be unsubstituted or at least one -H may be each independently substituted with one selected from the group consisting of -C₁₋₅ alkyl, -F, -Cl, - Br, -I, -NH₂, -NH(C₁₋₅ alkyl), -N(C₁₋₅ alkyl)₂, -NHCH₃, -N(CH₃)₂, -NHCH₂CH₃, -C(=O)NH₂, -C(=O)N(CH₃)₂ and -C(=O)NH(CH₃).

10. The urea compound, the optical isomer thereof, the pharmaceutically acceptable salt of them, or the hydrate or solvate of them of claim 1, wherein R₂ is 3- to 10-membered heterocycloalkyl comprising at least one heteroatom selected from the group consisting of N, O and S,
in which -H of the heterocycloalkyl may be unsubstituted or at least one -H may be each independently substituted with one selected from the group consisting of -C₁₋₅ alkyl (-H of the alkyl may be unsubstituted or at least one -H may be each independently substituted with one selected from the group consisting of phenyl, pyridinyl, morpholinyl, piperazine and piperidine), pyridinyl, phenyl (-H of the pyridinyl and phenyl is unsubstituted or at least one - H may be each independently substituted with one selected from the group consisting of C₁₋₃ alkyl and -CF₃), 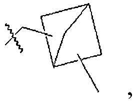 -NHC(=O)CH₃, -C(=O)CH₂CH₃, 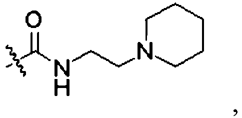 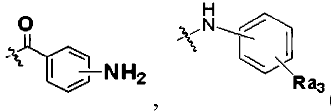 (the Ra₃ may be H, -C₁₋₃ alkyl or -CF₃), -OH, -S(=O)₂CH₃, - NH₂, -NH(C₁₋₅ alkyl), -N(C₁₋₅ alkyl)₂, -NHCH₃, -N(CH₃)₂ and -NHCH₂CH₃.

11. The urea compound, the optical isomer thereof, the pharmaceutically acceptable salt of them, or the hydrate or solvate of them of claim 1, wherein the R₂ is 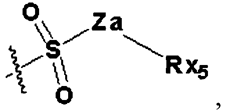
in which Za may be a single bond, -NH or -CH₂-, and
Rx₅ is 5- to 12-membered heteroaryl comprising at least one heteroatom selected from the group consisting of N, O and S, 6- to 10-membered aryl (e.g., C₆₋₁₂ aryl), 3- to 10-membered cycloalkyl, 5- to 12-membered heterocycloalkyl comprising at least one heteroatom selected from the group consisting of N, O and S, or -NYaYb (Ya or Yb is each independently H or C₁₋₆ alkyl),
in which -H of the 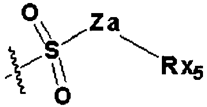 may be unsubstituted or at least one -H may be each independently substituted with C₁₋₅ alkyl (-H of the alkyl may be unsubstituted or at least one -H may be each independently substituted with one selected from the group consisting of phenyl, pyridinyl, morpholinyl, piperazine and piperidine), -CF₃, 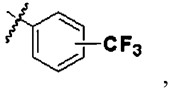 pyridinyl, pyrimidinyl, piperidinyl, piperazinyl, morpholinyl (-H of the pyridinyl, pyrimidinyl, piperidinyl, piperazinyl and morpholinyl may be unsubstituted or at least one -H may be substituted with one selected from the group consisting of -C₁₋₃ alkyl and -CF₃), 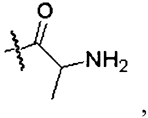 -NH₂, -F, -Cl, -Br or -I.

12. The urea compound, the optical isomer thereof, the pharmaceutically acceptable salt of them, or the hydrate or solvate of them of claim 1, wherein the R₂ is 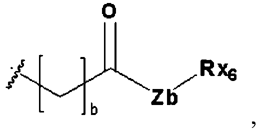
in which Zb is a single bond, -NH, -NHCH₂-, -NH(CH₂)₂ -CH₂-, -(CH₂)₂-, -(CH₂)₃-, -CH₂NH-, and
Rx₆ may be 5- to 12-membered heteroaryl comprising at least one heteroatom selected from the group consisting of N, O and S, 6- to 10-membered aryl, 3- to 10-membered heterocycloalkyl comprising at least one heteroatom selected from the group consisting of N, O and S, -NYcYd (Yc or Y_{d} may be each independently -H, -C₁₋₆ alkyl or C₃₋₆ cycloalkyl) or 3- to 10-membered cycloalkyl, and b is an integer of 0 to 4,
in which -H of the 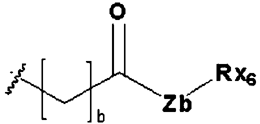 may be unsubstituted or at least one -H may be each independently substituted with -C₁₋₅ alkyl (-H of the alkyl may be unsubstituted or at least one -H may be each independently substituted with one selected from the group consisting of phenyl, pyridinyl, morpholinyl, piperazine and piperidine), -CF₃, 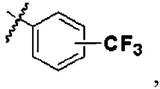 pyridinyl, pyrimidinyl, piperidinyl, piperazinyl, -morpholinyl (-H of the pyridinyl, pyrimidinyl, piperidinyl, piperazinyl and morpholinyl may be unsubstituted or at least one -H may be each independently substituted with one selected from the group consisting of -C₁₋₃ alkyl and -CF₃), 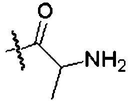 , -NH₂, -F, -Cl, -Br or -I.

13. The urea compound, the optical isomer thereof, the pharmaceutically acceptable salt of them, or the hydrate or solvate of them of claim 1, wherein the R₂ is 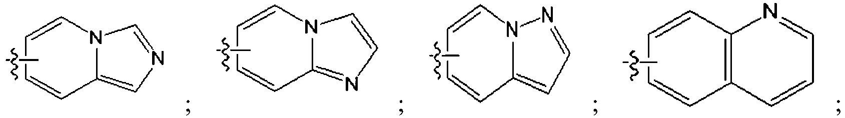 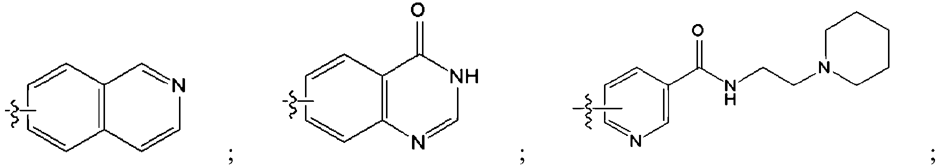 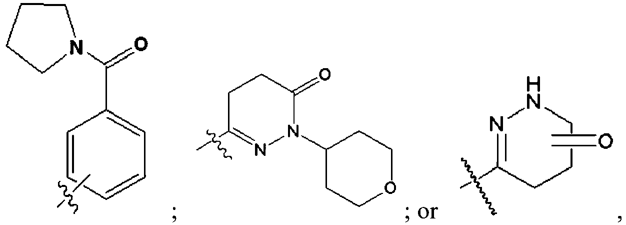 in which -H of the R₂ may be unsubstituted or at least one -H may be each independently substituted with one selected from the group consisting of C₁₋₅ alkyl, -NH₂, - NHCH₂CH₃, -(C₁₋₅ alkyl)NH₂, -F, -Cl, -Br, and -I.

14. A urea compound represented by formula I-1 below, a pharmaceutically acceptable salt of them, or a hydrate or solvate of them: 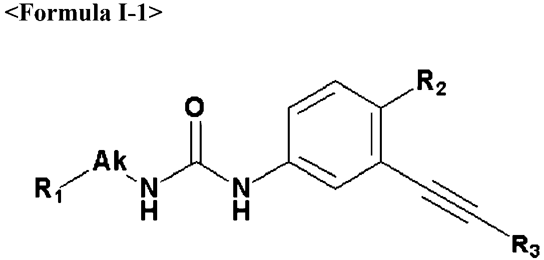
R₁ may be pyridinyl; pyrimidinyl; pyrazolyl; imidazolyl; pyrrolyl; furanyl; phenyl; phenolic; 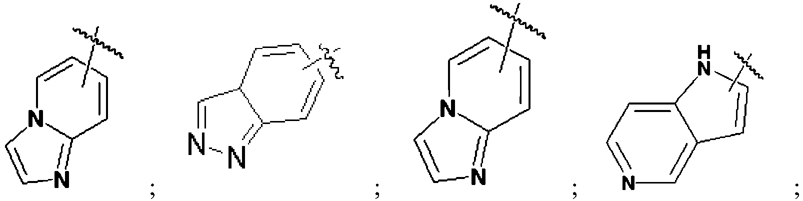 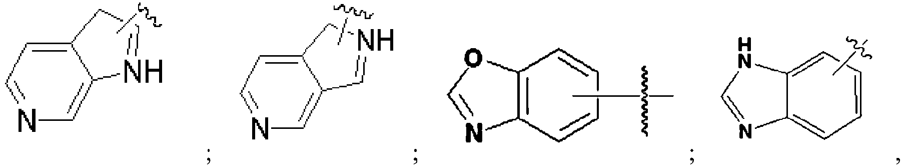 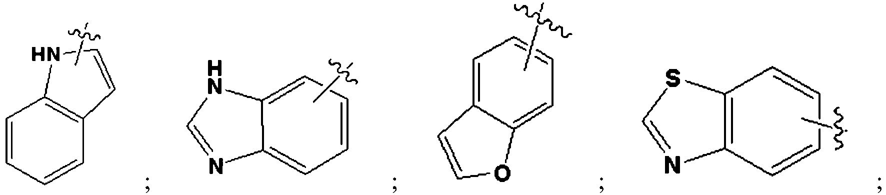 or 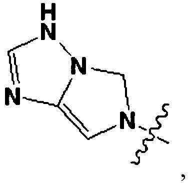
in which -H of the R₁ may be unsubstituted or at least one -H may be each independently substituted with -NH₂, -(C₁₋₃ alkyl)NH₂, -NH-(C₁₋₃ alkyl), -N(C₁₋₃ alkyl)₂, -OH, -NO₂, C₁₋₅ alkyl, -F, -Cl, -Br, or -I,
R₂ is pyridinyl; pyrimidinyl; phenyl; piperidinyl; piperazinyl; morpholinyl; pyrazolyl, imidazolyl, phenolic, phenyl; -CH₂C(=O)NH₂; -C(=O)NH₂; -C(=O)NHCH₂CH₃; - CH₂NHC(=O)CH₂CH₃; -NH₂; -NHCH₃, -NHCH₂CH₂CH₃; 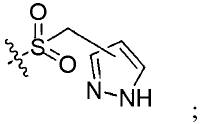 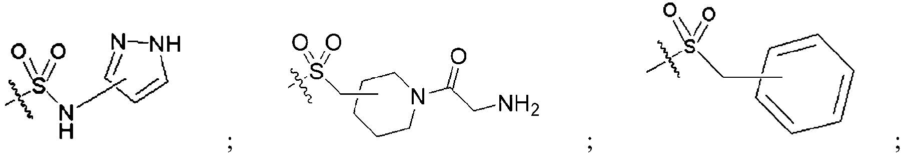 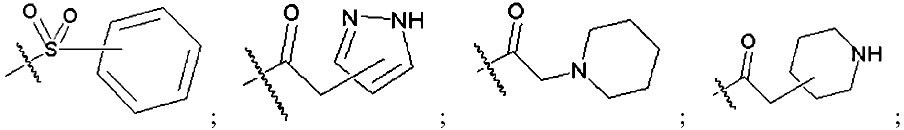 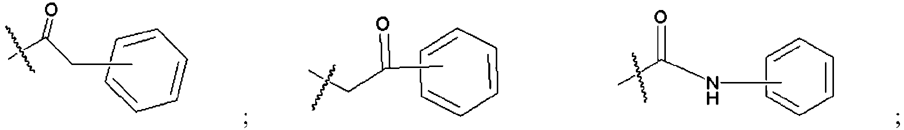 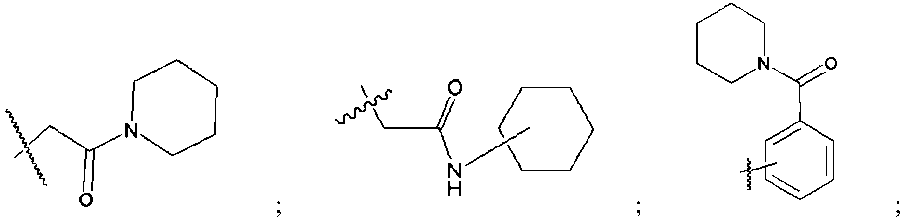 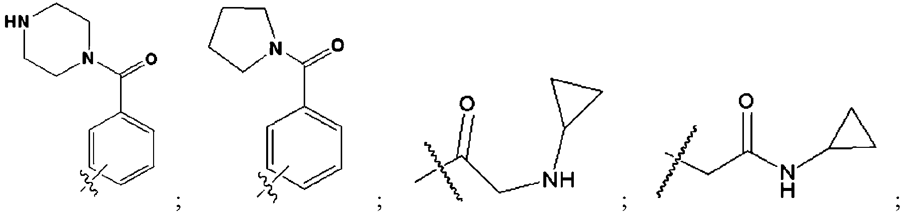 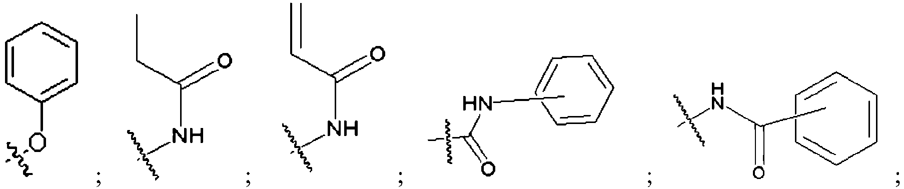 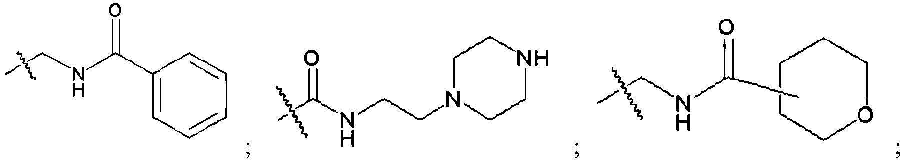 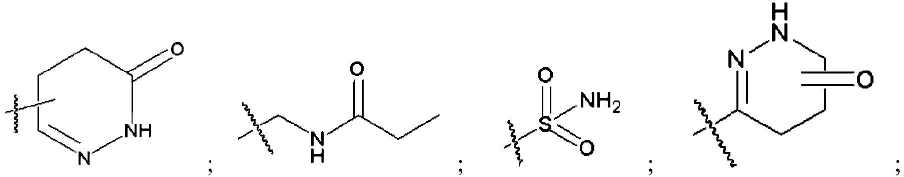 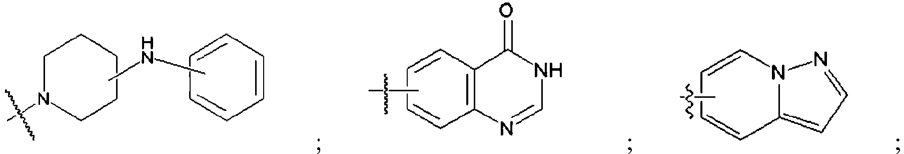 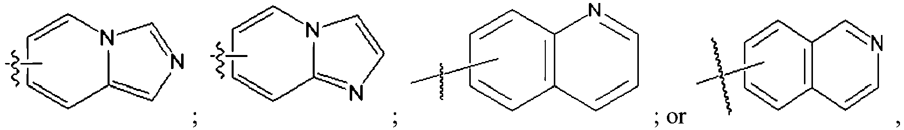
in which -H of the R₂ may be each independently unsubstituted or at least one -H may be each independently substituted with -NH₂, -NH(C₁₋₅ alkyl), -N(C₁₋₅ alkyl)₂, -NHCH₃, - N(CH₃)₂, -NHCH₂CH₃, -OH, -NO₂, -S(=O)₂CH₃, C₁₋₅ alkyl (-H of the alkyl may be unsubstituted or at least one -H may be each independently substituted with one selected from the group consisting of phenyl, pyridinyl, morpholinyl, piperazine and piperidine), - morpholidinyl, -piperidinyl, -piperazinyl, phenyl, -pyridinyl, -pyrimidinyl (-H of the - morpholidinyl, -piperidinyl, piperazinyl, phenyl, -pyridinyl and -pyrimidinyl may be unsubstituted or at least one -H may be each independently substituted with one selected from the group consisting of -C₁₋₃ alkyl and -CF₃), cyclohexyl, -cyclobutoxy, -cyclopropoxy, - cyclopentoxy, -F, -Cl, -Br, -I, -CHF₂, -CF₃, -C₁₋₃ alkoxy, -NHC(=O)CH₃, -C(=O)CH₂CH₃, - C(=O)N(CH₃)₂, -C(=O)NH(CH₃), -C(=O)NH₂, -S(=O)₂NHCH₃; -S(=O)₂, -S(=O)₂CH₃, - trifluoromethylphenyl, 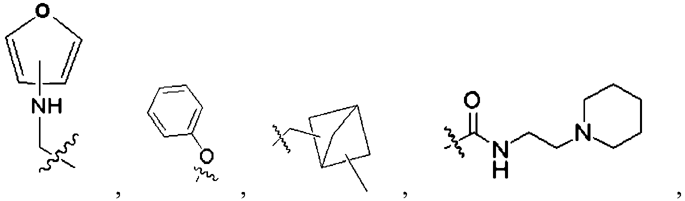 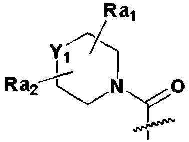 (the Ra₁ and Ra₂ may be each independently H, F, Cl, Br-, I or -C₁₋₅ alkyl, and Y₁ may be one selected from -CH₂, -NH, - and -O-), 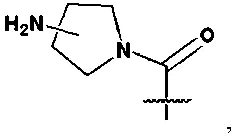 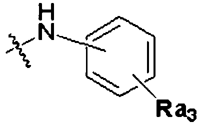 (the Ra₃ may be -H, -C₁₋₅ alkyl or -CF₃), 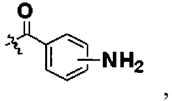 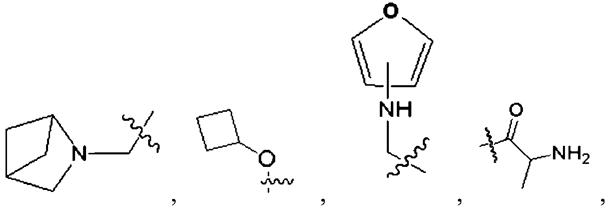
R₃ is phenyl, in which -H of the R₃ may be unsubstituted or at least one -H may be each independently substituted with one group selected from the group consisting of 1) to 18) below:
1) C₁₋₅ alkyl;
2) -F, -Cl, -Br, or -I
3) 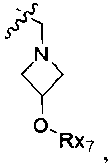 in which Rx₇ may be -CF₃;
4) 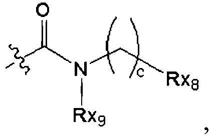
in which if c is 0 (in which Rx₈ is directly bonded to -N), Rx₈ may be C₁₋₅ alkyl or piperidine, and Rx₉ may be -H, C₁₋₅ alkyl;
if c is 1, Rx₈ may be 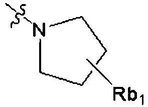
(the Rb₁ may be -NH₂ or -CH₂OH), and Rx₉ may be -H or C₁₋₅ alkyl;
if c is 2, Rx₈ may be C₁₋₅ alkyl, -NH₂, -NHCH₃, -N(CH₃)₂, piperidinyl, difluoropiperidinyl, piperazinyl, morpholinyl, 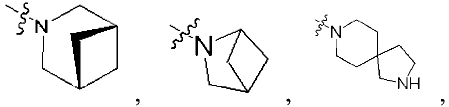 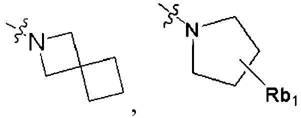
(the Rb₁ may be -NH₂ or -CH₂OH) pyridinyl, pyrrolyl, and Rx₉ may be -H or C₁₋₅ alkyl;
5) -COOH;
6) -NRx₁₀Rx₁₁, in which Rx₁₀ and Rx₁₁ may be each independently -H or -CH₃;
7) -CF₃;
8) -CN;
9) Morpholidinyl or morpholidinyl in which at least one -H is each independently substituted with C₁₋₅ alkyl, -F, -Cl, -Br, -I, -C(=O)NHCH₃, -C(=O)CH₃ or -C(=O)CH=CH₂;
10) Piperidinyl or piperidinyl in which at least one -H is each independently substituted with C₁₋₅ alkyl, -F, -Cl, -Br, -I, -C(=O)NHCH₃, -C(=O)CH₃ or -C(=O)CH=CH₂;
11) Piperazinyl or piperazinyl in which at least one -H is each independently substituted with C₁₋₅ alkyl, -F, -Cl, -Br, I, -C(=O)NHCH₃, -C(=O)CH₃ or -C(=O)CH=CH₂;
12) -ORx₁₂, in which R₁₂Rx₁₂ may be -CF₃; C₁₋₃ alkyl;
13) -OH;
14) 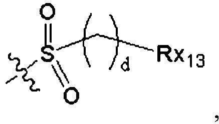 in which d may be 1, 2 or 3, and Rx₁₃ may be -NH₂, - NH(CH₃), -N(CH₃)₂pyrazolyl, methylpyrazolyl, pyrazolyl, piperazinyl, piperidinyl, or morpholinyl;
15) 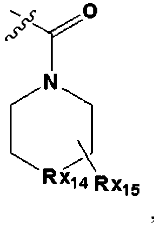 in which Rx₁₄ may be -CH₂-, NH- or -O-, and Rx₁₅ may be -H, C₁₋₅ alkyl, -NH₂, -F, -Cl, -Br, or I;
16) 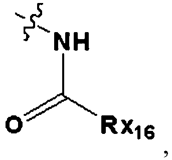 in which Rx₁₆ may be C₁₋₅ alkyl or -CH₂CH₂N(CH₃)₂;
17) 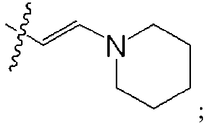 or
18) Ethylamine,
Ak is -CH₂-, -(CH₂)₂-, -(CH₂)₃- or 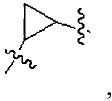 -H of -CH₂-, -(CH₂)₂-, -(CH₂)₃- or 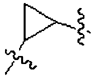 may be each independently unsubstituted or at least one -H may be each independently substituted with -NH₂, -OH, -NO₂, C₁₋₅ alkyl, -CH₂NH₂, -CF₃, OCF₃, -CN, -F, - Cl, -Br, or -I.

15. A urea compound represented by formula I-1 below, a pharmaceutically acceptable salt of them, or a hydrate or solvate of them: 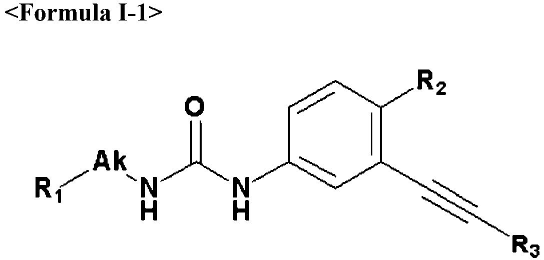
wherein the R₁ is a functional group of Table 1, -H of R₁ may be unsubstituted or at least one H may be each independently substituted with -NH₂, -OH, -NO₂, -CH₂NH₂, -CF₃, - OCF₃, -CN, -C₁₋₅ alkyl, -F, -Cl, -Br, or -I,
R₂ is a functional group of Table 2, -H of R₂ may be unsubstituted or at least one H may be each independently substituted with -NH₂, -OH, NO₂, -C₁₋₅ alkyl, -CH₂NH₂, -CF₃, - OCF₃, -CN, -F, -Cl, -Br, or -I,
R₃ is a functional group of Table 3, -H of the R₃ may be unsubstituted or at least one H may be each independently substituted with -NH₂, -NHCH₃, -OH, -NO₂, -C₁₋₃alkyl, -CH₂NH₂, -CF₃, -OCF₃, -CN, -F, -Cl, -Br, or -NH₂, -OH, -NO₂, -C₁₋₅alkyl, -CH₂NH₂, -CF₃, -OCF₃, -CN, - F, -Cl, -Br, or -I I, 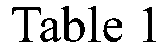 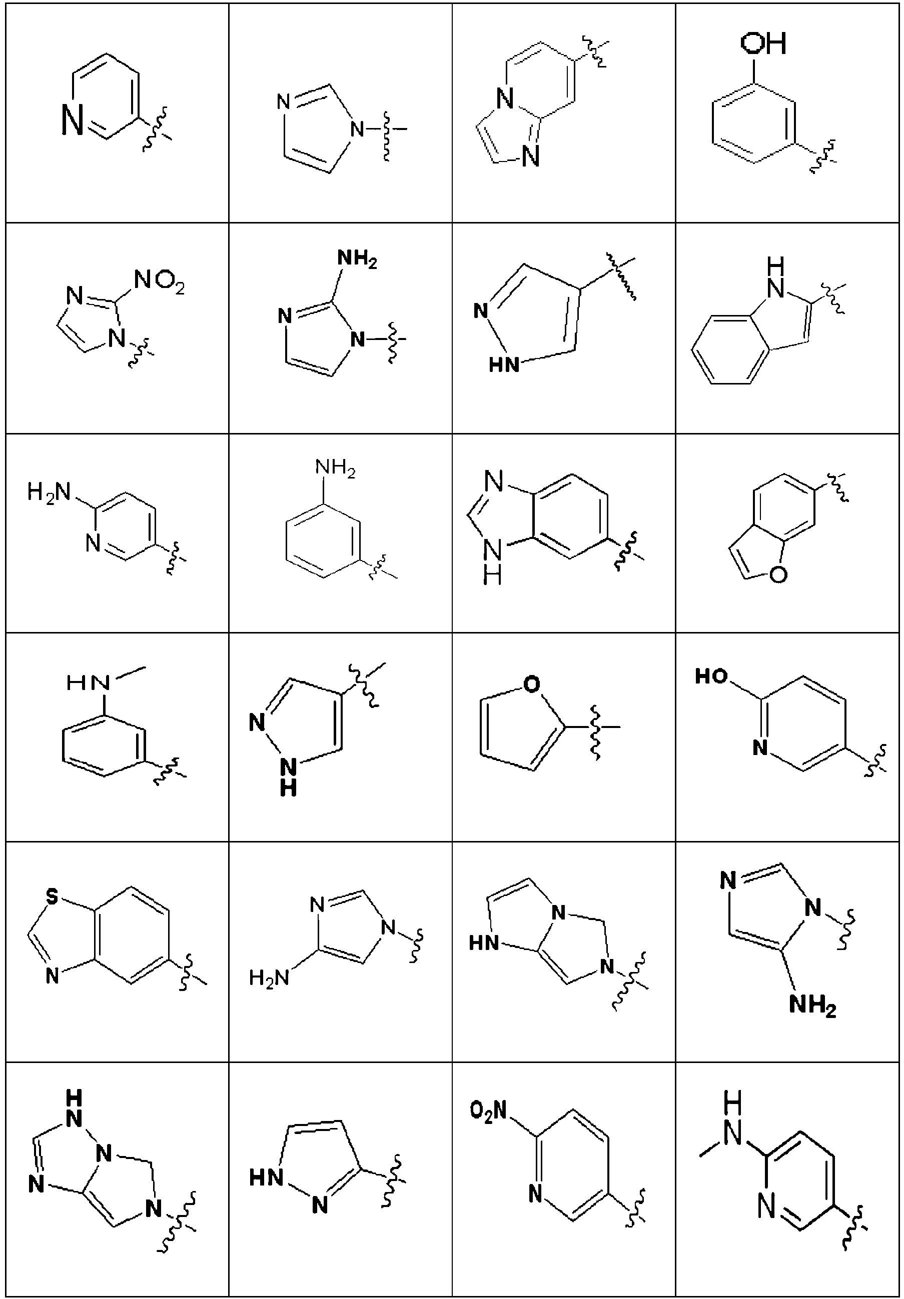 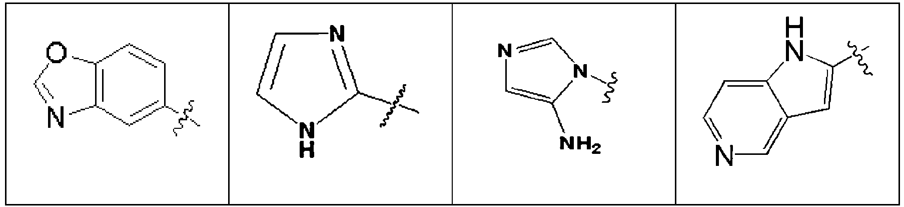 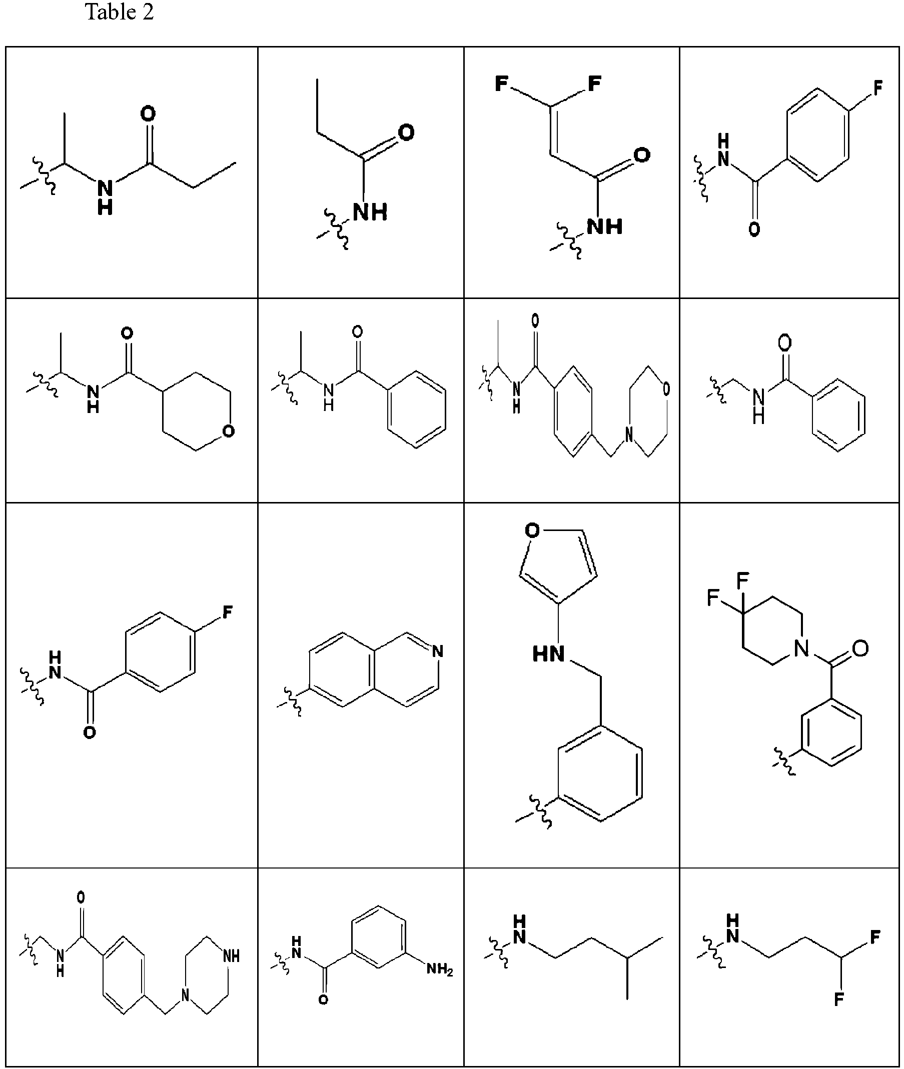 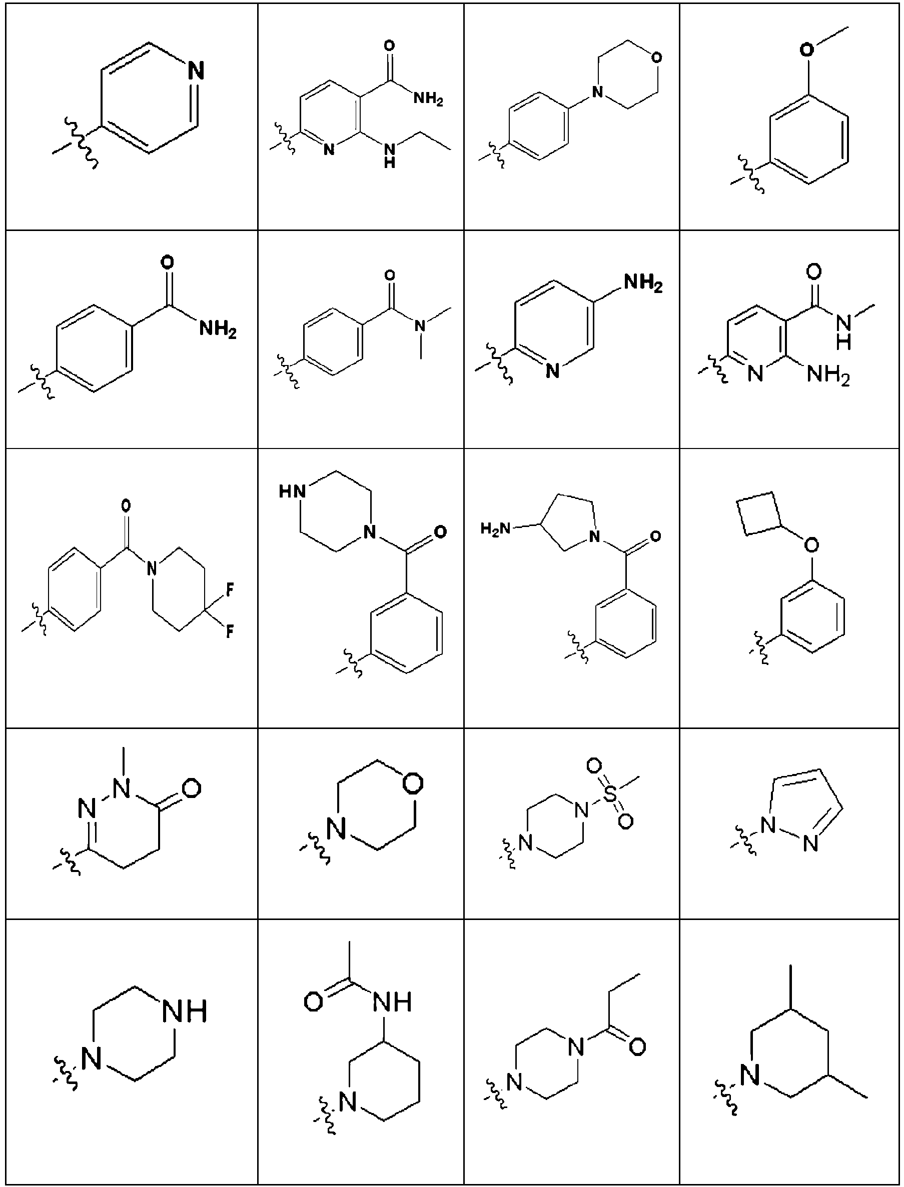 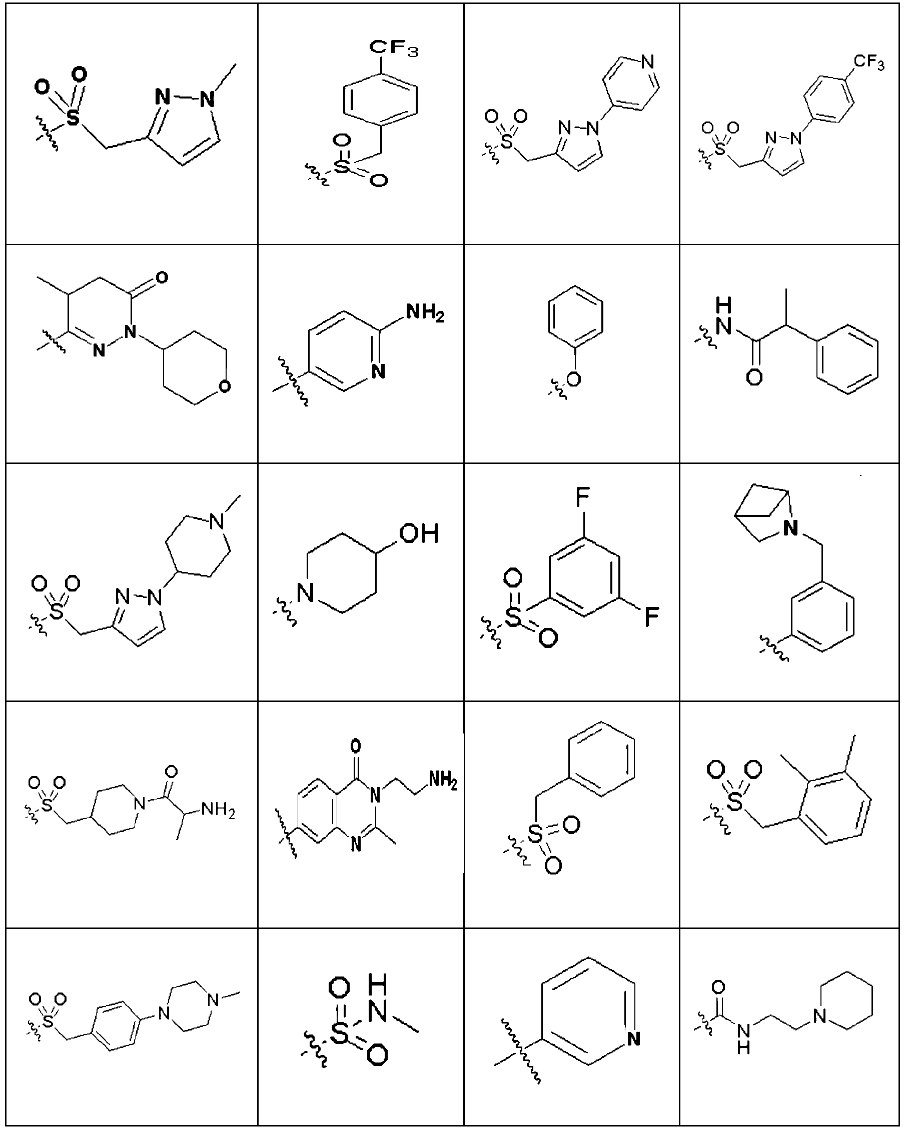 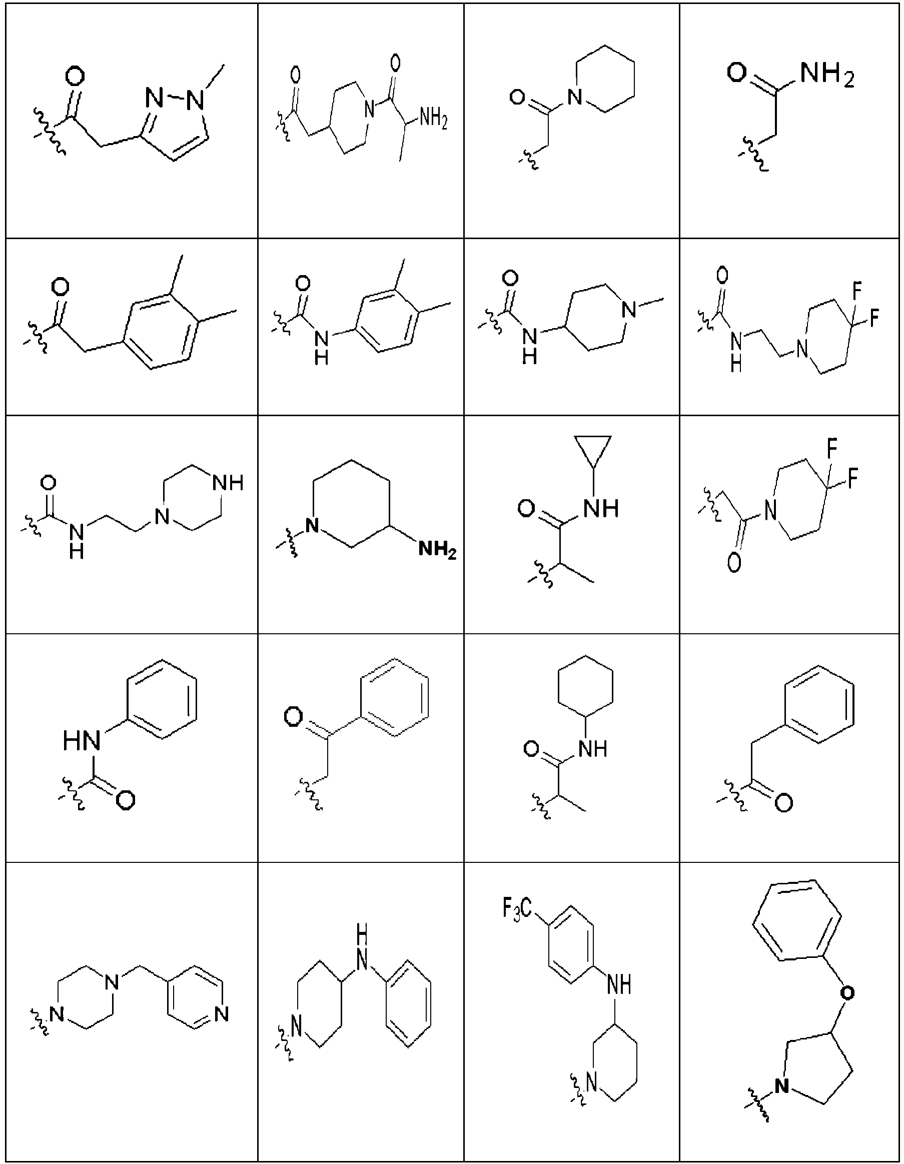 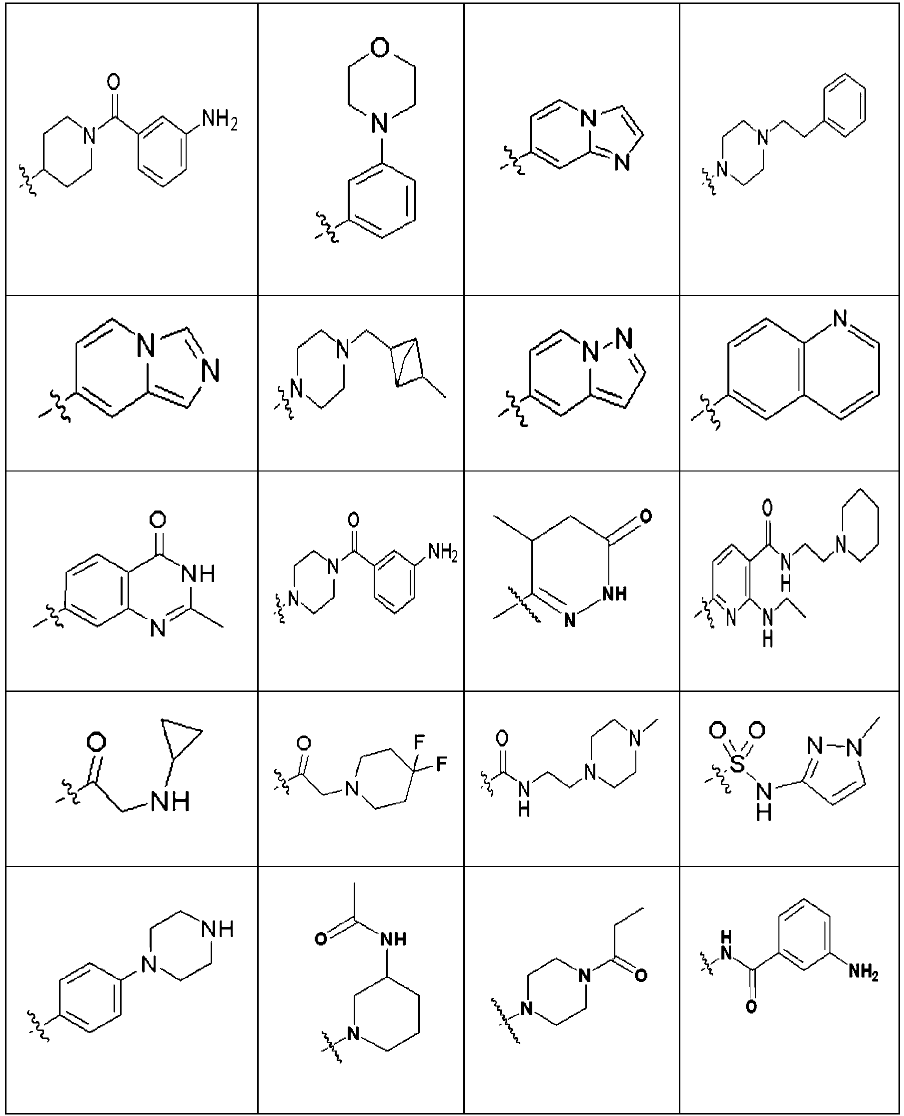 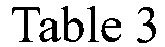 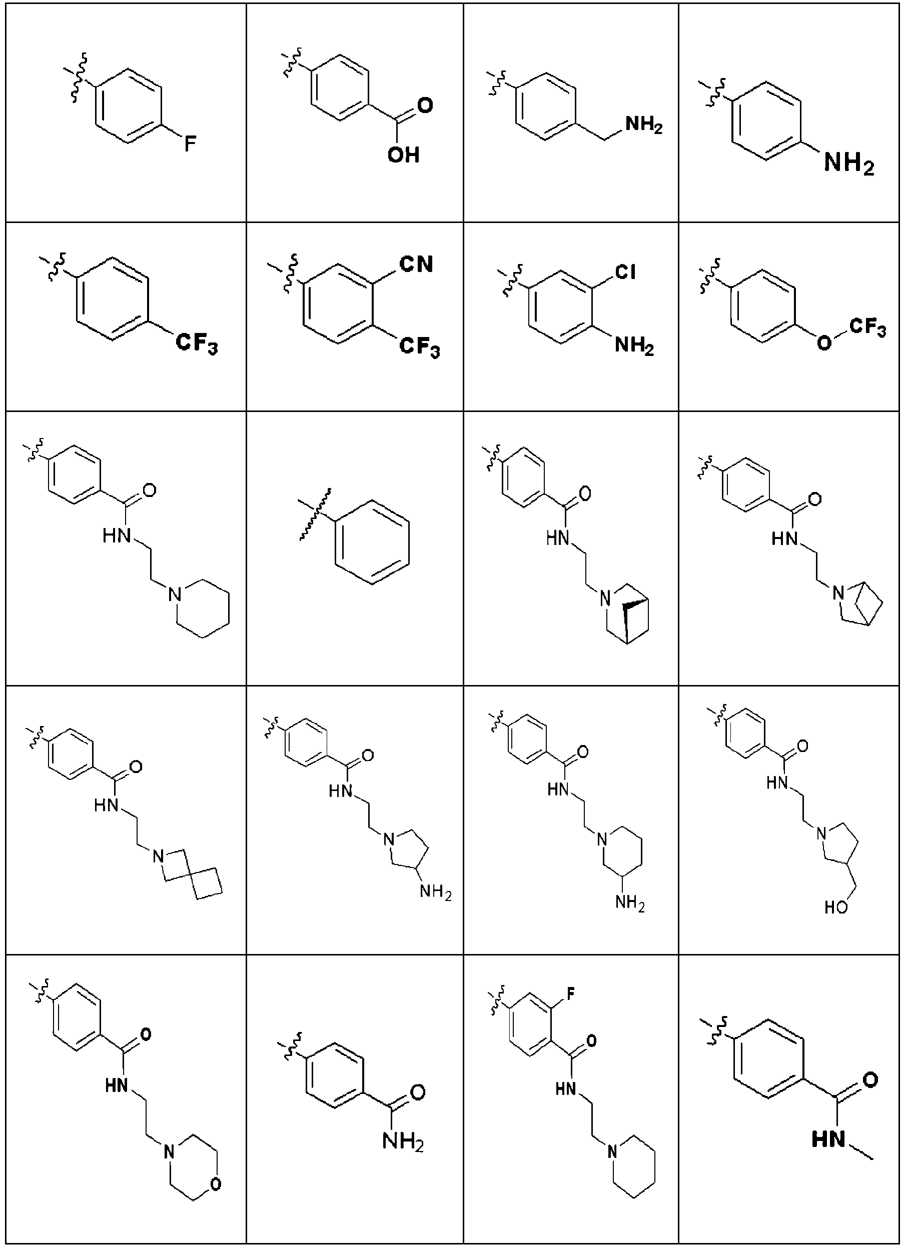 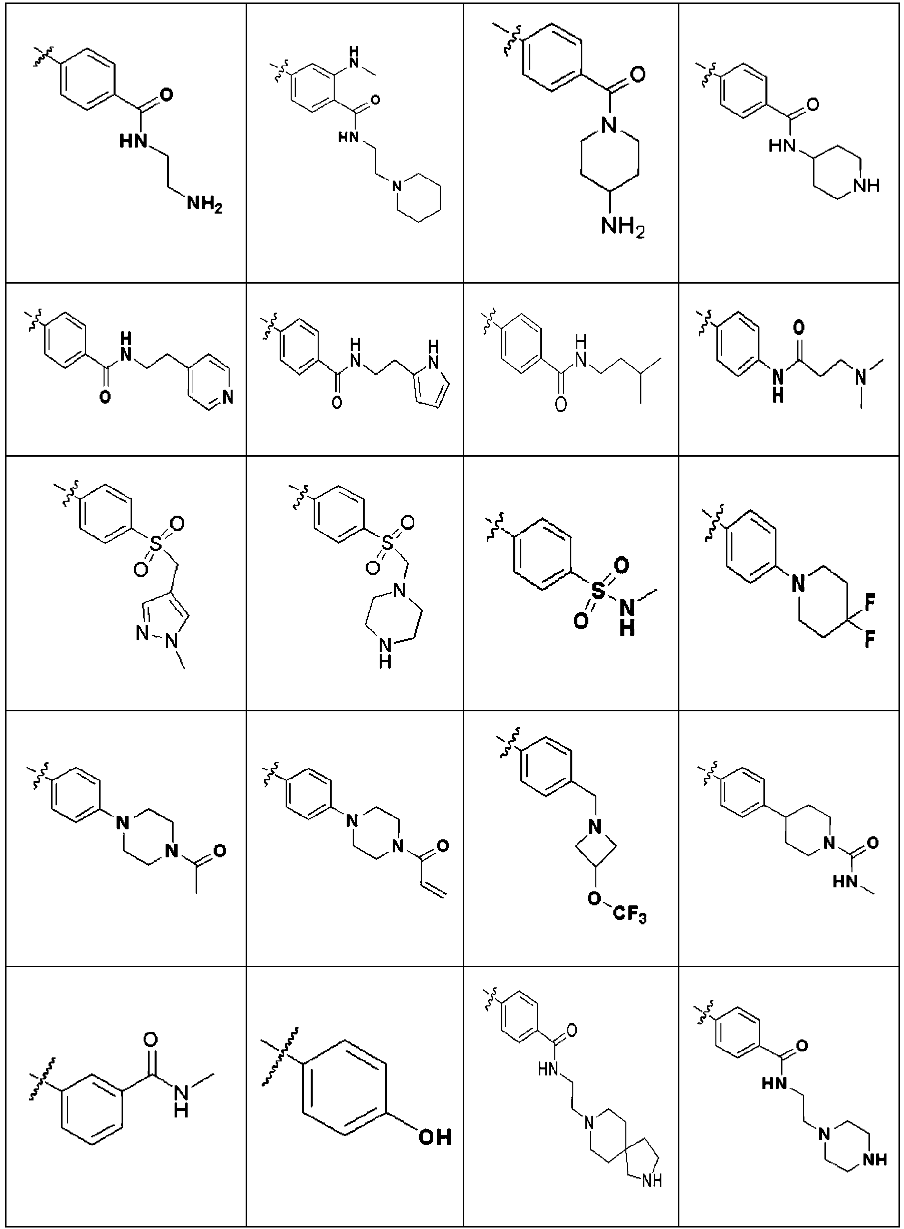 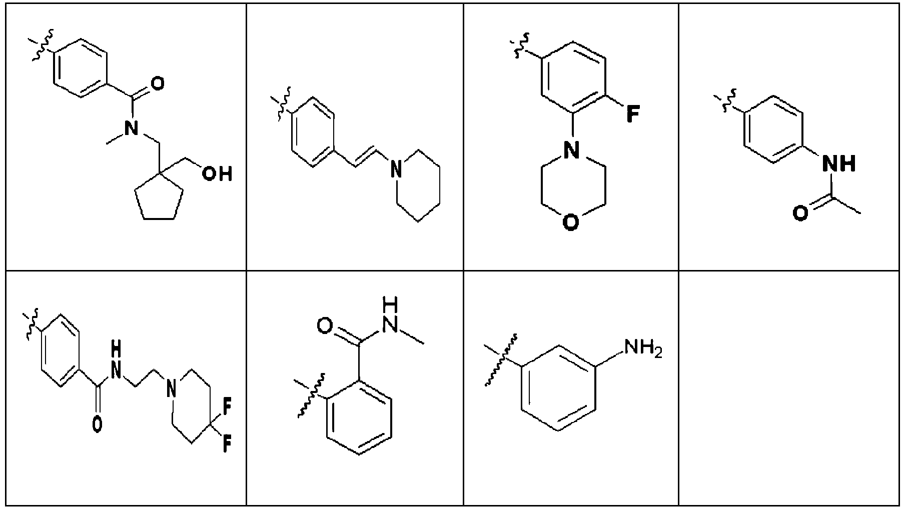
Ak may be -CH₂-, -(CH₂)₂-, -(CH₂)₃- or 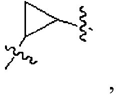 -H of -CH₂-, -(CH₂)₂-, -(CH₂)₃- or 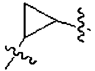 may be each independently unsubstituted or at least one -H may be each independently substituted with -NH₂, -OH, -NO₂, C₁₋₅ alkyl, -CH₂NH₂, -CF₃, OCF₃, -CN, -F, - Cl, -Br, or -I.

16. A urea compound represented by formula I-2 below, an optical isomer thereof, a pharmaceutically acceptable salt of them, or a hydrate or solvate of them: 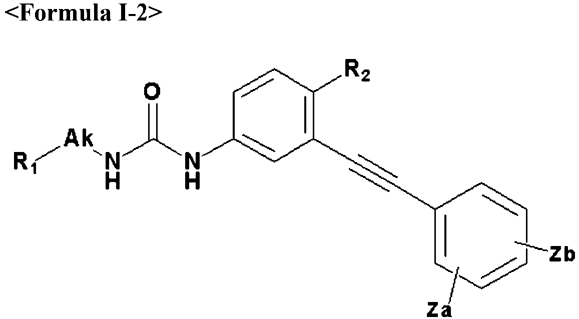
Wherein R₁, R₂ and Ak are same as defined in claim 14, and
Za and Zb are each independently -H or one selected from the group consisting of 1) to 18) below:
1) C₁₋₅ alkyl;
2) -F, -Cl, -Br, or -I
3) 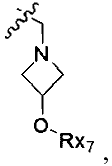 in which Rx₇ may be C₁₋₅ alkyl or -CF₃;
4) 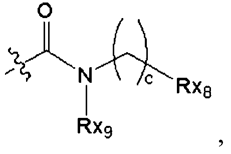 in which c is 0, 1, 2 or 3, Rx₈ may be C₁₋₅ alkyl, -NH₂, - NHCH₃, -N(CH₃)₂, piperidinyl, piperazinyl, morpholinyl, 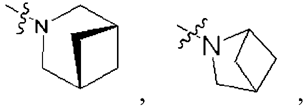 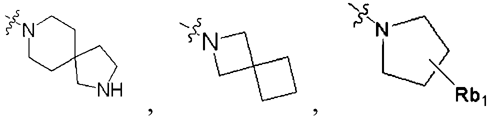 (the Rb₁ may be -NH₂ or -CH₂OH), 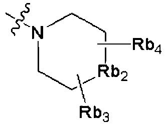 (the Rb₂ may be -CH₂-, -NH- or -O-, and Rb₃ and Rb₄ may be each independently -H, -F, -Cl, -Br, or -I), pyrimidinyl or pyrrolyl, and Rx₉ may be -H or C₁₋₅ alkyl;
5) -COOH;
6) -NRx₁₀Rx₁₁, in which Rx₁₀ or Rx₁₁ may be each independently -H or -CH₃;
7) -CF₃;
8) -CN;
9) Morpholinyl or morpholidinyl in which at least one -H is each independently substituted with C₁₋₅ alkyl, -F, -Cl, -Br, I, -C(=O)NHCH₃, -C(=O)CH₃ or -C(=O)CH=CH₂;
10) Piperidinyl or piperidinyl in which at least one -H is each independently substituted with C₁₋₅ alkyl, -F, -Cl, -Br, I, -C(=O)NHCH₃, -C(=O)CH₃ or -C(=O)CH=CH₂;
11) Piperazinyl or piperazinyl in which at least one -H is each independently substituted with C₁₋₅ alkyl, -F, -Cl, -Br, I, -C(=O)NHCH₃, -C(=O)CH₃ or -C(=O)CH=CH₂;
12) -ORx₁₂, in which R₁₂Rx₁₂ may be -CF₃; C₁₋₃ alkyl;
13) -OH;
14) 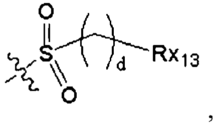 in which d may be 0, 1, 2 or 3, and Rx₁₃ may be -NH₂, - NH(CH₃), -N(CH₃)₂pyrazolyl, methylpyrazolyl, pyrazolyl, piperazinyl, piperidinyl, or morpholinyl;
15) 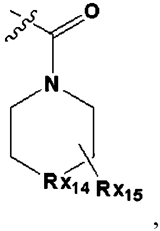 in which Rx₁₄ may be -CH₂-, -NH or -O-, and Rx₁₅ may be -H, linear or branched C₁₋₅ alkyl, -NH₂, -F, -Cl, -Br, or -I;
16) 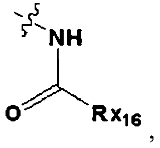 in which Rx₁₆ may be C₁₋₅ alkyl or -CH₂CH₂N(CH₃)₂,
17) 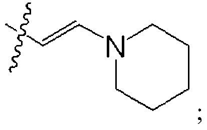 or
18) -(C₁₋₃ alkyl)-NH₂.

17. Compounds 1 to 189 below, optical isomers thereof, pharmaceutically acceptable salts of them, or hydrates or solvates of them:
| **No.** | **Compound** |
|---|---|
| **1** | 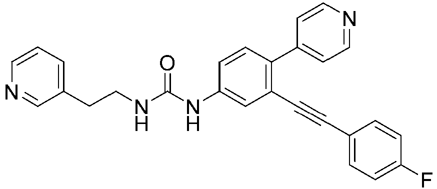 |
| **2** | 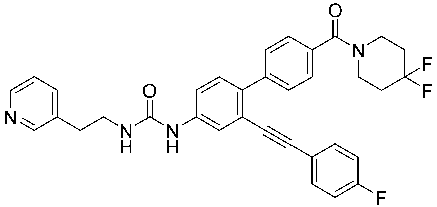 |
| **3** | 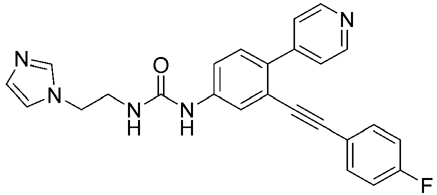 |
| **4** | 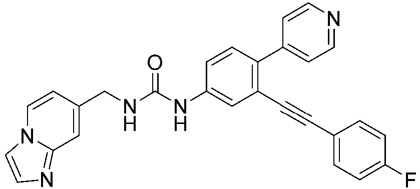 |
| **5** | 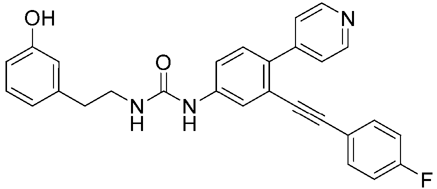 |
| **6** | 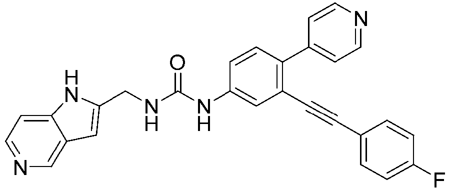 |
| **7** | 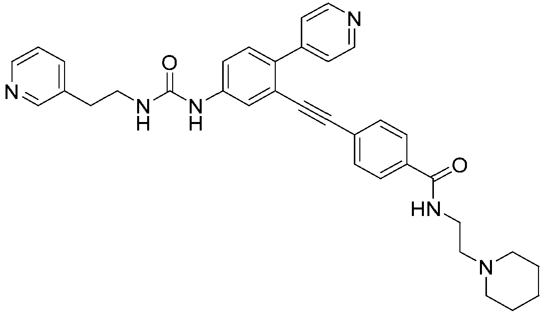 |
| **8** | 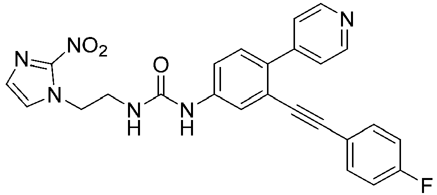 |
| **9** | 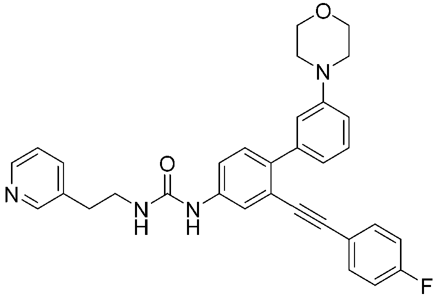 |
| **10** | 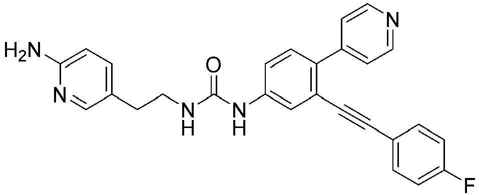 |
| **11** | 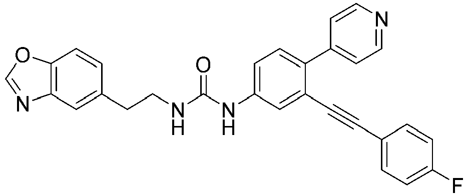 |
| **12** | 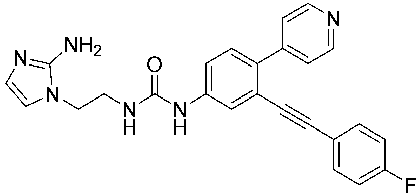 |
| **13** | 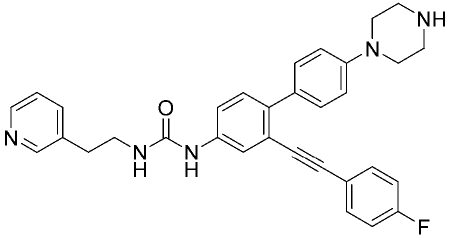 |
| **14** | 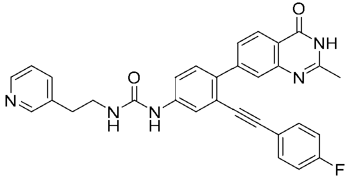 |
| **15** | 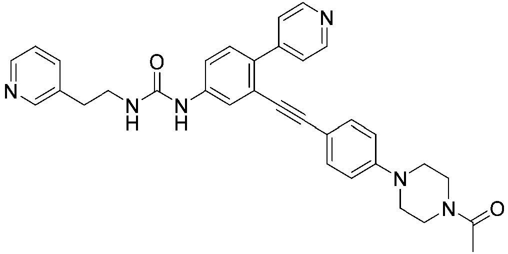 |
| **16** | 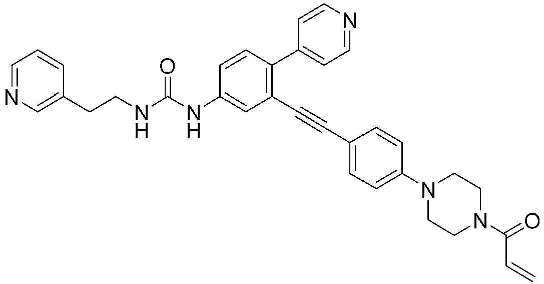 |
| **17** | 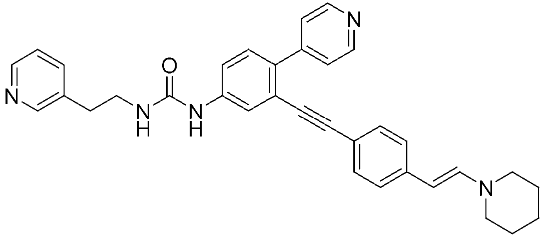 |
| **18** | 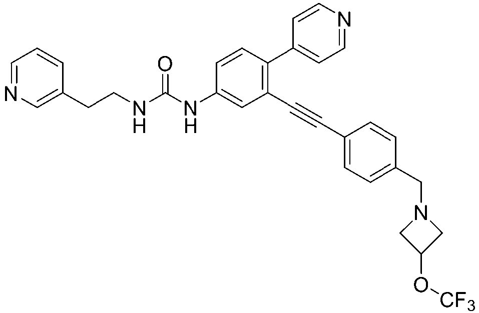 |
| **19** | 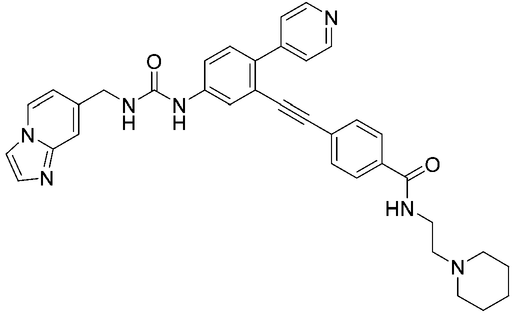 |
| **20** | 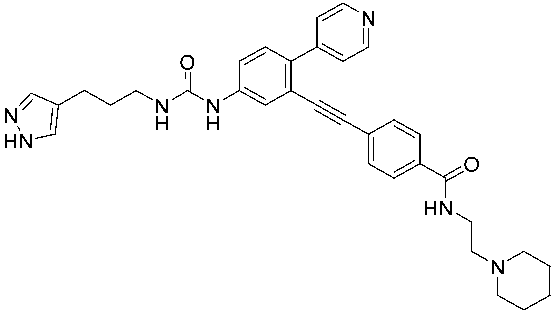 |
| **21** | 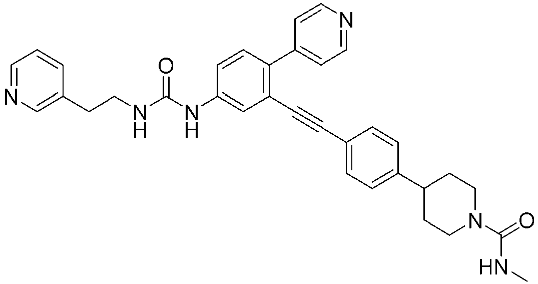 |
| **22** | 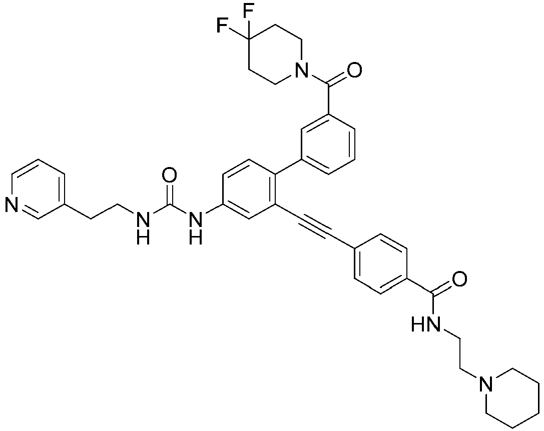 |
| **23** | 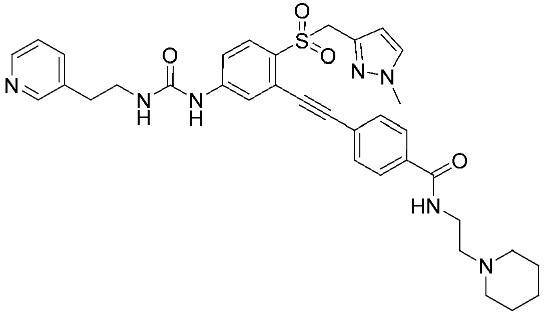 |
| **24** | 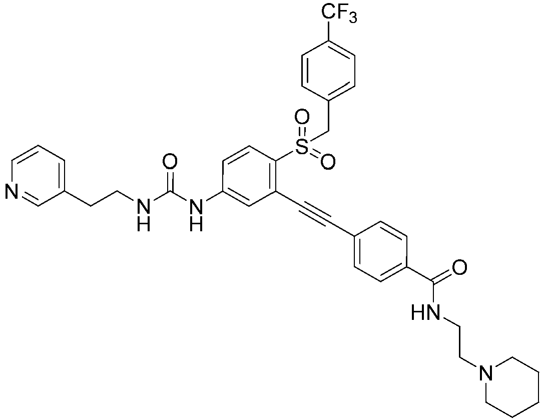 |
| **25** | 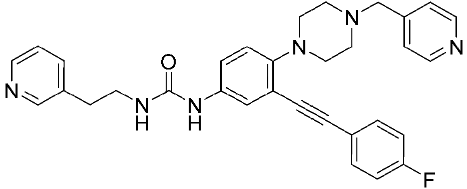 |
| **26** | 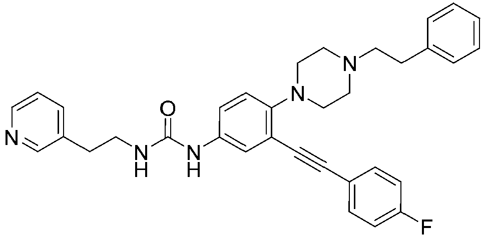 |
| **27** | 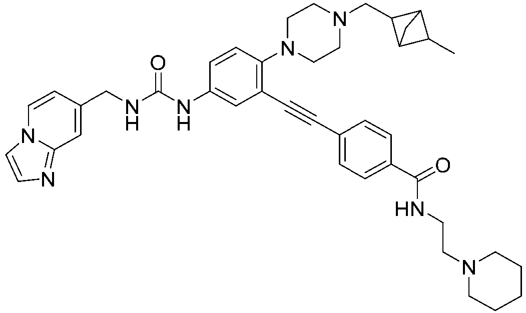 |
| **28** | 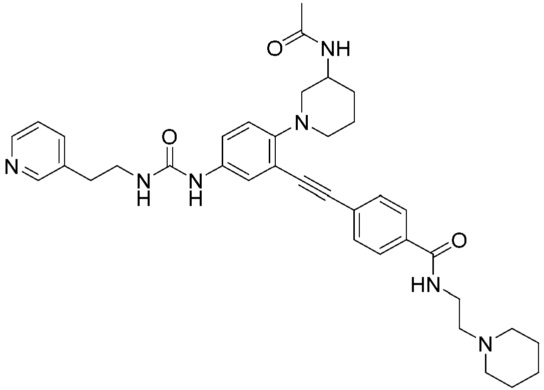 |
| **29** | 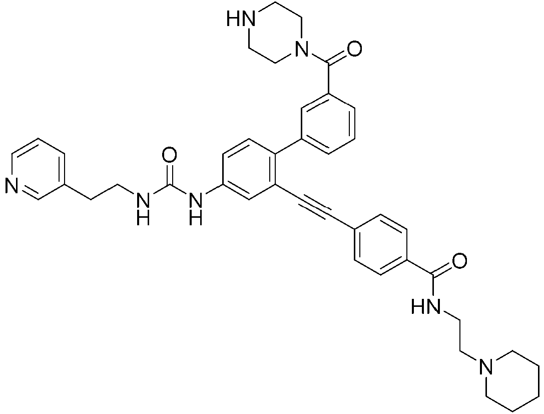 |
| **30** | 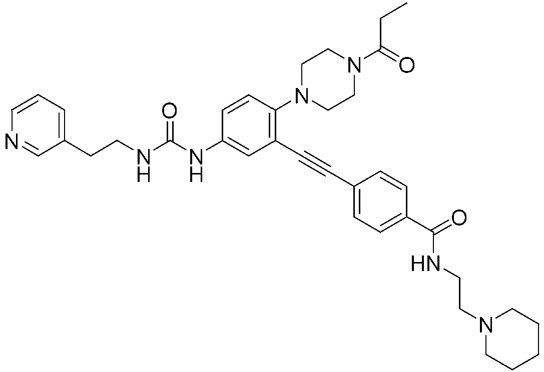 |
| **31** | 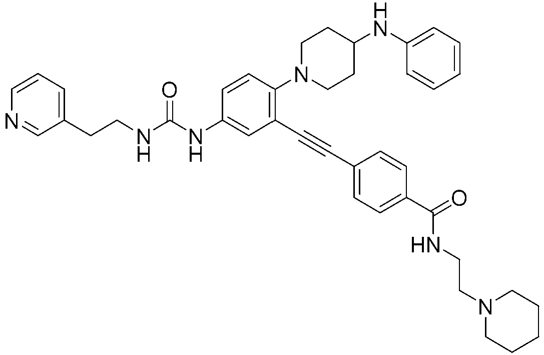 |
| **32** | 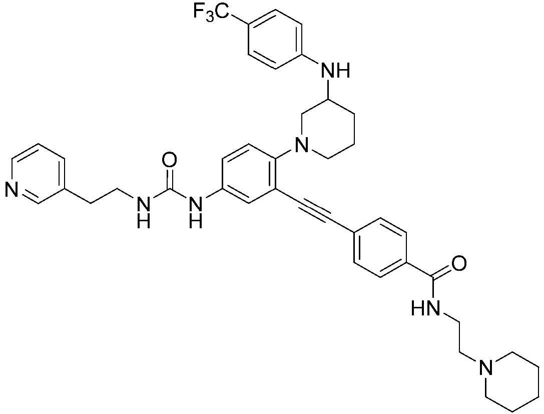 |
| **33** | 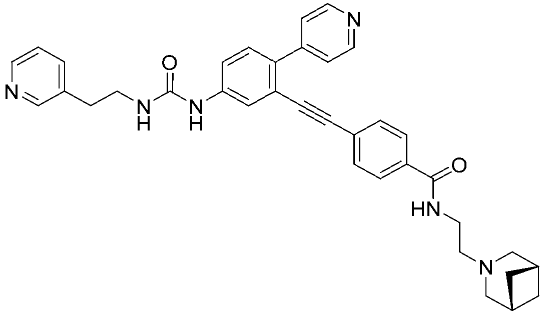 |
| **34** | 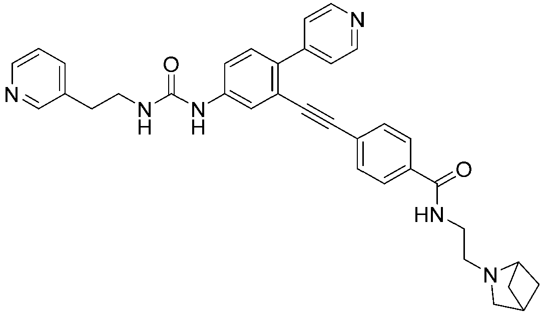 |
| **35** | 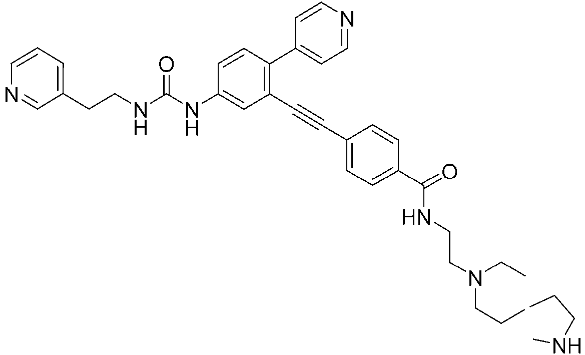 |
| **36** | 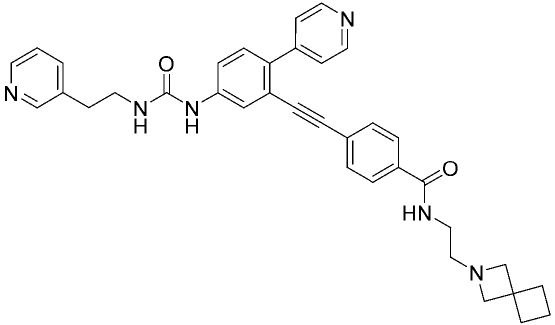 |
| **37** | 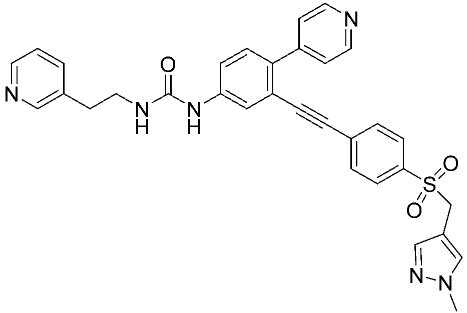 |
| **38** | 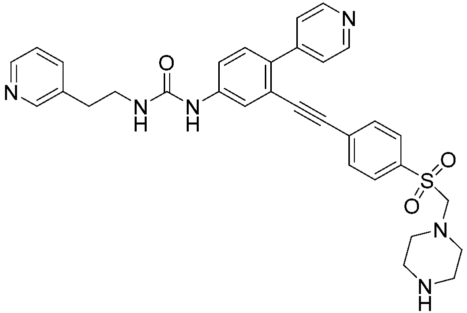 |
| **39** | 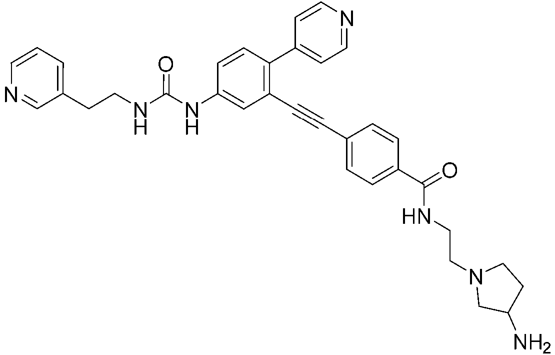 |
| **40** | 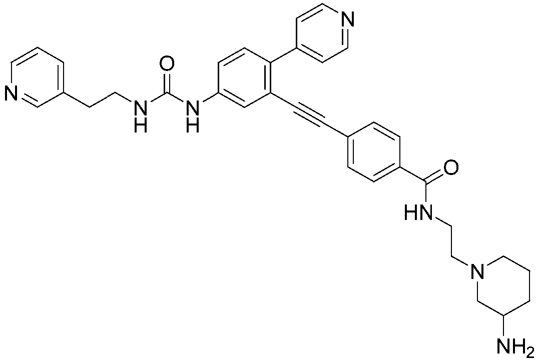 |
| **41** | 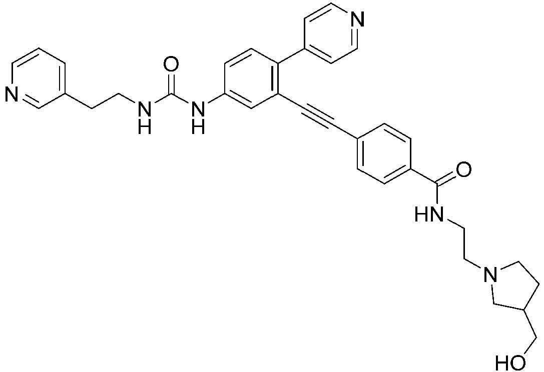 |
| **42** | 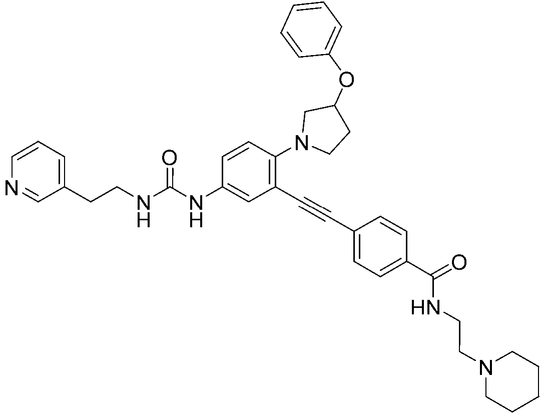 |
| **43** | 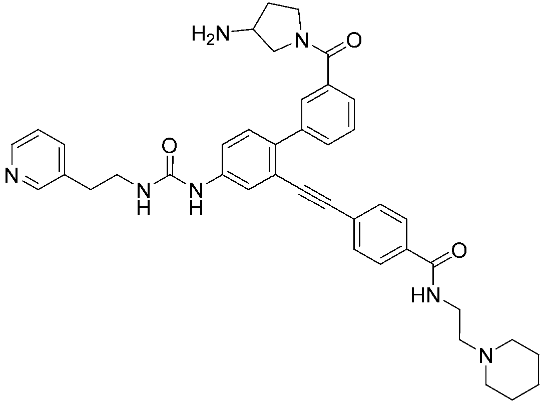 |
| **44** | 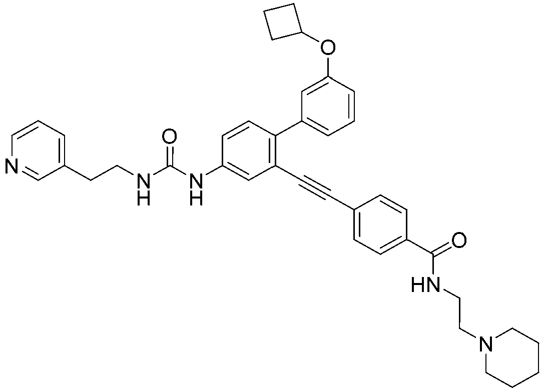 |
| **45** | 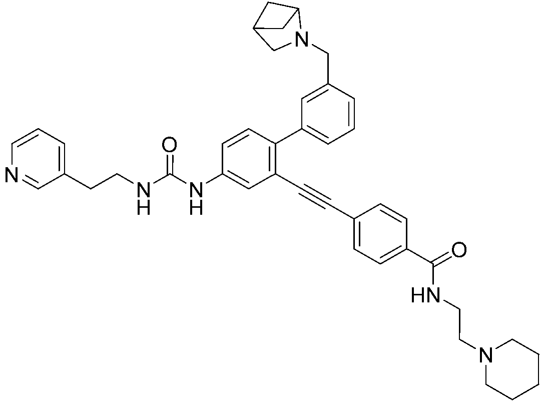 |
| **46** | 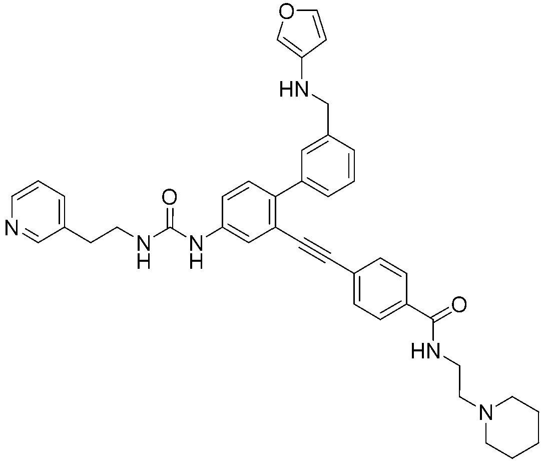 |
| **47** | 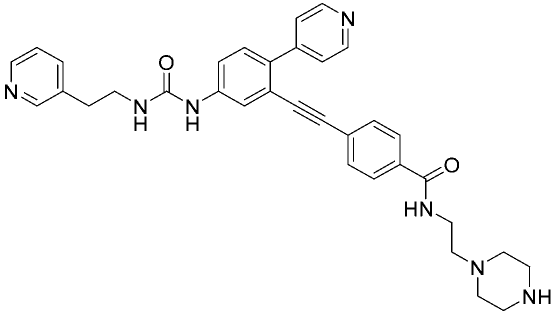 |
| **48** | 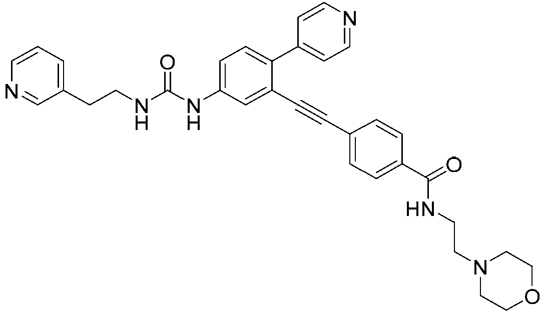 |
| **49** | 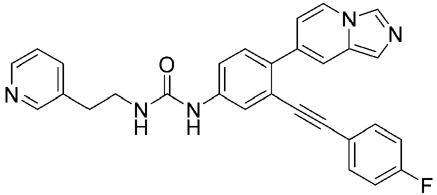 |
| **50** | 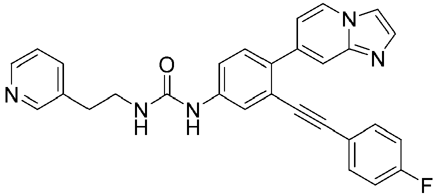 |
| **51** | 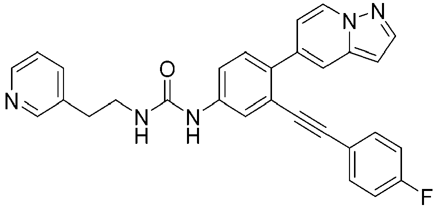 |
| **52** | 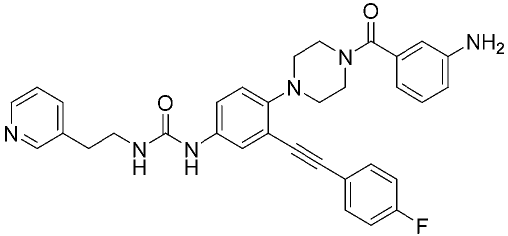 |
| **53** | 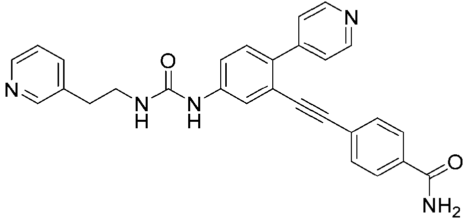 |
| **54** | 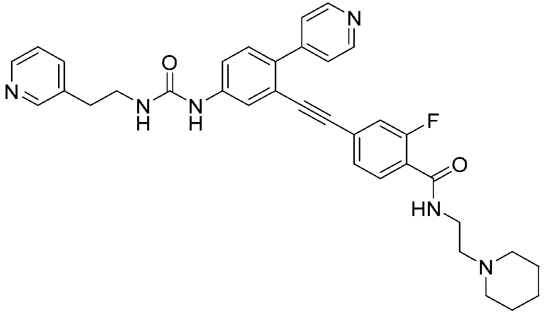 |
| **55** | 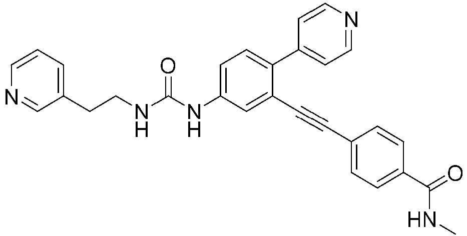 |
| **56** | 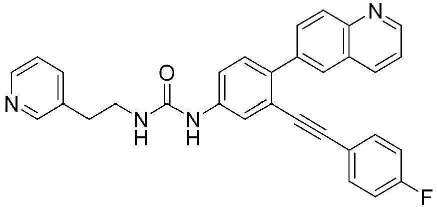 |
| **57** | 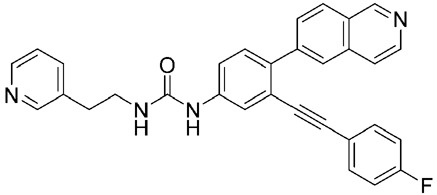 |
| **58** | 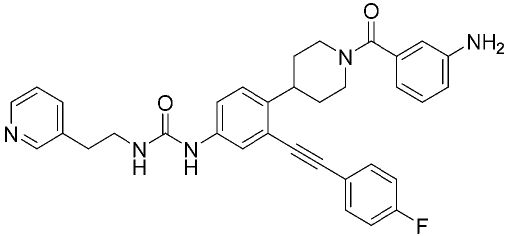 |
| **59** | 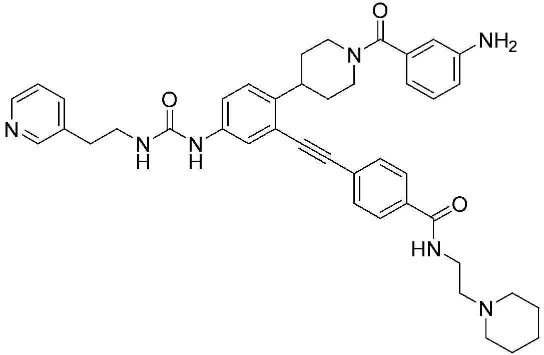 |
| **60** | 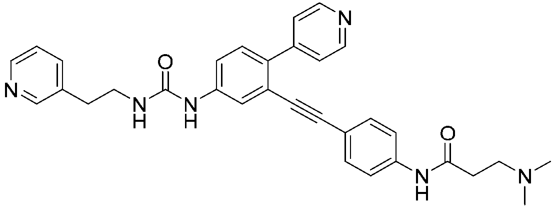 |
| **61** | 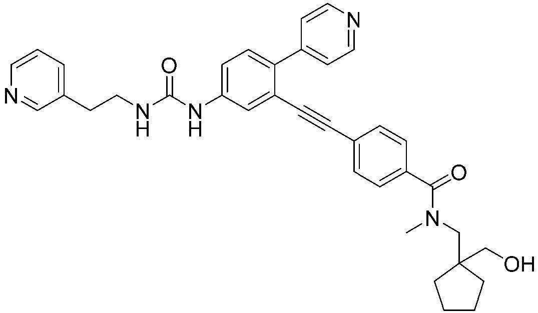 |
| **62** | 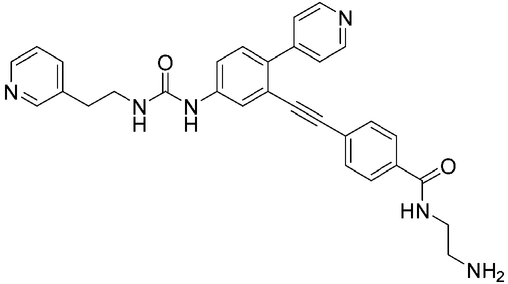 |
| **63** | 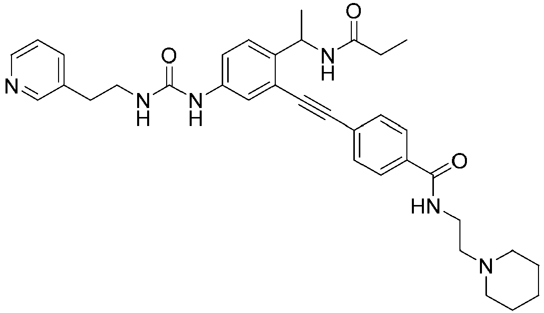 |
| **64** | 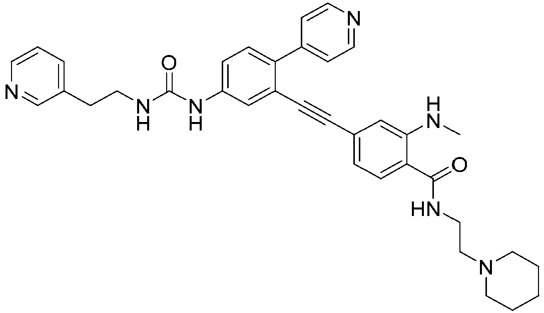 |
| **65** | 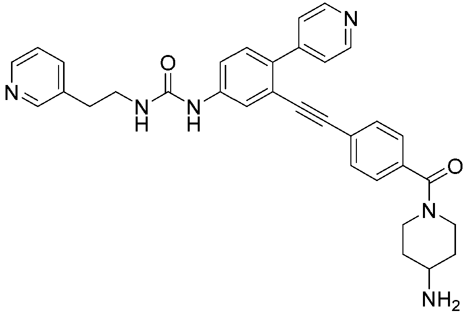 |
| **66** | 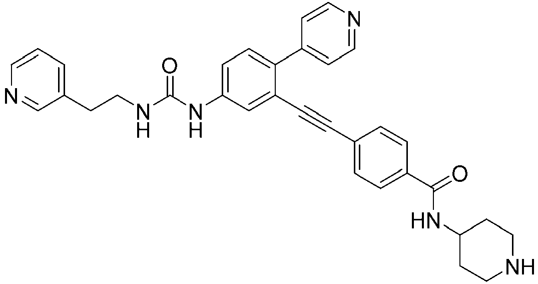 |
| **67** | 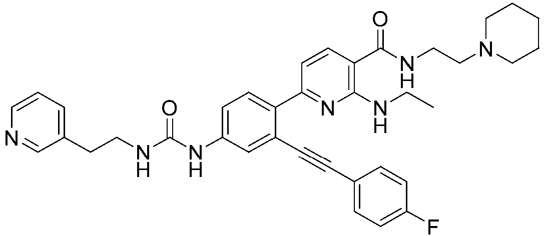 |
| **68** | 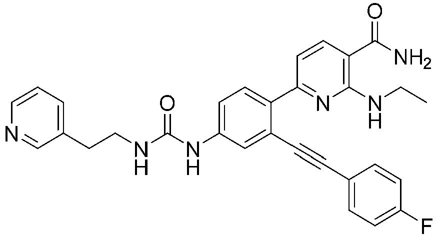 |
| **69** | 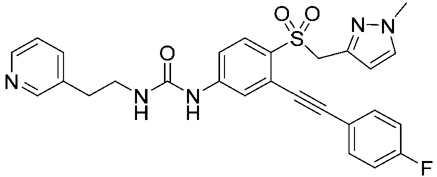 |
| **70** | 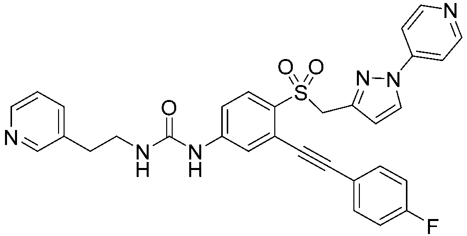 |
| **71** | 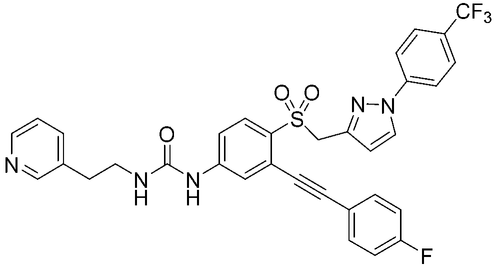 |
| **72** | 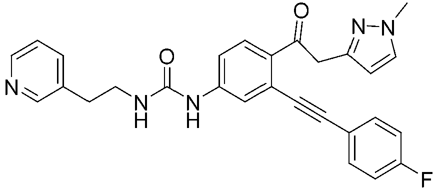 |
| **73** | 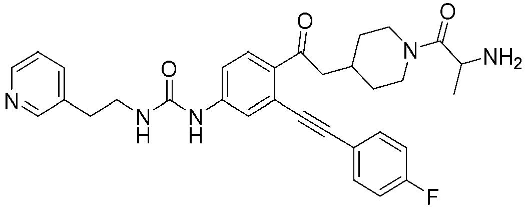 |
| **74** | 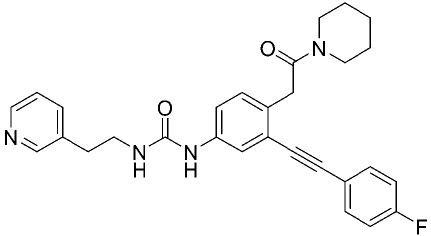 |
| **75** | 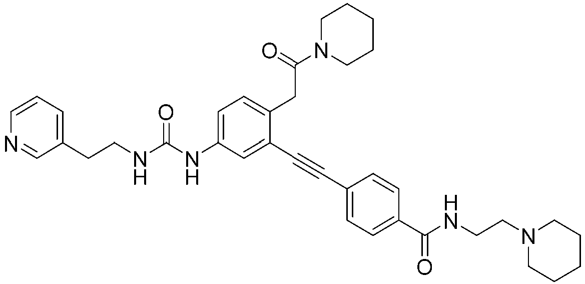 |
| **76** | 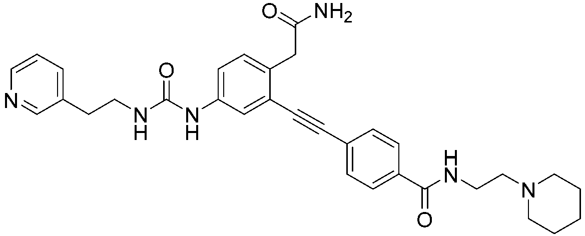 |
| **77** | 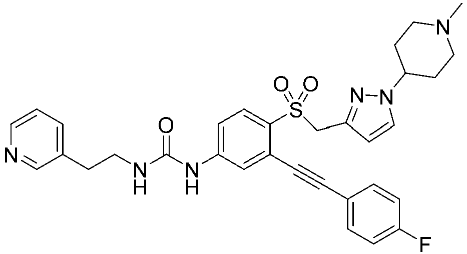 |
| **78** | 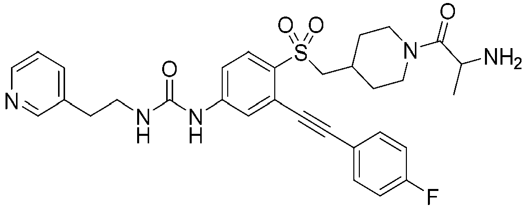 |
| **79** | 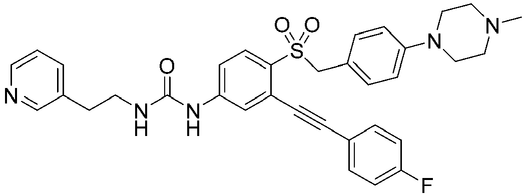 |
| **80** | 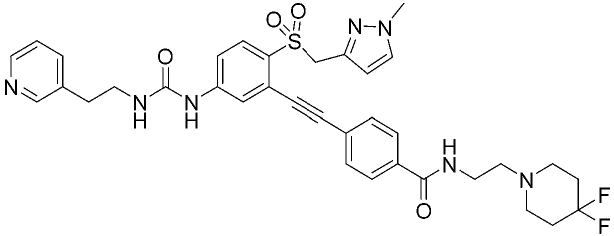 |
| **81** | 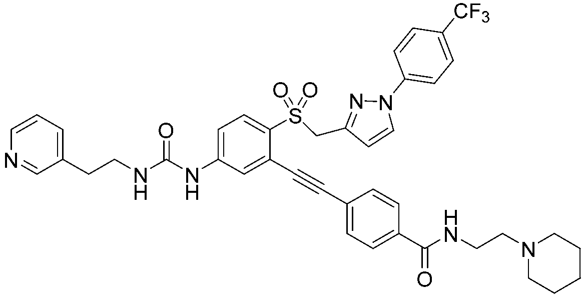 |
| **82** | 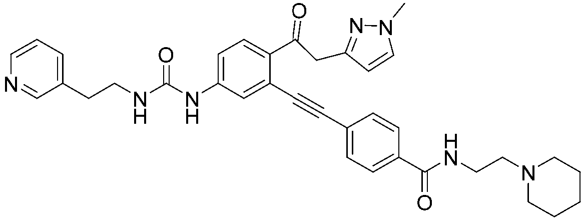 |
| **83** | 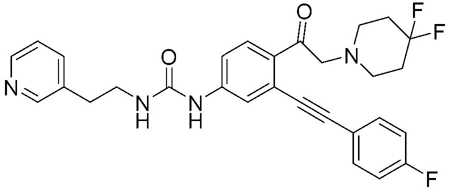 |
| **84** | 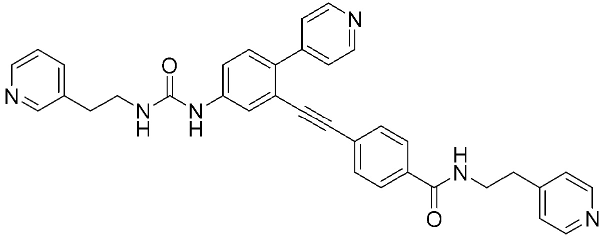 |
| **85** | 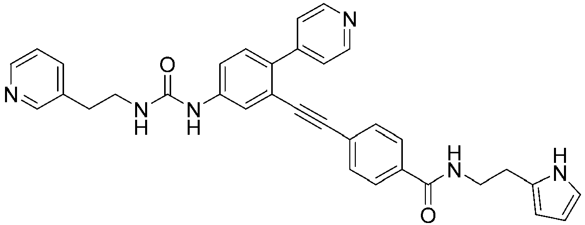 |
| **86** | 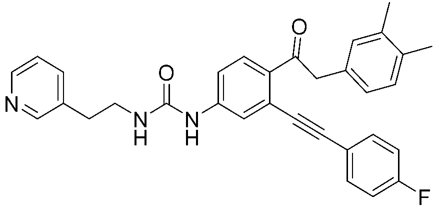 |
| **87** | 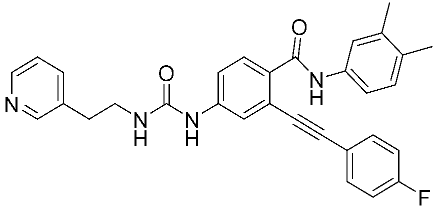 |
| **88** | 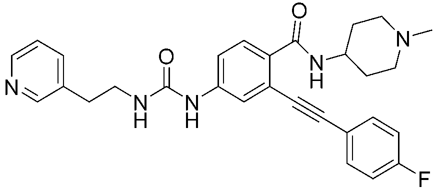 |
| **89** | 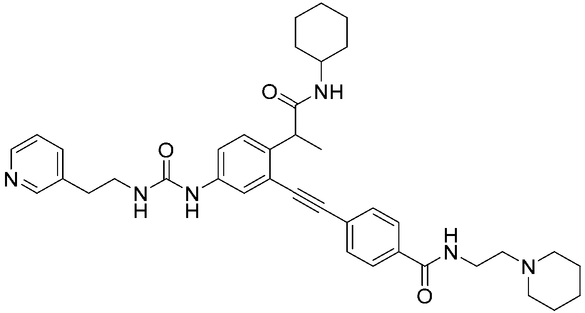 |
| **90** | 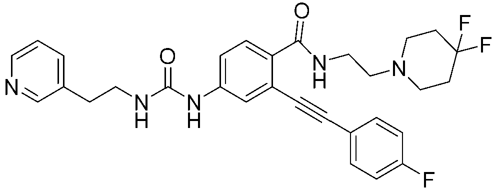 |
| **91** | 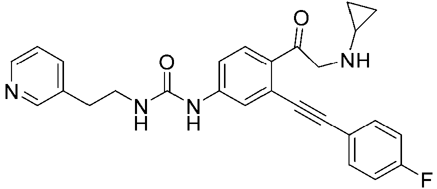 |
| **92** | 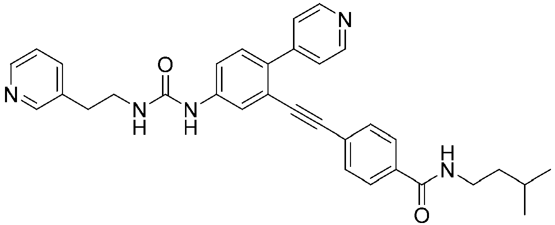 |
| **93** | 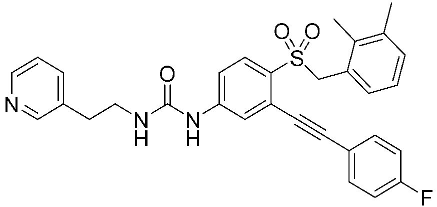 |
| **94** | 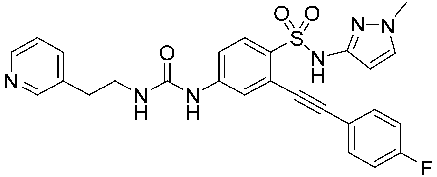 |
| **95** | 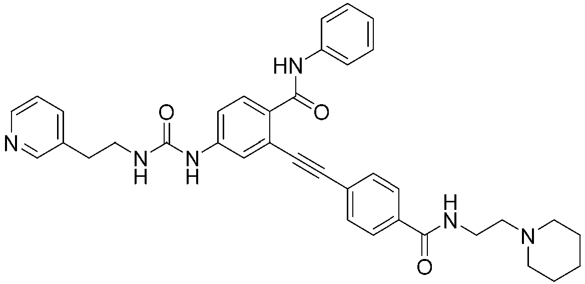 |
| **96** | 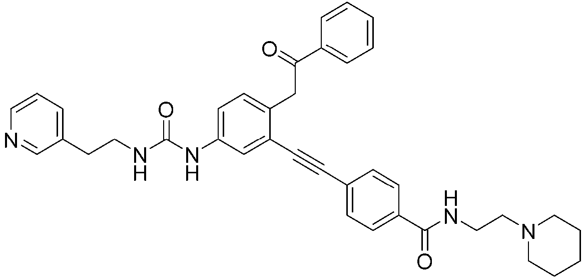 |
| **97** | 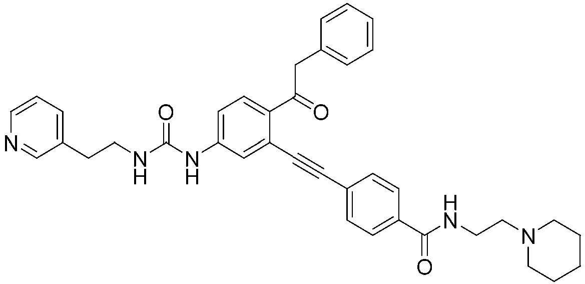 |
| **98** | 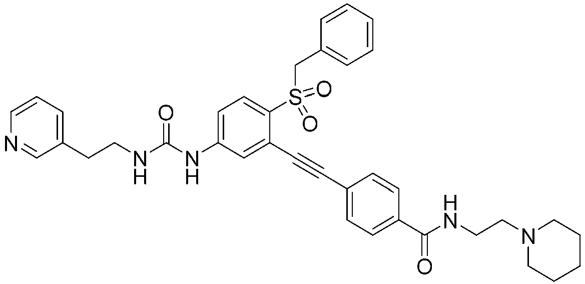 |
| **99** | 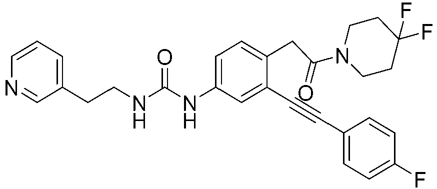 |
| **100** | 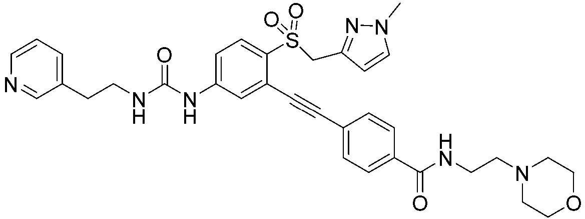 |
| **101** | 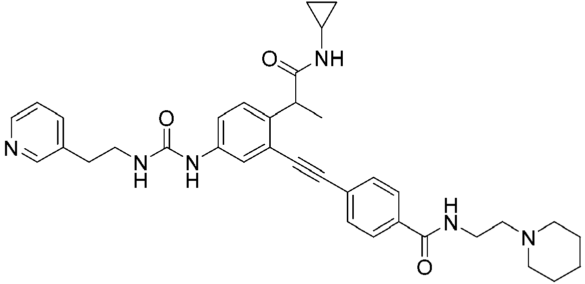 |
| **102** | 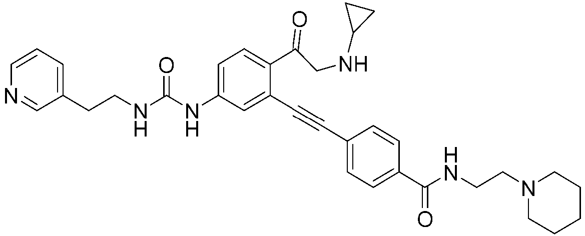 |
| **103** | 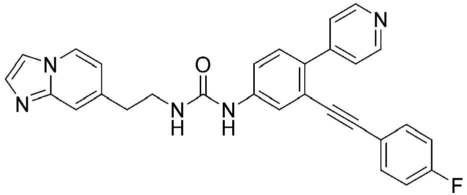 |
| **104** | 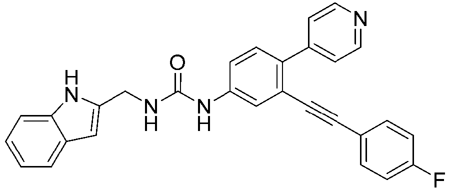 |
| **105** | 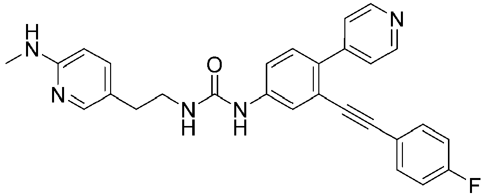 |
| **106** | 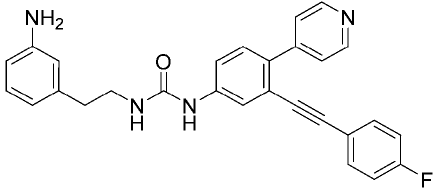 |
| **107** | 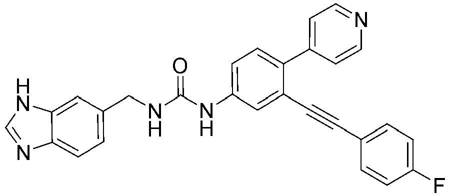 |
| **108** | 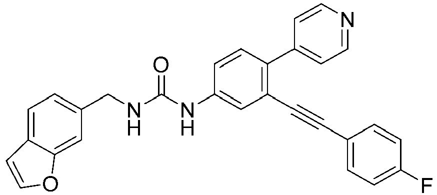 |
| **109** | 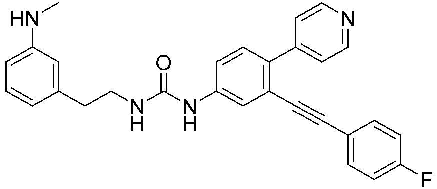 |
| **110** | 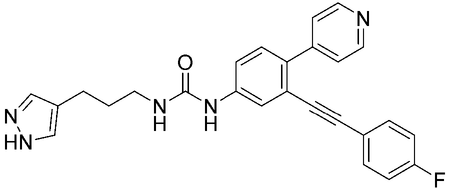 |
| **111** | 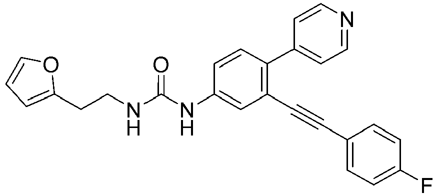 |
| **112** | 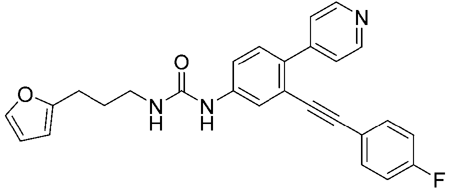 |
| **113** | 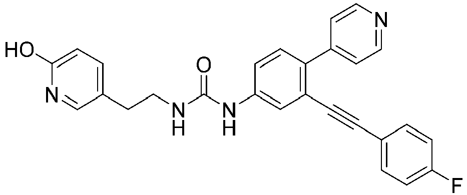 |
| **114** | 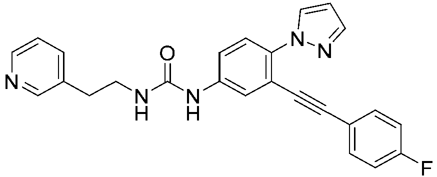 |
| **115** | 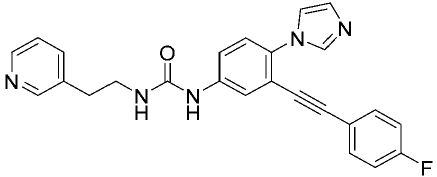 |
| **116** | 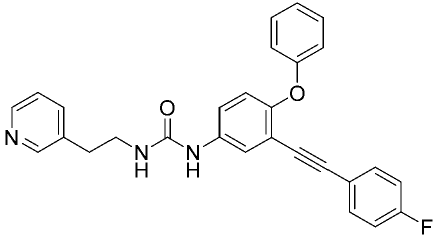 |
| **117** | 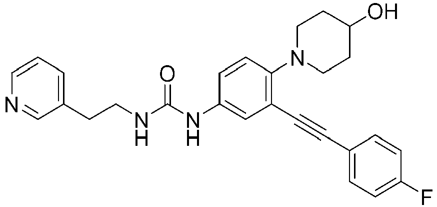 |
| **118** | 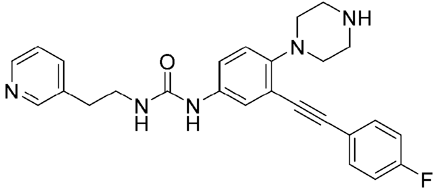 |
| **119** | 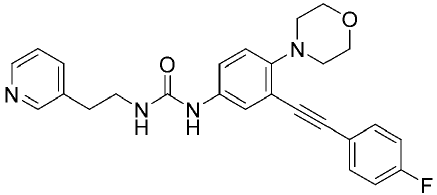 |
| **120** | 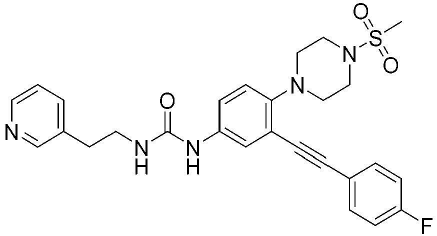 |
| **121** | 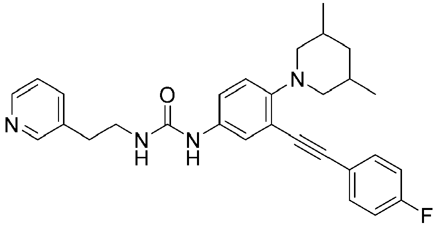 |
| **122** | 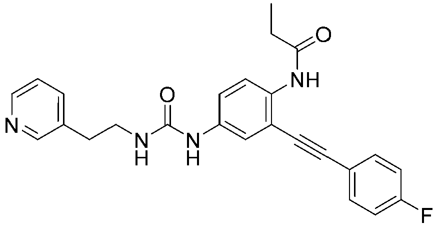 |
| **123** | 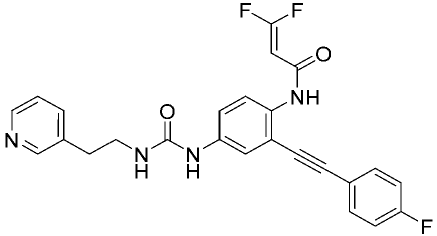 |
| **124** | 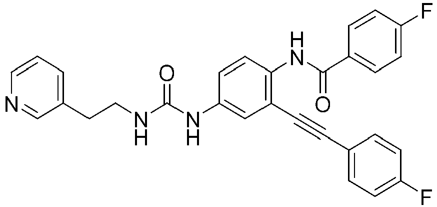 |
| **125** | 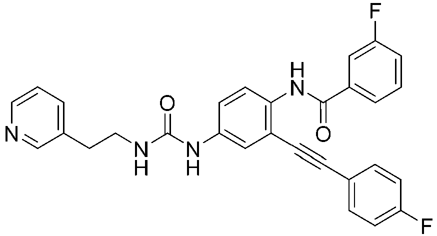 |
| **126** | 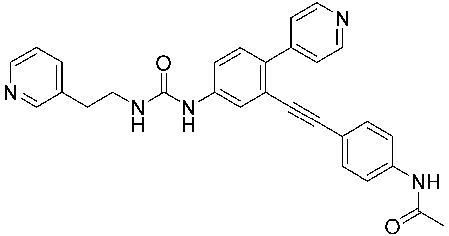 |
| **127** | 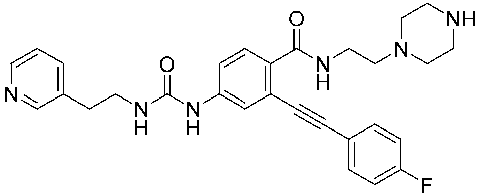 |
| **128** | 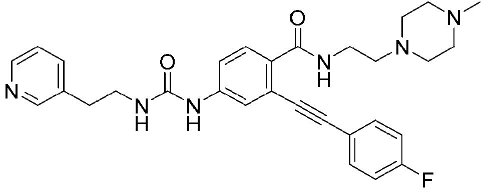 |
| **129** | 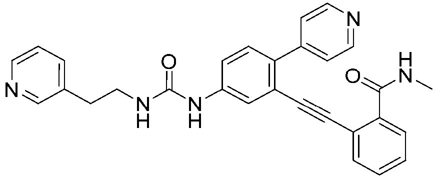 |
| **130** | 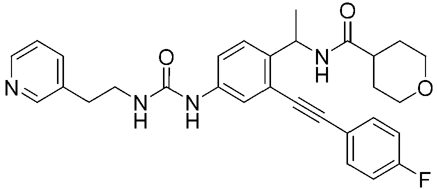 |
| **131** | 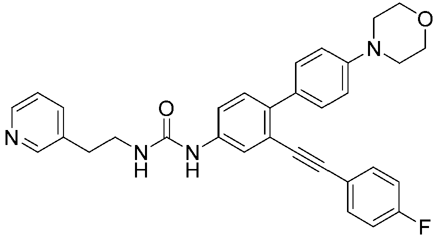 |
| **132** | 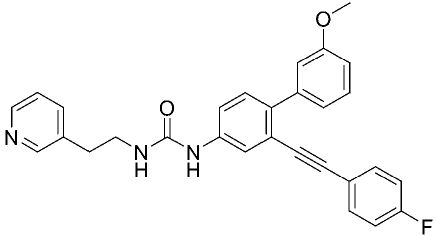 |
| **133** | 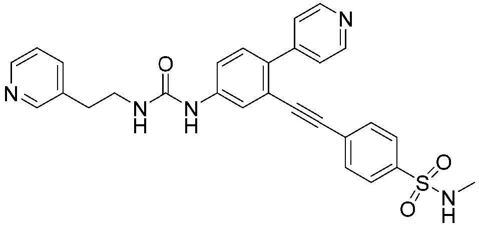 |
| **134** | 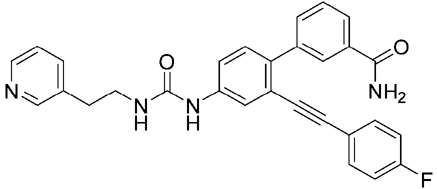 |
| **135** | 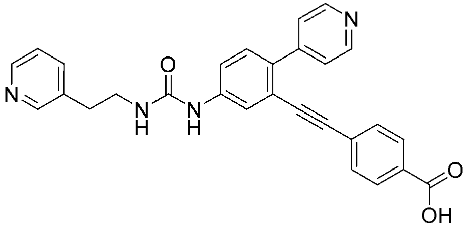 |
| **136** | 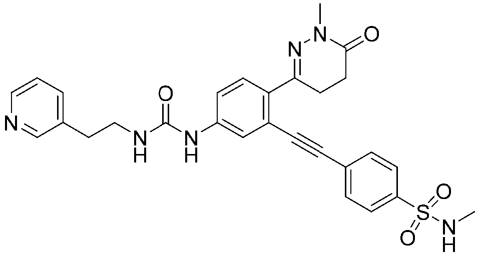 |
| **137** | 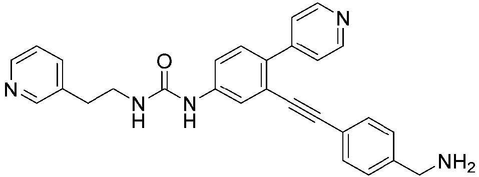 |
| **138** | 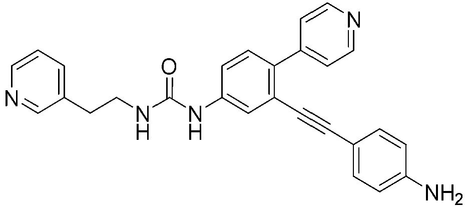 |
| **139** | 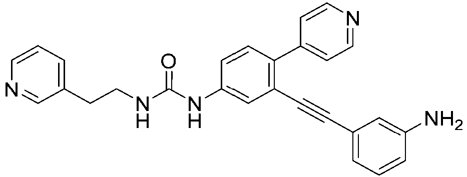 |
| **140** | 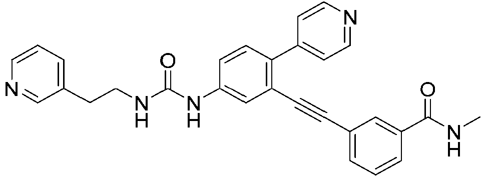 |
| **141** | 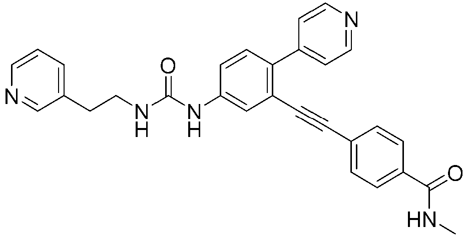 |
| **142** | 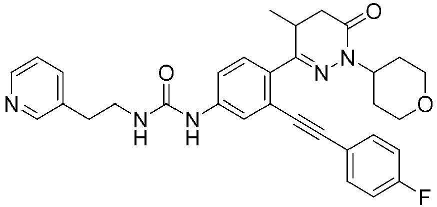 |
| **143** | 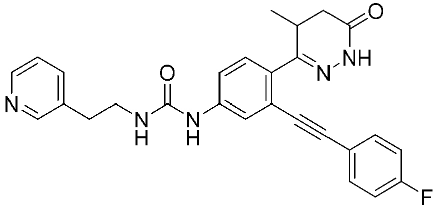 |
| **144** | 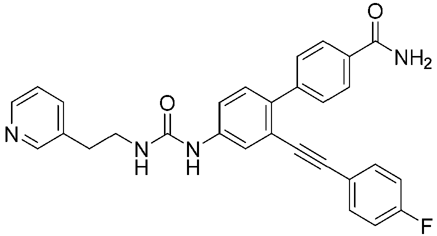 |
| **145** | 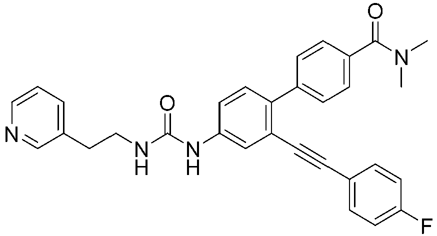 |
| **146** | 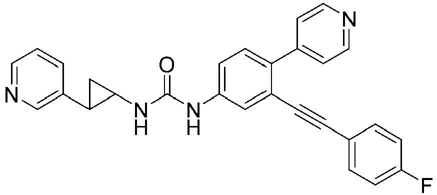 |
| **147** | 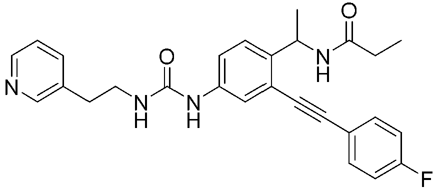 |
| **148** | 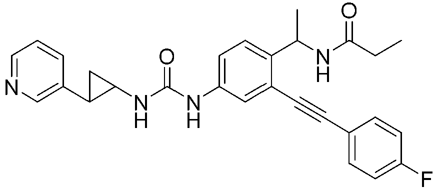 |
| **149** | 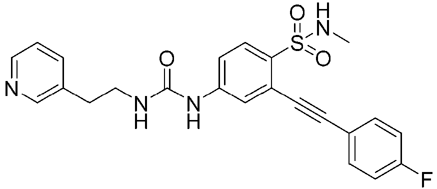 |
| **150** | 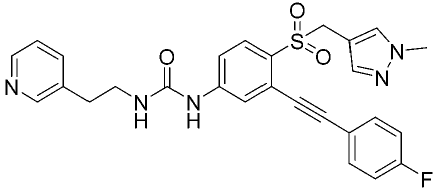 |
| **151** | 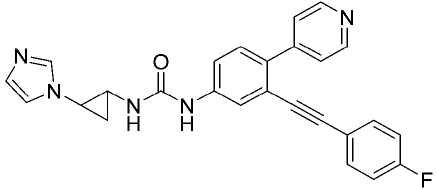 |
| **152** | 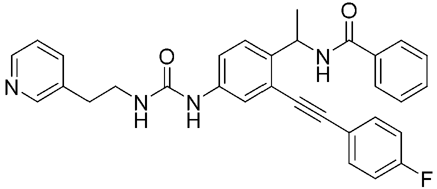 |
| **153** | 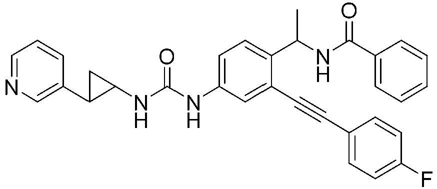 |
| **154** | 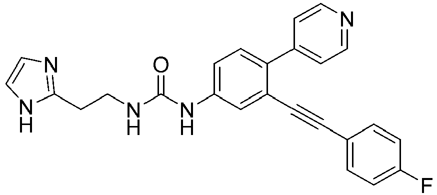 |
| **155** | 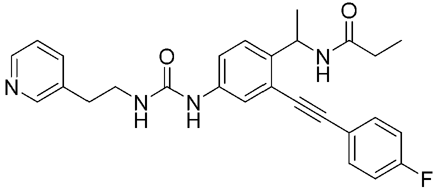 |
| **156** | 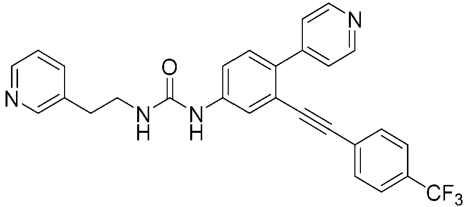 |
| **157** | 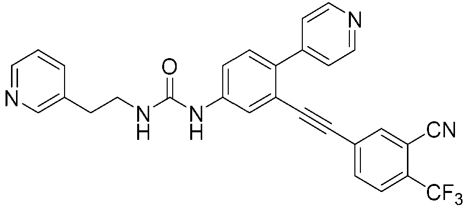 |
| **158** | 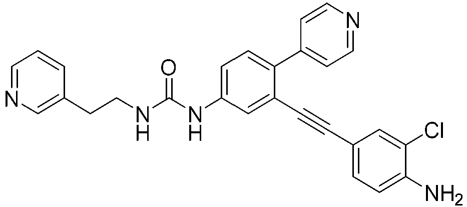 |
| **159** | 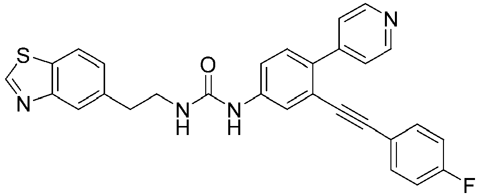 |
| **160** | 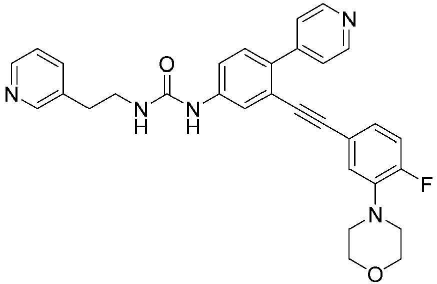 |
| **161** | 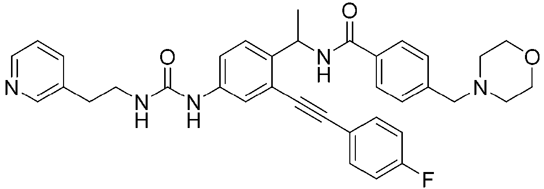 |
| **162** | 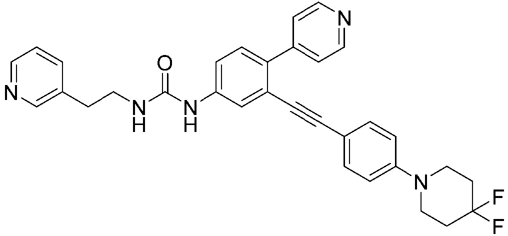 |
| **163** | 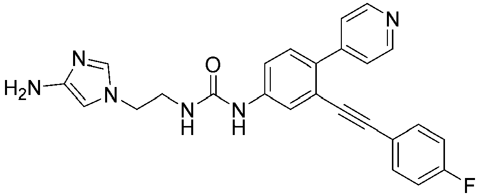 |
| **164** | 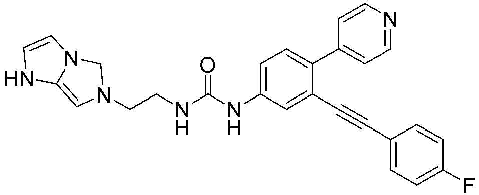 |
| **165** | 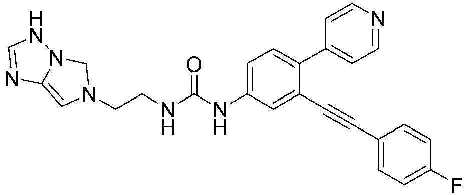 |
| **166** | 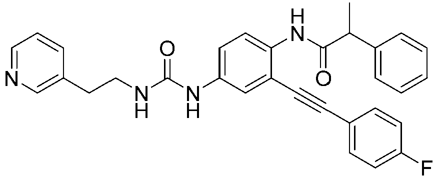 |
| **167** | 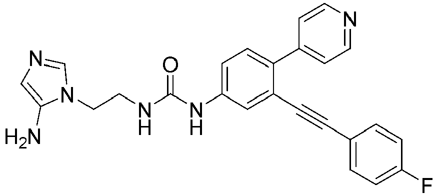 |
| **168** | 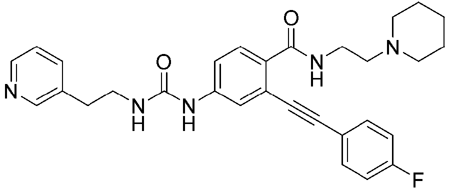 |
| **169** | 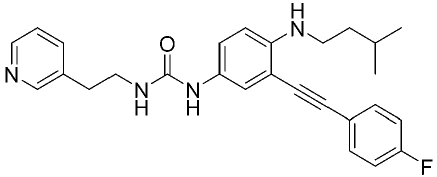 |
| **170** | 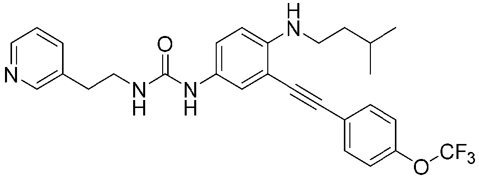 |
| **171** | 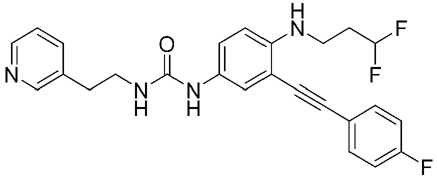 |
| **172** | 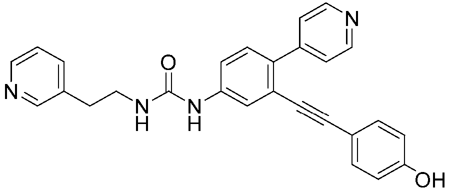 |
| **173** | 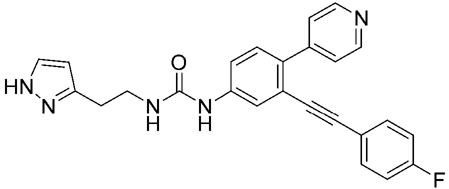 |
| **174** | 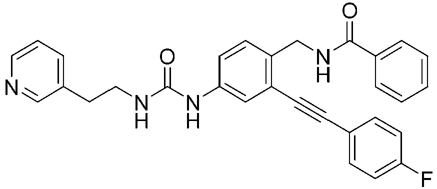 |
| **175** | 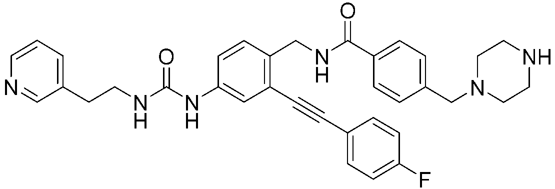 |
| **176** | 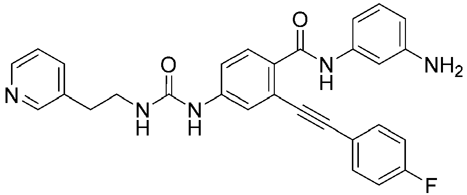 |
| **177** | 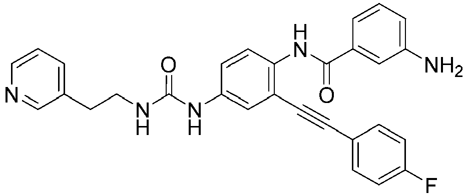 |
| **178** | 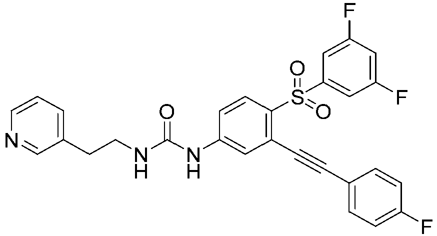 |
| **179** | 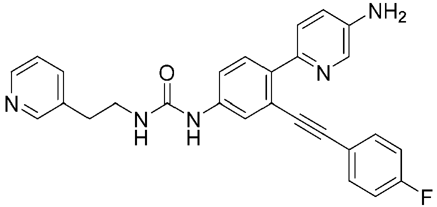 |
| **180** | 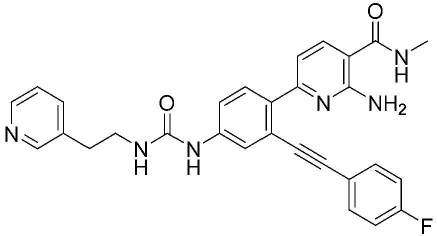 |
| **181** | 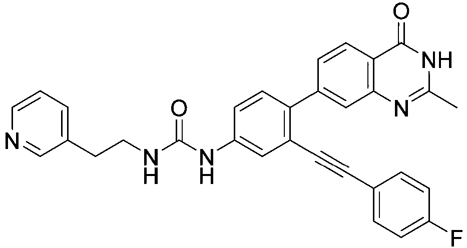 |
| **182** | 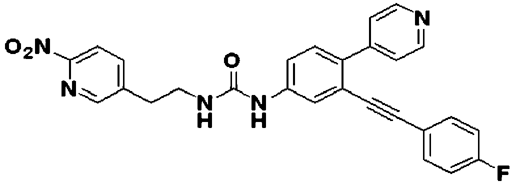 |
| **183** | 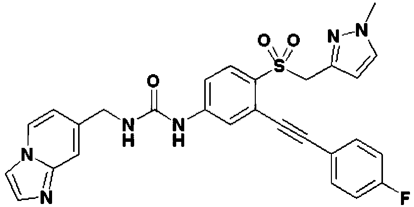 |
| **184** | 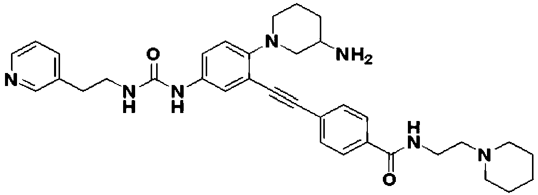 |
| **185** | 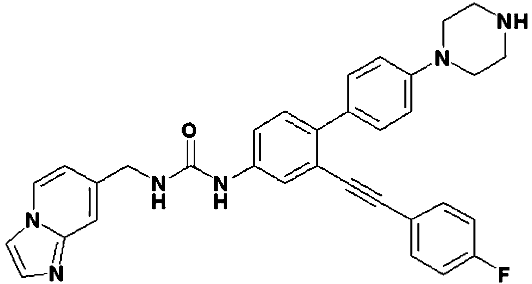 |
| **186** | 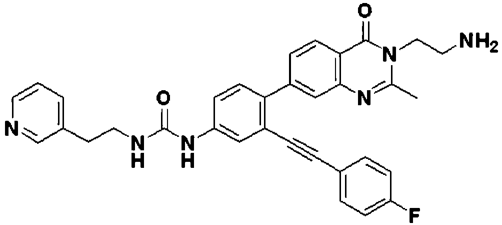 |
| **187** | 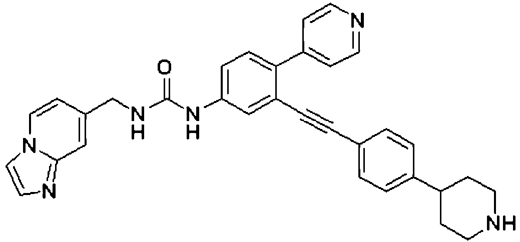 |
| **188** | 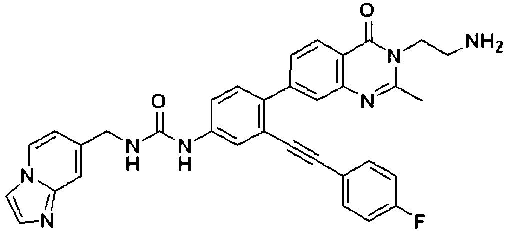 |
| **189** | 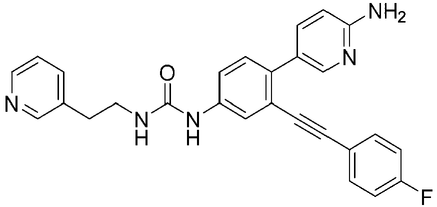 |

18. A urea compound represented by formula I-2 below, an optical isomer thereof, a pharmaceutically acceptable salt of them, or a hydrate or solvate of them: 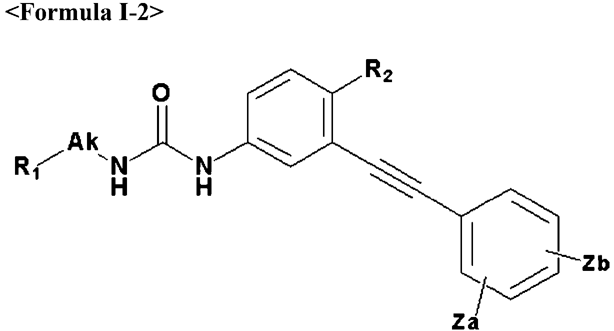
wherein R₁ may be pyridinyl; pyrimidinyl; imidazolyl; pyrazolyl; phenyl; 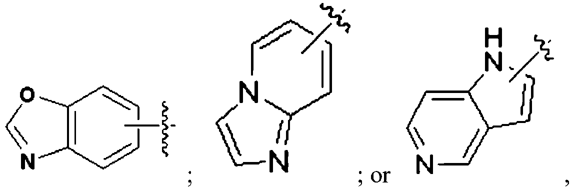
in which -H of R₁ may be each independently unsubstituted or at least one -H may be each independently substituted with morpholinyl, piperidinyl, piperazinyl, -NH₂, -(C₁₋₃ alkyl)NH₂, -NH-(C₁₋₃ alkyl), -N(C₁₋₃ alkyl)₂, -OH, -NO₂, C₁₋₅alkyl, -F, -Cl, -Br, or -I,
R₂ may be pyridinyl; pyrimidinyl; phenyl; piperazinyl; piperidinyl; 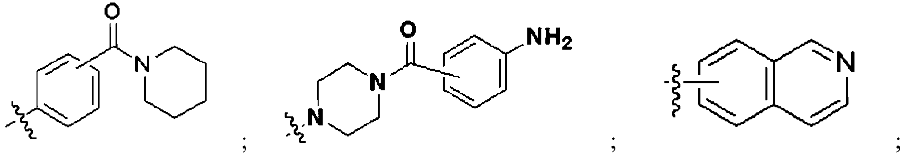 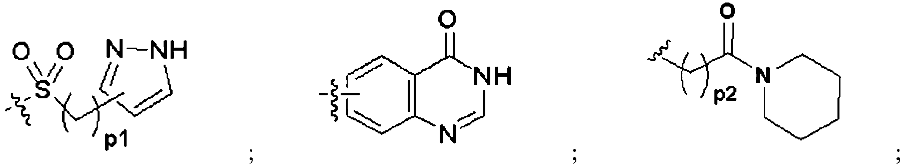 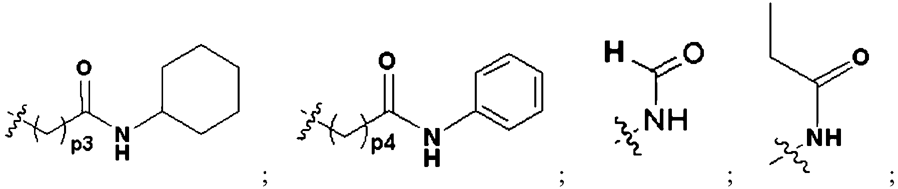 or 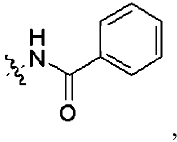
and the p1, p2, p3 or p4 are each independently an integer of 0 to 3, in which -H of R₂ may be each independently unsubstituted or at least one -H may be each independently substituted with morpholinyl, piperidinyl, piperazinyl, -NH₂, -(C₁₋₃ alkyl)NH₂, -NH-(C₁₋₃ alkyl), -N(C₁₋₃alkyl)₂, -OH, -NO₂, -C₁₋₅ alkyl, -F, -Cl, -Br, or -I,
Za or Zb may be each independently -H, morpholinyl, piperidinyl, piperazinyl, - NHC(=O)(C₁₋₃ alkyl), -NH₂, -(C₁₋₃ alkyl)NH₂, -NH(C₁₋₃ alkyl), -N(C₁₋₃ alkyl)₂, -OH, -NO₂, - C₁₋₅alkyl, -F, -Cl, -Br, -I, 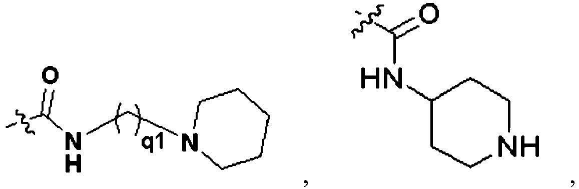 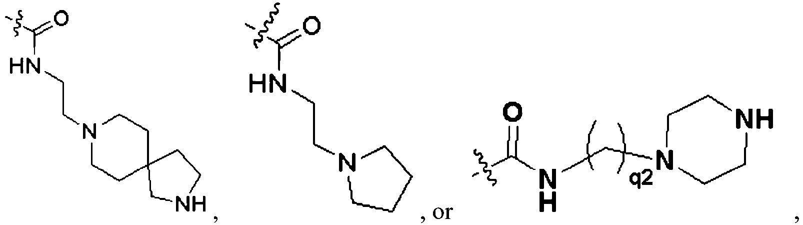 and the q1 or q2 may be each independently an integer of 0 to 3, in which -H of the Za or Zb may be each independently unsubstituted or at least one -H may be each independently substituted with -C₁₋₅alkyl, -NH₂, -(C₁₋₃ alkyl)NH₂, -NH-(C₁₋₃alkyl), or -N(C₁₋₃alkyl)₂, and
the Ak is -(CH₂)n-, in which the n may be 1, 2 or 3.

19. A pharmaceutical composition comprising a urea compound according to any one of claims 1 to 18, an optical isomer thereof, a pharmaceutically acceptable salt of them, or a hydrate or solvate of them.

20. The pharmaceutical composition of claim 19, wherein the pharmaceutical composition exhibits NAMPT inhibitory activity.

21. The pharmaceutical composition of claim 19, wherein the pharmaceutical composition is used for preventing or treating cancer.

22. The pharmaceutical composition of claim 19, wherein the cancer is a solid cancer or hematological cancer.

23. The pharmaceutical composition of claim 19, wherein the cancer is one selected from the group consisting of skin cancer, melanoma, lymphoma, breast cancer, cervical cancer, uterine cancer, gastrointestinal cancer, lung cancer, ovarian cancer, prostate cancer, colorectal cancer, colon cancer, rectal cancer, oral cancer, brain cancer, head and neck cancer, throat cancer, testicular cancer, kidney cancer, pancreatic cancer, bone cancer, spleen cancer, liver cancer, bladder cancer, larynx cancer, nasal cavity cancer, AIDS-related cancer, esophageal cancer, gastric cancer, stomach cancer, colorectal cancer, virus-related cancer, nasopharyngeal cancer, vaginal cancer, vulvar cancer, penile cancer, Kaposi sarcoma, Burkitt lymphoma, T-cell lymphoma and Merkel cell carcinoma, multiple myeloma, acute and chronic leukemia, lymphoblastic leukemia, myeloid leukemia, lymphocytic leukemia, and myelocytic leukemia.

24. A method for preventing or treating cancer, the method comprising administering a urea compound according to any one of claims 1 to 18, an optical isomer thereof, a pharmaceutically acceptable salt of them, or a hydrate or solvate of them into an individual in need.

25. A use of a urea compound according to any one of claims 1 to 18, an optical isomer thereof, a pharmaceutically acceptable salt of them, or a hydrate or solvate of them for preventing or treating cancer.

26. A use of a urea compound according to any one of claims 1 to 18, an optical isomer thereof, a pharmaceutically acceptable salt of them, or a hydrate or solvate of them, in preparation of a medication for preventing or treating cancer.
